(19) Europäisches Patentamt
European Patent Office
Office européen des brevets

(11) EP 0 882 794 B1

(12) **EUROPEAN PATENT SPECIFICATION**

(45) Date of publication and mention of the grant of the patent:
**02.06.2004 Bulletin 2004/23**

(51) Int Cl.[7]: **C12N 15/13**, C07K 16/46, A61K 39/395, G01N 33/574 // C07K16/18

(21) Application number: **98105047.9**

(22) Date of filing: **19.03.1998**

(54) **Human complementarity determining region (CDR)-grafted antibody to ganglioside gm2**

Menschliche-"CDR-grafted"-Antikörper gegen Gangliosid gm2

Anticorps anti-ganglioside gm2 comprenant des CDR d'origine humaine

(84) Designated Contracting States:
**AT BE CH DE DK ES FI FR GB GR IE IT LI LU MC NL PT SE**
Designated Extension States:
**AL LT LV MK RO SI**

(30) Priority: **19.03.1997 JP 6698197**

(43) Date of publication of application:
**09.12.1998 Bulletin 1998/50**

(73) Proprietor: **KYOWA HAKKO KOGYO CO., LTD.
Chiyoda-ku, Tokyo 100 (JP)**

(72) Inventors:
- **Kazuyasu, Nakamura
Machida-shi, Tokyo (JP)**
- **Nobuo, Hanai
Sagamihara-shi, Kanagawa (JP)**

(74) Representative: **Kinzebach, Werner, Dr. et al
Patentanwälte
Reitstötter, Kinzebach und Partner
Postfach 86 06 49
81633 München (DE)**

(56) References cited:
**EP-A- 0 598 998**

- **SHITARA K ET AL: "Immunoglobulin class switch of anti- ganglioside monoclonal antibody from IgM to IgG." JOURNAL OF IMMUNOLOGICAL METHODS, (1994 FEB 28) 169 (1) 83-92. JOURNAL CODE: IFE. ISSN: 0022-1759., XP002101158 Netherlands**
- **CLACKSON, TIM ET AL: "Making antibody fragments using phage display libraries" NATURE (LONDON) (1991), 352(6336), 624-8 CODEN: NATUAS;ISSN: 0028-0836, XP002101159**
- **HANIBUCHI M ET AL: "Therapeutic efficacy of mouse-human chimeric anti- ganglioside GM2 monoclonal antibody against multiple organ micrometastases of human lung cancer in NK cell-depleted SCID mice." INTERNATIONAL JOURNAL OF CANCER, (1998 NOV 9) 78 (4) 480-5. JOURNAL CODE: GQU. ISSN: 0020-7136., XP002101157 United States**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 882 794 B1

SEQUENCE LISTING

(1) GENERAL INFORMATION:

(i) APPLICANT: Kyowa Hakko Kogyo Co., Ltd

(ii) TITLE OF INVENTION: Human complementarity determining Region (CDR)-grafted antibody to ganglioside GM2

(iii) NUMBER OF SEQUENCES: 73

(iv) CORRESPONDENCE ADDRESS:

(A) ADDRESSEE: Kyowa Hakko Kogyo Co., Ltd.
(B) STREET: 6-1, Ohtemachi 1-chome, Chiyoda-ku
(C) CITY: Tokyo
(E) COUNTRY: Japan

(v) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:

(A) APPLICATION NUMBER: 98105047.9
(B) FILING DATE: 10.03.1998

(viii) ATTORNEY/AGENT INFORMATION:

(A) NAME: Kinzebach, Werner, Dr.
(C) REFERENCE/DOCKET NUMBER: M/39063

(ix) TELECOMMUNICATION INFORMATION:

(A) TELEPHONE: (089) 998397-0
(B) TELEFAX: (089) 987304

(2) INFORMATION FOR SEQ ID NO:1:

(i) SEQUENCE CHARACTERISTICS:

(A) LENGTH: 5 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:1:

```
Asp Tyr Asn Met Asp
 1               5
```

(2) INFORMATION FOR SEQ ID NO:2:

(i) SEQUENCE CHARACTERISTICS:

(A) LENGTH: 17 amino acids
(B) TYPE: amino acid
(C) STRANDEDNESS: single

(D) TOPOLOGY: linear

(ii) MOLECULE TYPE: peptide

(xi) SEQUENCE DESCRIPTION: SEQ ID NO:2:

```
Tyr Ile Tyr Pro Asn Asn Gly Gly Thr Gly Tyr Asn Gln Lys Phe Lys
 1               5                  10                  15

Ser
17
```

(2) INFORMATION FOR SEQ ID NO:3:

(i) SEQUENCE CHARACTERISTICS:

(A) LENGTH: 11 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO: 3:

```
Tyr Gly His Tyr Tyr Gly Tyr Met Phe Ala Tyr
 1               5                  10  11
```

(2) INFORMATION FOR SEQ ID NO:4:

(i) SEQUENCE CHARACTERISTICS:

(A) LENGTH: 10 amino acids
(B) TYPE: amino acid
(D) TOPOLOGY: linear

(ii) MOLECULE TYPE: peptide

(xi) SEQUENCE DESCRIPTION: SEQ ID NO:4:

```
Ser Ala Ser Ser Ser Val Ser Tyr Met His
 1               5                  10
```

(2) INFORMATION FOR SEQ ID NO:5:

(i) SEQUENCE CHARACTERISTICS:

(A) LENGTH: 7 amino acids
(B) TYPE: amino acid
(D) TOPOLOGY: linear

(ii) MOLECULE TYPE: peptide

(xi) SEQUENCE DESCRIPTION: SEQ ID NO:5:

```
Ser Thr Ser Asn Leu Ala Ser
 1               5       7
```

(2) INFORMATION FOR SEQ ID NO:6:

(i) SEQUENCE CHARACTERISTICS:

(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:6:

```
Gln Gln Arg Ser Ser Tyr Pro Tyr Thr
 1                   5           9
```

(2) INFORMATION FOR SEQ ID NO:7:

(i) SEQUENCE CHARACTERISTICS:

(A) LENGTH: 433 base pairs
(B) TYPE: nucleic acid
(C) STRANDEDNESS: double
(D) TOPOLOGY: linear

(ii) MOLECULE TYPE: other nucleic acid, synthetic DNA

(ix) FEATURES:

(A) NAME/KEY: sig peptide
(B) LOCATION: -19..-1
(C) IDENTIFICATION METHOD: S

(A) NAME/KEY: domain
(B) LOCATION: 31..35
(C) IDENTIFICATION METHOD: S
(D) OTHER INFORMATION: hypervariable region 1

(A) NAME/KEY: domain
(B) LOCATION: 50..66
(C) IDENTIFICATION METHOD: S
(D) OTHER INFORMATION: hypervariable region 2

(A) NAME/KEY: domain
(B) LOCATION: 99..109
(C) IDENTIFICATION METHOD: S
(D) OTHER INFORMATION: hypervariable region 3

(xi) SEQUENCE DESCRIPTION: SEQ ID NO:7:

```
ATG GGA TGG AGC TGG ATC TTT CTC TTC CTC CTG TCA GGA ACT GCA GGT   48
Met Gly Trp Ser Trp Ile Phe Leu Phe Leu Leu Ser Gly Thr Ala Gly
 1               5                  10                  15

GTC CTC TCT GAG GTG CAG CTG GTG CAG TCT GGA GCA GAG GTG AAG AAG   96
Val Leu Ser Glu Val Gln Leu Val Gln Ser Gly Ala Glu Val Lys Lys
            20                  25                  30

CCT GGG GCC TCA GTG AAG GTC TCC TGC AAG GCT TCC GGA TAC ACC TTC  144
Pro Gly Ala Ser Val Lys Val Ser Cys Lys Ala Ser Gly Tyr Thr Phe
        35                  40                  45

ACT GAC TAC AAC ATG GAC TGG GTG CGA CAG GCC CCT GGA CAA GGG CTC  192
Thr Asp Tyr Asn Met Asp Trp Val Arg Gln Ala Pro Gly Gln Gly Leu
    50                  55                  60

GAG TGG ATG GGA TAT ATT TAT CCT AAC AAT GGT GGT ACT GGC TAC AAC  240
Glu Trp Met Gly Tyr Ile Tyr Pro Asn Asn Gly Gly Thr Gly Tyr Asn
65                  70                  75                  80

CAG AAG TTC AAG AGC AAG GTC ACC ATT ACC GTA GAC ACA TCC ACG AGC  288
Gln Lys Phe Lys Ser Lys Val Thr Ile Thr Val Asp Thr Ser Thr Ser
                85                  90                  95

ACA GCC TAC ATG GAG CTG CAC AGC CTG AGA TCT GAG GAC ACG GCC GTG  336
Thr Ala Tyr Met Glu Leu His Ser Leu Arg Ser Glu Asp Thr Ala Val
            100                 105                 110

TAT TAC TGT GCG ACC TAC GGT CAT TAC TAC GGC TAC ATG TTT GCT TAC  384
Tyr Tyr Cys Ala Thr Tyr Gly His Tyr Tyr Gly Tyr Met Phe Ala Tyr
        115                 120                 125

TGG GGC CAG GGA ACC CTG GTC ACC GTC TCC TCA GCC TCC ACC AAG GGC  442
Trp Gly Gln Gly Thr Leu Val Thr Val Ser Ser Ala Ser Thr Lys Gly
    130                 135                 140                 144

C                                                                443
```

(2) INFORMATION FOR SEQ ID NO:8:

(i) SEQUENCE CHARACTERISTICS:

(A) LENGTH: 390 base pairs
(B) TYPE: nucleic acid
(C) STRANDEDNESS: double
(D) TOPOLOGY: linear

(ii) MOLECULE TYPE: other nucleic acid, synthetic DNA

(ix) FEATURES:

(A) NAME/KEY: sig peptide
(B) LOCATION: -22..-1
(C) IDENTIFICATION METHOD: S

(A) NAME/KEY: domain
(B) LOCATION: 24..33
(C) IDENTIFICATION METHOD: S
(D) OTHER INFORMATION: hypervariable region 1

(A) NAME/KEY: domain
(B) LOCATION: 49..55
(C) IDENTIFICATION METHOD: S
(D) OTHER INFORMATION: hypervariable region 2

(A) NAME/KEY: domain
(B) LOCATION: 86..96
(C) IDENTIFICATION METHOD: S
(D) OTHER INFORMATION: hypervariable region 3

(xi) SEQUENCE DESCRIPTION: SEQ ID NO:8:

```
ATG CAT TTT CAA GTG CAG ATT TTC AGC TTC CTG CTA ATC AGT GCC TCA    48
Met His Phe Gln Val Gln Ile Phe Ser Phe Leu Leu Ile Ser Ala Ser
 1               5                  10                  15

GTC ATA ATG TCC AGA GGA GAT ATC CAG CTG ACC CAG AGC CCA AGC AGC    96
Val Ile Met Ser Arg Gly Asp Ile Gln Leu Thr Gln Ser Pro Ser Ser
            20                  25                  30

CTG AGC GCT AGC CCA GGT GAC AGA GTG ACC ATC ACG TGC AGT GCC AGC   144
Leu Ser Ala Ser Pro Gly Asp Arg Val Thr Ile Thr Cys Ser Ala Ser
        35                  40                  45

TCA AGT GTA AGT TAC ATG CAC TGG TTC CAG CAG AAA CCA GGT AAG GCT   192
Ser Ser Val Ser Tyr Met His Trp Phe Gln Gln Lys Pro Gly Lys Ala
    50                  55                  60

CCA AAG CTT TGG ATC TAC AGC ACA TCC AAC CTG GCT TCT GGT GTG CCA   240
Pro Lys Leu Trp Ile Tyr Ser Thr Ser Asn Leu Ala Ser Gly Val Pro
65                  70                  75                  80

TCT AGA TTC AGC GGT AGC GGT AGC GGT ACA TCT TAC TCT CTC ACC ATC   288
Ser Arg Phe Ser Gly Ser Gly Ser Gly Thr Ser Tyr Ser Leu Thr Ile
                85                  90                  95

AGC CGA CTC CAG CCA GAG GAC ATC GCT ACA TAC TAC TGC CAG CAA AGG   336
Ser Arg Leu Gln Pro Glu Asp Ile Ala Thr Tyr Tyr Cys Gln Gln Arg
            100                 105                 110

AGT AGT TAC CCG TAC ACG TTC GGC GGG GGG ACC AAG GTG GAA ATC AAA   384
Ser Ser Tyr Pro Tyr Thr Phe Gly Gly Gly Thr Lys Val Glu Ile Lys
        115                 120                 125

CGT ACG                                                           390
Arg Thr
    130
```

(2) INFORMATION FOR SEQ ID NO:9:

(i) SEQUENCE CHARACTERISTICS:

(A) LENGTH: 390 base pairs
(B) TYPE: nucleic acid
(C) STRANDEDNESS: double
(D) TOPOLOGY: linear

(ii) MOLECULE TYPE: other nucleic acid, synthetic DNA

(ix) FEATURES:

(A) NAME/KEY: sig peptide
(B) LOCATION: -22..-1
(C) IDENTIFICATION METHOD: S

(A) NAME/KEY: domain
(B) LOCATION: 24..33
(C) IDENTIFICATION METHOD: S
(D) OTHER INFORMATION: hypervariable region 1

(A) NAME/KEY: domain
(B) LOCATION: 49..55
(C) IDENTIFICATION METHOD: S
(D) OTHER INFORMATION: hypervariable region 2

(A) NAME/KEY: domain
(B) LOCATION: 86..96
(C) IDENTIFICATION METHOD: S
(D) OTHER INFORMATION: hypervariable region 3

(xi) SEQUENCE DESCRIPTION: SEQ ID NO:9:

```
ATG CAT TTT CAA GTG CAG ATT TTC AGC TTC CTG CTA ATC AGT GCC TCA    48
Met His Phe Gln Val Gln Ile Phe Ser Phe Leu Leu Ile Ser Ala Ser
 1               5                  10                  15

GTC ATA ATG TCC AGA GGA GAC ATC CAG CTG ACC CAG TCT CCA TCC TCC    96
Val Ile Met Ser Arg Gly Asp Ile Gln Leu Thr Gln Ser Pro Ser Ser
            20                  25                  30

ATG TCT GCA TCT CCA GGG GAC AGA GTC ACC ATC ACT TGT AGT GCA AGT   144
Met Ser Ala Ser Pro Gly Asp Arg Val Thr Ile Thr Cys Ser Ala Ser
        35                  40                  45

TCA AGT GTA AGT TAC ATG CAC TGG TTT CAG CAG AAA CCA GGG AAA TCA   192
Ser Ser Val Ser Tyr Met His Trp Phe Gln Gln Lys Pro Gly Lys Ser
    50                  55                  60

CCT AAG CTC TGG ATC TAC TCA ACT TCA AAT TTA GCT TCT GGT GTG CCA   240
Pro Lys Leu Trp Ile Tyr Ser Thr Ser Asn Leu Ala Ser Gly Val Pro
65                  70                  75                  80
```

```
TCT AGA TTC AGC GGT AGC GGT AGC GGT ACA TCT TAC TCT CTC ACC ATC   288
Ser Arg Phe Ser Gly Ser Gly Ser Gly Thr Ser Tyr Ser Leu Thr Ile
                 85                  90                  95

AGC AGC ATG CAG CCT GAA GAT TTT GCA ACT TAT TAC TGT CAG CAA AGG   336
Ser Ser Met Gln Pro Glu Asp Phe Ala Thr Tyr Tyr Cys Gln Gln Arg
            100                 105                 110

AGT AGT TAC CCG TAC ACG TTC GGC CAG GGG ACC AAG CTG GAA ATC AAA   384
Ser Ser Tyr Pro Tyr Thr Phe Gly Gln Gly Thr Lys Leu Glu Ile Lys
        115                 120                 125

CGT ACG                                                            390
Arg Thr
    130
```

(2) INFORMATION FOR SEQ ID NO:10:

(i) SEQUENCE CHARACTERISTICS:

(A) LENGTH: 433 base pairs
(B) TYPE: nucleic acid
(C) STRANDEDNESS: double
(D) TOPOLOGY: linear

(ii) MOLECULE TYPE: other nucleic acid, synthetic DNA

(ix) FEATURES:

(A) NAME/KEY: sig peptide
(B) LOCATION: -19..-1
(C) IDENTIFICATION METHOD: S

(A) NAME/KEY: domain
(B) LOCATION: 31..35
(C) IDENTIFICATION METHOD: S
(D) OTHER INFORMATION: hypervariable region 1

(A) NAME/KEY: domain
(B) LOCATION: 50..66
(C) IDENTIFICATION METHOD: S
(D) OTHER INFORMATION: hypervariable region 2

(A) NAME/KEY: domain
(B) LOCATION: 99..109
(C) IDENTIFICATION METHOD: S
(D) OTHER INFORMATION: hypervariable region 3

(xi) SEQUENCE DESCRIPTION: SEQ ID NO:10:

```
ATG GGA TGG AGC TGG ATC TTT CTC TTC CTC CTG TCA GGA ACT GCA GGT    48
Met Gly Trp Ser Trp Ile Phe Leu Phe Leu Leu Ser Gly Thr Ala Gly
 1               5                  10                  15

GTC CTC TCT GAG GTG CAG CTG GTG CAG TCT GGA GCA GAG GTG AAG AAG    96
Val Leu Ser Glu Val Gln Leu Val Gln Ser Gly Ala Glu Val Lys Lys
            20                  25                  30

CCT GGG GCC TCA GTG AAG GTC TCC TGC AAG GCT TCC GGA TAC ACC TTC   144
Pro Gly Ala Ser Val Lys Val Ser Cys Lys Ala Ser Gly Tyr Thr Phe
        35                  40                  45

ACT GAC TAC AAC ATG GAC TGG GTG AAG CAG AGC CCT GGA CAA GGG CTC   192
Thr Asp Tyr Asn Met Asp Trp Val Lys Gln Ser Pro Gly Gln Gly Leu
    50                  55                  60

GAG TGG ATG GGA TAT ATT TAT CCT AAC AAT GGT GGT ACT GGC TAC AAC   240
Glu Trp Met Gly Tyr Ile Tyr Pro Asn Asn Gly Gly Thr Gly Tyr Asn
65                  70                  75                  80

CAG AAG TTC AAG AGC AAG GTC ACC ATT ACC GTA GAC ACA TCC ACG AGC   288
Gln Lys Phe Lys Ser Lys Val Thr Ile Thr Val Asp Thr Ser Thr Ser
                85                  90                  95

ACA GCC TAC ATG GAG CTG CAC AGC CTG AGA TCT GAG GAC ACG GCC GTG   336
Thr Ala Tyr Met Glu Leu His Ser Leu Arg Ser Glu Asp Thr Ala Val
            100                 105                 110

TAT TAC TGT GCG ACC TAC GGT CAT TAC TAC GGC TAC ATG TTT GCT TAC   384
Tyr Tyr Cys Ala Thr Tyr Gly His Tyr Tyr Gly Tyr Met Phe Ala Tyr
        115                 120                 125

TGG GGC CAG GGA ACC CTG GTC ACC GTC TCC TCA GCC TCC ACC AAG GGC   432
Trp Gly Gln Gly Thr Leu Val Thr Val Ser Ser Ala Ser Thr Lys Gly
    130                 135                 140                 144

C                                                                 433
```

(2) INFORMATION FOR SEQ ID NO:11:

(i) SEQUENCE CHARACTERISTICS:

(A) LENGTH: 390 base pairs
(B) TYPE: nucleic acid
(C) STRANDEDNESS: double
(D) TOPOLOGY: linear

(ii) MOLECULE TYPE: other nucleic acid, synthetic DNA

(ix) FEATURES:

(A) NAME/KEY: sig peptide
(B) LOCATION: -22..-1
(C) IDENTIFICATION METHOD: S

(A) NAME/KEY: domain
(B) LOCATION: 24..33
(C) IDENTIFICATION METHOD: S

(D) OTHER INFORMATION: hypervariable region 1

(A) NAME/KEY: domain
(B) LOCATION: 49..55
(C) IDENTIFICATION METHOD: S
(D) OTHER INFORMATION: hypervariable region 2

(A) NAME/KEY: domain
(B) LOCATION: 86..96
(C) IDENTIFICATION METHOD: S
(D) OTHER INFORMATION: hypervariable region 3

(xi) SEQUENCE DESCRIPTION: SEQ ID NO:11:

```
ATG CAT TTT CAA GTG CAG ATT TTC AGC TTC CTG CTA ATC AGT GCC TCA    48
Met His Phe Gln Val Gln Ile Phe Ser Phe Leu Leu Ile Ser Ala Ser
  1               5                  10                  15

GTC ATA ATG TCC AGA GGA GAT ATC CAG CTG ACC CAG AGC CCA AGC AGC    96
Val Ile Met Ser Arg Gly Asp Ile Gln Leu Thr Gln Ser Pro Ser Ser
             20                  25                  30

CTG AGC GCT AGC CCA GGT GAC AGA GTG ACC ATC ACG TGC AGT GCC AGC   144
Leu Ser Ala Ser Pro Gly Asp Arg Val Thr Ile Thr Cys Ser Ala Ser
         35                  40                  45

TCA AGT GTA AGT TAC ATG CAC TGG TTC CAG CAG AAA CCA GGT AAG GCT   192
Ser Ser Val Ser Tyr Met His Trp Phe Gln Gln Lys Pro Gly Lys Ala
     50                  55                  60

CCA AAG CTT TGG ATC TAC AGC ACA TCC AAC CTG GCT TCT GGT GTG CCA   240
Pro Lys Leu Trp Ile Tyr Ser Thr Ser Asn Leu Ala Ser Gly Val Pro
 65                  70                  75                  80

GCT AGA TTC AGC GGT AGC GGT AGC GGT ACA TCT TAC TCT CTC ACC ATC   288
Ala Arg Phe Ser Gly Ser Gly Ser Gly Thr Ser Tyr Ser Leu Thr Ile
                 85                  90                  95

AGC CGA CTC CAG CCA GAG GAC ATC GCT ACA TAC TAC TGC CAG CAA AGG   336
Ser Arg Leu Gln Pro Glu Asp Ile Ala Thr Tyr Tyr Cys Gln Gln Arg
            100                 105                 110

AGT AGT TAC CCG TAC ACG TTC GGC GGG GGG ACC AAG GTG GAA ATC AAA   384
Ser Ser Tyr Pro Tyr Thr Phe Gly Gly Gly Thr Lys Val Glu Ile Lys
        115                 120                 125

CGT ACG                                                           390
Arg Thr
    130
```

(2) INFORMATION FOR SEQ ID NO:12:

(i) SEQUENCE CHARACTERISTICS:

(A) LENGTH: 32 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:12:

CACTCAGTGT TAACTGAGGA GCAGGTGAAT TC 32

(2) INFORMATION FOR SEQ ID NO:13:

(i) SEQUENCE CHARACTERISTICS:

(A) LENGTH: 40 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:13:

AGCTGAATTC ACCTGCTCCT CAGTTAACAC TGAGTGGTAC 40

(2) INFORMATION FOR SEQ ID NO:14:

(i) SEQUENCE CHARACTERISTICS:

(A) LENGTH: 21 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:14:

AATTCGTACG GTGGCTGCAC C 21

(2) INFORMATION FOR SEQ ID NO:15:

(i) SEQUENCE CHARACTERISTICS:

(A) LENGTH: 17 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:15:

GGTGCAGCCA CCGTACG 17

(2) INFORMATION FOR SEQ ID NO:16:

(i) SEQUENCE CHARACTERISTICS:

(A) LENGTH: 26 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:16:

CTCGCGACTA GTGGGCCCGC GGCCGC 26

(2) INFORMATION FOR SEQ ID NO:17:

(i) SEQUENCE CHARACTERISTICS:

(A) LENGTH: 34 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:17:

AGCTGCGGCC GCGGGCCCAC TAGTCGCGAG GTAC 34

(2) INFORMATION FOR SEQ ID NO:18:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:18:

GTGGCGGCCG CTTGGGCCCG 20

(2) INFORMATION FOR SEQ ID NO:19:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:19:

CGGGCCCAAG CGGCCGCCAC 20

(2) INFORMATION FOR SEQ ID NO:20:

(i) SEQUENCE CHARACTERISTICS:

(A) LENGTH: 36 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:20:

CATGAATTCT TCGTACGGTT CGATAAATCG ATACCG 36

(2) INFORMATION FOR SEQ ID NO:21:

(i) SEQUENCE CHARACTERISTICS:

(A) LENGTH: 40 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:21:

CGGTATCGAT TTATCGAACC GTACGAAGAA TTCATGAGCT 40

(2) INFORMATION FOR SEQ ID NO:22:

(i) SEQUENCE CHARACTERISTICS:

(A) LENGTH: 35 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:22:

CACGTTCGGA GGGGGGACCA AGCTGGAAAT AAAAC 35

(2) INFORMATION FOR SEQ ID NO:23:

(i) SEQUENCE CHARACTERISTICS:

(A) LENGTH: 35 base pairs
(B) TYPE: nucleic acid

(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:23:

GTACGTTTTA TTTCCAGCTT GGTCCCCCCT CCGAA 35

(2) INFORMATION FOR SEQ ID NO:24:

(i) SEQUENCE CHARACTERISTICS:

(A) LENGTH: 61 base pairs
(B) TYPE: nucleic acid
(C) STRANDEDNESS: double
(D) TOPOLOGY: linear

(ii) MOLECULE TYPE: other nucleic acid, synthetic DNA

(xi) SEQUENCE DESCRIPTION: SEQ ID NO:24:

TCGACACCAG CAAGAACACA GCCTACCTGA GACTCAGCAG CGTGACAGCC GCCGACACCG 60

C 61

(2) INFORMATION FOR SEQ ID NO:25:

(i) SEQUENCE CHARACTERISTICS:

(A) LENGTH: 60 base pairs
(B) TYPE: nucleic acid
(C) STRANDEDNESS: double
(D) TOPOLOGY: linear

(ii) MOLECULE TYPE: other nucleic acid, synthetic DNA

(xi) SEQUENCE DESCRIPTION: SEQ ID NO:25:

CCGGATACAC ATTCACTGAC TACAACATGG ACTGGGTGAG ACAGAGCCAT GGACGAGGTC 60

(2) INFORMATION FOR SEQ ID NO:26:

(i) SEQUENCE CHARACTERISTICS:

(A) LENGTH: 442 base pairs
(B) TYPE: nucleic acid
(C) STRANDEDNESS: double
(D) TOPOLOGY: linear

(ii) MOLECULE TYPE: other nucleic acid, synthetic DNA

(ix) FEATURES:

(A) NAME/KEY: sig peptide
(B) LOCATION: -19..-1
(C) IDENTIFICATION METHOD: S

(A) NAME/KEY: domain
(B) LOCATION: 31..35
(C) IDENTIFICATION METHOD: S
(D) OTHER INFORMATION: hypervariable region 1

(A) NAME/KEY: domain
(B) LOCATION: 50..66
(C) IDENTIFICATION METHOD: S
(D) OTHER INFORMATION: hypervariable region 2

(A) NAME/KEY: domain
(B) LOCATION: 99..109
(C) IDENTIFICATION METHOD: S
(D) OTHER INFORMATION: hypervariable region 3

(xi) SEQUENCE DESCRIPTION: SEQ ID NO:26:

```
GGCCGCACC ATG GGA TGG AGC TGG ATC TTT CTC TTC CTC CTG TCA GGA ACT 51
          Met Gly Trp Ser Trp Ile Phe Leu Phe Leu Leu Ser Gly Thr
            1               5                  10

GCT GGT GTC CTC TCT CAG GTC CAA CTG CAG GAG AGC GGT CCA GGT CTT   99
Ala Gly Val Leu Ser Gln Val Gln Leu Gln Glu Ser Gly Pro Gly Leu
15                  20                  25                  30

GTG AGG CCT AGC CAG ACC CTG AGC CTG ACC TGC ACC GTG TCC GGA TTC  147
Val Arg Pro Ser Gln Thr Leu Ser Leu Thr Cys Thr Val Ser Gly Phe
                35                  40                  45

ACC TTC AGC GAC TAC AAC ATG GAC TGG GTG AGA CAG CCA CCT GGA CGA  195
Thr Phe Ser Asp Tyr Asn Met Asp Trp Val Arg Gln Pro Pro Gly Arg
            50                  55                  60

GGT CTC GAG TGG ATT GGA TAT ATT TAT CCT AAC AAT GGT GGT ACT GGC  243
Gly Leu Glu Trp Ile Gly Tyr Ile Tyr Pro Asn Asn Gly Gly Thr Gly
        65                  70                  75

TAC AAC CAG AAG TTC AAG AGC AGA GTG ACA ATG CTG GTC GAC ACC AGC  291
Tyr Asn Gln Lys Phe Lys Ser Arg Val Thr Met Leu Val Asp Thr Ser
    80                  85                  90

AAG AAC ACA GCC TAC CTG AGA CTC AGC AGC GTG ACA GCC GCC GAC ACC  339
Lys Asn Thr Ala Tyr Leu Arg Leu Ser Ser Val Thr Ala Ala Asp Thr
95                  100                 105                 110

GCG GTC TAT TAT TGT GCA ACC TAC GGT CAT TAC TAC GGC TAC ATG TTT  387
Ala Val Tyr Tyr Cys Ala Thr Tyr Gly His Tyr Tyr Gly Tyr Met Phe
                115                 120                 125

GCT TAC TGG GGT CAA GGT ACC ACC GTC ACA GTC TCC TCA GCC TCC ACC  435
Ala Tyr Trp Gly Gln Gly Thr Thr Val Thr Val Ser Ser Ala Ser Thr
            130                 135                 140

AAG GGC C                                                        442
Lys Gly
    144
```

(2) INFORMATION FOR SEQ ID NO: 27:

(i) SEQUENCE CHARACTERISTICS:

(A) LENGTH: 442 base pairs
(B) TYPE: nucleic acid
(C) STRANDEDNESS: double
(D) TOPOLOGY: linear

(ii) MOLECULE TYPE: other nucleic acid, synthetic DNA

(ix) FEATURES:

(A) NAME/KEY: sig peptide
(B) LOCATION: -19..-1
(C) IDENTIFICATION METHOD: S

(A) NAME/KEY: domain
(B) LOCATION: 31..35
(C) IDENTIFICATION METHOD: S
(D) OTHER INFORMATION: hypervariable region 1

(A) NAME/KEY: domain
(B) LOCATION: 50..66
(C) IDENTIFICATION METHOD: S
(D) OTHER INFORMATION: hypervariable region 2

(A) NAME/KEY: domain
(B) LOCATION: 99..109
(C) IDENTIFICATION METHOD: S
(D) OTHER INFORMATION: hypervariable region 3

(xi) SEQUENCE DESCRIPTION: SEQ ID NO:27:

```
GGCCGCACC ATG GGA TGG AGC TGG ATC TTT CTC TTC CTC CTG TCA GGA ACT 51
          Met Gly Trp Ser Trp Ile Phe Leu Phe Leu Leu Ser Gly Thr
           1               5                  10

GCT GGT GTC CTC TCT CAG GTC CAA CTG CAG GAG AGC GGT CCA GGT CTT   99
Ala Gly Val Leu Ser Gln Val Gln Leu Gln Glu Ser Gly Pro Gly Leu
15                  20                  25                  30

GTG AGG CCT AGC CAG ACC CTG AGC CTG ACC TGC ACC GTG TCC GGA TAC  147
Val Arg Pro Ser Gln Thr Leu Ser Leu Thr Cys Thr Val Ser Gly Tyr
                35                  40                  45

ACC TTC ACT GAC TAC AAC ATG GAC TGG GTG AGA CAG AGC CAT GGA CGA  195
Thr Phe Thr Asp Tyr Asn Met Asp Trp Val Arg Gln Ser His Gly Arg
            50                  55                  60

GGT CTC GAG TGG ATT GGA TAT ATT TAT CCT AAC AAT GGT GGT ACT GGC  243
Gly Leu Glu Trp Ile Gly Tyr Ile Tyr Pro Asn Asn Gly Gly Thr Gly
        65                  70                  75

TAC AAC CAG AAG TTC AAG AGC AGA GTG ACA ATG CTG GTC GAC ACC AGC  291
Tyr Asn Gln Lys Phe Lys Ser Arg Val Thr Met Leu Val Asp Thr Ser
    80                  85                  90

AAG AAC CAG TTC AGC CTG AGA CTC AGC AGC GTG ACA GCC GCC GAC ACC  339
Lys Asn Gln Phe Ser Leu Arg Leu Ser Ser Val Thr Ala Ala Asp Thr
95                  100                 105                 110

GCG GTC TAT TAT TGT GCA ACC TAC GGT CAT TAC TAC GGC TAC ATG TTT  387
Ala Val Tyr Tyr Cys Ala Thr Tyr Gly His Tyr Tyr Gly Tyr Met Phe
                115                 120                 125

GCT TAC TGG GGT CAA GGT ACC ACC GTC ACA GTC TCC TCA GCC TCC ACC  435
Ala Tyr Trp Gly Gln Gly Thr Thr Val Thr Val Ser Ser Ala Ser Thr
            130                 135                 140

AAG GGC C                                                        442
Lys Gly
    144
```

(2) INFORMATION FOR SEQ ID NO:28:

(i) SEQUENCE CHARACTERISTICS:

(A) LENGTH: 100 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:28:

```
CAGGAAACAG CTATGACGCG GCCGCCACCA TGGGATGGAG CTGGATCTTT CTCTTCCTCC 60

TGTCAGGAAC TGCAGGTGTC CTCTCTGAGG TGCAGCTGGT                       100
```

(2) INFORMATION FOR SEQ ID NO:29:

(i) SEQUENCE CHARACTERISTICS:

(A) LENGTH: 100 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:29:

```
AGTCAGTGAA GGTGTATCCG GAAGCCTTGC AGGAGACCTT CACTGAGGCC CCAGGCTTCT 60
TCACCTCTGC TCCAGACTGC ACCAGCTGCA CCTCAGAGAG                       100
```

(2) INFORMATION FOR SEQ ID NO:30:

(i) SEQUENCE CHARACTERISTICS:

(A) LENGTH: 100 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:30:

```
CGGATACACC TTCACTGACT ACAACATGGA CTGGGTGCGA CAGGCCCCTG GACAAGGGCT 60
CGAGTGGATG GGATATATTT ATCCTAACAA TGGTGGTACT                       100
```

(2) INFORMATION FOR SEQ ID NO:31:

(i) SEQUENCE CHARACTERISTICS:

(A) LENGTH: 94 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:31:

```
AGCTCCATGT AGGCTGTGCT CGTGGATGTG TCTACGGTAA TGGTGACCTT GCTCTTGAAC 60
TTCTGGTTGT AGCCAGTACC ACCATTGTTA GGAT                             94
```

(2) INFORMATION FOR SEQ ID NO:32:

(i) SEQUENCE CHARACTERISTICS:

(A) LENGTH: 96 base pairs
(B) TYPE: nucleic acid

(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:32:

AGCACAGCCT ACATGGAGCT GCACAGCCTG AGATCTGAGG ACACGGCCGT GTATTACTGT 60

GCGACCTACG GTCATTACTA CGGCTACATG TTTGCT 96

(2) INFORMATION FOR SEQ ID NO:33:

(i) SEQUENCE CHARACTERISTICS:

(A) LENGTH: 90 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:33:

GTTTTCCCAG TCACGACGGG CCCTTGGTGG AGGCTGAGGA GACGGTGACC AGGGTTCCCT 60

GGCCCCAGTA AGCAAACATG TAGCCGTAGT 90

(2) INFORMATION FOR SEQ ID NO:34:

(i) SEQUENCE CHARACTERISTICS:

(A) LENGTH: 68 base pairs
(B) TYPE: nucleic acid
(C) STRANDEDNESS: double
(D) TOPOLOGY: linear

(ii) MOLECULE TYPE: other nucleic acid, synthetic DNA

(xi) SEQUENCE DESCRIPTION: SEQ ID NO:34:

GTACTACTGC CAGCAAAGGA GTAGTTACCC GTACACGTTC GGCGGGGGGA CCAAGGTGGA 60

AATCAAAC 68

(2) INFORMATION FOR SEQ ID NO:35:

(i) SEQUENCE CHARACTERISTICS:

(A) LENGTH: 25 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:35:

ACTCTGTCAC CTGGGCTAGC GCTCA 25

(2) INFORMATION FOR SEQ ID NO:36:

(i) SEQUENCE CHARACTERISTICS:

(A) LENGTH: 25 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:36:

TGAGCGCTAG CCCAGGTGAC AGAGT 25

(2) INFORMATION FOR SEQ ID NO:37:

(i) SEQUENCE CHARACTERISTICS:

(A) LENGTH: 390 base pairs
(B) TYPE: nucleic acid
(C) STRANDEDNESS: double
(D) TOPOLOGY: linear

(ii) MOLECULE TYPE: other nucleic acid, synthetic DNA

(ix) FEATURES:

(A) NAME/KEY: sig peptide
(B) LOCATION: -22..-1
(C) IDENTIFICATION METHOD: S

(A) NAME/KEY: domain
(B) LOCATION: 24..33
(C) IDENTIFICATION METHOD: S
(D) OTHER INFORMATION: hypervariable region 1

(A) NAME/KEY: domain
(B) LOCATION: 49..55
(C) IDENTIFICATION METHOD: S
(D) OTHER INFORMATION: hypervariable region 2

(A) NAME/KEY: domain
(B) LOCATION: 86..96
(C) IDENTIFICATION METHOD: S
(D) OTHER INFORMATION: hypervariable region 3

(xi) SEQUENCE DESCRIPTION: SEQ ID NO:37:

```
ATG CAT TTT CAA GTG CAG ATT TTC AGC TTC CTG CTA ATC AGT GCC TCA    48
Met His Phe Gln Val Gln Ile Phe Ser Phe Leu Leu Ile Ser Ala Ser
 1               5                  10                  15

GTC ATA ATG TCC AGA GGA GAT ATC CAG CTG ACC CAG AGC CCA AGC AGC    96
Val Ile Met Ser Arg Gly Asp Ile Gln Leu Thr Gln Ser Pro Ser Ser
            20                  25                  30

CTG AGC GCT AGC CCA GGT GAC AGA GTG ACC ATC ACG TGC AGT GCC AGC   144
Leu Ser Ala Ser Pro Gly Asp Arg Val Thr Ile Thr Cys Ser Ala Ser
        35                  40                  45

TCA AGT GTA AGT TAC ATG CAC TGG TAT CAG CAG AAA CCA GGT AAG GCT   192
Ser Ser Val Ser Tyr Met His Trp Tyr Gln Gln Lys Pro Gly Lys Ala
    50                  55                  60

CCA AAG CTT CTG ATC TAC AGC ACA TCC AAC CTG GCT TCT GGT GTG CCA   240
Pro Lys Leu Leu Ile Tyr Ser Thr Ser Asn Leu Ala Ser Gly Val Pro
65                  70                  75                  80

TCT AGA TTC AGC GGT AGC GGT AGC GGT ACA GAC TTC ACC TTC ACC ATC   288
Ser Arg Phe Ser Gly Ser Gly Ser Gly Thr Asp Phe Thr Phe Thr Ile
                85                  90                  95

AGC AGC CTC CAG CCA GAG GAC ATC GCT ACA TAC TAC TGC CAG CAA AGG   336
Ser Ser Leu Gln Pro Glu Asp Ile Ala Thr Tyr Tyr Cys Gln Gln Arg
            100                 105                 110

AGT AGT TAC CCG TAC ACG TTC GGC GGG GGG ACC AAG GTG GAA ATC AAA   384
Ser Ser Tyr Pro Tyr Thr Phe Gly Gly Gly Thr Lys Val Glu Ile Lys
        115                 120                 125

CGT ACG                                                           390
Arg Thr
    130
```

(2) INFORMATION FOR SEQ ID NO:38:

(i) SEQUENCE CHARACTERISTICS:

(A) LENGTH: 25 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ-ID NO:38:

```
GTGCTGTAGA TCCAAAGCTT TGGAG                                        25
```

(2) INFORMATION FOR SEQ ID NO:39:

(i) SEQUENCE CHARACTERISTICS:

(A) LENGTH: 25 base pairs
(B) TYPE: nucleic acid

(C) STRANDEDNESS: single
(D) TOPOLOGY: linear

(ii) MOLECULE TYPE: other nucleic acid, synthetic DNA

(xi) SEQUENCE DESCRIPTION: SEO ID NO:39:

CTCCAAAGCT TTGGATCTAC AGCAC 25

(2) INFORMATION FOR SEQ ID NO:40:

(i) SEQUENCE CHARACTERISTICS:

(A) LENGTH: 390 base pairs
(B) TYPE: nucleic acid
(C) STRANDEDNESS: double
(D) TOPOLOGY: linear

(ii) MOLECULE TYPE: other nucleic acid, synthetic DNA

(ix) FEATURES:

(A) NAME/KEY: sig peptide
(B) LOCATION: -22..-1
(C) IDENTIFICATION METHOD: S

(A) NAME/KEY: domain
(B) LOCATION: 24..33
(C) IDENTIFICATION METHOD: S
(D) OTHER INFORMATION: hypervariable region 1

(A) NAME/KEY: domain
(B) LOCATION: 49..55
(C) IDENTIFICATION METHOD: S
(D) OTHER INFORMATION: hypervariable region 2

(A) NAME/KEY: domain
(B) LOCATION: 86..96
(C) IDENTIFICATION METHOD: S
(D) OTHER INFORMATION: hypervariable region 3

(xi) SEQUENCE DESCRIPTION: SEQ ID NO:40:

```
ATG CAT TTT CAA GTG CAG ATT TTC AGC TTC CTG CTA ATC AGT GCC TCA    48
Met His Phe Gln Val Gln Ile Phe Ser Phe Leu Leu Ile Ser Ala Ser
 1               5                  10                  15

GTC ATA ATG TCC AGA GGA GAT ATC CAG CTG ACC CAG AGC CCA AGC AGC    96
Val Ile Met Ser Arg Gly Asp Ile Gln Leu Thr Gln Ser Pro Ser Ser
            20                  25                  30

CTG AGC GCT AGC GTG GGT GAC AGA GTG ACC ATC ACC TGC AGT GCC AGC   144
Leu Ser Ala Ser Val Gly Asp Arg Val Thr Ile Thr Cys Ser Ala Ser
        35                  40                  45

TCA AGT GTA AGT TAC ATG CAC TGG TAT CAG CAG AAA CCA GGT AAG GCT   192
Ser Ser Val Ser Tyr Met His Trp Tyr Gln Gln Lys Pro Gly Lys Ala
    50                  55                  60

CCA AAG CTT TGG ATC TAC AGC ACA TCC AAC CTG GCT TCT GGT GTG CCA   240
Pro Lys Leu Trp Ile Tyr Ser Thr Ser Asn Leu Ala Ser Gly Val Pro
65                  70                  75                  80

TCT AGA TTC AGC GGT AGC GGT AGC GGT ACA GAC TTC ACC TTC ACC ATC   288
Ser Arg Phe Ser Gly Ser Gly Ser Gly Thr Asp Phe Thr Phe Thr Ile
                85                  90                  95

AGC AGC CTC CAG CCA GAG GAC ATC GCT ACA TAC TAC TGC CAG CAA AGG   336
Ser Ser Leu Gln Pro Glu Asp Ile Ala Thr Tyr Tyr Cys Gln Gln Arg
            100                 105                 110

AGT AGT TAC CCG TAC ACG TTC GGC GGG GGG ACC AAG GTG GAA ATC AAA   384
Ser Ser Tyr Pro Tyr Thr Phe Gly Gly Gly Thr Lys Val Glu Ile Lys
        115                 120                 125

CGT ACG                                                           390
Arg Thr
    130
```

(2) INFORMATION FOR SEQ ID NO:41:

(i) SEQUENCE CHARACTERISTICS:

(A) LENGTH: 25 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:41:

```
ACGTAGCAGC ATCTTCAGCC TGGAG                                       25
```

(2) INFORMATION FOR SEQ ID NO:42:

(i) SEQUENCE CHARACTERISTICS:

(A) LENGTH: 25 base pairs
(B) TYPE: nucleic acid
(C) STRANDEDNESS: single

(D) TOPOLOGY: linear

(ii) MOLECULE TYPE: other nucleic acid, synthetic DNA

(xi) SEQUENCE DESCRIPTION: SEQ ID NO:42:

```
CTCCAGGCTG AAGATGCTGC TACGT                                        25
```

(2) INFORMATION FOR SEQ ID NO:43:

(i) SEQUENCE CHARACTERISTICS:

(A) LENGTH: 390 base pairs
(B) TYPE: nucleic acid
(C) STRANDEDNESS: double
(D) TOPOLOGY: linear

(ii) MOLECULE TYPE: other nucleic acid, synthetic DNA

(ix) FEATURES:

(A) NAME/KEY: sig peptide
(B) LOCATION: -22..-1
(C) IDENTIFICATION METHOD: S

(A) NAME/KEY: domain
(B) LOCATION: 24..33
(C) IDENTIFICATION METHOD: S
(D) OTHER INFORMATION: hypervariable region 1

(A) NAME/KEY: domain
(B) LOCATION: 49..55
(C) IDENTIFICATION METHOD: S
(D) OTHER INFORMATION: hypervariable region 2

(A) NAME/KEY: domain
(B) LOCATION: 86..96
(C) IDENTIFICATION METHOD: S
(D) OTHER INFORMATION: hypervariable region 3

(xi) SEQUENCE DESCRIPTION: SEQ ID NO:43:

```
ATG CAT TTT CAA GTG CAG ATT TTC AGC TTC CTG CTA ATC AGT GCC TCA    48
Met His Phe Gln Val Gln Ile Phe Ser Phe Leu Leu Ile Ser Ala Ser
 1               5                  10                  15
```

```
GTC ATA ATG TCC AGA GGA GAT ATC CAG CTG ACC CAG AGC CCA AGC AGC    96
Val Ile Met Ser Arg Gly Asp Ile Gln Leu Thr Gln Ser Pro Ser Ser
            20                  25                  30

CTG AGC GCT AGC GTG GGT GAC AGA GTG ACC ATC ACG TGC AGT GCC AGC   144
Leu Ser Ala Ser Val Gly Asp Arg Val Thr Ile Thr Cys Ser Ala Ser
        35                  40                  45

TCA AGT GTA AGT TAC ATG CAC TGG TAT CAG CAG AAA CCA GGT AAG GCT   192
Ser Ser Val Ser Tyr Met His Trp Tyr Gln Gln Lys Pro Gly Lys Ala
    50                  55                  60

CCA AAG CTT CTG ATC TAC AGC ACA TCC AAC CTG GCT TCT GGT GTG CCA   240
Pro Lys Leu Leu Ile Tyr Ser Thr Ser Asn Leu Ala Ser Gly Val Pro
65                  70                  75                  80

TCT AGA TTC AGC GGT AGC GGT AGC GGT ACA GAC TTC ACC TTC ACC ATC   288
Ser Arg Phe Ser Gly Ser Gly Ser Gly Thr Asp Phe Thr Phe Thr Ile
                85                  90                  95

AGC AGC CTC CAG GCT GAA GAT GCT GCT ACA TAC TAC TGC CAG CAA AGG   336
Ser Ser Leu Gln Ala Glu Asp Ala Ala Thr Tyr Tyr Cys Gln Gln Arg
            100                 105                 110

AGT AGT TAC CCG TAC ACG TTC GGC GGG GGG ACC AAG GTG GAA ATC AAA   384
Ser Ser Tyr Pro Tyr Thr Phe Gly Gly Gly Thr Lys Val Glu Ile Lys
        115                 120                 125

CGT ACG                                                           390
Arg Thr
    130
```

(2) INFORMATION FOR SEQ ID NO:44:

(i) SEQUENCE CHARACTERISTICS:

(A) LENGTH: 25 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:44:

```
ATGGTGAAAG AGTAAGATGT ACCGC                                       25
```

(2) INFORMATION FOR SEQ ID NO:45:

(i) SEQUENCE CHARACTERISTICS:

(A) LENGTH: 25 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:45:

GCGGTACATC TTACTCTTTC ACCAT 25

(2) INFORMATION FOR SEQ ID NO:46:

(i) SEQUENCE CHARACTERISTICS:

(A) LENGTH: 390 base pairs
(B) TYPE: nucleic acid
(C) STRANDEDNESS: double
(D) TOPOLOGY: linear

(ii) MOLECULE TYPE: other nucleic acid, synthetic DNA

(ix) FEATURES:

(A) NAME/KEY: sig peptide
(B) LOCATION: -22..-1
(C) IDENTIFICATION METHOD: S

(A) NAME/KEY: domain
(B) LOCATION: 24..33
(C) IDENTIFICATION METHOD: S
(D) OTHER INFORMATION: hypervariable region 1

(A) NAME/KEY: domain
(B) LOCATION: 49..55
(C) IDENTIFICATION METHOD: S
(D) OTHER INFORMATION: hypervariable region 2

(A) NAME/KEY: domain
(B) LOCATION: 86..96
(C) IDENTIFICATION METHOD: S
(D) OTHER INFORMATION: hypervariable region 3

(xi) SEQUENCE DESCRIPTION: SEQ ID NO:46:

```
ATG CAT TTT CAA GTG CAG ATT TTC AGC TTC CTG CTA ATC AGT GCC TCA    48
Met His Phe Gln Val Gln Ile Phe Ser Phe Leu Leu Ile Ser Ala Ser
 1               5                  10                  15

GTC ATA ATG TCC AGA GGA GAT ATC CAG CTG ACC CAG AGC CCA AGC AGC    96
Val Ile Met Ser Arg Gly Asp Ile Gln Leu Thr Gln Ser Pro Ser Ser
            20                  25                  30

CTG AGC GCT AGC CCA GGT GAC AGA GTG ACC ATC ACG TGC AGT GCC AGC   144
Leu Ser Ala Ser Pro Gly Asp Arg Val Thr Ile Thr Cys Ser Ala Ser
         35                  40                  45

TCA AGT GTA AGT TAC ATG CAC TGG TAT CAG CAG AAA CCA GGT AAG GCT   192
Ser Ser Val Ser Tyr Met His Trp Tyr Gln Gln Lys Pro Gly Lys Ala
     50                  55                  60

CCA AAG CTT TGG ATC TAC AGC ACA TCC AAC CTG GCT TCT GGT GTG CCA   240
Pro Lys Leu Trp Ile Tyr Ser Thr Ser Asn Leu Ala Ser Gly Val Pro
65                   70                  75                  80

TCT AGA TTC AGC GGT AGC GGT AGC GGT ACA TCT TAC TCT TTC ACC ATC   288
Ser Arg Phe Ser Gly Ser Gly Ser Gly Thr Ser Tyr Ser Phe Thr Ile
                85                  90                  95

AGC AGC CTC CAG CCA GAG GAC ATC GCT ACA TAC TAC TGC CAG CAA AGG   336
Ser Ser Leu Gln Pro Glu Asp Ile Ala Thr Tyr Tyr Cys Gln Gln Arg
            100                 105                 110

AGT AGT TAC CCG TAC ACG TTC GGC GGG GGG ACC AAG GTG GAA ATC AAA   384
Ser Ser Tyr Pro Tyr Thr Phe Gly Gly Gly Thr Lys Val Glu Ile Lys
        115                 120                 125

CGT ACG                                                           390
Arg Thr
    130
```

(2) INFORMATION FOR SEQ ID NO:47:

(i) SEQUENCE CHARACTERISTICS:

(A) LENGTH: 40 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:47:

```
TCTGGCTCCA TTCGGCTGAT GGTGAAAGAG TAAGATGTAC    40
```

(2) INFORMATION FOR SEQ ID NO:48:

(i) SEQUENCE CHARACTERISTICS:

(A) LENGTH: 40 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:48:

```
GTACATCTTA CTCTTTCACC ATCAGCCGAA TGGAGCCAGA          40
```

(2) INFORMATION FOR SEQ ID NO:49:

(i) SEQUENCE CHARACTERISTICS:

(A) LENGTH: 390 base pairs
(B) TYPE: nucleic acid
(C) STRANDEDNESS: double
(D) TOPOLOGY: linear

(ii) MOLECULE TYPE: other nucleic acid, synthetic DNA

(ix) FEATURES:

(A) NAME/KEY: sig peptide
(B) LOCATION: -22..-1
(C) IDENTIFICATION METHOD: S

(A) NAME/KEY: domain
(B) LOCATION: 24..33
(C) IDENTIFICATION METHOD: S
(D) OTHER INFORMATION: hypervariable region 1

(A) NAME/KEY: domain
(B) LOCATION: 49..55
(C) IDENTIFICATION METHOD: S
(D) OTHER INFORMATION: hypervariable region 2

(A) NAME/KEY: domain
(B) LOCATION: 86..96
(C) IDENTIFICATION METHOD: S
(D) OTHER INFORMATION: hypervariable region 3

(xi) SEQUENCE DESCRIPTION: SEQ ID NO:49:

```
ATG CAT TTT CAA GTG CAG ATT TTC AGC TTC CTG CTA ATC AGT GCC TCA    48
Met His Phe Gln Val Gln Ile Phe Ser Phe Leu Leu Ile Ser Ala Ser
 1               5                  10                  15
```

```
GTC ATA ATG TCC AGA GGA GAT ATC CAG CTG ACC CAG AGC CCA AGC AGC    96
Val Ile Met Ser Arg Gly Asp Ile Gln Leu Thr Gln Ser Pro Ser Ser
            20                  25                  30

CTG AGC GCT AGC CCA GGT GAC AGA GTG ACC ATC ACG TGC AGT GCC AGC   144
Leu Ser Ala Ser Pro Gly Asp Arg Val Thr Ile Thr Cys Ser Ala Ser
        35                  40                  45

TCA AGT GTA AGT TAC ATG CAC TGG TAT CAG CAG AAA CCA GGT AAG GCT   192
Ser Ser Val Ser Tyr Met His Trp Tyr Gln Gln Lys Pro Gly Lys Ala
    50                  55                  60

CCA AAG CTT TGG ATC TAC AGC ACA TCC AAC CTG GCT TCT GGT GTG CCA   240
Pro Lys Leu Trp Ile Tyr Ser Thr Ser Asn Leu Ala Ser Gly Val Pro
65                  70                  75                  80

TCT AGA TTC AGC GGT AGC GGT AGC GGT ACA TCT TAC TCT TTC ACC ATC   288
Ser Arg Phe Ser Gly Ser Gly Ser Gly Thr Ser Tyr Ser Phe Thr Ile
                85                  90                  95

AGC CGA ATG GAG CCA GAG GAC ATC GCT ACA TAC TAC TGC CAG CAA AGG   336
Ser Arg Met Glu Pro Glu Asp Ile Ala Thr Tyr Tyr Cys Gln Gln Arg
            100                 105                 110

AGT AGT TAC CCG TAC ACG TTC GGC GGG GGG ACC AAG GTG GAA ATC AAA   384
Ser Ser Tyr Pro Tyr Thr Phe Gly Gly Gly Thr Lys Val Glu Ile Lys
        115                 120                 125

CGT ACG                                                           390
Arg Thr
    130
```

(2) INFORMATION FOR SEQ ID NO:50:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:50:

```
TTCTGCTGGA ACCAGTGCAT                                              20
```

(2) INFORMATION FOR SEQ ID NO:51:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:51:

ATGCACTGGT TCCAGCAGAA 20

(2) INFORMATION FOR SEQ ID NO:52:

(i) SEQUENCE CHARACTERISTICS:

(A) LENGTH: 390 base pairs
(B) TYPE: nucleic acid
(C) STRANDEDNESS: double
(D) TOPOLOGY: linear

(ii) MOLECULE TYPE: other nucleic acid, synthetic DNA

(ix) FEATURES:

(A) NAME/KEY: sig peptide
(B) LOCATION: -22..-1
(C) IDENTIFICATION METHOD: S

(A) NAME/KEY: domain
(B) LOCATION: 24..33
(C) IDENTIFICATION METHOD: S
(D) OTHER INFORMATION: hypervariable region 1

(A) NAME/KEY: domain
(B) LOCATION: 49..55
(C) IDENTIFICATION METHOD: S
(D) OTHER INFORMATION: hypervariable region 2

(A) NAME/KEY: domain
(B) LOCATION: 86..96
(C) IDENTIFICATION METHOD: S
(D) OTHER INFORMATION: hypervariable region 3

(xi) SEQUENCE DESCRIPTION: SEQ ID NO:52:

```
ATG CAT TTT CAA GTG CAG ATT TTC AGC TTC CTG CTA ATC AGT GCC TCA    48
Met His Phe Gln Val Gln Ile Phe Ser Phe Leu Leu Ile Ser Ala Ser
 1               5                  10                  15

GTC ATA ATG TCC AGA GGA GAT ATC CAG CTG ACC CAG AGC CCA AGC AGC    96
Val Ile Met Ser Arg Gly Asp Ile Gln Leu Thr Gln Ser Pro Ser Ser
            20                  25                  30

CTG AGC GCT AGC CCA GGT GAC AGA GTG ACC ATC ACG TGC AGT GCC AGC   144
Leu Ser Ala Ser Pro Gly Asp Arg Val Thr Ile Thr Cys Ser Ala Ser
        35                  40                  45

TCA AGT GTA AGT TAC ATG CAC TGG TTC CAG CAG AAA CCA GGT AAG GCT   192
Ser Ser Val Ser Tyr Met His Trp Phe Gln Gln Lys Pro Gly Lys Ala
    50                  55                  60

CCA AAG CTT TGG ATC TAC AGC ACA TCC AAC CTG GCT TCT GGT GTG CCA   240
Pro Lys Leu Trp Ile Tyr Ser Thr Ser Asn Leu Ala Ser Gly Val Pro
65                  70                  75                  80

TCT AGA TTC AGC GGT AGC GGT AGC GGT ACA TCT TAC TCT TTC ACC ATC   288
Ser Arg Phe Ser Gly Ser Gly Ser Gly Thr Ser Tyr Ser Phe Thr Ile
                85                  90                  95

AGC AGC CTC CAG CCA GAG GAC ATC GCT ACA TAC TAC TGC CAG CAA AGG   336
Ser Ser Leu Gln Pro Glu Asp Ile Ala Thr Tyr Tyr Cys Gln Gln Arg
            100                 105                 110

AGT AGT TAC CCG TAC ACG TTC GGC GGG GGG ACC AAG GTG GAA ATC AAA   384
Ser Ser Tyr Pro Tyr Thr Phe Gly Gly Gly Thr Lys Val Glu Ile Lys
        115                 120                 125

CGT ACG                                                           390
Arg Thr
    130
```

(2) INFORMATION FOR SEQ ID NO: 53:

(i) SEQUENCE CHARACTERISTICS:

(A) LENGTH: 25 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:53:

```
TGGAGTCGGC TGATGGTGAG AGAGT                                        25
```

(2) INFORMATION FOR SEQ ID NO:54:

(i) SEQUENCE CHARACTERISTICS:

(A) LENGTH: 25 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:54:

```
ACTCTCTCAC CATCAGCCGA CTCCA                                    25
```

(2) INFORMATION FOR SEQ ID NO:55:

(i) SEQUENCE CHARACTERISTICS:

(A) LENGTH: 390 base pairs
(B) TYPE: nucleic acid
(C) STRANDEDNESS: double
(D) TOPOLOGY: linear

(ii) MOLECULE TYPE: other nucleic acid, synthetic DNA

(ix) FEATURES:

(A) NAME/KEY: sig peptide
(B) LOCATION: -22..-1
(C) IDENTIFICATION METHOD: S

(A) NAME/KEY: domain
(B) LOCATION: 24..33
(C) IDENTIFICATION METHOD: S
(D) OTHER INFORMATION: hypervariable region 1

(A) NAME/KEY: domain
(B) LOCATION: 49..55
(C) IDENTIFICATION METHOD: S
(D) OTHER INFORMATION: hypervariable region 2

(A) NAME/KEY: domain
(B) LOCATION: 86..96
(C) IDENTIFICATION METHOD: S
(D) OTHER INFORMATION: hypervariable region 3

(xi) SEQUENCE DESCRIPTION: SEQ ID NO:55:

```
ATG CAT TTT CAA GTG CAG ATT TTC AGC TTC CTG CTA ATC AGT GCC TCA    48
Met His Phe Gln Val Gln Ile Phe Ser Phe Leu Leu Ile Ser Ala Ser
 1               5                  10                  15
```

```
GTC ATA ATG TCC AGA GGA GAT ATC CAG CTG ACC CAG AGC CCA AGC AGC    96
Val Ile Met Ser Arg Gly Asp Ile Gln Leu Thr Gln Ser Pro Ser Ser
            20                  25                  30

CTG AGC GCT AGC CCA GGT GAC AGA GTG ACC ATC ACG TGC AGT GCC AGC   144
Leu Ser Ala Ser Pro Gly Asp Arg Val Thr Ile Thr Cys Ser Ala Ser
        35                  40                  45

TCA AGT GTA AGT TAC ATG CAC TGG TAT CAG CAG AAA CCA GGT AAG GCT   192
Ser Ser Val Ser Tyr Met His Trp Tyr Gln Gln Lys Pro Gly Lys Ala
    50                  55                  60

CCA AAG CTT TGG ATC TAC AGC ACA TCC AAC CTG GCT TCT GGT GTG CCA   240
Pro Lys Leu Trp Ile Tyr Ser Thr Ser Asn Leu Ala Ser Gly Val Pro
65                  70                  75                  80

TCT AGA TTC AGC GGT AGC GGT AGC GGT ACA TCT TAC TCT CTC ACC ATC   288
Ser Arg Phe Ser Gly Ser Gly Ser Gly Thr Ser Tyr Ser Leu Thr Ile
                85                  90                  95

AGC CGA CTC CAG CCA GAG GAC ATC GCT ACA TAC TAC TGC CAG CAA AGG   336
Ser Arg Leu Gln Pro Glu Asp Ile Ala Thr Tyr Tyr Cys Gln Gln Arg
            100                 105                 110

AGT AGT TAC CCG TAC ACG TTC GGC GGG GGG ACC AAG GTG GAA ATC AAA   384
Ser Ser Tyr Pro Tyr Thr Phe Gly Gly Gly Thr Lys Val Glu Ile Lys
        115                 120                 125

CGT ACG                                                           390
Arg Thr
    130
```

(2) INFORMATION FOR SEQ ID NO:56:

(i) SEQUENCE CHARACTERISTICS:

(A) LENGTH: 94 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:56:

```
CAGGAAACAG CTATGACGAA TTCCACCATG CATTTTCAAG TGCAGATTTT CAGCTTCCTG 60
CTAATCAGTG CCTCAGTCAT AATGTCCAGA GGAG                             94
```

(2) INFORMATION FOR SEQ ID NO:57:

(i) SEQUENCE CHARACTERISTICS:

(A) LENGTH: 88 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:57:

ACAAGTGATG GTGACTCTGT CTCCTGGAGA TGCAGACATG GAGGATGGAG ACTGGGTCAG 60

CTGGATGTCT CCTCTGGACA TTATGACT 88

(2) INFORMATION FOR SEQ ID NO:58:

(i) SEQUENCE CHARACTERISTICS:

(A) LENGTH: 92 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:58:

ACAGAGTCAC CATCACTTGT AGTGCAAGTT CAAGTGTAAG TTACATGCAC TGGTTTCAGC 60

AGAAACCAGG GAAATCACCT AAGCTCTGGA TC 92

(2) INFORMATION FOR SEQ ID NO:59:

(i) SEQUENCE CHARACTERISTICS:

(A) LENGTH: 87 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:59:

AAGATGTACC GCTACCGCTA CCGCTGAATC TAGATGGCAC ACCAGAAGCT AAATTTGAAG 60

TTGAGTAGAT CCAGAGCTTA GGTGATT 87

(2) INFORMATION FOR SEQ ID NO:60:

(i) SEQUENCE CHARACTERISTICS:

(A) LENGTH: 89 base pairs
(B) TYPE: nucleic acid
(D) TOPOLOGY: linear

(ii) MOLECULE TYPE: other nucleic acid, synthetic DNA

(xi) SEQUENCE DESCRIPTION: SEQ ID NO:60:

TAGCGGTAGC GGTACATCTT ACTCTCTCAC CATCAGCAGC ATGCAGCCTG AAGATTTTGC 60

AACTTATTAC TGTCAGCAAA GGAGTAGTT 89

(2) INFORMATION FOR SEQ ID NO:61:

(i) SEQUENCE CHARACTERISTICS:

(A) LENGTH: 84 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:61:

GTTTTCCCAG TCACGACCGT ACGTTTGATT TCCAGCTTGG TCCCCTGGCC GAACGTGTAC 60

GGGTAACTAC TCCTTTGCTG ACAG 84

(2) INFORMATION FOR SEQ ID NO:62:

(i) SEQUENCE CHARACTERISTICS:

(A) LENGTH: 35 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:62:

ACTCGAGGCT CTTTCCAGGG CTCTGCTTCA CCCAG 35

(2) INFORMATION FOR SEQ ID NO:63:

(i) SEQUENCE CHARACTERISTICS:

(A) LENGTH: 35 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:63:

CTGGGTGAAG CAGAGCCCTG GAAAGAGCCT CGAGT 35

(2) INFORMATION FOR SEQ ID NO:64:

(i) SEQUENCE CHARACTERISTICS:

(A) LENGTH: 433 base pairs
(B) TYPE: nucleic acid
(C) STRANDEDNESS: double
(D) TOPOLOGY: linear

(ii) MOLECULE TYPE: other nucleic acid, synthetic DNA

(ix) FEATURES:

(A) NAME/KEY: sig peptide
(B) LOCATION: -19..-1
(C) IDENTIFICATION METHOD: S

(A) NAME/KEY: domain
(B) LOCATION: 31..35
(C) IDENTIFICATION METHOD: S
(D) OTHER INFORMATION: hypervariable region 1

(A) NAME/KEY: domain
(B) LOCATION: 50..66
(C) IDENTIFICATION METHOD: S
(D) OTHER INFORMATION: hypervariable region 2

(A) NAME/KEY: domain
(B) LOCATION: 99..109
(C) IDENTIFICATION METHOD: S
(D) OTHER INFORMATION: hypervariable region 3

(xi) SEQUENCE DESCRIPTION: SEQ NO ID:64:

```
ATG GGA TGG AGC TGG ATC TTT CTC TTC CTC CTG TCA GGA ACT GCA GGT   48
Met Gly Trp Ser Trp Ile Phe Leu Phe Leu Leu Ser Gly Thr Ala Gly
 1               5                  10                  15

GTC CTC TCT GAG GTG CAG CTG GTG CAG TCT GGA GCA GAG GTG AAG AAG   96
Val Leu Ser Glu Val Gln Leu Val Gln Ser Gly Ala Glu Val Lys Lys
            20                  25                  30

CCT GGG GCC TCA GTG AAG GTC TCC TGC AAG GCT TCC GGA TAC ACC TTC  144
Pro Gly Ala Ser Val Lys Val Ser Cys Lys Ala Ser Gly Tyr Thr Phe
        35                  40                  45

ACT GAC TAC AAC ATG GAC TGG GTG AAG CAG AGC CCT GGA AAG AGC CTC  192
Thr Asp Tyr Asn Met Asp Trp Val Lys Gln Ser Pro Gly Lys Ser Leu
    50                  55                  60

GAG TGG ATG GGA TAT ATT TAT CCT AAC AAT GGT GGT ACT GGC TAC AAC  240
Glu Trp Met Gly Tyr Ile Tyr Pro Asn Asn Gly Gly Thr Gly Tyr Asn
65                  70                  75                  80

CAG AAG TTC AAG AGC AAG GTC ACC ATT ACC GTA GAC ACA TCC ACG AGC  288
Gln Lys Phe Lys Ser Lys Val Thr Ile Thr Val Asp Thr Ser Thr Ser
                85                  90                  95

ACA GCC TAC ATG GAG CTG CAC AGC CTG AGA TCT GAG GAC ACG GCC GTG  336
Thr Ala Tyr Met Glu Leu His Ser Leu Arg Ser Glu Asp Thr Ala Val
            100                 105                 110

TAT TAC TGT GCG ACC TAC GGT CAT TAC TAC GGC TAC ATG TTT GCT TAC  384
Tyr Tyr Cys Ala Thr Tyr Gly His Tyr Tyr Gly Tyr Met Phe Ala Tyr
        115                 120                 125

TGG GGC CAG GGA ACC CTG GTC ACC GTC TCC TCA GCC TCC ACC AAG GGC  432
Trp Gly Gln Gly Thr Leu Val Thr Val Ser Ser Ala Ser Thr Lys Gly
    130                 135                 140                 144

C                                                                433
```

(2) INFORMATION FOR SEQ ID NO:65:

(i) SEQUENCE CHARACTERISTICS:

(A) LENGTH: 24 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:65:

```
TGTCCAGGGC TCTGCTTCAC CCAG                                        24
```

(2) INFORMATION FOR SEQ ID NO:66:

(i) SEQUENCE CHARACTERISTICS:

(A) LENGTH: 24 base pairs
(B) TYPE: nucleic acid

(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:66:

CTGGGTGAAG CAGAGCCCTG GACA 24

(2) INFORMATION FOR SEQ ID NO:67:

(i) SEQUENCE CHARACTERISTICS:

(A) LENGTH: 25 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:67:

TCTACGGTCA AGGTGGCCTT GCTCT 25

(2) INFORMATION FOR SEQ ID NO:68:

(i) SEQUENCE CHARACTERISTICS:

(A) LENGTH: 25 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:68:

AGAGCAAGGC CACCTTGACC GTAGA 25

(2) INFORMATION FOR SEQ ID NO:69:

(i) SEQUENCE CHARACTERISTICS:

(A) LENGTH: 433 base pairs
(B) TYPE: nucleic acid
(C) STRANDEDNESS: double
(D) TOPOLOGY: linear

(ii) MOLECULE TYPE: other nucleic acid, synthetic DNA

(ix) FEATURES:

(A) NAME/KEY: sig peptide
(B) LOCATION: -19..-1

(C) IDENTIFICATION METHOD: S

(A) NAME/KEY: domain
(B) LOCATION: 31..35
(C) IDENTIFICATION METHOD: S
(D) OTHER INFORMATION: hypervariable region 1

(A) NAME/KEY: domain
(B) LOCATION: 50..66
(C) IDENTIFICATION METHOD: S
(D) OTHER INFORMATION: hypervariable region 2

(A) NAME/KEY: domain
(B) LOCATION: 99..109
(C) IDENTIFICATION METHOD: S
(D) OTHER INFORMATION: hypervariable region 3

(xi) SEQUENCE DESCRIPTION: SEQ ID NO:69:

```
ATG GGA TGG AGC TGG ATC TTT CTC TTC CTC CTG TCA GGA ACT GCA GGT    48
Met Gly Trp Ser Trp Ile Phe Leu Phe Leu Leu Ser Gly Thr Ala Gly
 1               5                  10                  15

GTC CTC TCT GAG GTG CAG CTG GTG CAG TCT GGA GCA GAG GTG AAG AAG    96
Val Leu Ser Glu Val Gln Leu Val Gln Ser Gly Ala Glu Val Lys Lys
            20                  25                  30

CCT GGG GCC TCA GTG AAG GTC TCC TGC AAG GCT TCC GGA TAC ACC TTC   144
Pro Gly Ala Ser Val Lys Val Ser Cys Lys Ala Ser Gly Tyr Thr Phe
        35                  40                  45

ACT GAC TAC AAC ATG GAC TGG GTG CGA CAG GCC CCT GGA CAA GGG CTC   192
Thr Asp Tyr Asn Met Asp Trp Val Arg Gln Ala Pro Gly Gln Gly Leu
    50                  55                  60

GAG TGG ATG GGA TAT ATT TAT CCT AAC AAT GGT GGT ACT GGC TAC AAC   240
Glu Trp Met Gly Tyr Ile Tyr Pro Asn Asn Gly Gly Thr Gly Tyr Asn
65                  70                  75                  80

CAG AAG TTC AAG AGC AAG GCC ACC TTG ACC GTA GAC ACA TCC ACG AGC   288
Gln Lys Phe Lys Ser Lys Ala Thr Leu Thr Val Asp Thr Ser Thr Ser
                85                  90                  95

ACA GCC TAC ATG GAG CTG CAC AGC CTG AGA TCT GAG GAC ACG GCC GTG   336
Thr Ala Tyr Met Glu Leu His Ser Leu Arg Ser Glu Asp Thr Ala Val
            100                 105                 110

TAT TAC TGT GCG ACC TAC GGT CAT TAC TAC GGC TAC ATG TTT GCT TAC   384
Tyr Tyr Cys Ala Thr Tyr Gly His Tyr Tyr Gly Tyr Met Phe Ala Tyr
        115                 120                 125

TGG GGC CAG GGA ACC CTG GTC ACC GTC TCC TCA GCC TCC ACC AAG GGC   432
Trp Gly Gln Gly Thr Leu Val Thr Val Ser Ser Ala Ser Thr Lys Gly
    130                 135                 140                 144

C                                                                 433
```

(2) INFORMATION FOR SEQ ID NO:70:

(i) SEQUENCE CHARACTERISTICS:

(A) LENGTH: 433 base pairs
(B) TYPE: nucleic acid
(C) STRANDEDNESS: double
(D) TOPOLOGY: linear

(ii) MOLECULE TYPE: other nucleic acid, synthetic DNA

(ix) FEATURES:

(A) NAME/KEY: sig peptide
(B) LOCATION: -19..-1
(C) IDENTIFICATION METHOD: S

(A) NAME/KEY: domain
(B) LOCATION: 31..35
(C) IDENTIFICATION METHOD: S
(D) OTHER INFORMATION: hypervariable region 1

(A) NAME/KEY: domain
(B) LOCATION: 50..66
(C) IDENTIFICATION METHOD: S
(D) OTHER INFORMATION: hypervariable region 2

(A) NAME/KEY: domain
(B) LOCATION: 99..109
(C) IDENTIFICATION METHOD: S
(D) OTHER INFORMATION: hypervariable region 3

(xi) SEQUENCE DESCRIPTION: SEQ ID NO:70:

```
ATG GGA TGG AGC TGG ATC TTT CTC TTC CTC CTG TCA GGA ACT GCA GGT   48
Met Gly Trp Ser Trp Ile Phe Leu Phe Leu Leu Ser Gly Thr Ala Gly
 1               5                  10                  15

GTC CTC TCT GAG GTG CAG CTG GTG CAG TCT GGA GCA GAG GTG AAG AAG   96
Val Leu Ser Glu Val Gln Leu Val Gln Ser Gly Ala Glu Val Lys Lys
            20                  25                  30

CCT GGG GCC TCA GTG AAG GTC TCC TGC AAG GCT TCC GGA TAC ACC TTC  144
Pro Gly Ala Ser Val Lys Val Ser Cys Lys Ala Ser Gly Tyr Thr Phe
        35                  40                  45

ACT GAC TAC AAC ATG GAC TGG GTG AAG CAG AGC CCT GGA AAG AGC CTC  192
Thr Asp Tyr Asn Met Asp Trp Val Lys Gln Ser Pro Gly Lys Ser Leu
    50                  55                  60

GAG TGG ATG GGA TAT ATT TAT CCT AAC AAT GGT GGT ACT GGC TAC AAC  240
Glu Trp Met Gly Tyr Ile Tyr Pro Asn Asn Gly Gly Thr Gly Tyr Asn
65                  70                  75                  80

CAG AAG TTC AAG AGC AAG GCC ACC TTG ACC GTA GAC ACA TCC ACG AGC  288
Gln Lys Phe Lys Ser Lys Ala Thr Leu Thr Val Asp Thr Ser Thr Ser
                85                  90                  95

ACA GCC TAC ATG GAG CTG CAC AGC CTG AGA TCT GAG GAC ACG GCC GTG  336
Thr Ala Tyr Met Glu Leu His Ser Leu Arg Ser Glu Asp Thr Ala Val
            100                 105                 110

TAT TAC TGT GCG ACC TAC GGT CAT TAC TAC GGC TAC ATG TTT GCT TAC  384
Tyr Tyr Cys Ala Thr Tyr Gly His Tyr Tyr Gly Tyr Met Phe Ala Tyr
        115                 120                 125

TGG GGC CAG GGA ACC CTG GTC ACC GTC TCC TCA GCC TCC ACC AAG GGC  432
Trp Gly Gln Gly Thr Leu Val Thr Val Ser Ser Ala Ser Thr Lys Gly
    130                 135                 140                 144

C                                                                433
```

(2) INFORMATION FOR SEQ ID NO:11:

(i) SEQUENCE CHARACTERISTICS:

(A) LENGTH: 433 base pairs
(B) TYPE: nucleic acid
(C) STRANDEDNESS: double
(D) TOPOLOGY: linear

(ii) MOLECULE TYPE: other nucleic acid, synthetic DNA

(ix) FEATURES:

(A) NAME/KEY: sig peptide
(B) LOCATION: -19..-1
(C) IDENTIFICATION METHOD: S

(A) NAME/KEY: domain
(B) LOCATION: 31..35

(C) IDENTIFICATION METHOD: S
(D) OTHER INFORMATION: hypervariable region 1

(A) NAME/KEY: domain
(B) LOCATION: 50..66
(C) IDENTIFICATION MHTHOD: S
(D) OTHER INFORMATION: hypervariable region 2

(A) NAME/KEY: domain
(B) LOCATION: 99..109
(C) IDENTIFICATION METHOD: S
(D) OTHER INFORMATION: hypervariable region 3

(xi) SEQUENCE DESCRIPTION: SEQ ID NO:71:

```
ATG GGA TGG AGC TGG ATC TTT CTC TTC CTC CTG TCA GGA ACT GCA GGT    48
Met Gly Trp Ser Trp Ile Phe Leu Phe Leu Leu Ser Gly Thr Ala Gly
 1               5                  10                  15

GTC CTC TCT GAG GTG CAG CTG GTG CAG TCT GGA GCA GAG GTG AAG AAG    96
Val Leu Ser Glu Val Gln Leu Val Gln Ser Gly Ala Glu Val Lys Lys
            20                  25                  30

CCT GGG GCC TCA GTG AAG GTC TCC TGC AAG GCT TCC GGA TAC ACC TTC   144
Pro Gly Ala Ser Val Lys Val Ser Cys Lys Ala Ser Gly Tyr Thr Phe
        35                  40                  45

ACT GAC TAC AAC ATG GAC TGG GTG AAG CAG AGC CCT GGA CAA GGG CTC   192
Thr Asp Tyr Asn Met Asp Trp Val Lys Gln Ser Pro Gly Gln Gly Leu
    50                  55                  60

GAG TGG ATG GGA TAT ATT TAT CCT AAC AAT GGT GGT ACT GGC TAC AAC   240
Glu Trp Met Gly Tyr Ile Tyr Pro Asn Asn Gly Gly Thr Gly Tyr Asn
65                  70                  75                  80

CAG AAG TTC AAG AGC AAG GCC ACC TTG ACC GTA GAC ACA TCC ACG AGC   288
Gln Lys Phe Lys Ser Lys Ala Thr Leu Thr Val Asp Thr Ser Thr Ser
                85                  90                  95

ACA GCC TAC ATG GAG CTG CAC AGC CTG AGA TCT GAG GAC ACG GCC GTG   336
Thr Ala Tyr Met Glu Leu His Ser Leu Arg Ser Glu Asp Thr Ala Val
            100                 105                 110

TAT TAC TGT GCG ACC TAC GGT CAT TAC TAC GGC TAC ATG TTT GCT TAC   384
Tyr Tyr Cys Ala Thr Tyr Gly His Tyr Tyr Gly Tyr Met Phe Ala Tyr
        115                 120                 125

TGG GGC CAG GGA ACC CTG GTC ACC GTC TCC TCA GCC TCC ACC AAG GGC   432
Trp Gly Gln Gly Thr Leu Val Thr Val Ser Ser Ala Ser Thr Lys Gly
    130                 135                 140                 144

C                                                                 433
```

(2) INFORMATION FOR SEQ ID NO:72:

(i) SEQUENCE CHARACTERISTICS:

(A) LENGTH: 20 base pairs

(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:72:

TGAATCTAGC TGGCACACCA 20

(2) INFORMATION FOR SEQ ID NO:73:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: other nucleic acid, synthetic DNA

(xi) SEQUENCE DESCRIPTION: SEQ ID NO:73:

TGGTGTGCCA GCTAGATTCA 20

## Description

FIELD OF THE INVENTION

This invention relates to a human complementarity determining region (referred to as "CDR" hereinafter) grafted antibody to ganglioside $GM_2$ (referred to as "$GM_2$" hereinafter). This invention also relates to a DNA fragment encoding the above-described antibody, particularly its variable region (referred to as "V region" hereinafter). This invention relates to an expression vector which contains the DNA fragment and to a host transformed with the expression vector. This invention further relates to a method for the production of the human CDR-grafted antibody specific for $GM_2$ and to its therapeutic and diagnostic use.

BACKGROUND OF THE INVENTION

It is known in general that, when a mouse antibody is administered to human, the mouse antibody is recognized as foreign matter in the human body and thus induces a human antibody to a mouse antibody (human anti-mouse antibody, referred to as "HAMA" hereinafter) which reacts with the administered mouse antibody to produce adverse effects (Dillman, R.O. *et al., J. Clin. Oncol.*, 2, 881 (1984); Meeker, T.C. *et al., Blood*, 65, 1349 (1985); LoBuglio, A.F. *et al., J. Natl. Cancer Inst.,* 80, 932 (1988); Houghton, A.N. *et al., Proc. Natl. Acad. Sci. U.S.A.,* 82, 1242 (1985)), and the administered mouse antibody is quickly cleared (Pimm, M.V. et *al., J. Nucl. Med.*, 26, 1011 (1985); Meeker, T.C. *et al*., Blood, 65, 1349 (1985); Khazaeli, M.B. *et al*., *J. Natl. Cancer Inst.*, 80, 937 (1988)) to reduce effects of the antibody (Shawler, D.L. *et al*., *J. Immunol*., 135, 1530 (1985); Courtenay-Luck, N.S. *et al., Cancer Res.,* **46, 6489** (1986)).

In order to solve these problems, attempts have been made to convert a mouse antibody into a humanized antibody such as a human chimeric antibody or a human CDR-grafted antibody. The human chimeric antibody is an antibody in which its V region is derived from an antibody of nonhuman animal and its constant region (referred to as "C region" hereinafter) is derived from a human antibody (Morrison, S.L. *et al., Proc. Natl. Acad. Sci. U.S.A.,* 81, 6851 (1984)). Furthermore, it is reported that, when this type of antibody is administered to human, HAMA is hardly induced and its half-life in blood increases six times (LoBuglio, A.F. *et al., Proc. Natl. Acad. Sci. U.S.A.,* 86, 4220 (1989)). The human CDR-grafted antibody is an antibody in which the CDR of human antibody is replaced by other CDR derived from nonhuman animal (Jones, P.T. *et al., Nature,* 321, 522 (1986)), which is also called a reshaped human antibody. It is reported that, in a test of a human CDR-grafted antibody in monkeys, its immunogenicity is reduced and its half-life in blood is increased four to five times, in comparison with a mouse antibody (Hakimi, J. *et al.*, J. *Immunol*., 147, 1352 (1991)).

Also, with regard to the cytotoxicity of antibodies, it is reported that the Fc region of a human antibody activates human complement and human effector cells more effectively than the Fc region of mouse antibody. For example, it is reported that human effector cell-mediated anti-tumor effects of a mouse antibody to $GD_2$ is increased when the antibody is converted into a human chimeric antibody having human antibody Fc region (Mueller, B.M. *et al*., *J. Immunol.*, 144, 1382 (1990)), and similar results are reported on a human CDR-grafted antibody to CAMPATH-1 antigen (Reichmann, L. *et al., Nature,* 332, 323 (1988)). These results indicate that humanized antibodies are more desirable than mouse antibodies as antibodies to be clinically used in human.

Ganglioside as a glycolipid having sialic acid is a molecule which constitutes an animal cell membrane, and comprises a carbonhydrate chain as a hydrophilic side chain and sphingosine and fatty acid as hydrophobic side chains. It is known that types and expression quantities of ganglioside vary depending on the cell species, organ species, animal species. It is known also that the expression of ganglioside changes quantitatively and qualitatively in the process of cancer development of cells (Hakomori, S. *et al., Cancer Res.,* 45, 2405 (1985)). For example, it is reported that gangliosides $GD_2$, $GD_3$, $GM_2$ which are hardly observed in normal cells are expressed in nerve ectoderm system tumors considered to have high malignancy, such as neuroblastoma, pulmonary small cell carcinoma and melanoma (Pukel, C.S. *et al., J. Exp. Med.,* 155, 1133 (1982); Nudelman, E. *et al., J. Biol. Chem.*, 257, 12752 (1982); Werkmeister, J.A. *et al., Cancer Res.,* 47, 225 (1987); Mujoo, K. *et al., Cancer Res.,* 47, 1098 (1987); Cheung, N.V. *et al*., *Cancer Res.,* 45, 2642 (1985); Tai, T. *et al., Proc. Natl. Acad. Sci. U.S.A.,* 80, 5392 (1983)), and antibodies to these gangliosides are considered to be useful for diagnosis and treatment of various cancers in human.

It is indicated that human antibodies to $GM_2$ are useful for treatment of human melanoma (Irie, R.F. *et al., Lancet*, I, 786 (1989)). However, the antibodies to $GM_2$ so far reported are either those which are derived from nonhuman animal or a human antibody belonging to the IgM class (Natoli, E. J. *et al*., *Cancer Res*., 46, 4116 (1986); Miyake, M. *et al., Cancer Res*., 48, 6154 (1988); Cahan, L.D. *et al*., *Proc. Natl. Acad. Sci. U.S.A.,* 79, 7629 (1982); Fredman, P. *et al., J. Biol. Chem*., 264, 12122 (1989)). The antibody of the IgM class, however, is unsuitable for applying to human, because it has a pentameric structure having a large molecular weight (about 900,000) in comparison with the antibody of IgG class which has a molecular weight of about 150,000, thus posing a problem in carrying out its purification, in addition to other problems such as its short half-life in blood and weak anti-tumor effect (Bernstein, I.D. *et al.*, *Monoclonal Antibodies, Plenum Press,* p.275 (1980)).

Because of the above, it is desirable to develop a humanized antibody to $GM_2$ of the IgG class which, when applied to human, does not induce HAMA in the human body, causes less adverse effects, shows prolonged half-life in blood and has improved anti-tumor effect, so that its high diagnostic and therapeutic effects on human cancers can be expected.

The inventors of the present invention disclose in JP-A-6-205694 (the term "JP-A" as used herein means an "unexamined published Japanese patent application") (corresponding to EP-A-0 598 998) a method for producing an IgG class human chimeric antibody and a human CDR-grafted antibody, which can specifically react with $GM_2$ and are useful for diagnosis and treatment of human cancers. However, there are no reports on a human CDR-grafted antibody which, when compared with a human chimeric antibody, has similar levels of binding activity and binding specificity for $GM_2$ and anti-tumor effects upon $GM_2$-positive cells.

## SUMMARY OF THE INVENTION

As described in the foregoing, it is considered that human CDR-grafted antibodies are useful for diagnosis and treatment of human cancers. However, the antibody activity is reduced when only the CDRs of the heavy chain (referred to as "H chain" hereinafter) V region and light chain (referred to as "L chain" hereinafter) V region of an antibody of a nonhuman animal replace the CDRs of the H chain V region and L chain V region of a human antibody, so that great concern has been directed toward the establishment of a method for the production of a human CDR-grafted antibody to $GM_2$ belonging to the IgG class (referred to as "human CDR-grafted anti-$GM_2$ antibody" hereinafter) which, when compared with a human chimeric antibody, has similar levels of binding activity and binding specificity for $GM_2$ and anti-tumor effects upon $GM_2$-positive cells, as well as a method for producing a human CDR-grafted antibody, which can be applied to all antibodies.

This invention relates to a human CDR-grafted antibody which specifically reacts with ganglioside $GM_2$, wherein said antibody comprises CDR 1, CDR 2 and CDR 3 of a H chain V region comprising amino acid sequences of SEQ ID NO:1, SEQ ID NO:2 and SEQ ID NO:3, and CDR 1, CDR 2 and CDR 3 of a L chain V region comprising amino acid sequences of SEQ ID NO:4, SEQ ID NO:5 and SEQ ID NO:6, and frameworks (referred to as "FR" hereinafter) of Kabat's Human Sub Group (HSG), which comprise an amino acid sequence in: which at least one amino acid of positions 38, 40, 67, 72, 84 and 98 in the FR of the H chain V region and positions 4, 11, 15, 35, 42, 46, 59, 69, 70, 71, 72, 76, 77 and 103 in the FR of the L chain V region is replaced by another amino acid.

Furthermore, the present invention relates to the above human CDR-grafted antibody, wherein said antibody has antigen-binding activity, binding specificity, antibody dependent cell mediated cytotoxicity (ADCC), and complement dependent cytotoxicity (CDC) comparable to those of a human chimeric antibody having a V region of a monoclonal antibody derived from a nonhuman animal which specifically reacts with ganglioside $GM_2$ .

Moreover, the present invention relates to the above human CDR-grafted antibody, wherein said H chain C region of the antibody is derived from an antibody belonging to the human antibody IgG class.

## BRIEF EXPLANATION OF THE DRAWINGS

Fig. 1 shows a construction scheme for a plasmid named pBSA.
Fig. 2 shows a construction scheme for a plasmid named pBSAE.
Fig. 3 shows a construction scheme for a plasmid named pBSH-S.
Fig. 4 shows a construction scheme for a plasmid named pBSK-H.
Fig. 5 shows a construction scheme for plasmids named pBSH-SA and pBSK-HA.
Fig. 6 shows a construction scheme for plasmids named pBSH-SAE and pBSK-HAE.
Fig. 7 shows a construction scheme for plasmids named pBSH-SAEE and pBSK-HAEE.
Fig. 8 shows a construction scheme for a plasmid named pBSK-HAEESal.
Fig. 9 shows a construction scheme for a plasmid named pBSX-S.
Fig. 10 shows a construction scheme for a plasmid named pBSX-SA.
Fig. 11 shows a construction scheme for a plasmid named pBSSC.
Fig. 12 shows a construction scheme for a plasmid named pBSMo.
Fig. 13 shows a construction scheme for a plasmid named pBSMoS.
Fig. 14 shows a construction scheme for a plasmid named pChiIgLA1S.
Fig. 15 shows a construction scheme for a plasmid named pMohCκ.
Fig. 16 shows a construction scheme for a plasmid named pBSMoSal.
Fig. 17 shows a construction scheme for a plasmid named pBSMoSalS.
Fig. 18 shows a construction scheme for a plasmid named pBShCγ1.

Fig. 19 shows a construction scheme for a plasmid named pMohCγ1.
Fig. 20 shows a construction scheme for a plasmid named pMoγ1SP.
Fig. 21 shows a construction scheme for a plasmid named pMoκγ1SP.
Fig. 22 shows a construction scheme for a plasmid named pKANTEX93.
Fig. 23 shows a construction scheme for a plasmid named pBSNA.
Fig. 24 shows a construction scheme for a plasmid named pBSH3.
Fig. 25 shows a construction scheme for a plasmid named pBSES.
Fig. 26 shows a construction scheme for a plasmid named pBSL3.
Fig. 27 shows a construction scheme for a plasmid named pKANTEX796H.
Fig. 28 shows a construction scheme for a plasmid named pKANTEX796.
Fig. 29 shows a construction scheme for a plasmid named pT796.
Fig. 30 is a graphic representation of transient mouse-human chimeric anti-$GM_2$ antibody expression by the plasmids pKANTEX796 and pT796. The ordinate donotes the antibody concentration that showed $GM_2$-binding activity, and the abscissa denotes the time after introduction of the plasmid.
Fig. 31 shows a construction scheme for a plasmid named pBSH10.
Fig. 32 shows a construction scheme for a plasmid named pBSL16.
Fig. 33 illustrates a process for mutagenesis by PCR and a process for cloning DNA fragments mutated.
Fig. 34 shows a construction scheme for a plasmid named pBSLV1+2.
Fig. 35 shows a construction scheme for a plasmid named pBSLm-28.
Fig. 36 shows a construction scheme for a plasmid named pBSBSGL.
Fig. 37 shows a construction scheme for a plasmid named pT796LCDR.
Fig. 38 shows a construction scheme for plasmids named pT796HLCDR, pT796HLCDRHV2 and pT796HLCDRHV4.
Fig. 39 shows a construction scheme for a plasmid named pT796HLCDRH10.
Fig. 40 shows construction scheme for plasmids named pT796HCDR, pT796HCDRHV2, pT796HCDRHV4 and pT796HCDRH10.
Fig. 41 is a graphic representation of the results of human CDR-grafted anti-$GM_2$ antibody activity evaluation in terms of transient expression as obtained using the plasmids pT796, pT796HCDR, pT796HCDRHV2, pT796HCDRHV4 and pT796HCDRH10. The ordinate denotes the plasmid used, and the abscissa denotes the relative activity value with the activity obtained with the chimera antibody being taken as 100%.
Fig. 42 shows a construction scheme for plasmids named pT796HLCDRLV1, pT796HLCDRLV2, pT796HLCDRLV3, pT796HLCDRLV4, pT796HLCDRLV8, pT796HLCDRLm-2, pT796HLCDRLm-8, pT796HLCDRLm-28 and pT796HLCDRHSGL.
Fig. 43 is a graphic representation of the results of human CDR-grafted anti-$GM_2$ antibody activity evaluation in terms of transient expression as obtained using the plasmids pT796, pT796HLCDR, pT796HLCDRLV1, pT796HLCDRLV2, pT796HLCDRLV3, pT796HLCDRLV4, pT796HLCDRLV8, pT796HLCDRLm-2, pT796HLCDRLm-8, pT796HLCDRLm-28 and pT796HLCDRHSGL. The ordinate denotes the plasmid used, and the abscissa denotes the relative activity value with the activity obtained with the chimera antibody being taken as 100%.
Fig. 44 shows a construction scheme for plasmids named pKANTEX796HLCDRLm-28 and pKANTEX796HLCDRHSGL.
Fig. 45 shows electrophoretic patterns obtained for mouse-human chimeric anti-$GM_2$ antibody KM966 and purified human CDR-grafted anti-$GM_2$ antibodies KM8966 and KM8967 by SDS-PAGE (4 to 15% gradient gels used). The patterns shown on the left side are those obtained under reducing conditions, and those on the right under nonreducing conditions. From the left of each lane, the electrophoretic patterns for high-molecular-weight marker, KM966, KM8966, KM8967, low-molecular-weight marker, KM966, KM8966 and KM8967 are shown in that order.
Fig. 46 is a graphic representation of the $GM_2$-binding activities of mouse-human chimeric anti-$GM_2$ antibody KM966 and purified human CDR-grafted anti-$GM_2$ antibodies KM8966 and KM8967. The ordinate denotes the $GM_2$-binding activity, and the abscissa the antibody concentration.
Fig. 47 is a graphic representation of the reactivities of mouse-human chimeric anti-$GM_2$ antibody KM966 and purified human CDR-grafted anti-$GM_2$ antibodies KM8966 and KM8967 against various gangliosides. The ordinate denotes the ganglioside species, and the abscissa the binding activity. $AcGM_2$ stands for N-acetyl-$GM_2$, $GcGM_2$ for N-glycolyl-$GM_2$, AcGM3 for N-acetyl-$GM_3$ and GcGM3 for N-N-glycolyl-$GM_3$.
Fig. 48 is a graphic representation of the reactivities of mouse-human chimeric anti-$GM_2$ antibody KM966 and purified human CDR-grafted anti-$GM_2$ antibodies KM8966 and KM8967 against the human lung small cell carcinoma cell line SBC-3. The ordinate denotes the number of cells, and the abscissa the fluorescence intensity. From the lowermost graph, the reactivities of control, KM8967, KM8966 and KM966 are shown in that order.
Fig. 49 graphically shows the CDC activities of mouse-human chimeric anti-$GM_2$ antibody KM966 and purified human CDR-grafted anti-$GM_2$ antibodies KM8966 and KM8967 against the human lung small cell carcinoma cell line SBC-3. The ordinate indicates the cytotoxic activity and the abscissa the concentration of the antibody.

Fig. 50 graphically shows the ADCC activities of mouse-human chimeric anti-$GM_2$ antibody KM966 and purified human CDR-grafted anti-$GM_2$ antibodies KM8966 and KM8967 against the human lung small cell carcinoma cell line SBC-3. The ordinate indicates the cytotoxicity and the abscissa the concentration of the antibody.

Fig. 51 shows a construction scheme for plasmids, pKANTEX796HM1Lm-28, pKANTEX796HM2Lm-28, pKANTEX796HM3Lm-28, pKANTEX796HM31Lm-28 and pMU4TEX796M432Lm-28.

Fig. 52 shows the electrophoretic patterns in SDS-PAGE (using 4-15% gradient gels) of mouse-human chimeric anti-$GM_2$ antibody KM966, human CDR-grafted anti-$GM_2$ antibody KM8966 and human CDR-grafted anti-$GM_2$ antibodies each having various types of substitution. The pattern obtained under nonreducing conditions is shown on the left side and that obtained under reducing conditions on the right side. M stands for molecular weight markers (from the top, the arrows indicate the molecular weight of 205 Kd, 140 Kd, 83 Kd, 45 Kd, 32.6 Kd, 18 Kd and 7.5 Kd in that order) and 1, 2, 3, 4, 5, 6 and 7 stand for the electrophoretic patterns of KM966, KM8966, M1-28, M2-28, M3-28, M31-28 and M32-28, respectively.

Fig. 53 graphically shows the CDC activities of mouse-human chimeric anti-$GM_2$ antibody KM966, human CDR-grafted anti-$GM_2$ antibody KM8966 and human CDR-grafted anti-$GM_2$ antibodies each having various types of substitution against the human lung small cell carcinoma cell line SBC-3. The ordinate indicates the cytotoxic activity and the abscissa the concentration of the antibody.

Fig. 54 shows a construction scheme for plasmids, pKANTEX796HLm-28 No.1, pKANTEX796HM1Lm-28.No. 1, pKANTEX796HM2Lm-28 No.1 and pKANTEX796HM3Lm-28 No.1.

Fig. 55 shows the electrophoretic patterns in SDS-PAGE (using 4-15% gradient gels) of mouse-human chimeric anti-$GM_2$ antibody KM966 and human CDR-grafted anti-$GM_2$ antibodies each having various types of substitution. The pattern obtained under nonreducing conditions is shown on the left side and that obtained under reducing conditions on the right side. M stands for molecular weight markers (from the top, the arrows indicate the molecular weight of 205 Kd, 140 Kd, 83 Kd, 45 Kd, 32.6 Kd, 18 Kd and 7.5 Kd in that order) and 1, 2, 3, 4 and 5 stand for the electrophoretic patterns of KM966, h796H-No.1, M1-No.1, M2-No.1 and M3-No.1, respectively.

Fig. 56 graphically shows the CDC activities of mouse-human chimeric anti-$GM_2$ antibody KM966, human CDR-grafted anti-$GM_2$ antibodies KM8966 and KM8970 and human CDR-grafted anti-$GM_2$ antibodies each having various types of substitution against the human lung small cell carcinoma cell line SBC-3. The ordinate indicates the cytotoxic activity and the abscissa the concentration of the antibody.

Fig. 57 graphically shows the $GM_2$-binding activities of mouse-human chimeric anti-$GM_2$ antibody KM966 and human CDR-grafted anti-$GM_2$ antibodies KM8969 and KM8970. The ordinate indicates the $GM_2$-binding activity and the abscissa the concentration of the antibody.

Fig. 58 graphically shows the reactivities of mouse-human chimeric anti-$GM_2$ antibody KM966 and human CDR-grafted anti-$GM_2$ antibodies KM8969 and KM8970 against various gangliosides. The ordinate indicates the ganglioside species and the abscissa the binding activity. $AcGM_2$ stands for N-acetyl-$GM_2$, $GcGM_2$ for N-glycolyl-$GM_2$, $AcGM_3$ for N-acetyl-$GM_3$ and $GcGM_3$ for N-glycolyl-$GM_3$.

Fig. 59 graphically shows the reactivities of mouse-human chimeric anti-$GM_2$ antibody KM966 and human CDR-grafted anti-$GM_2$ antibodies KM8969 and KM8970 against the human lung small cell carcinoma cell line SBC-3. The ordinate indicates the number of cells and the abscissa the fluorescence intensity. From the lowermost graph, the reactivities of control, KM966, KM8970 and KM8969 are shown in that order.

Fig. 60 graphically shows the ADCC activities of mouse-human chimeric anti-$GM_2$ antibody KM966 and human CDR-grafted anti-$GM_2$ antibodies KM8966, KM8969 and KM8970 against the human lung small cell carcinoma cell line SBC-3. The ordinate indicates the cytotoxicity and the abscissa the concentration of the antibody.

Fig. 61 graphically shows the CDC activities of mouse-human chimeric anti-$GM_2$ antibody KM966 and human CDR-grafted anti-$GM_2$ antibodies KM8966, KM8969 and KM8970 against the human lung small cell carcinoma cell line SBC-3 obtained when the reaction was carried out for 1 hour and 4 hours after the addition of the human complement. The ordinate indicates the cytotoxicity and the abscissa the concentration of the antibody.

## DETAILED DESCRIPTION OF THE INVENTION

In the human CDR-grafted antibody, only the CDRs of the H chain and L chain V regions comprise amino acid sequences of an antibody derived from a nonhuman animal, and the FRs of the H and L chain V regions and the C region comprise amino acid sequences of a human antibody. Examples of the nonhuman animal include mouse, rat, hamster, rabbit, as long as a hybridoma can be prepared therefrom.

With regard to the FR of the V regions of the H chain and the L chain, any amino acid sequence of known human antibodies can be used, such as an amino acid sequence selected from human antibody amino acid sequences, HMHCS, registered at the Protein Data Bank. Preferably, an amino acid sequence of the FR of HMHCS, which has a high homology with the FR of a monoclonal antibody of a nonhuman animal, may be used.

As described in the foregoing, the antibody activity is reduced when only the CDRs of the H chain V region and L chain V region of an antibody of a nonhuman animal replace the CDRs of the H chain V region and L chain V region of a human antibody. In consequence, the present invention relates to a human CDR-grafted antibody wherein at least one amino acid of the afore-mentioned positions, in the FR of H chain and L chain V regions of the human CDR-grafted antibody is replaced by another amino acid, so that it can show certain levels of antigen-binding activity, binding specificity and antibody dependent cell mediated cytotoxicity (ADCC), as well as complement dependent cytotoxicity (CDC), which are comparable to those of a human chimeric antibody having the V region of a monoclonal antibody derived from a nonhuman animal which specifically reacts with ganglioside $GM_2$, and to a method for producing the same.

The replacement of at least one amino acid in the FR of H chain and L chain V regions of the human CDR-grafted antibody of the present invention means that the amino acid residues desired to be replaced in the FR of H chain and L chain V regions of the human CDR-grafted antibody having a human antibody amino acid sequence are replaced by an other amino acid residues at corresponding positions in the FR of H chain and L chain V regions of a monoclonal antibody derived from a nonhuman animal which specifically reacts with ganglioside $GM_2$. Accordingly, at least one amino acid of positions 38, 40, 67, 72, 84 and 98 in the FR of the H chain V region and positions 4, 11, 15, 35, 42, 46, 59, 69, 70, 71, 72, 76, 77 and 103 in the FR of the L chain V region is replaced by another amino acid.

Mouse anti-$GM_2$ monoclonal antibody KM796 (FERM BP-3340, JP-A-4-311385) can be cited as an example of a monoclonal antibody derived from a nonhuman animal which specifically reacts with ganglioside $GM_2$. A chimeric anti-$GM_2$ antibody KM966 (FERM BP-3931, JP-A- 6-205694) can be cited as an example of the human chimeric antibody having the V region of a monoclonal antibody which is derived from a nonhuman animal which specifically reacts with ganglioside $GM_2$.

Examples of an antibody having certain levels of antigen-binding activity, binding specificity and antibody dependent cell mediated cytotoxicity (ADCC), which are comparable to those of a human chimeric antibody having a V region of a monoclonal antibody derived from a nonhuman animal which specifically reacts with ganglioside $GM_2$ include KM8966 produced by a transformant cell line KM8966 (FERM BP-5105), KM8967 produced by a transformant cell line KM8967 (FERM BP-5106) and KM8970 produced by a transformant cell line KM8970 (FERM BP-5528).

KM8969 produced by a transformant cell line KM8969 (FERM BP-5527) can be cited as an example of an antibody having certain levels of antigen-binding activity, binding specificity, antibody dependent cell mediated cytotoxicity (ADCC) and complement dependent cytotoxicity (CDC), which are comparable to those of a human chimeric antibody having a V region of a monoclonal antibody derived from a nonhuman animal which specifically reacts with ganglioside $GM_2$.

A method for producing the human CDR-grafted anti-$GM_2$ antibody is discussed below.

1. Construction of humanized antibody expression vector

The humanized antibody expression vector is an expression vector for use in animal cells, in which cDNA molecules encoding the C regions of H chain and L chain of a human antibody are integrated, and can be constructed by inserting the cDNA molecules encoding the C regions of H chain and L chain of a human antibody into respective expression vectors for animal cell use or by inserting the cDNA molecules which encode the C regions of H chain and L chain of a human antibody into a single expression vector for animal cell use (such a vector is called a tandem cassette vector). The C regions of human antibody can be any of C regions of human antibody H chain and L chain, and examples thereof include γ1 type C region (referred to as "Cγ1" hereinafter) and γ4 type C region (referred to as "Cγ4" hereinafter) of the human antibody H chain and κ type C region (referred to as "Cκ" hereinafter) of the human antibody L chain. Any expression vector for animal cell use can be used, as long as the cDNA encoding the human antibody C region can be integrated and expressed. Examples thereof include pAGE107 (Miyaji, H. *et al., Cytotechnology*, 3, 133 (1990)), pAGE103 (Mizukami, T. *et al.*, *J. Biochem.,* 101, 1307 (1987)), pHSG274 (Brady, G. *et al.*, Gene, 27, 223 (1984)), pKCR (O'Hare, K. *et al, Proc. Natl. Acad. Sci. U.S.A.,* 78, 1527 (1981)), and pSG1βd2-4 (Miyaji, H. *et al., Cytotechnology*, 4, 173 (1990)). Examples of the promoter and enhancer to be used in the expression vector for animal cell use include early promoter and enhancer of SV40 (Mizukami, T. *et al., J. Biochem.*, 101, 1397 (1987)), LTR promoter and enhancer of Moloney mouse leukemia virus (Kuwana, Y. *et al.*, *Biochem. Biophys. Res. Comm.*, 149, 960 (1987)) and promoter (Mason, J.O. *et al., Cell*, 41, 479 (1985)) and enhancer (Gillies, S.D. *et al.*, *Cell,* 33, 717 (1983)) of immunoglobulin H chain. The thus constructed humanized antibody expression vector can be used for expressing the human chimeric antibody and human CDR-grafted antibody in animal cells.

2. Preparation of cDNA encoding the V region of antibody of nonhuman animal

The cDNA encoding the H chain V region and L chain V region of the antibody of nonhuman animal to $GM_2$ is obtained in the following manner.

cDNA molecules are synthesized by extracting mRNA from cells of a hybridoma which produces the anti-$GM_2$ monoclonal antibody. A library is prepared from the thus synthesized cDNA using a phage or a plasmid. Using cDNA corresponding to the C region moiety or cDNA corresponding to the V region moiety of each chain of a mouse antibody as a probe, a recombinant phage or recombinant plasmid having a cDNA which encodes the V region of H chain or a recombinant phage or recombinant plasmid having a cDNA encoding the V region of L chain is isolated from the library, and complete nucleotide sequences of the intended H chain V region and L chain V region of the antibody on the recombinant phage or recombinant plasmid are determined. Complete amino acid sequences of the H chain V region and L chain V region are deduced from the thus determined nucleotide sequences.

KM796 (FERM BP-3340, JP-A-4-311385) can be cited as an example of the hybridoma cells which produce the anti-$GM_2$ monoclonal antibody.

The guanidine thiocyanate-cesium trifluoroacetate method [*Methods in Enzymol*., 154, 3 (1987)] can be exemplified as a method for preparing total RNA from hybridoma cells KM796, and the oligo (dT) immobilized cellulose column method [*Molecular Cloning*; A Laboratory Manual (2nd ed.)] can be- exemplified as a method for preparing poly $(A)^+$ RNA from the total RNA. As a kit for use in the preparation of mRNA from the hybridoma KM796 cells, Fast Track mRNA Isolation Kit; manufactured by Invitrogen), Quick Prep mRNA Purification Kit; manufactured by Pharmacia) can be exemplified.

With regard to the method for synthesizing cDNA and preparing cDNA library, the methods described in Molecular Cloning; A Laboratory Manual (2nd ed.) and Current Protocols in Molecular Biology, supplements 1 - 34, or a method which uses a commercially available kit such as Super Script™ Plasmid System for cDNA Synthesis and Plasmid Cloning (manufactured by Life Technologies) or Zap-cDNA Synthesis Kit (manufactured by Stratagene) can be exemplified. In preparing a cDNA library, any vector can be used as the vector into which the cDNA synthesized using the mRNA extracted from the hybridoma cells KM796 is to be integrated, as long as the cDNA can be integrated therein. Examples of such vectors include ZAP Express [*Strategies,* 5, 58 (1992)], pBluescript II SK(+) [*Nucleic Acids Research,* 17, 9494 (1989)], λzap II (manufactured by Stratagene), λgt10, λgt11 [*DNA Cloning,* A Practical Approach, Vol.1, 49 (1985)], Lambda BlueMid (manufactured by Clontech), λExCell, pT7T3 18U (manufactured by Pharmacia), pcD2 [*Mol. Cell. Biol.*, 3, 280 (1983)] and pUC18 [*Gene,* 33, 103 (1985).

As *Escherichia coli* into which a cDNA library constructed by the vector is to be introduced, any strain can be used, as long as the cDNA library can be introduced, expressed and maintained. Examples of such strains include XL1-Blue NRF' [*Strategies*, 5, 81 (1992)], , C600 [*Genetics*, 39, 440 (1954)], Y1088, Y1090 [*Science,* 222, 778 (1983)], NM522 [*J. Mol.* Biol., 166, 1 (1983)], K802 [*J. Mol. Biol.,* 16, 118 (1966))] and JM105 [*Gene*, 38, 275 (1985)]. Selection of cDNA clones encoding the V regions of H chain and L chain of the antibody of nonhuman animal from the cDNA library can be carried out by a colony hybridization or plaque hybridization method in which a probe labeled with an isotope or a fluorescence is used [*Molecular Cloning*; A Laboratory Manual (2nd ed.)]. Also, a DNA fragment encoding the V regions of H chain and L chain can be prepared by preparing primers and carrying out the polymerase chain reaction (referred to as "PCR" hereinafter) method [*Molecular Cloning*; A Laboratory Manual (2nd ed.), Current Protocols in Molecular Biology, supplements 1 - 34] using cDNA or cDNA library synthesized from $poly(A)^+$ RNA or mRNA as the template.

Nucleotide sequence of the DNA can be determined by digesting the cDNA clone selected by the aforementioned method with appropriate restriction enzymes, cloning the digests into a plasmid such as pBluescript SK(-) (manufactured by Stratagene) and then analyzing the resulting clones by a generally used nucleotide sequence analyzing method such as the dideoxy-method of Sanger *et al*. [*Proc. Natl. Acad. Sci., U.S.A.,* 74, 5463 (1977)]. Analysis of the nucleotide sequence can be carried out using an automatic nucleotide sequence analyzer such as 373A DNA Sequencer (manufactured by Applied Biosystems).

3. Identification of CDR of the antibody of nonhuman animal

Each V region of H chain and L chain of the antibody forms an antigen binding site. Each of the V regions of H chain and L chain comprises four FRs whose sequences are relatively stable and three CDRs which connect them and are rich in sequence changes (Kabat, E.A. *et al*., "*Sequences of Proteins* of *Immunological Interest",* US Dept. Health and Human Services, 1991). Each CDR can be found by comparing it with the V region amino acid sequences of known antibodies (Kabat, E.A. *et al., Sequences of Proteins of Immunological Interest*, US Dept. Health and Human Services, 1991).

4. Construction of CDR of the antibody of nonhuman animal

The DNA sequences encoding the H chain V region and L chain V region of the human CDR-grafted anti-$GM_2$ antibody are obtained in the following manner.

First, an amino acid sequence of the V region of each of the H chain and L chain of the human antibody is selected for grafting the CDR of the V region of the anti-$GM_2$ antibody of nonhuman animal. As the amino acid sequence of the human antibody V region, any of the known V region amino acid sequences derived from human antibodies can be used. For example, an amino acid sequence selected from human antibody V region amino acid sequences, HMHCS, registered at the Protein Data Bank may be used. However, in order to create a human CDR-grafted antibody having activities of interest such as binding activity and binding specificity for $GM_2$ or anti-tumor effect on $GM_2$-positive cells, it is desirable that the sequence has a high homology with the amino acid sequence of the V region of monoclonal antibody derived from nonhuman animal. Next, the DNA sequence encoding the FR in the selected V region amino acid sequence of human antibody is connected with the DNA sequence which encodes the amino acid sequence of the CDR, that becomes the source of the creation, of the V region of monoclonal antibody originated from nonhuman animal, thereby designing a DNA sequence which encodes the amino acid sequence of the V region of each of the H chain and L chain. A total of 6 synthetic DNA fragments are designed for each chain in such a manner that they can cover the thus designed DNA sequence, and PCR is carried out using them. Alternatively, 6 or 7 of each of anti-sense and sense DNA sequences, each comprising 35 to 84 bases, are synthesized in such a manner that they can cover the thus designed DNA sequence, and they are annealed to form double-stranded DNA fragments which are then subjected to the linking reaction. Thereafter, the amplification reaction product or the linking reaction product is subcloned into an appropriate vector and then its nucleotide sequence is determined, thereby obtaining a plasmid which contains the DNA sequence that encodes the amino acid sequence of the V region of each chain of the human CDR-grafted antibody of interest.

5. Modification of amino acid sequence of the V region of human CDR-grafted antibody

Modification of amino acid sequence of the V region of human CDR-grafted antibody is carried out by a mutation introducing method using PCR. Illustratively, a sense mutation primer and an anti-sense mutation primer, comprising 20 to 40 bases and containing a DNA sequence which encodes amino acid residues after the modification, are synthesized and PCR is carried out using, as the template, a plasmid containing a DNA sequence which encodes the amino acid sequence of the V region to be modified. The amplified fragments are subcloned into an appropriate vector and then their nucleotide sequences are determined to obtain a plasmid which contains a DNA sequence in which the mutation of interest is introduced.

6. Construction of human CDR-grafted antibody expression vector

The human CDR-grafted antibody expression vector can be constructed by inserting the DNA sequences obtained in the above paragraphs 4 and 5, encoding V regions of H chain and L chain of the human CDR-grafted antibody, into upstream of the cDNA, corresponding to the C regions of H chain and L chain of human antibody, of the humanized antibody expression vector prepared in the above paragraph 1. For example, they are inserted into upstream of the cDNA of desired human antibody C regions so that they are properly expressed, by introducing appropriate restriction enzyme recognition sequences into the 5'- and 3'-termini of a synthetic DNA when PCR is carried out in order to construct a DNA sequence which encodes amino acid sequences of the V regions of H chain and L chain of the human CDR-grafted antibody.

7. Expression of the human CDR-grafted antibody and its activity evaluation

A transformant cell line capable of producing the human CDR-grafted antibody can be obtained by introducing the human CDR-grafted antibody expression vector prepared in the above paragraph 6.

Electroporation (JP-A-2-257891; Miyaji, H. *et al., Cytotechnology*, 3, 133 (1990)) can be used as the introduction method of the expression vector into host cells.

With regard to the host cells into which the human CDR-grafted antibody expression vector is introduced, any type of host cells can be used with the proviso that the human CDR-grafted antibody can be expressed therein. Examples of such cells include mouse SP2/0-Ag14 cells (ATCC CRL1581, referred to as "SP2/0 cells" hereinafter), mouse P3X63-Ag8.653 cells (ATCC CRL1580), dihydrofolate reductase gene (referred to as "DHFR gene" hereinafter)-deficient CHO cells (Urlaub, G. *et al.*, *Proc. Natl. Acad. Sci. U.S.A.,* 77, 4216 (1980)), rat YB2/3HL.P2.G11.16Ag.20 cells (ATCC CRL1662, referred to as "YB2/0 cells" hereinafter).

After introduction of the vector, a transformant cell line capable of producing the human CDR-grafted antibody is selected in accordance with the method disclosed in JP-A-2-257891, using the RPMI 1640 medium containing geneticin (manufactured by Gibco, referred to as "G418" hereinafter) and fetal calf serum (referred to as "FCS" hereinafter). By culturing the thus obtained transformant cell line in a medium, the human CDR-grafted antibody can be produced and accumulated in the culture supernatant. Activity of the human CDR-grafted antibody in the culture supernatant is measured, for example, by the enzyme-linked immunosorbent assay (referred to as "ELISA method" hereinafter; Barlow, E. *et al.*, Antibodies, *A laboratory Manual,* Cold Spring Harbor Laboratory, Chapter 14 (1988)). In addition, production of the human CDR-grafted antibody by the transformant cell line can be improved in accordance with the method disclosed in JP-A-2-257891 making use of a DHFR gene amplifying system and the like.

The human CDR-grafted antibody can be purified from the aforementioned culture supernatant using a protein A column (Harlow, E. *et al.*, *Antibodies, A Laboratory Manual,* Cold Spring Harbor Laboratory, Chapter 8 (1988)). Alternatively, other purification methods usually used for proteins can be employed. For example, it can be purified by carrying out gel filtration, ion exchange chromatography, ultrafiltration in an appropriate combination. Molecular weight of the H chain, L chain or entire antibody molecule of the thus purified human CDR-grafted antibody is measured for example by polyacrylamide gel electrophoresis (referred to as "SDS-PAGE" hereinafter; Laemmli, U.K. *et al.*, *Nature,* 227, 680 (1970) or western blot technique (Harlow, E. *et al.*, *Antibodies, A Laboratory Manual*, Cold Spring Harbor Laboratory, Chapter 12 (1988).

Reactivity of the purified human CDR-grafted antibody with antigens and its binding activity to cultured cancer cell lines are measured by ELISA method, fluorescent antibody technique. Its complement dependent cytotoxicity (referred to as "CDC" hereinafter) activity and antibody dependent cell mediated cytotoxicity (referred to as "ADCC" hereinafter) activity upon cultured cancer cell lines are measured by the method of Shitara, K. *et al*. (*Cancer Immunol. Immunother*., 36, 373 (1993)).

Since the human CDR-grafted antibody of the present invention binds to cultured cancer cell lines of human origin in a specific fashion and shows cytotoxic activities such as CDC activity and ADCC activity, it is useful in the diagnosis and treatment of human cancers. In addition, since most portions of said antibody are originated from the amino acid sequence of a human antibody, when compared with monoclonal antibodies of animal origins excluding human, it is expected that it will exert strong anti-tumor effect without showing immunogenicity and that the effect will be maintained for a prolonged period of time.

The human CDR-grafted antibody of the present invention can be used as an anti-tumor composition, alone or together with at least one pharmaceutically acceptable auxiliary (carrier). For example, the human CDR-grafted antibody is made into an appropriate pharmaceutical composition by dissolving it in physiological saline or an aqueous solution of glucose, lactose, mannitol. Alternatively, the human CDR-grafted antibody is freeze-dried in the usual way and then mixed with sodium chloride to prepare powder injections. As occasion demands, the pharmaceutical composition may contain pharmaceutically acceptable salts and additives commonly known in the field of pharmaceutical preparations.

Though the dosage of the pharmaceutical preparation varies depending on the age, symptoms of each patient, the human CDR-grafted antibody is administered to animals including human at a dose of from 0.2 to 20 mg/kg/day. The administration is carried out once a day (single administration or every day administration) or 1 to 3 times a week or once in 2 to 3 weeks, by intravenous injection.

The present invention will be illustrated by the following Examples; however, the present invention is not limited thereto.

EXAMPLE 1

Construction of tandem cassette humanized antibody expression vector, pKANTEX93:

A tandem cassette humanized antibody expression vector, pKANTEX93, for the expression of a human CDR-grafted antibody in animal cells was constructed based on the plasmid pSE1UK1SEd1-3 described in JP-A-2-257891 by inserting a cDNA fragment coding for a human CDR-grafted antibody H chain V region and a cDNA fragment coding for a human CDR-grafted antibody L chain V region into said plasmid upstream of the human antibody Cγ1 cDNA and human antibody Cκ cDNA, respectively, in the following manner. The humanized antibody expression vector thus constructed can be also used for expressing a mouse-human chimeric antibody.

1. Modification of *Apa*I and *Eco*RI restriction enzyme sites occurring in rabbit β-globin gene splicing and poly A signals

For making it possible to construct a human CDR-grafted antibody expression vector by inserting human CDR-grafted antibody V regions cassette-wise in the form of NotI-*Apa*I (H chain) and *Eco*RI-*Spl*I (L chain) restriction fragments into a vector for humanized antibody expression, the *Apa*I and *Eco*RI restriction sites occurring in the rabbit β-globin gene splicing and poly A signals of the plasmid pSE1UK1SEd1-3 were modified in the following manner.

Three μg of the plasmid pBluescript® SK(-) (Stratagene) was added to 10 μl of 10 mM Tris-hydrochloride buffer (pH 7.5) containing 10 mM magnesium chloride and 1 mM DTT, 10 units of the restriction enzyme *Apa*I (Takara Shuzo) was further added, and the digestion reaction was allowed to proceed at 37°C for 1 hour. The reaction mixture was subjected to ethanol precipitation, and the 3' cohesive ends resulting from *Apa*I digestion were rendered blunt using DNA Blunting Kit (Takara Shuzo), followed by ligation using DNA Ligation Kit (Takara Shuzo). The thus-obtained recombinant plasmid DNA solution was used to transform *Escherichia coli* HB101. Thus was obtained a plasmid, pBSA, shown in Fig. 1.

Furthermore, 3 μg of the plasmid pBSA thus obtained was added to 10 μl of 50 mM Tris-hydrochloride buffer (pH 7.5) containing 10 mM magnesium chloride, 100 mM sodium chloride and 1 mM DTT, 10 units of the restriction enzyme *Eco*RI (Takara Shuzo) was further added, and the reaction was allowed to proceed at 37°C for 1 hour. The reaction mixture was subjected to ethanol precipitation, and the 5' cohesive ends resulting from *Eco*RI digestion were rendered blunt using DNA Blunting Kit (Takara Shuzo), followed by ligation using DNA Ligation Kit (Takara Shuzo). The thus-obtained recombinant plasmid DNA solution was used to transform *Escherichia coli* HB101. Thus was obtained the plasmid pBSAE shown in Fig. 2.

Then, 3 μg of the thus-obtained plasmid pBSAE was added to 10 μl of 10 mM Tris-hydrochloride buffer (pH 7.5) containing 10 mM magnesium chloride, 50 mM sodium chloride and 1 mM DTT, 10 units of the restriction enzyme HindIII (Takara Shuzo) was further added, and the reaction was allowed to proceed at 37°C for 1 hour. The reaction mixture was subjected to ethanol precipitation, the precipitate was dissolved in 20 μl of 10 mM Tris-hydrochloride buffer (pH 7.5) containing 10 mM magnesium chloride and 1 mM DTT, and the solution was divided into two 10-μl portions. To one portion, 10 units of the restriction enzyme *Sac*II (Toyobo) was further added and, to the other, 10 units of the restriction enzyme *Kpn*I (Takara Shuzo) was further added, and the reaction was allowed to proceed at 37°C for 1 hour. Both the reaction mixtures were fractionated by agarose gel electrophoresis, whereby about 0.3 μg each of a *Hin*dIII-*Sac*II fragment (about 2.96 kb) and a *Kpn*I-*Hin*dIII fragment (about 2.96 kb) were recovered.

Then, 3 μg of the plasmid pSE1UK1SEd1-3 was added to 10 μl of 10 mM Tris-hydrochloride buffer (pH 7.5) containing 10 mM magnesium chloride and 1 mM DTT, 10 units of the restriction enzyme *Sac*II (Toyobo) and 10 units of the restriction enzyme *Kpn*I (Takara Shuzo) were further added, and the reaction was allowed to proceed at 37°C for 1 hour. The reaction mixture was subjected to ethanol precipitation, the precipitate was dissolved in 10 μl of 10 mM Tris-hydrochloride buffer (pH 7.5) containing 10 mM magnesium chloride, 50 mM sodium chloride and 1 mM DTT, 10 units of the restriction enzyme *Hin*dIII (Takara Shuzo) was further added, and the reaction was allowed to proceed at 37°C for 1 hour. The reaction mixture was fractionated by agarose gel electrophoresis, whereby about 0.2 μg each of a *Hin*dIII-*Sac*II fragment (about 2.42 kb) and a *Kpn*I-*Hin*dIII fragment (about 1.98 kb) were recovered.

Then, 0.1 μg of the thus-obtained *Hin*dIII-*Sac*II fragment of pSE1UK1SEd1-3 and 0.1 μg of the above *Hin*dIII-*Sac*II fragment of pBSAE were dissolved in a total of 20 μl of sterilized water and ligated to each other using Ready-To-Go T4 DNA Ligase (Pharmacia Biotech). The thus-obtained recombinant plasmid DNA solution was used to transform *Escherichia coli* HB101 and, as a result, a plasmid, pBSH-S, shown in Fig. 3 was obtained. Furthermore, 0.1 μg of the above-mentioned *Kpn*I-*Hin*dIII fragment of pSE1UK1SEd1-3 and 0.1 μg of the above-mentioned *Kpn*I-*Hin*dIII fragment of pBSAE were dissolved in a total of 20 μl of sterilized water and ligated to each other using Ready-To-Go T4 DNA Ligase (Pharmacia Biotech). The thus-obtained recombinant plasmid DNA solution was used to transform *Escherichia coli* HB101, and the plasmid pBSK-H shown in Fig. 4 was obtained.

Then, 3 μg each of the thus-obtained plasmids pBSH-S and pBSK-H were respectively added to 10-μl portions of 10 mM Tris-hydrochloride buffer (pH 7.5) containing 10 mM magnesium chloride and 1 mM DTT, 10 units of the restriction enzyme *Apa*I (Takara Shuzo) was further added to each mixture, and the reaction was allowed to proceed at 37°C for 1 hour. Both the reaction mixtures were subjected to ethanol precipitation. With each precipitate, the 3' cohesive ends resulting from *Apa*I digestion were rendered blunt using DNA Blunting Kit (Takara Shuzo), followed by ligation using DNA Ligation Kit (Takara Shuzo). The thus-obtained recombinant DNA solution were used to transform *Escherichia coli* HB101, and the plasmids pBSH-SA and pBSK-HA shown in Fig. 5 were obtained.

Then, 5 μg each of the thus-obtained plasmids pBSH-SA and pBSK-HA were respectively added to 10-μl portions of 50 mM Tris-hydrochloride buffer (pH 7.5) containing 10 mM magnesium chloride, 100 mM sodium chloride and 1 mM DTT, 1 unit of the restriction enzyme *Eco*RI (Takara Shuzo) was further added to each mixture, and the reaction was allowed to proceed at 37°C for 10 minutes for partial digestion. Both the reaction mixtures were subjected to ethanol precipitation. With each precipitate, the 5' cohesive ends resulting from *Eco*RI digestion were rendered blunt using DNA Blunting Kit (Takara Shuzo), followed by fractionation by agarose gel electrophoresis, whereby about 0.5 μg each of a fragment about 5.38 kb in length and a fragment about 4.94 kb in length were recovered. The thus-recovered fragments (0.1 μg each) were each dissolved in a total of 20 μl of sterilized water and subjected to ligation treatment using Ready-To-Go T4 DNA Ligase (Pharmacia Biotech). The thus-obtained recombinant DNA solutions were respectively used to transform *Escherichia coli* HB101, and the plasmids pBSH-SAE and pBSK-HAE shown in Fig. 6 were obtained.

Then, 3 μg each of the thus-obtained plasmids pBSH-SAE and pBSK-HAE were respectively added to 10-μl portions of 50 mM Tris-hydrochloride buffer (pH 7.5) containing 10 mM magnesium chloride, 100 mM sodium chloride and 1 mM DTT, 10 units of the restriction enzyme *Eco*RI (Takara Shuzo) was further added to each mixture, and the reaction was allowed to proceed at 37°C for 1 hour. Both the reaction mixtures were subjected to ethanol precipitation. With each precipitate, the 5' cohesive ends resulting from *Eco*RI digestion were rendered blunt using DNA Blunting Kit (Takara Shuzo), followed by ligation using DNA Ligation Kit (Takara Shuzo). The thus-obtained recombinant plasmid DNA solutions were each used to transform *Escherichia coli* HB101, and two plasmids, pBSH-SAEE and pBSK-HAEE, shown in Fig. 7 were obtained. Ten μg each of the thus-obtained plasmids were subjected to sequencing reaction according to the instructions attached to AutoRead Sequencing Kit (Pharmacia Biotech), followed by base sequence determination by electrophoresis on A.L.F. DNA Sequencer (Pharmacia Biotech), whereby it was confirmed that both the *Apa*I and *Eco*RI sites had disappeared as a result of the above modification.

2. *Sal*I restriction site introduction downstream from rabbit β-globin gene splicing and poly A signals and SV40 early gene poly A signal

For making it possible to exchange the antibody H chain and L chain expression promoters of the humanized antibody expression vector each for an arbitrary promoter, a *Sal*I restriction site was introduced into the plasmid pSE1UK1SEd1-3 downstream from the rabbit β-globin gene splicing and poly A signals and from the SV40 early gene poly A signal in the following manner.

Three μg of the plasmid pBSK-HAEE obtained in Paragraph 1 of Example 1 was added to 10 μl of 10 mM Tris-hydrochloride buffer (pH 7.5) containing 10 mM magnesium chloride and 1 mM DTT, 10 units of the restriction enzyme *Nae*I (Takara Shuzo) was further added, and the reaction was allowed to proceed at 37°C for 1 hour. The reaction mixture was subjected to ethanol precipitation, the precipitate was dissolved in 20 μl of 50 mM Tris-hydrochloride buffer (pH 9.0) containing 1 mM magnesium chloride, 1 unit of alkaline phosphatase (*E. coli C75,* Takara Shuzo) was further added, and the reaction was allowed to proceed at 37°C for 1 hour for dephosphorylation at the 5' termini. The reaction mixture was further subjected to phenol-chloroform extraction and then to ethanol precipitation, and the precipitate was dissolved in 20 μl of 10 mM Tris-hydrochloride buffer (pH 8.0) containing 1 mM disodium ethylenediaminetetraacetate (hereinafter briefly referred to as "TE buffer"). One μl of said reaction solution and 0.1 μg of a phosphorylated *Sal*I linker (Takara Shuzo) were added to sterilized water to make a total volume of 20 μl, followed by ligation treatment using Ready-To-Go T4 DNA Ligase (Pharmacia Biotech). The thus-obtained recombinant plasmid DNA solution was used to transform Escherichia coli HB101, and a plasmid, pBSK-HAEESal, shown in Fig. 8 was obtained. Ten μg of the plasmid thus obtained was subjected to sequencing reaction according to the instructions attached to AutoRead Sequencing Kit (Pharmacia Biotech), followed by electrophoresis on A.L.F. DNA Sequencer (Pharmacia Biotech) for base sequence determination, whereby it was confirmed that one *Sal*I restriction site had been introduced downstream from the rabbit β-globin gene splicing and poly A signals and from the SV40 early gene poly A signal.

3. Modification of *Apa*I restriction site occurring in poly A signal of Herpes simplex virus thymidine kinase (hereinafter referred to as "HSVtk") gene

The *Apa*I restriction site occurring in the HSVtk gene poly A signal downstream from the Tn5 kanamycin phosphotransferase gene of the plasmid pSE1UK1SEd1-3 was modified in the following manner.

Three μg of the plasmid pBSA obtained in Paragraph 1 of Example 1 was added to 10 μl of 10 mM Tris-hydrochloride buffer (pH 7.5) containing 10 mM magnesium chloride and 1 mM DTT, 10 units of the restriction enzyme *Sac*II (Toyobo) was further added, and the reaction was allowed to proceed at 37°C for 1 hour. The reaction mixture was subjected to ethanol precipitation, the precipitate was added to 10 μl of 50 mM Tris-hydrochloride buffer (pH 7.5) containing 100 mM sodium chloride, 10 mM magnesium chloride and 1 mM DTT, 10 units of the restriction enzyme *Xho*I (Takara Shuzo) was further added, and the reaction was allowed to proceed at 37°C for 1 hour. The reaction mixture was fractionated by agarose gel electrophoresis, whereby about 1 μg of a SacII-*Xho*I fragment (about 2.96 kb) was recovered.

Then, 5 μg of the plasmid pSE1UK1SEd1-3 was added to 10 μl of 10 mM Tris-hydrochloride buffer (pH 7.5) containing 10 mM magnesium chloride and 1 mM DTT, 10 units of the restriction enzyme *Sac*II (Toyobo) was further added, and the reaction was allowed to proceed at 37°C for 1 hour. The reaction mixture was subjected to ethanol precipitation, the precipitate was added to 10 μl of 50 mM Tris-hydrochloride buffer (pH 7.5) containing 100 mM sodium chloride, 10 mM magnesium chloride and 1 mM DTT, 10 units of the restriction enzyme *Xho*I (Takara Shuzo) was further added, and the reaction was fractionated by agarose gel electrophoresis, whereby about 1 μg of a *Sac*II-*Xho*I fragment (about 4.25 kb) was recovered.

Then, 0.1 μg of the above *Sac*II-*Xbo*I fragment of pBSA and the above *Sac*II-*Xho*I fragment of pSE1UK1SEd1-3 were added to a total of 20 μl of sterilized water, followed by ligation using Ready-To-Go T4 DNA Ligase (Pharmacia Biotech). The thus-obtained recombinant plasmid DNA solution was used to transform *Escherichia coli* HB101, and the plasmid pBSX-S shown in Fig. 9 was obtained.

Then, 3 μg of the thus-obtained plasmid pBSX-S was added to 10 μl of 10 mM Tris-hydrochloride buffer (pH 7.5) containing 10 mM magnesium chloride and 1 mM DTT, 10 units of the restriction enzyme *Apa*I (Takara Shuzo) was further added, and the reaction was allowed to proceed at 37°C for 1 hour. The reaction mixture was subjected to ethanol precipitation, the 3' cohesive ends resulting from *Apa*I digestion were rendered blunt using DNA Blunting Kit (Takara Shuzo) and then ligation was carried out using DNA Ligation Kit (Takara Shuzo). The thus-obtained recombinant plasmid DNA solution was used to transform *Escherichia coli* HB101, and a plasmid, pBSX-SA, shown in Fig. 10 was obtained. Ten μg of the thus-obtained plasmid was subjected to sequencing reaction according to the instructions attached to AutoRead Sequencing Kit (Pharmacia Biotech), followed by electrophoresis on A.L.F. DNA Sequencer (Pharmacia Biotech) for base sequence determination, whereby it was confirmed that the *Apa*I restriction site in the HSVtk gene poly A signal had disappeared.

4. Construction of humanized antibody L chain expression unit

A plasmid, pMohCκ, containing a human antibody Cκ cDNA downstream from the promoter/enhancer of the Moloney mouse leukemia virus long terminal repeat and having a humanized antibody L chain expression unit allowing cassette-wise insertion thereinto of a humanized antibody L chain V region was constructed in the following manner.
Three μg of the plasmid pBluescript® SK(-) (Stratagene) was added to 10 μl of 10 mM Tris-hydrochloride buffer (pH 7.5) containing 10 mM magnesium chloride and 1 mM DTT, 10 units of the restriction enzyme *Sac*I (Takara Shuzo) was further added, and the reaction was allowed to proceed at 37°C for 1 hour. The reaction mixture was subjected to ethanol precipitation, the precipitate was added to 10 μl of 10 mM Tris-hydrochloride buffer (pH 7.5) containing 50 mM sodium chloride, 10 mM magnesium chloride and 1 mM DTT, 10 units of the restriction enzyme *Cla*I (Takara Shuzo) was further added, and the reaction was allowed to proceed at 37°C for 1 hour. The reaction mixture was subjected to ethanol precipitation, and the cohesive ends resulting from *Sac*I and *Cla*I digestion were rendered blunt using DNA Blunting Kit (Takara Shuzo), followed by fractionation by agarose gel electrophoresis, whereby about 1 μg of a DNA fragment about 2.96 kb in length was recovered. A 0.1-μg portion of the DNA fragment recovered was added to a total of 20 μl of sterilized water and subjected to ligation reaction using Ready-To-Go T4 DNA Ligase (Pharmacia Biotech). The thus-obtained recombinant plasmid DNA solution was used to transform *Escherichia coli* HB101, and the plasmid pBSSC shown in Fig. 11 was obtained.
Then, 3 μg of the thus-obtained plasmid pBSSC was added to 10 μl of 10 mM Tris-hydrochloride buffer (pH 7.5) containing 10 mM magnesium chloride and 1 mM DTT, 10 units of the restriction enzyme *Kpn*I (Takara Shuzo) was further added, and the reaction was allowed to proceed at 37°C for 1 hour. The reaction mixture was subjected to ethanol precipitation, the precipitate was dissolved in 10 μl of 50 mM Tris-hydrochloride buffer (pH 7.5) containing 100 mM sodium chloride, 10 mM magnesium chloride and 1 mM DTT, 10 units of the restriction enzyme *Xho*I (Takara Shuzo) was further added, and the reaction was allowed to proceed at 37°C for 1 hour. The reaction mixture was fractionated by agarose gel electrophoresis, whereby about 1 μg of a *Kpn*I-*Xho*I fragment (about 2.96 kb) was recovered.
Then, 5 μg of the plasmid pAGE147 described in JP-A-6-205694 was added to 10 μl of 10 mM Tris-hydrochloride buffer (pH7.5) containing 10 mM magnesium chloride and 1 mM DTT, 10 units of the restriction enzyme *Kpn*I (Takara Shuzo) was further added, and the reaction was allowed to proceed at 37°C for 1 hour. The reaction mixture was subjected to ethanol precipitation, the precipitate was dissolved in 10 μl of 50 mM Tris-hydrochloride buffer (pH 7.5) containing 100 mM sodium chloride, 10 mM magnesium chloride and 1 mM DTT, 10 units of the restriction enzyme *Xho*I (Takara Shuzo) was further added, and the reaction was fractionated by agarose gel electrophoresis, whereby about 0.3 μg of a *Kpn*I-*Xho*I fragment (about 0.66 kb) containing the Moloney mouse leukemia virus long terminal repeat promoter/enhancer was recovered.
Then, 0.1 μg of the *Kpn*I-*Xho*I fragment of pBSSC and 0.1 μg of the *Kpn*I-*Xho*I fragment of pAGE147 each obtained as mentioned above were dissolved in a total of 20 μl of sterilized water and subjected to ligation using Ready-To-Go T4 DNA Ligase (Pharmacia Biotech). The thus-obtained recombinant plasmid DNA solution was used to transform *Escherichia coli* HB101, and the plasmid pBSMo shown in Fig. 12 was obtained.
Then, 3 μg of the above plasmid pBSMo was added to 10 μl of 10 mM Tris-hydrochloride buffer (pH 7.5) containing 10 mM magnesium chloride and 1 mM DTT, 10 units of the restriction enzyme KpnI (Takara Shuzo) was further added, and the reaction was allowed to proceed at 37°C for 1 hour. The reaction mixture was subjected to ethanol precipitation, the precipitate was dissolved in 10 μl of 10 mM Tris-hydrochloride buffer (pH 7.5) containing 50 mM sodium chloride, 10 mM magnesium chloride and 1 mM DTT, 10 units of the restriction enzyme *Hin*dIII (Takara Shuzo) was further added, and the reaction was allowed to proceed at 37°C for 1 hour. The reaction mixture was fractionated by agarose gel electrophoresis, whereby about 1 μg of a *Kpn*I-*Hin*dIII fragment (about 3.62 kb) was recovered.
Then, synthetic DNAs respectively having the base sequences shown in SEQ ID NO:12 and SEQ ID NO:13 were synthesized using an automatic DNA synthesizer (Applied Biosystems model 380A). To 15 μl of sterilized water were added 0.3 μg each of the thus-obtained synthetic DNAs, and the mixture was heated at 65°C for 5 minutes. The reaction mixture was allowed to stand at room temperature for 30 minutes and then 2 μl of 10-fold concentrated buffer [500 mM Tris-hydrochloride (pH 7.6), 100 mM magnesium chloride, 50 mM DTT] and 2 μl of 10 mM ATP were added, 10 units of T4 polynucleotide kinase was further added, and the reaction was allowed to proceed at 37°C for 30 minutes for phosphorylation of the 5' termini. To a total of 20 µl of sterilized water were added 0.1 µg of the above *Kpn*I-*Hin*dIII fragment (3.66 kb) derived from the plasmid pBSMo and 0.05 µg of the phsophorylated synthetic DNA pair, and ligation was effected using Ready-To-Go T4 DNA Ligase (Pharmacia Biotech). The thus-obtained recombinant plasmid DNA solution was used to transform *Escherichia coli* HB101, and the plasmid pBSMoS shown in Fig. 13 was obtained. Ten µg of the plasmid thus obtained was subjected to sequencing reaction according to the instructions attached to AutoRead Sequencing Kit (Pharmacia Biotech), followed by electrophoresis on A.L.F. DNA Sequencer (Pharmacia Biotech) for base sequence determination, whereby it was confirmed that the synthetic DNA pair had been introduced as desired.

Then, 3 µg of the plasmid pChiIgLA1 described in JP-A-5-304989 was dissolved in 10 µl of 50 mM Tris-hydrochloride buffer (pH 7.5) containing 100 mM sodium chloride, 10 mM magnesium chloride and 1 mM DTT, 10 units each of the restriction enzymes *Eco*RI (Takara Shuzo) and *Eco*RV (Takara Shuzo) were further added, and the reaction was allowed to proceed at 37°C for 1 hour. The reaction mixture was fractionated by agarose gel electrophoresis, whereby about 1 pg of an EcoRI-EcoRV fragment (about 9.70 kb) was recovered.

Then, synthetic DNAs respectively having the base sequences shown in SEQ ID NO:14 and SEQ ID NO:15 were synthesized using an automatic DNA synthesizer (Applied Biosystems model 380A). To 15 µl of sterilized water were added 0.3 µg each of the thus-obtained synthetic DNAs, and the mixture was heated at 65°C for 5 minutes. The reaction mixture was allowed to stand at room temperature for 30 minutes. Then, 2 µl of 10-fold concentrated buffer [500 mM Tris-hydrochloride (pH 7.6), 100 mM magnesium chloride, 50 mM DTT] and 2 µl of 10 mM ATP were added, 10 units of T4 polynucleotide kinase was further added, and the reaction was allowed to proceed at 37°C for 30 minutes for phosphorylation of the 5' termini. To a total of 20 µl of sterilized water were added 0.1 µg of the above *Eco*RI-*Eco*RV fragment (9.70 kb) derived from the plasmid pChiIgLA1 and 0.05 µg of the phsophorylated synthetic DNA, and ligation was effected using Ready-To-Go T4 DNA Ligase (Pharmacia Biotech). The thus-obtained recombinant plasmid DNA solution was used to transform *Escherichia coli* HB101, and the plasmid pChiIgLA1S shown in Fig. 14 was obtained.

Then, 3 µg of the plasmid pBSMoS obtained in the above manner was dissolved in 10 µl of 20 mM Tris-hydrochloride buffer (pH 8.5) containing 100 mM potassium chloride, 10 mM magnesium chloride and 1 mM DTT, 10 units of the restriction enzyme *Hpa*I (Takara Shuzo) was further added, and the reaction was allowed to proceed at 37°C for 1 hour. The reaction mixture was subjected to ethanol precipitation, the precipitate was dissolved in 10 µl of 50 mM Tris-hydrochloride buffer (pH 7.5) containing 100 mM sodium chloride, 10 mM magnesium chloride and 1 mM DTT, 10 units of the restriction enzyme *Eco*RI (Takara Shuzo) was further added, and the reaction was allowed to proceed at 37°C for 1 hour. The reaction mixture was fractionated by agarose gel electrophoresis, whereby about 1 µg of an *Hpa*I-*Eco*RI fragment (about 3.66 kb) was recovered.

Then, 10 µg of the plasmid pChiIgLA1S obtained as mentioned above was dissolved in 10 µl of 20 mM Tris-acetate buffer (pH 7.9) containing 50 mM potassium acetate, 10 mM magnesium acetate, 1 mM DTT and 100 µg/ml BSA, 10 units of the restriction enzyme *Nla*IV (New England BioLabs) was further added, and the reaction was allowed to proceed at 37°C for 1 hour. The reaction mixture was subjected to ethanol precipitation, the precipitate was dissolved in 10 µl of 50 mM Tris-hydrochloride buffer (pH 7.5) containing 100 mM sodium chloride, 10 mM magnesium chloride and 1 mM DTT, 10 units of the restriction enzyme *Eco*RI (Takara Shuzo) was further added, and the reaction was allowed to proceed at 37°C for 1 hour. The reaction mixture was fractionated by agarose gel electrophoresis, whereby about 0.3 µg of an *Nla*IV-*Eco*RI fragment (about 0.41 kb) was recovered.

Then, 0.1 µg of the above *Hpa*I-*Eco*RI fragment of pBSMoS and 0.1 µg of the above *Nla*IV-*Eco*RI fragment of pChiIgLA1S were added to a total of 20 µl of sterilized water, and ligation was effected using Ready-To-Go T4 DNA Ligase (Pharmacia Biotech). The thus-obtained recombinant plasmid DNA solution was used to transform *Escherichia coli* HB101, and the plasmid pMohCκ shown in Fig. 15 was obtained.

5. Construction of humanized antibody H chain expression unit

A plasmid, pMohCγ1, containing a human antibody Cγ1 cDNA downstream from the promoter/enhancer of the Moloney mouse leukemia virus long terminal repeat and having a humanized antibody H chain expression unit allowing cassette-wise insertion thereinto of a humanized antibody H chain V region was constructed in the following manner.

Three µg of the plasmid pBSMo obtained in Paragraph 4 of Example 1 was added to 10 µl of 50 mM Tris-hydrochloride buffer (pH 7.5) containing 100 mM sodium chloride, 10 mM magnesium chloride and 1 mM DTT, 10 units of the restriction enzyme *Xho*I (Takara Shuzo) was further added, and the reaction was allowed to proceed at 37°C for 1 hour. The reaction mixture was subjected to ethanol precipitation, the precipitate was dissolved in 10 µl of 30 mM sodium acetate buffer (pH 5.0) containing 100 mM sodium chloride, 1 mM zinc acetate and 10% glycerol, 10 units of Mung bean nuclease (Takara Shuzo) was further added, and the reaction was allowed to proceed at 37°C for 10 minutes. The reaction mixture was subjected to phenol-chloroform extraction and then to ethanol precipitation, the cohesive ends of the precipitate were rendered blunt using DNA Blunting Kit (Takara Shuzo) and ligation was effected using DNA Ligation Kit (Takara Shuzo). The thus-obtained recombinant plasmid DNA solution was used to transform *Escherichia coli* HB101, and the plasmid pBSMoSal shown in Fig. 16 was obtained. A 10-µg portion of the plasmid obtained was subjected to sequencing reaction according to the instructions attached to AutoRead Sequencing Kit (Pharmacia Biotech), followed by electrophoresis on A.L.F. DNA Sequencer (Pharmacia Biotech) for base sequence determination, whereby it was confirmed that the XhoI restriction site upstream of the Moloney mouse leukemia virus long terminal repeat promoter/enhancer had disappeared.

Then, 3 µg of the plasmid pBSMoSal obtained as mentioned above was added to 10 µl of 10 mM Tris-hydrochloride buffer (pH 7.5) containing 10 mM magnesium chloride and 1 mM DTT, 10 units of the restriction enzyme *Kpn*I (Takara Shuzo) was further added, and the reaction was allowed to proceed at 37°C for 1 hour. The reaction mixture was subjected to ethanol precipitation, the precipitate was dissolved in 10 µl of 10 mM Tris-hydrochloride buffer (pH 7.5) containing 50 mM sodium chloride, 10 mM magnesium chloride and 1 mM DTT, 10 units of the restriction enzyme *Hin*dIII (Takara Shuzo) was further added, and the reaction was allowed to proceed at 37°C for 1 hour. The reaction mixture was fractionated by agarose gel electrophoresis, whereby about 1 µg of a *Kpn*I-*Hin*dIII fragment (about 3.66 kb) was recovered.

Then, synthetic DNAs respectively having the base sequences shown in SEQ ID NO:16 and SEQ ID NO:17 were synthesized using an automatic DNA synthesizer (Applied Biosystems model 380A). To 15 µl of sterilized water were added 0.3 µg each of the thus-obtained synthetic DNAs, and the mixture was heated at 65°C for 5 minutes. The reaction mixture was allowed to stand at room temperature for 30 minutes. Then, 2 µl of 10-fold concentrated buffer [500 mM Tris-hydrochloride (pH 7.6), 100 mM magnesium chloride, 50 mM DTT] and 2 µl of 10 mM ATP were added, 10 units of T4 polynucleotide kinase was further added, and the reaction was allowed to proceed at 37°C for 30 minutes for phosphorylation of the 5' termini. To a total of 20 µl of sterilized water were added 0.1 µg of the above *Kpn*I-*Hin*dIII fragment (3.66 kb) derived from the plasmid pBSMoSa1 and 0.05 µg of the phosphorylated synthetic DNA, and ligation was effected using Ready-To-Go T4 DNA Ligase (Pharmacia Biotech). The thus-obtained recombinant plasmid DNA solution was used to transform *Escherichia coli* HB101, and the plasmid pBSMoSa1S shown in Fig. 17 was obtained. A 10-µg portion of the thus-obtained plasmid was subjected to sequencing reaction according to the instructions attached to AutoRead Sequencing Kit (Pharmacia Biotech), followed by electrophoresis on A.L.F. DNA Sequencer (Pharmacia Biotech), for base sequence determination whereby it was confirmed that the synthetic DNA had been introduced as desired.

Then, 10 µg of the plasmid pChiIgHB2 described in JP-A-5-304989 was dissolved in 10 µl of 50 mM Tris-hydrochloride buffer (pH 7.5) containing 100 mM sodium chloride, 10 mM magnesium chloride and 1 mM DTT, 10 units of the restriction enzyme Eco52I (Toyobo) was further added, and the reaction was allowed to proceed at 37°C for 1 hour. The reaction mixture was subjected to ethanol precipitation, the precipitate was dissolved in 10 µl of 30 mM sodium acetate buffer (pH 5.0) containing 100 mM sodium chloride, 1 mM zinc acetate and 10% glycerol, 10 units of Mung bean nuclease (Takara Shuzo) was further added, and the reaction was allowed to proceed at 37°C for 10 minutes. The reaction mixture was subjected to phenol-chloroform extraction and then to ethanol precipitation, and the cohesive ends were rendered blunt using DNA Blunting Kit (Takara Shuzo). After ethanol precipitation, the precipitate was dissolved in 10 µl of 10 mM Tris-hydrochloride buffer (pH 7.5) containing 10 mM magnesium chloride and 1 mM DTT, 10 units of the restriction enzyme *Apa*I (Takara Shuzo) was further added, and the reaction was allowed to proceed at 37°C for 1 hour. The reaction mixture was fractionated by agarose gel electrophoresis, whereby about 0.7 µg of *Apa*I-blunt end fragment (about 0.99 kb) was recovered.

Then, 3 µg of the plasmid pbluescript® SK(-) (Stratagene) was added to 10 µl of 10 mM Tris-hydrochloride buffer (pH 7.5) containing 10 mM magnesium chloride and 1 mM DTT, 10 units of the restriction enzyme *Apa*I ( 2UTakara Shuzo) was further added, and the reaction was allowed to proceed at 37°C for 1 hour. The reaction mixture was subjected to ethanol precipitation, the precipitate was dissolved in 10 µl of 33 mM Tris-acetate buffer (pH 7.9) containing 10 mM magnesium acetate, 66 mM potassium acetate, 0.5 mM DTT and 100 µg/ml BSA, 10 units of the restriction enzyme *Sma*I (Takara Shuzo) was further added, and the reaction was allowed to proceed at 37°C for 1 hour. The reaction mixture was fractionated by agarose gel electrophoresis, whereby about 1 µg of an *Apa*I-*Sma*I fragment (about 3.0 kb) was recovered.

Then, 0.1 µg of the *Apa*I-blunt end fragment of pChiIgHB2 and 0.1 µg of the *Apa*I-*Sma*I fragment of pBluescript® SK(-), each obtained as mentioned above, were added to a total of 20 µl of sterilized water and ligated to each other using Ready-To-Go T4 DNA Ligase (Pharmacia Biotech). The thus-obtained recombinant plasmid DNA solution was used to transform *Escherichia coli* HB101, and the plasmid pBShCγ1 shown in Fig. 18 was obtained.

Then, 5 µg of the above plasmid pBShCγ1 was dissolved in 10 µl of 10 mM Tris-hydrochloride buffer (pH 7.5) containing 10 mM magnesium chloride and 1 mM DTT, 10 units of the restriction enzyme *Apa*I (Takara Shuzo) was further added, and the reaction was allowed to proceed at 37°C for 1 hour. The reaction mixture was subjected to ethanol precipitation, the precipitate was dissolved in 10 µl of 10 mM Tris-hydrochloride buffer (pH 7.5) containing 50 mM sodium chloride, 10 mM magnesium chloride and 1 mM DTT, 10 units of the restriction enzyme *Spe*I (Takara Shuzo) was further added, and the reaction was allowed to proceed at 37°C for 1 hour. The reaction mixture was fractionated by agarose gel electrophoresis, whereby about 1 μg of an *Apa*I-*Spe*I fragment (about 1.0 kb) was recovered.

Then, 3 μg of the plasmid pBSMoSa1S obtained as mentioned above was dissolved in 10 μl of 10 mM Tris-hydrochloride buffer (pH 7.5) containing 10 mM magnesium chloride and 1 mM DTT, 10 units of the restriction enzyme *Apa*I (Takara Shuzo) was further added, and the reaction was allowed to proceed at 37°C for 1 hour. The reaction mixture was subjected to ethanol precipitation, the precipitate was dissolved in 10 μl of 10 mM Tris-hydrochloride buffer (pH 7.5) containing 50 mM sodium chloride, 10 mM magnesium chloride and 1 mM DTT, 10 units of the restriction enzyme *Spe*I (Takara Shuzo) was further added, and the reaction was allowed to proceed at 37°C for 1 hour. The reaction mixture was fractionated by agarose gel electrophoresis, whereby about 1 μg of an *Apa*I-*Spe*I fragment (about 3.66 kb) was recovered.

Then, 0.1 μg of the *Apa*I-*Spe*I fragment of pBShCγ1 and 0.1 μg of the *Apa*I-*Spe*I fragment of pBSMoSa1S, each obtained as mentioned above, were added to a total of 20 μl of sterilized water and ligated to each other using Ready-To-Go T4 DNA Ligase (Pharmacia Biotech). The thus-obtained recombinant plasmid DNA solution was used to transform *Escherichia coli* HB101, and the plasmid pMohCγ1 shown in Fig. 19 was obtained.

6. Construction of tandem cassette humanized antibody expression vector, pKANTEX93

A tandem cassette humanized antibody expression vector, pKANTEX93, was constructed using the various plasmids obtained in Paragraphs 1 through 5 of Example 1 in the following manner.

Three μg of the plasmid pBSH-SAEE obtained in Paragraph 1 of Example 1 was added to 10 μl of 10 mM Tris-hydrochloride buffer (pH 7.5) containing 50 mM sodium chloride, 10 mM magnesium chloride and 1 mM DTT, 10 units of the restriction enzyme *Hin*dIII (Takara Shuzo) was further added, and the reaction was allowed to proceed at 37°C for 1 hour. The reaction mixture was subjected to ethanol precipitation, the precipitate was dissolved in 10 μl of 50 mM Tris-hydrochloride buffer (pH 7.5) containing 100 mM sodium chloride, 10 mM magnesium chloride and 1 mM DTT, 10 units of the restriction enzyme *Sal*I (Takara Shuzo) was further added, and the reaction was allowed to proceed at 37°C for 1 hour. The reaction mixture was fractionated by agarose gel electrophoresis, whereby about 1 μg of a *Hin*dIII -*Sal*I fragment (about 5.42 kb) was recovered.

Then, 5 μg of the plasmid pBSK-HAEE obtained in Paragraph 1 of Example 1 was added to 10 μl of 10 mM Tris-hydrochloride buffer (ph 7.5) containing 10 mM magnesium chloride and 1 mM DTT, 10 units of the restriction enzyme *Kpn*I (Takara Shuzo) was further added, and the reaction was allowed to proceed at 37°C for 1 hour. The reaction mixture was subjected to ethanol precipitation, the precipitate was dissolved in 10 μl of 10 mM Tris-hydrochloride buffer (pH 7.5) containing 50 mM sodium chloride, 10 mM magnesium chloride and 1 mM DTT, 10 units of the restriction enzyme *Hin*dIII (Takara Shuzo) was further added, and the reaction was allowed to proceed at 37°C for 1 hour. The reaction mixture was fractionated by agarose gel electrophoresis, whereby about 0.8 μg of a *Kpn*I-*Hin*dIII fragment (about 1.98 kb) containing the rabbit β-globin gene splicing and poly A signals, the SV40 early gene poly A signal and the SV40 early gene promoter was recovered.

Then, 5 μg of the plasmid pMohCγ1 obtained in Paragraph 5 of Example 1 was added to 10 μl of 10 mM Tris-hydrochloride buffer (pH 7.5) containing 10 mM magnesium chloride and 1 mM DTT, 10 units of the restriction enzyme *Kpn*I (Takara Shuzo) was further added, and the reaction was allowed to proceed at 37°C for 1 hour. The reaction mixture was subjected to ethanol precipitation, the precipitate was dissolved in 10 μl of 50 mM Tris-hydrochloride buffer (pH 7.5) containing 100 mM sodium chloride, 10 mM magnesium chloride and 1 mM DTT, 10 units of the restriction enzyme *Sal*I (Takara Shuzo) was further added, and the reaction was allowed to proceed at 37°C for 1 hour. The reaction mixture was fractionated by agarose gel electrophoresis, whereby about 0.8 μg of a human CDR-grafted antibody H chain expression unit-containing *Kpn*I-*Sal*I fragment (about 1.66 kb) was recovered.

Then, 0.1 μg of the *Hin*dIII-*Sal*I fragment of pBSH-SAEE, 0.1 μg of the *Kpn*I-*Hin*dIII fragment of pBSK-HAEE and 0.1 μg of the *Kpn*I-*Sal*I fragment of pMohCγ1, each obtained as mentioned above, were added to a total of 20 μl of sterilized water and ligated together using Ready-To-Go T4 DNA Ligase (Pharmacia Biotech). The thus-obtained recombinant plasmid DNA solution was used to transform *Escherichia coli* HB101, and the plasmid pMoγ1SP shown in Fig. 20 was obtained.

Then, 3 μg of the above plasmid pMoγ1SP was added to 10 μl of 50 mM Tris-hydrochloride buffer (pH 7.5) containing 100 mM sodium chloride, 10 mM magnesium chloride and 1 mM DTT, 10 units of the restriction enzyme *Sal*I (Takara Shuzo) and 10 units of the restriction enzyme *Xho*I were further added, and the reaction was allowed to proceed at 37°C for 1 hour. The reaction mixture was fractionated by agarose gel electrophoresis, whereby about 1 μg of a *Sal*I-*Xho*I fragment (about 9.06 kb) was recovered.

Then, 5 μg of the plasmid pBSK-HAEESal obtained in Paragraph 2 of Example 1 was added to 10 μl of 10 mM Tris-hydrochloride buffer (pH 7.5) containing 10 mM magnesium chloride and 1 mM DTT, 10 units of the restriction enzyme *Kpn*I (Takara Shuzo) was further added, and the reaction was allowed to proceed at 37°C for 1 hour. The reaction mixture was subjected to ethanol precipitation, the precipitate was dissolved in 10 μl of 50 mM Tris-hydrochloride buffer (pH 7.5) containing 100 mM sodium chloride, 10 mM magnesium chloride and 1 mM DTT, 10 units of the restriction enzyme *Sal*I (Takara Shuzo) was further added, and the reaction was allowed to proceed at 37°C for 1 hour. The reaction mixture was fractionated by agarose gel electrophoresis, whereby about 0.7 μg of a *Kpn*I-*Sal*I fragment (about 1.37 kb) containing the rabbit β-globin gene splicing and poly A signals and the SV40 early gene poly A signal was recovered.

Then, 5 μg of the plasmid pMohCκ obtained in Paragraph 4 of Example 1 was added to 10 μl of 10 mM Tris-hydrochloride buffer (pH 7.5) containing 10 mM magnesium chloride and 1mM DTT, 10 units of the restriction enzyme *Kpn*I (Takara Shuzo) was further added, and the reaction was allowed to proceed at 37°C for 1 hour. The reaction mixture was subjected to ethanol precipitation, the precipitate was dissolved in 10 μl of 50 mM Tris-hydrochloride buffer (pH 7.5) containing 100 mM sodium chloride, 10 mM magnesium chloride and 1 mM DTT, 10 units of the restriction enzyme *Xho*I (Takara Shuzo) was further added, and the reaction was allowed to proceed at 37°C for 1 hour. The reaction mixture was fractionated by agarose gel electrophoresis, whereby about 0.7 μg of a human CDR-grafted antibody L chain expression unit-containing *Kpn*I-*Xho*I fragment (about 1.06 kb) was recovered.

Then, 0.1 μg of the *Sal*I-*Xho*I fragment of pMoγ1SP, 0.1 μg of the *Kpn*I-*Sal*I fragment of pBSK-HAEESal and 0.1 μg of the *Kpn*I-*Xho*I fragment of pMohCκ, each obtained as mentioned above, were added to a total of 20 μl of sterilized water and ligated together using Ready-To-Go T4 DNA Ligase (Pharmacia Biotech). The thus-obtained recombinant plasmid DNA solution was used to transform *Escherichia coli* HB101, and the plasmid pMoκγ1SP shown in Fig. 21 was obtained.

Then, 3 μg of the above plasmid pMoκγ1SP was added to 10 μl of 50 mM Tris-hydrochloride buffer (pH 7.5) containing 100 mM sodium chloride, 10 mM magnesium chloride and 1 mM DTT, 10 units of the restriction enzyme *Xho*I (Takara Shuzo) was further added, and the reaction was allowed to proceed at 37°C for 1 hour. The reaction mixture was subjected to ethanol precipitation, the precipitate was added to 10 μl of 10 mM Tris-hydrochloride buffer (pH 7.5) containing 10 mM magnesium chloride and 1 mM DTT, 1 units of the restriction enzyme *Sac*II (Toyobo) was further added, and the reaction was allowed to proceed at 37°C for 10 minutes for partial digestion. The reaction mixture was fractionated by agarose gel electrophoresis, and about 0.2 μg of a SacII-XhoI fragment (about 8.49 kb) was recovered.

Then, 3 μg of the plasmid pBSX-SA obtained in Paragraph 3 of Example 1 was added to 10 μl of 10 mM Tris-hydrochloride buffer (pH 7.5) containing 10 mM magnesium chloride and 1 mM DTT, 10 units of the restriction enzyme *Sac*II (Toyobo) was further added, and the reaction was allowed to proceed at 37°C for 1 hour. The reaction mixture was subjected to ethanol precipitation, the precipitate was dissolved in 10 μl of 50 mM Tris-hydrochloride buffer (pH 7.5) containing 100 mM sodium chloride, 10 mM magnesium chloride and 1 mM DTT, 10 units of the restriction enzyme *Xho*I (Takara Shuzo) was further added, and the reaction was allowed to proceed at 37°C for 1 hour. The reaction mixture was fractionated by agarose gel electrophoresis, and about 1 μg of a *Sac*II-*Xho*I fragment (about 4.25 kb) was recovered.

Then, 0.1 μg of the *Sac*II-*Xho*I fragment of pMoκγ1SP and 0.1 μg of the *Sac*II-*Xho*I fragment of pBSX-SA, each obtained as mentioned above, were added to a total of 20 μl of sterilized water and ligated to each other using Ready-To-Go T4 DNA Ligase (Pharmacia Biotech). The thus-obtained recombinant plasmid DNA solution was used to transform *Escherichia coli* HB101, and the plasmid pKANTEX93 shown in Fig. 22 was obtained.

## EXAMPLE 2

### 1. Expression of mouse-human chimeric anti-$GM_2$ antibody

Mouse-human chimeric anti-$GM_2$ antibody expression was effected using the humanized antibody expression vector pKANTEX93 mentioned above in Example 1 in the following manner.

#### (1) Construction of plasmid pBSH3 containing mouse anti-$GM_2$ antibody KM796 H chain V region cDNA

Three μg of the plasmid pBluescript® SK(-) (Stratagene) was added to 10 μl of 10 mM Tris-hydrochloride buffer (pH 7.5) containing 10 mM magnesium chloride and 1 mM DTT, 10 units each of the restriction enzymes SacII (Toyobo) and *Kpn*I (Takara Shuzo) were further added, and the reaction was allowed to proceed at 37°C for 1 hour. The reaction mixture was subjected to ethanol precipitation, and the precipitate was subjected to blunting treatment for rendering blunt the 3' cohesive ends resulting from the restriction enzyme digestion using DNA Blunting Kit (Takara Shuzo). The resulting reaction was precipitated with ethanol, the precipitate thus obtained was dissolved in 20 μl of a buffer containing 50 mM Tris-hydrochloride buffer (pH 9.0) and 1 mM magnesium chloride, and the mixture thus obtained was allowed to react by adding one unit of alkali phosphatase (*E. coli* C75, Takara Shuzo) at 37°C for 1 hour for dephosphorylation of the 5' termini. Then, fractionation by agarose gel electrophoresis was carried out, and about 1 μg of a DNA fragment about 2.95 kb in size was recovered.

Then, synthetic DNAs respectively having the base sequences shown in SEQ ID NO:18 and SEQ ID NO:19 were synthesized using an automatic DNA synthesizer (Applied Biosystems model 380A). To 15 µl of sterilized water were added 0.3 µg each of the synthetic DNAs obtained, and the mixture was heated at 65°C for 5 minutes. The reaction mixture was allowed to stand at room temperature for 30 minutes and then 2 µl of 10-fold concentrated buffer [500 mM Tris-hydrochloride (pH 7.6), 100 mM magnesium chloride, 50 mM DTT] and 2 µl of 10 mM ATP were added, 10 units of T4 polynucleotide kinase was further added, and the reaction was allowed to proceed at 37°C for 30 minutes for phosphorylating the 5' termini. To a total of 20 µl of sterilized water were added 0.1 µg of the DNA fragment (2.95 kb) derived from the plasmid pBluescript® SK(-) and 0.05 µg of the phosphorylated synthetic DNA, each obtained as mentioned above, followed by ligation to each other using Ready-To-Go T4 DNA Ligase (Pharmacia Biotech): The thus-obtained recombinant plasmid DNA solution was used to transform *Escherichia coli* HB101, and the plasmid pBSNA shown in Fig. 23 was obtained. Ten µg of the plasmid obtained was subjected to sequencing reaction treatment according to the instructions attached to AutoRead Sequencing Kit (Pharmacia Biotech), followed by electrophoresis on A.L.F. DNA Sequencer (Pharmacia Biotech) for base sequence determination, whereby it was confirmed that the synthetic DNA had been introduced as desired.

Then, 3 µg of the plasmid pBSNA obtained as mentioned above was added to 10 µl of 10 mM Tris-hydrochloride buffer (pH 7.5) containing 10 mM magnesium chloride and 1 mM DTT, 10 units of the restriction enzyme *Apa*I (Takara Shuzo) was further added, and the reaction was allowed to proceed at 37°C for 1 hour. The reaction mixture was subjected to ethanol precipitation, the precipitate was added to 10 µl of 50 mM Tris-hydrochloride buffer (pH 7.5) containing 100 mM sodium chloride, 10 mM magnesium chloride, 1 mM DTT, 100 µg/ml BSA and 0.01% Triton® X-100, 10 units of the restriction enzyme *Not*I (Takara Shuzo) was further added, and the reaction was allowed to proceed at 37°C for 1 hour. The reaction mixture was fractionated by agarose gel electrophoresis, and about 1 µg of a DNA fragment about 2.95 kb in size was recovered.

Then, 10 µg of the plasmid pChi796HM1 described in JP-A-6-205964 was added to 10 µl of 10 mM Tris-hydrochloride buffer (pH 7.5) containing 10 mM magnesium chloride and 1 mM DTT, 10 units of the restriction enzyme *Apa*I (Takara Shuzo) was further added, and the reaction was allowed to proceed at 37°C for 1 hour. The reaction mixture was subjected to ethanol precipitation, the precipitate was added to 10 µl of 50 mM Tris-hydrochloride buffer (pH 7.5) containing 100 mM sodium chloride, 10 mM magnesium chloride, 1 mM DTT, 100 µg/ml BSA and 0.01% Triton® X-100, 10 units of the restriction enzyme *Not*I (Takara Shuzo) was further added, and the reaction was allowed to proceed at 37°C for 1 hour. The reaction mixture was fractionated by agarose gel electrophoresis, and about 0.3 µg of a DNA fragment about 0.45 kb in size was recovered.

Then, 0.1 µg of the *Apa*I-*Not*I fragment of pBSNA and 0.1 µg of the *Apa*I-*Not*I fragment of pChi796HM1, each obtained as mentioned above, were added to a total of 20 µl of sterilized water and ligated to each other using Ready-To-Go T4 DNA Ligase (Pharmacia Biotech). The thus-obtained recombinant plasmid DNA solution was used to transform *Escherichia coli* HB101, and the plasmid pBSH3 shown in Fig. 24 was obtained.

(2) Construction of plasmid pBSL3 containing mouse anti-$GM_2$ antibody KM796 L chain V region cDNA

Three µg of the plasmid pBluescript® - SK(-) (Stratagene) was added to 10 µl of 10 mM Tris-hydrochloride buffer (pH 7.5) containing 10 mM magnesium chloride and 1 mM DTT, 10 units of the restriction enzyme *Kpn*I (Takara Shuzo) was further added, and the reaction was allowed to proceed at 37°C for 1 hour. The reaction mixture was subjected to ethanol precipitation, and the precipitate was subjected to blunting treatment for rendering blunt the 3' cohesive ends resulting from *Kpn*I digestion using DNA Blunting Kit (Takara Shuzo) and then to ethanol precipitation, the precipitate was added to 10 µl of 10 mM Tris-hydrochloride buffer (pH 7.5) containing 10 mM magnesium chloride and 1 mM DTT, 10 units of the restriction enzyme *Sac*I (Takara Shuzo) was further added, and the reaction was allowed to proceed at 37°C for 1 hour. The reaction mixture was fractionated by agarose gel electrophoresis, whereby about 1 µg of a DNA fragment about 2.95 kb in size was recovered.

Then, synthetic DNAs respectively having the base sequences shown in SEQ ID NO:20 and SEQ ID NO:21 were synthesized using an automatic DNA synthesizer (Applied Biosystems model 380A). To 15 µl of sterilized water were added 0.3 µg each of the synthetic DNAs obtained, and the mixture was heated at 65°C for 5 minutes. The reaction mixture was allowed to stand at room temperature for 30 minutes. Then, 2 µl of 10-fold concentrated buffer [500 mM Tris-hydrochloride (pH 7.5), 100 mM magnesium chloride, 50 mM DTT] and 2 µl of 10 mM ATP were added, 10 units of T4 polynucleotide kinase was further added, and the reaction was allowed to proceed at 37°C for 30 minutes for phosphorylating the 5' termini. The, 0.1 µg of the DNA fragment (2.95 kb) derived from the plasmid pBluescript® SK (-) and 0.05 µg of the phosphorylated synthetic DNA, each obtained as mentioned above, were added to a total of 20 µl of sterilized water and ligated to each other using Ready-To-Go T4 DNA Ligase (Pharmacia Biotech). The thus-obtained recombinant plasmid DNA solution was used to transform *Escherichia coli* HB101, and the plasmid pBSES shown in Fig. 25 was obtained. Ten µg of the plasmid obtained was subjected to sequencing reaction treatment according to the instructions attached to AutoRead Sequencing Kit (Pharmacia Biotech), followed by electrophoresis on A.L.F. DNA Sequencer (Pharmacia Biotech) for base sequence determination, whereby it was confirmed that the synthetic DNA had been introduced as desired.

Then, 3 μg of the plasmid pBSES obtained as mentioned above was added to 10 μl of 50 mM Tris-hydrochloride buffer (pH 7.5) containing 100 mM sodium chloride, 10 mM magnesium chloride, 1 mM DTT and 100 μg/ml BSA, 10 units each of the restriction enzymes *Eco*RI (Takara Shuzo) and *Spl*I (Takara Shuzo) were further added, and the reaction was allowed to proceed at 37°C for 1 hour. The reaction mixture was fractionated by agarose gel electrophoresis, and about 1 μg of a DNA fragment about 2.95 kb in size was recovered.

Then, 5 μg of the plasmid pKM796L1 described in JP-A-6-205694 was added to 10 μl of 50 mM Tris-hydrochloride buffer (pH 7.5) containing 10 mM magnesium chloride and 1 mM DTT, 10 units each of the restriction enzymes *Eco*RI (Takara Shuzo) and *Afl*III (Takara Shuzo) were further added, and the reaction was allowed to proceed at 37°C for 1 hour. The reaction mixture was fractionated by agarose gel electrophoresis, and about 0.3 μg of an *Eco*RI-*Afl*III fragment about 0.39 kb in size was recovered. Then, synthetic DNAs respectively having the base sequences shown in SEQ ID NO:22 and SEQ ID NO:23 were synthesized using an automatic DNA synthesizer (Applied Biosystems model 380A). To 15 μl of sterilized water were added 0.3 μg each of the synthetic DNAs obtained, and the mixture was heated at 65°C for 5 minutes. The reaction mixture was allowed to stand at room temperature for 30 minutes. Then, 2 μl of 10-fold concentrated buffer [500 mM Tris-hydrochloride (pH 7.6), 100 mM magnesium chloride, 50 mM DTT) and 2 μl of 10 mM ATP were added, 10 units of T4 polynucleotide kinase was further added, and the reaction was allowed to proceed at 37°C for 30 minutes for phosphorylating the 5' termini.

Then, 0.1 μg of the pBSES-derived *Eco*RI-*Spl*I fragment (2.95 kb), 0.1 μg of the pKM796L1-derived *Eco*RI-*Afl*III fragment and 0.05 μg of the phosphorylated synthetic DNA, each obtained as mentioned above, were added to a total of 20 μl of sterilized water and ligated together using Ready-To-Go T4 DNA Ligase (Pharmacia Biotech). The thus-obtained recombinant plasmid DNA solution was used to transform *Escherichia coli* HB101, and the plasmid pBSL3 shown in Fig. 26 was obtained. Ten μg of the plasmid obtained was subjected to sequencing reaction treatment according to the instructions attached to AutoRead Sequencing Kit (Pharmacia Biotech), followed by electrophoresis on A.L.F. DNA Sequencer (Pharmacia Biotech) for base sequence determination, whereby it was confirmed that the synthetic DNA had been introduced as desired.

(3) Construction of mouse-human chimeric anti-$GM_2$ antibody expression vector, pKANTEX796

A mouse-human chimeric anti-$GM_2$ antibody expression vector, pKANTEX796, was constructed using the plasmid pKANTEX93 obtained in Example 1 and the plasmids pBSH3 and pBSL3 respectively obtained in Paragraph 1 (1) and (2) of Example 2, in the following manner.

Three μg of the plasmid pBSH3 was added to 10 μl of 10 mM Tris-hydrochloride buffer (pH 7.5) containing 10 mM magnesium chloride and 1 mM DTT, 10 units of the restriction enzyme *Apa*I (Takara Shuzo) was further added, and the reaction was allowed to proceed at 37°C for 1 hour. The reaction mixture was subjected to ethanol precipitation, the precipitate was dissolved in 10 μl of 50 mM Tris-hydrochloride buffer (pH 7.5) containing 100 mM sodium chloride, 10 mM magnesium chloride, 1 mM DTT, 100 μg/ml BSA and 0.01% Triton® X-100, 10 units of the restriction enzyme *Not*I (Takara Shuzo) was further added, and the reaction was allowed to proceed at 37°C for 1 hour. The reaction mixture was fractionated by agarose gel electrophoresis, and about 0.3 μg of an *Apa*I-*Not*I fragment about 0.46 kb in size was recovered.

Then, 3 μg of the plasmid pKANTEX93 was added to 10 μl of 10 mM Tris-hydrochloride buffer (pH 7.5) containing 10 mM magnesium chloride and 1 mM DTT, 10 units of the restriction enzyme ApaI (Takara Shuzo) was further added, and the reaction was allowed to proceed at 37°C for 1 hour. The reaction mixture was subjected to ethanol precipitation, the precipitate was dissolved in 10 μl of 50 mM Tris-hydrochloride buffer (pH 7.5) containing 100 mM sodium chloride, 10 mM magnesium chloride, 1 mM DTT, 100 μg/ml BSA and 0.01% Triton® X-100, 10 units of the restriction enzyme *Not*I (Takara Shuzo) was further added, and the reaction was allowed to proceed at 37°C for 1 hour. The reaction mixture was fractionated by agarose gel electrophoresis, whereby about 1 μg of an *Apa*I-*Not*I fragment about 12.75 kb in size was recovered.

Then, 0.1 μg of the pBSH3-derived *Apa*I-*Not*I fragment and 0.1 μg of the pKANTEX93-derived *Apa*I-*Not*I fragment, each obtained as mentioned above, were added to a total of 20 μl of sterilized water and ligated to each other using Ready-To-Go T4 DNA Ligase (Pharmacia Biotech). The thus-obtained recombinant plasmid DNA solution was used to transform *Escherichia coli* HB101, and the plasmid pKANTEX796H shown in Fig. 27 was obtained.

Then, 3 μg of the plasmid pBSL3 was added to 10 μl of 50 mM Tris-hydrochloride buffer (pH 7.5) containing 100 mM sodium chloride, 10 mM magnesium chloride, 1 mM DTT and 100 μg/ml BSA, 10 units each of the restriction enzymes *Eco*RI (Takara Shuzo) and *Spl*I (Takara Shuzo) were further added, and the reaction was allowed to proceed at 37°C for 1 hour. The reaction mixture was fractionated by agarose gel electrophoresis, and about 0.3 μg of an *Eco*RI-*Spl*I fragment about 0.4 kb in size was recovered.

Then, 3 μg of the plasmid pKANTEX796H was added to 10 μl of 50 mM Tris-hydrochloride buffer (pH 7.5) containing 100 mM sodium chloride, 10 mM magnesium chloride, 1 mM DTT and 100 μg/ml BSA, 10 units each of the restriction enzymes EcoRI (Takara Shuzo) and *Spl*I (Takara Shuzo) were further added, and the reaction was allowed to proceed at 37°C for 1 hour. The reaction mixture was fractionated by agarose gel electrophoresis, and about 1 μg of an *Eco*RI-*Spl*I fragment about 13.20 kb in size was recovered.

Then, 0.1 μg of the pBSL3-derived *Eco*RI-*Spl*I fragment and 0.1 μg of the pKANTEX796H-derived *Eco*RI-*Spl*I fragment, each obtained as mentioned above, were added to a total of 20 μl of sterilized water and ligated to each other using Ready-To-Go T4 DNA Ligase (Pharmacia Biotech). The thus-obtained recombinant plasmid DNA solution was used to transform *Escherichia coli* HB101, and the plasmid pKANTEX796 shown in Fig. 28 was obtained.

(4) Expression of mouse-human chimeric anti-$GM_2$ antibody in YB2/0 cells using pKANTEX796

Introduction of the plasmid into YB2/0 cells (ATCC CRL1662) was carried out by the electroporation method (Miyaji, H. *et al., Cytotechnology*, 3, 133 (1990)). A 4 μg portion of pKANTEX796 obtained in Paragraph 1 (3) of Example 2 was introduced into $4 \times 10^6$ cells of YB2/0 cells, and the resulting cells were suspended in 40 ml of RPMI1640-FCS (10) medium [RPMI1640 medium (manufactured by Nissui Pharmaceutical) supplemented with 10% of FCS, an appropriate amount of 7.5% sodium bicarbonate solution, 3% of 200 mM L-glutamine solution (manufactured by Gibco) and 0.5% of penicillin-streptomycin solution (manufactured by Gibco, contains 5,000 U/ml of penicillin and 5 mg/ml of streptomycin)] and dispensed in 200 μl portions into wells of a 96 well microplate. After 24 hours of culturing at 37°C in a 5% $CO_2$ incubator, G418 was added to each well to a final concentration of 0.5 mg/ml, and the cells were cultured for 1 to 2 weeks. Culture supernatants were recovered from wells in which colonies of transformant cell lines have been formed, and the activity of the mouse-human chimeric anti-$GM_2$ antibody in the culture supernatants was measured by the ELISA method described in the following paragraph (5). Cells in wells in which the activity was found were subjected to gene amplification in the following manner with an attempt to increase expression quantity of the chimera antibody. Firstly, the cells were suspended in the RPMI1640-FCS (10) medium supplemented with 0.5 mg/ml of G418 and 50 nM of methotrexate (manufactured by Sigma, to be referred to as "MTX" hereinafter), to a density of 1-2 $\times 10^5$ cells/ml, and the suspension was dispensed in 2 ml portions in wells of a 24 well plate. The cells were cultured at 37°C for 1 to 2 weeks in a 5% $CO_2$ incubator to induce resistant cells to 50 nM MTX. In wells in which the cells resistant to 50 nM MTX have been formed, the final concentration of MTX was increased to 100 nM and then to 200 nM and the expression quantity was evaluated by the ELISA method to select cells having the highest expression quantity. The thus selected cells were subjected twice to cloning by the limiting dilution analysis and then established as the final chimera antibody stable expression cells. The thus established mouse-human chimeric anti-$GM_2$ antibody stable expression cells showed an expression quantity of about 1 to 2 μg/ml, so that it was confirmed that efficient and stable expression of the humanized antibody can be effected by the use of pKANTEX93.

(5) ELISA method

A 2 ng portion of ganglioside was dissolved in 2 ml of ethanol solution containing 5 ng of phosphatidylcholine (manufactured by Sigma) and 2.5 ng of cholesterol (manufactured by Sigma). This solution or a diluted solution thereof was dispensed in 20 μl portions in wells of a 96 well microplate (manufactured by Greiner), air-dried and then subjected to blocking with a phosphate buffer containing 1% BSA (to be referred to as "PBS" hereinafter). To the resulting plate was added culture supernatant of a transformant cell line, a purified mouse monoclonal antibody, a purified mouse-human chimeric antibody or a purified humanized antibody in an amount of from 50 to 100 μl, subsequently carrying out 1 to 2 hours of reaction at room temperature. After the reaction and subsequent washing of each well with PBS, 50 to 100 μl of a peroxidase-labeled rabbit anti-mouse IgG antibody (manufactured by Dako, used by 400 times dilution) or a peroxidase-labeled goat anti-human γ chain antibody (manufactured by Kiyukegard & Perry Laboratory, used by 1,000 times dilution) was added thereto, and 1 to 2 hours of reaction was carried out at room temperature. After washing with PBS, 50 to 100 μl of an ABTS substrate solution [a solution prepared by dissolving 550 mg of 2,2'-azinobis(3-ethylbenzothiazoline-6-sulfonic acid) in 1 liter of 0.1 M citrate buffer (pH 4.2) and adding 1 μl/ml of hydrogen peroxide to the solution just before its use] was added to each well to effect development of color which was then measured at $OD_{415}$.

2. Transient mouse-human chimeric antibody expression in COS-7 (ATCC CRL 1651) cells

For enabling more rapid activity evaluation of various versions of human CDR-grafted anti-$GM_2$ antibody, transient expression of mouse-human chimeric anti-$GM_2$ antibody expression was caused in COS-7 cells by the Lipofectamine method using pKANTEX796 and a variant thereof in the following manner.

(1) Construction of variant of pKANTEX796

Since transient antibody expression in animal cells is dependent on the copy number of an expression vector introduced, it was supposed that an expression vector smaller in size would show a higher expression efficiency. Therefore, a smaller humanized antibody expression vector, pT796, was constructed by deleting a region supposedly having no effect on humanized antibody expression from pKANTEX796 in the following manner.

Thus, 3 µg of the plasmid pKANTEX796 was added to 10 µl of 10 mM Tris-hydrochloride buffer (pH 7.5) containing 50 mM sodium chloride, 10 mM magnesium chloride and 1 mM DTT, 10 units of the restriction enzyme *Hin*dIII (Takara Shuzo) was further added, and the reaction was allowed to proceed at 37°C for 1 hour. The reaction mixture was subjected to ethanol precipitation, the precipitate was dissolved in 10 µl of 50 mM Tris-hydrochloride buffer (pH 7.5) containing 100 mM sodium chloride, 10 mM magnesium chloride and 1 mM DTT, 10 units of the restriction enzyme *Mlu*I (Takara Shuzo) was further added, and the reaction was allowed to proceed at 37°C for 1 hour. The reaction mixture was subjected to ethanol precipitation, and the 5' cohesive ends resulting from the restriction enzyme digestion were rendered blunt using DNA Blunting Kit (Takara Shuzo). The reaction mixture was fractionated by agarose gel electrophoresis and about 1 µg of a DNA fragment about 9.60 kb in size was recovered. A 0.1-µg portion of the thus-recovered DNA fragment was added to a total of 20 µl of sterilized water and subjected to ligation treatment using Ready-To-Go T4 DNA Ligase (Pharmacia Biotech). The thus-obtained recombinant plasmid DNA solution was used to transform *Escherichia coli* HB101, and the plasmid pT796 shown in Fig. 29 was obtained.

(2) Transient expression of mouse-human chimeric anti-$GM_2$ antibody using pKANTEX796 and pT796

A 1 × $10^5$ cells/ml suspension of COS-7 cells was distributed in 2-ml portions into wells of a 6-well plate (Falcon) and cultured overnight at 37°C. Two µg of pKANTEX796 or pT796 was added to 100 µl of OPTI-MEM medium (Gibco), a solution prepared by adding 10 µl of LIPOFECTAMINE® reagent (Gibco) to 100 µl of OPTI-MEM medium (Gibco) was further added, and the reaction was allowed to proceed at room temperature for 40 minutes to cause DNA-liposome complex formation. The COS-7 cells cultured overnight were washed twice with 2 ml of OPTI-MEM medium (Gibco), the complex-containing solution was added, and the cells were cultured at 37°C for 7 hours. Then, the solution was removed, 2 ml of DMEM medium (Gibco) containing 10% FCS was added to each well, and the cells were cultured at 37°C. After 24 hours, 48 hours, 72 hours, 96 hours and 120 hours of cultivation, the culture supernatant was recovered and, after concentration procedure as necessary, evaluated for mouse-human chimeric anti-$GM_2$ antibody activity in the culture supernatant by the ELISA method described in Paragraph 1 (5) of Example 2. The results are shown in Fig. 30. As shown in Fig. 30, higher levels of transient mouse-human chimeric anti-$GM_2$ antibody expression was observed with pT796 as compared with pKANTEX796. For pT796, the level of expression was highest at 72 to 96 hours, the concentration being about 30 ng/ml (in terms of $GM_2$ binding activity). The above results indicate that construction of a pKANTEX93-derived vector having a reduced size and introduction thereof into COS-7 cells make it possible to make activity evaluation of expression vector-derived humanized antibodies in a transient expression system. Furthermore, for close activity comparison of various versions of human CDR-grafted anti-$GM_2$ antibody as mentioned hereinafter, the ELISA method described below under (3) was used to determine antibody concentrations in transient expression culture supernatants.

(3) Determination by sandwich ELISA of humanized antibody concentrations in various culture supernatants

A solution prepared by 400-fold dilution of goat anti-human γ chain antibody (Igaku Seibutugaku Kenkyusho) with PBS was distributed in 50-µl portions into wells of a 96-well microtiter plate and allowed to stand overnight at 4°C for binding to the wells. After removing the antibody solution, blocking was effected with 100 µl of PBS containing 1% BSA at 37°C for 1 hour. Fifty µl of a transient expression culture supernatant or purified mouse-human chimeric anti-$GM_2$ antibody was added thereto and allowed to react at room temperature for 1 hour. Thereafter, the solution was removed, the wells were washed with PBS, and 50 µl of a solution prepared by 500-fold dilution of peroxidase-labeled mouse anti-human κ chain antibody (Zymet) with PBS was added and allowed to react at room temperature for 1 hour. After washing with PBS, 50 µl of an ABTS substrate solution was added for causing color development, and the $OD_{415}$ was measured.

EXAMPLE 3

Production of human CDR-grafted anti-$GM_2$ antibody I

A human CDR-grafted anti-$GM_2$ antibody higher in $GM_2$-binding activity than the human CDR-grafted anti-$GM_2$ antibody described in Example 2 of JP-A-6-105694 was produced in the following manner.

1. Modification of human CDR-grafted anti-$GM_2$ antibody H chain V region described in Paragraph 1 (1) of Example 2 of JP-A-6-205694

DNAs coding for some versions of the human CDR-grafted anti-$GM_2$ antibody H chain V region described in Example 2 as derived by replacing several amino acid in the FR with original mouse antibody amino acids were constructed in the following manner. Based on a computer model for the V region of mouse antibody KM796, those amino acid residues that were expected to contribute to restoration of antigen-binding activity as a result of replacement were selected as the amino acid residues to be replacement. First, DNAs respectively having the base sequences of SEQ ID NO:24 and SEQ ID NO:25 were synthesized using an automatic DNA synthesize (Applied Biosystems model 380A).

Then, a version (version 2) of human CDR-grafted antibody H chain V region shown in SEQ ID NO:26 and having replacement in positions 78 (threonine in lieu of glutamine), 79 (alanine in lieu of phenylalanine) and 80 (tyrosine in lieu of serine) was constructed in the same manner as in Paragraph 1 (1) of Example 2 of JP-A-6-205964 using a synthetic DNA of SEQ ID NO:24 in lieu of the synthetic DNA of SEQ ID NO:21 of JP-A-6-205964.

Then, another version (version 4) of human CDR-grafted antibody H chain V region shown in SEQ ID NO:27 and having replacements in positions 27 (tyrosine in lieu of phenylalanine), 30 (threonine in lieu of serine), 40 (serine in lieu of proline) and 41 (histidine in lieu of proline) was constructed in the same manner as in Paragraph 1 (1) of Example 2 of JP-A-6-205694 using a synthetic DNA of SEQ ID NO:25 in lieu of the synthetic DNA of SEQ ID NO:25 of JP-A-6-205694.

(2) Construction of human CDR-grafted anti-$GM_2$ antibody H chain V region using known HMHCS of human antibody H chain V region

According to Kabat *et al*. (Kabat E. A. *et al*., *Sequences of Proteins of Immunological Interest*, US Dept. of Health and Human Services, 1991), known human antiobdy H chain V regions are classifiable into subgroups I to III (Human Sub Groups (HSG) I to III) based on the homology of their FR regions, and common sequences have been identified for respective subgroups. The present inventors identified HMHCS as one meaning from the common sequences, a human CDR-grafted anti-$GM_2$ antibody H chain V region was constructed based on the HMHCS. First, for selecting HMHCS to serve as the base, the homology was examined between the FR of the mouse antibody KM796 H chain V region and the FR of the HMHCS of the human antibody H chain V region of each subgroup (Table 1).

TABLE 1

| Homology (%) between mouse antibody KM796 H chain V region FR and human antibody H chain V region common sequence FR | | |
|---|---|---|
| HSG I | HSG II | HSG III |
| 72.1 | 52.9 | 58.6 |

As a result, it was confirmed that subgroup I shows the greatest similarity. Thus, based on the HMHCS of subgroup I, a human CDR-grafted anti-$GM_2$ antibody H chain V region was constructed by the PCR method in the following manner.

Synthetic DNAs respectively having the base sequences of SEQ ID NO:28 through SEQ ID NO:33 were synthesized using an automatic DNA synthesizer (Applied Systems model 380A). The DNAs synthesized were added, each to a final concentration of 0.1 μM, to 50 μl of 10 mM Tris-hydrochloride buffer (pH 8.3) containing 50 mM potassium chloride, 1.5 mM magnesium chloride, 0.001% gelatin, 200 μM dNTP, 0.5 μM M13 primer RV (Takara Shuzo), 0.5 μM M13 primer M4 (Takara Shuzo) and 2 units of TaKaRa Taq DNA polymerase, the mixture was covered with 50 μl of mineral oil, a DNA thermal cycler (Perkin Elmer model PJ480) was loaded with the mixture, and 30 PCR cycles (2 minutes at 94°C, 2 minutes at 55°C and 2 minutes at 72°C per cycle) were conducted. The reaction mixture was purified using QIAquick PCR Purification Kit (Qiagen) and then made into a solution in 30 μl of 10 mM Tris-hydrochloride buffer (pH 7.5) containing 10 mM magnesium chloride and 1 mM DTT, 10 units of the restriction enzyme *Apa*I (Takara Shuzo) was further added, and the reaction was allowed to proceed at 37°C for 1 hour. The reaction mixture was subjected to ethanol precipitation, the precipitate was added to 10 μl of 50 mM Tris-hydrochloride (pH 7.5) containing 100 mM sodium chloride, 10 mM magnesium chloride, 1 mM DTT, 100 μg/ml BSA and 0.01% Triton® X-100, 10 units of the restriction enzyme *Not*I (Takara Shuzo) was further added, and the reaction was allowed to proceed at 37°C for 1 hour. The reaction mixture was fractionated by agarose gel electrophoresis, and about 0.2 μg of an *Apa*I-*Not*I fragment about 0.44 kb in size was recovered.

Then, 3 μg of the plasmid pBSH3 obtained in Paragraph 1 (1) of Example 2 was added to 10 μl of 10 mM Tris-hydrochloride buffer (pH 7.5) containing 10 mM magnesium chloride and 1 mM DTT, 10 units of the restriction enzyme *Apa*I (Takara Shuzo) was further added, and the reaction was allowed to proceed at 37°C for 1 hour. The reaction mixture was subjected to ethanol precipitation, the precipitate was added to 10 μl of 50 mM Tris-hydrochloride buffer (pH 7.5) containing 100 mM sodium chloride, 10 mM magnesium chloride, 1 mM DTT, 100 μg/ml BSA and 0.01% Triton® X-100, 10 units of the restriction enzyme *Not*I (Takara Shuzo) was further added, and the reaction was allowed to proceed at 37°C for 1 hour. The reaction mixture was fractionated by agraose gel electrophoresis, and about 1 μg of an *Apa*I-*Not*I fragment about 2.95 kb in size was recovered.

Then, 0.1 μg of the *Apa*I-*Not*I fragment of the human CDR-grafted anti-$GM_2$ antibody H chain V region and 0.1 μg of the *Apa*I-*Not*I fragment of pBSH3, each obtained as mentioned above, were added to a total of 20 μl of sterilized water and ligated to each other using Ready-To-Go T4 DNA Ligase (Pharmacia Biotech). The thus-obtained recombinant plasmid DNA solution was used to transform *Escherichia coli* HB101. Plasmid DNAs were prepared from 10 transformant clones and their base sequences were determined. As a result, a plasmid, pBSH10, shown in Fig. 31 and having the desired base sequence was obtained. The amino acid sequence and base sequence of the human CDR-grafted anti-$GM_2$ antibody H chain V region contained in pBSH10 are shown in SEQ ID NO:7. In the amino acid sequence of the thus-constructed human CDR-grafted anti-$GM_2$ antibody H chain V region, arginine in position 67, alanine in position 72, serine in position 84 and arginine in position 98 in the FR as selected based on a computer model for the V region are replaced by lysine, valine, histidine and threonine, respectively, that are found in the mouse antibody KM796 H chain V region. This is for the purpose of retaining the antigen-binding capacity of mouse antibody KM796.

(3) Modification of human CDR-grafted anti-$GM_2$ antibody L chain V region described in Paragraph 1 (2) of Example 2 of JP-A-6-205694

First, a DNA having the base sequence of SEQ ID NO:34 was synthesized using an automatic DNA synthesizer (Applied Biosystems model 380A), and a human CDR-grafted anti-$GM_2$ antibody L chain V region cDNA with a 3' terminus capable of pairing with the restriction enzyme *Spl*I was constructed by following the same reaction procedure as in Paragraph 1 (2) of Example 2 of JP-A-6-205694 using the synthetic DNA in lieu of the synthetic DNA of SEQ ID NO:35 of JP-A-6-205964.

Then, 3 μg of the plasmid pBSL3 obtained in Paragraph 1 (2) of Example 2 was added to 10 μl of 50 mM Tris-hydrochloride buffer (pH 7.5) containing 100 mM sodium chloride, 10 mM magnesium chloride, 1 mM DTT and 100 μg/ml BSA, 10 units each of the restriction enzymes *Eco*RI (Takara Shuzo) and *Spl*I (Takara Shuzo) were further added, and the reaction was allowed to proceed at 37°C for 1 hour. The reaction mixture was fractionated by agarose gel electrophoresis, and about 1 μg of an *Eco*RI-*Spl*I fragment about 2.95 kb in size was recovered.

Then, 0.1 μg of the *Eco*RI-*Spl*I fragment of the human CDR-grafted anti-$GM_2$ antibody L chain V region obtained as mentioned above and 0.1 pg of the above *Eco*RI-*Spl*I fragment of pBSL3 were added to a total of 20 μl of sterilized water and ligated to each other using Ready-To-Go T4 DNA Ligase (Pharmacia Biotech). The thus-obtained recombinant plasmid DNA solution was used to transform *Escherichia coli* HB101, and the plasmid pBSL16 shown in Fig. 32 was obtained.

Then, DNAs coding for certain versions of the human CDR-grafted anti-$GM_2$ antibody L chain V region contained in the above plasmid pBSL16 were constructed by replacing a certain number of amino acids in the FR with original mouse antibody amino acids by mutagenesis by means of PCR in the following manner (Fig. 33). Based on a computer model for the V region of mouse antibody KM796, those amino acid residues that were expected to contribute to restoration of antigen-binding activity as a result of replacement were selected as the amino acid residues to be replaced.

Antisense and sense DNA primers for introducing mutations were synthesized using an automatic DNA synthesizer (Applied Biosystems model 380A). A first PCR reaction was conducted in the same manner as in Paragraph 1 (2) of Example 3 using a final concentration each of 0.5 μM of M13 primer RV (Takara Shuzo) and the antisense DNA primer and of M13 primer M4 (Takara Shuzo) and the sense DNA primer, with 1 ng of pBSL16 as the template. Each reaction mixture was purified using QIAquick PCR Purification Kit (Qiagen) with elution with 20 μl of 10 mM Tris-hydrochloride (pH 8.0). Using 5 μl of each elute, a second PCR reaction was conducted in the same manner as in Paragraph 1 (2) of Example 3. The reaction mixture was purified using QIA quick® PCR Purification Kit (Qiagen) and then made into a solution in 30 μl of 50 mM Tris-hydrochloride buffer (pH 7.5) containing 100 mM sodium chloride, 10 mM magnesium chloride, 1 mM DTT and 100 μg/ml BSA, 10 units each of the restriction enzymes *Eco*RI (Takara Shuzo) and *Spl*I (Takara Shuzo) were further added, and the reaction was allowed to proceed at 37°C for 1 hour. The reaction mixture was fractionated by agarose gel electrophoresis, and about 0.2 μg of an *Eco*RI-*Spl*I fragment (about 0.39 kb) of each replacement version of the human CDR-grafted anti-$GM_2$ antiobdy L chain V region was recovered.

Then, 0.1 μg of the above *Eco*RI-*Spl*I fragment of each replacement version of the human CDR-grafted anti-$GM_2$ antibody L chain V region and 0.1 μg of the *Eco*RI-*Spl*I fragment of pBSL3 were added to a total of 20 μl of sterilized water and ligated to each other using Ready-To-Go T4 DNA ligase (Pharmacia Biotech). The thus-obtained recombinant plasmid DNA solution was used to transform *Escherichia coli* HB101, and a plasmid DNA was prepared from a transformant clone, and the base sequence of said plasmid was determined. In this way, plasmids respectively containing a base sequence having a desired mutation or mutations were obtained.

Thus, a plasmid, pBSLV1, containing version 1, shown in SEQ ID NO:37, of the human CDR-grafted anti-$GM_2$ antibody L chain V region was obtained following the above procedure using the synthetic DNA of SEQ ID NO:35 as the mutant antisense primer and the synthetic DNA of SEQ ID NO:36 as the mutant sense primer. In the amino acid sequence of the version 1 human CDR-grafted anti-$GM_2$ antibody L chain V region, the amino acid valine in position 15 in the FR is replaced by proline that is found in the mouse antibody KM796 L chain V region. This is for the purpose of retaining the antigen-binding capacity of mouse antibody KM796.

A plasmid, pBSLV2, containing version 2, shown in SEQ ID NO:40, of the human CDR-grafted anti-$GM_2$ antibody L chain V region was obtained following the above procedure using the synthetic DNA of SEQ ID NO:38 as the mutant antisense primer and the synthetic DNA of SEQ ID NO:39 as the mutant sense primer. In the amino acid sequence of the version 2 human CDR-grafted anti-$GM_2$ antibody L chain V region, the amino acid leucine in positions 46 in the FR is replaced by tryptophan that is found in the mouse antibody KM796 L chain V region. This is for the purpose of retaining the antigen-binding capacity of mouse antibody KM796.

A plasmid, pBSLV3, containing version 3, shown in SEQ ID NO:43, of the human CDR-grafted anti-$GM_2$ antibody L chain V region was obtained following the above procedure using the synthetic DNA of SEQ ID NO:41 as the mutant antisense primer and the synthetic DNA of SEQ ID NO:42 as the mutant sense primer. In the amino acid sequence of the version 3 human CDR-grafted anti-$GM_2$ antibody L chain V region, proline in position 79 and isoleucine in position 82 in the FR are both replaced by alanine that is found in the mouse antibody KM796 L chain V region. This is for the purpose of retaining the antigen-binding capacity of mouse antibody KM796.

Then, a plasmid, pBSLV1+2, containing a human CDR-grafted anti-$GM_2$ antibody L chain V region having both the version 1 and version 2 replacements was constructed in the following manner.

Three μg of the plasmid pBSLV1 obtained as mentioned above was added to 10 μl of 10 mM Tris-hydrochloride buffer (pH 7.5) containing 50 mM sodium chloride, 10 mM magnesium chloride and 1 mM DTT, 10 units each of the restriction enzymes *Eco*RI (Takara Shuzo) and *Hin*dIII (Takara Shuzo) were further added, and the reaction was allowed to proceed at 37°C for 1 hour. The reaction mixture was fractionated by agarose gel electrophoresis, and about 0.2 μg of an EooRI-*Hin*dIII fragment about 0.20 kb in size was recovered.

Then, 3 μg of the plasmid pBSLV2 obtained as mentioned above was added to 10 μl of 10 mM Tris-hydrochloride buffer (pH 7.5) containing 50 mM sodium chloride, 10 mM magnesium chloride and 1 mM DTT, 10 units each of the restriction enzymes *Eco*RI (Takara Shuzo) and *Hin*dIII (Takara Shuzo) were further added, and the reaction was allowed to proceed at 37°C for 1 hour. The reaction mixture was fractionated by agarose gel electrophoresis, and about 1 μg of an *Eco*RI-*Hin*dIII fragment about 3.2 kb in size was recovered.

Then, 0.1 μg of the *Eco*RI-*Hin*dIII fragment of pBSLV1 and 0.1 μg of the *Eco*RI-*Hin*dIII fragment of pBSLV2, each obtained as mentioned above, were added to a total of 20 μl of sterilized water and ligated to each other using Ready-To-Go T4 DNA Ligase (Pharmacia Biotech). The thus-obtained recombinant plasmid DNA solution was used to transform *Escherichia coli* HB101, and the plasmid pBSLV1+2 shown in Fig. 34 was obtained.

Then, the PCR reaction procedure mentioned above was followed using 1 ng of the plasmid pBSLV1+2 obtained as mentioned above as the template, a synthetic DNA having the base sequence of SEQ ID NO:44 as the mutant antisense primer and a synthetic DNA having the base sequence of SEQ ID NO:45 as the mutant sense primer, whereby a plasmid, pBSLV4, containing a version 4 human CDR-grafted anti-$GM_2$ antibody L chain V region set forth in SEQ ID NO:46 was obtained. In the amino acid sequence of the version 4 human CDR-grafted anti-$GM_2$ antibody L chain V region, valine in position 15, leucine in position 46, aspartic acid in position 69, phenylalanine in position 70 and threonine in position 71 in the FR are replaced by proline, tryptophan, serine, tyrosine and serine, respectively, that are found in the mouse antibody KM796 L chain V region. This is for the purpose of retaining the antigen-binding capacity of mouse antibody KM796.

Then, the PCR reaction procedure mentioned above was followed using 1 ng of the plasmid pBSLV1+2 obtained as mentioned above as the template, a synthetic DNA having the base sequence of SEQ ID NO:47 as the mutant antisense primer and a synthetic DNA having the base sequence of SEQ ID NO:48 as the mutant sense primer, whereby a plasmid, pBSLV8, containing a version 8 human CDR-grafted anti-$GM_2$ antibody L chain V region set forth in SEQ ID NO:49 was obtained. In the amino acid sequence of the version 8 human CDR-grafted anti-$GM_2$ antibody L chain V region, valine in position 15, leucine in position 46, aspartic acid in position 69, phenylalanine in position 70, threonine in position 71, serine in position 76, leucine in position 77 and glutamine in position 78 in the FR are replaced by proline, tryptophan, serine, tyrosine, serine, arginine, methionine and glutamic acid, respectively, that are found in the mouse antibody KM796 L chain V region. This is for the purpose of retaining the antigen-binding capacity of mouse antibody KM796.

Then, the PCR reaction procedure mentioned above was followed using 1 ng of the plasmid pBSLV4 obtained as mentioned above as the template, a synthetic DNA having the base sequence of SEQ ID NO:50 as the mutant antisense primer and a synthetic DNA having the base sequence of SEQ ID NO:51 as the mutant sense primer, whereby a plasmid, pBSLm-2, containing a version Lm-2 human CDR-grafted anti-$GM_2$ antibody L chain V region set forth in SEQ ID NO:52 was obtained. In the amino acid sequence of the version Lm-2 human CDR-grafted anti-$GM_2$ antibody L chain V region, valine in position 15, tyrosine in position 35, leucine in position 46, aspartic acid in position 69, phenylalanine in position 70 and threonine in position 71 in the FR are replaced by proline, phenylalanine, tryptophan, serine, tyrosine and serine, respectively, that are found in the mouse antibody KM796 L chain V region. This is for the purpose of retaining the antigen-binding capacity of mouse antibody KM796.

Then, the PCR reaction procedure mentioned above was followed using 1 ng of the plasmid pBSLV4 obtained as mentioned above as the template, a synthetic DNA having the base sequence of SEQ ID NO:53 as the mutant antisense primer and a synthetic DNA having the base sequence of SEQ ID NO:54 as the mutant sense primer, whereby a plasmid, pBSLm-8, containing a version Lm-8 human CDR-grafted anti-$GM_2$ antibody L chain V region set forth in SEQ ID NO:55 was obtained. In the amino acid sequence of the version Lm-8 human CDR-grafted anti-$GM_2$ antibody L chain V region, valine in position 15, leucine in position 46, aspartic acid in position 69, phenylalanine in position 70, threonine in position 71, phenylalanine in position 72 and serine in position 76 in the FR are replaced by proline, tryptophan, serine, tyrosine, serine, leucine and arginine, respectively, that are found in the mouse antibody KM796 L chain V region. This is for the purpose of retaining the antigen-binding capacity of mouse antibody KM796.

Then, a plasmid, pBSLm-28, containing a human CDR-grafted anti-$GM_2$ antibody L chain V region having both the version Lm-2 and version Lm-8 replacements was constructed in the following manner.

Three µg of the plasmid pBSLm-2 obtained as mentioned above was added to 10 µl of 50 mM Tris-hydrochloride buffer (pH 7.5) containing 100 mM sodium chloride, 10 mM magnesium chloride and 1 mM DTT, 10 units of the restriction enzyme *Eco*RI (Takara Shuzo) was further added, and the reaction was allowed to proceed at 37°C for 1 hour. The reaction mixture was subjected to ethanol precipitation, the precipitate was added to 10 µl of 10 mM Tris-hydrochloride buffer (pH 7.5) containing 50 mM sodium chloride, 10 mM magnesium chloride, 1 mM DTT and 100 µg/ml BSA, 10 units of the restriction enzyme *Xba*I (Takara Shuzo) was further added, and the reaction as allowed to proceed at 37°C for 1 hour. The reaction mixture was fractionated by agarose gel electrophoresis, and about 0.2 µg of an *Eco*RI-*Xba*I fragment about 0.24 kb in size was recovered.

Then, 3 µg of the plasmid pBSLm-8 obtained as mentioned above was added to 10 µl of 50 mM Tris-hydrochloride buffer (pH 7.5) containing 100 mM sodium chloride, 10 mM magnesium chloride and 1 mM DTT, 10 units of the restriction enzyme *Eco*RI (Takara Shuzo) was further added, and the reaction was allowed to proceed at 37°C for 1 hour. The reaction mixture was subjected to ethanol precipitation, the precipitate was added to 10 µl of 10 mM Tris-hydrochloride buffer (pH 7.5) containing 50 mM sodium chloride, 10 mM magnesium chloride, 1 mM DTT and 100 µg/ml BSA, 10 units of the restriction enzyme *Xba*I (Takara Shuzo) was further added, and the reaction was allowed to proceed at 37°C for 1 hour. The reaction mixture was fractionated by agarose gel electrophoresis, and about 1 µg of an *Eco*RI-*Xba*I fragment about 3.16 kb in size was recovered.

Then, 0.1 µg of the *Eco*RI-*Xba*I fragment of pBSLm-2 and 0.1 µg of the *Eco*RI-*Xba*I fragment of pBSLm-8, each obtained as mentioned above, were added to a total of 20 µl of sterilized water and ligated to each other using Ready-To-go T4 DNA Ligase (Pharmacia Biotech). The thus-obtained recombinant plasmid DNA solution was used to transform *Escherichia coli* HB101, and the plasmid pBSLm-28 shown in Fig. 35 was obtained. The version Lm-28 human CDR-grafted anti-$GM_2$ antibody L chain V region contained in the plasmid pBSLm-28 is shown in SEQ ID NO: 8. In the amino acid sequence of the version Lm-28 human CDR-grafted anti-$GM_2$ antibody L chain V region thus constructed, valine in position 15, tyrosine in position 35, leucine in position 46, aspartic acid in position 69, phenylalanine in position 70, threonine in position 71, phenylalanine in position 72 and serine in position 76 are replaced by proline, phenylalanine, tryptophan, serine, tyrosine, serine, leucine and arginine, respectively, that are found in the mouse antibody KM796 L chain V region. This is for the intended purpose of retaining the antigen-binding capacity of mouse antibody KM796.

(4) Construction of human CDR-grafted anti-$GM_2$ antibody L chain V region using known HMHCS of human antibody L chain V region

According to Kabat *et al*. (Kabat E. A. *et al*., *"Sequences of Proteins of Immunological Interest*", US Dept. of Health and Human Services, 1991), known human antibody L chain V regions are classifiable into subgroups I to IV based on the homology of their FR regions, and common sequences have been identified for respective subgroups. The present inventors identified HMHCS as one meaning from the common sequences, a human CDR-grafted anti-$GM_2$ antibody L chain V region was constructed based on the HMHCS. First, for selecting common sequences to serve as the base, the homology was examined between the FR of the mouse antibody KM796 L chain V region and the FR of the HMHCS of the human antibody L chain V region of each subgroup (Table 2).

TABLE 2

| Homology (%) between mouse antibody KM796 L chain V region FR and human antibody L chain V region common sequence FR | | | |
|---|---|---|---|
| HSG I | HSG II | HSG III | HSG IV |
| 70.0 | 65.0 | 68.8 | 67.5 |

As a result, it was confirmed that subgroup I shows the greatest similarity. Thus based on the common sequence of subgroup I, a human CDR-grafted anti-$GM_2$ antibody L chain V region was constructed by the PCR method in the following manner.

Synthetic DNAs respectively having the base sequences of SEQ ID NO:56 through SEQ ID NO:61 were synthesized using an automatic DNA synthesizer (Applied Systems model 380A). The DNAs synthesized were added, each to a final concentration of 0.1 μM, to 50 μl of 10 mM Tris-hydrochloride buffer (pH 8.3) containing 50 mM potassium chloride, 1.5 mM magnesium chloride, 0.001% gelatin, 200 μM dNTP, 0.5 μM M13 primer RV (Takara Shuzo), 0.5 μM M13 primer M4 (Takara Shuzo) and 2 units of TaKaRa Taq DNA polymerase. The mixture was covered with 50 μl of mineral oil, a DNA thermal cycler (Perkin Elmer model PJ480) was loaded with the mixture, and 30 PCR cycles (2 minutes at 94°C, 2 minutes at 55°C and 2 minutes at 72°C per cycle) were conducted. The reaction mixture was purified using QIAquick® PCR Purification Kit (Qiagen) and then made into a solution in 30 μl of 50 mM Tris-hydrochloride buffer (pH 7.5) containing 100 mM sodium chloride, 10 mM magnesium chloride, 1 mM DTT and 100 μg/ml BSA, 10 units each of the restriction enzymes *Eco*RI (Takara Shuzo) and *Spl*I (Takara Shuzo) were further added, and the reaction was allowed to proceed at 37°C for 1 hour. The reaction mixture was fractionated by agarose gel electrophoresis, and about 0.2 μg of an *Eco*RI-*Spl*I fragment about 0.39 kb in size was recovered.

Then, 0.1 μg of the above *Eco*RI-*Spl*I fragment of the human CDR-grafted anti-$GM_2$ antibody L chain V region and 0.1 μg of the *Eco*RI-*Spl*I fragment of pBSL3 were added to a total of 20 μl of sterilized water and ligated to each other using Ready-To-Go T4 DNA Ligase (Pharmacia Biotech). The thus-obtained recombinant plasmid DNA solution was used to transform *Escherichia coli* HB101. Plasmid DNAs were prepared from 10 transformant clones and their base sequences were determined. As a result, a plasmid, pBSHSGL, shown in Fig. 36 and having the desired base sequence was obtained. The amino acid sequence and base sequence of the human CDR-grafted anti-$GM_2$ antibody L chain V region contained in pBSHSGL are shown in SEQ ID NO:9. In the amino acid sequence of the thus-constructed human CDR-grafted anti-$GM_2$ antibody L chain V region, methionine in position 4, leucine in position 11, valine in position 15, tyrosine in position 35, alanine in position 42, leucine in position 46, aspartic acid in position 69, phenylalanine in position 70, threonine in position 71, leucine in position 77 and valine in position 103 in the FR as selected based on a computer model for the V region are replaced by leucine, methionine, proline, phenylalanine, serine, tryptophan, serine, tyrosine, serine, methionine and leucine, respectively, that are found in the mouse antibody KM796 L chain V region. This is for the intended purpose of retaining the antigen-binding capacity of mouse antibody KM796.

2. Activity evaluation of replacement versions of human CDR-grafted anti-$GM_2$ antibody in terms of transient expression

Various replacement version human CDR-grafted anti-$GM_2$ antibodies composed of the human CDR-grafted anti-$GM_2$ antibody H chain and L chain V regions constructed in Paragraphs 3 (1) through (4) of Example 3 and having various replacements were evaluated for activity in terms of transient expression in the following manner.

First, for evaluating the human CDR-grafted anti-$GM_2$ antibody H chain V regions having various replacements, expression vectors, pT796HCDRHV2, pT796HCDRHV4 and pT796HCDRH10, were constructed by replacing the mouse H chain V region of the mouse-human chimeric anti-$GM_2$ antibody transient expression vector pT796 obtained in Paragraph 1 (1) of Example 2 of JP-A-6-205694 with the human CDR-grafted anti-$GM_2$ antibody H chain V regions having various replacements, in the following manner. For comparison, an expression vector, pT796HCDR was constructed by replacing the mouse H chain V region of pT796 with the human CDR-grafted anti-$GM_2$ antibody H chain V region obtained in Paragraph 1 (1) of Example 2.

Three μg of the plasmid pT796 was added to 10 μl of 50 mM Tris-hydrochloride buffer (pH 7.5) containing 100 mM sodium chloride, 10 mM magnesium chloride, 1mM DTT and 100 μg/ml BSA, 10 units each of the restriction enzymes *Eco*RI (Takara Shuzo) and *Spl*I (Takara Shuzo) were further added, and the reaction was allowed to proceed at 37°C for 1 hour. The reaction mixture was fractionated by agarose gel electrophoresis, and about 1 μg of an *Eco*RI-*Spl*I fragment about 9.20 kb in size was recovered. Then, 3 μg of the plasmid pBSL16 obtained in Paragraph 1 (3) of Example 3 was added to 10 μl of 50 mM Tris-hydrochloride buffer (pH 7.5) containing 100 mM sodium chloride, 10 mM magnesium chloride, 1 mM DTT and 100 μg/ml BSA, 10 units each of the restriction enzymes *Eco*RI (Takara Shuzo) and *Spl*I (Takara Shuzo) were further added, and the reaction was allowed to proceed at 37°C for 1 hour. The reaction mixture was fractionated by agarose gel electrophoresis, and about 0.3 μg of an *EcoR*I-*Spl*I fragment about 0.39 kb in size was recovered.

Then, 0.1 μg of the *Eco*RI-*Spl*I fragment of pT796 and 0.1 μg of the *Eco*RI-*Spl*I fragment of pBSL16, each obtained as mentioned above, were added to a total of 20 μl of sterilized water and ligated to each other using Ready-To-Go T4 DNA Ligase (Pharmacia Biotech). The thus-obtained recombinant plasmid DNA solution was used to transform *Escherichia coli* HB101, and the plasmid pT796LCDR shown in Fig. 37 was obtained.

Then, 3 μg of the above plasmid pT796LCDR was added to 10 μl of 10 mM Tris-hydrochloride buffer (pH 7.5) containing 10 mM magnesium chloride and 1 mM DTT, 10 units of the restriction enzyme *Apa*I (Takara Shuzo) was further added, and the reaction was allowed to proceed at 37°C for 1 hour. The reaction mixture was subjected to ethanol precipitation, the precipitate was added to 10 μl of 50 mM Tris-hydrochloride buffer (pH 7.5) containing 100 mM sodium chloride, 10 mM magnesium chloride, 1 mM DTT, 100 μg/ml BSA and 0.01% Triton® X-100, 10 units of the restriction enzyme *Not*I (Takara Shuzo) was further added, and the reaction was allowed to proceed at 37°C for 1 hour. The reaction mixture was fractionated by agarose gel electrophoresis, and about 1 μg of an *Apa*I-*Not*I fragment about 9.11 kb in size was recovered.

Then, 0.1 μg of the human CDR-grafted anti-$GM_2$ antibody H chain V region obtained in Paragraph 1 (1) of Example 2 of JP-A-6-205694 or the replacement version 2 or 4 human CDR-grafted anti-$GM_2$ antibody H chain V region obtained in Paragraph 1 (1) of Example 3 and 0.1 μg of the *Apa*I-*Not*I fragment of pT796LCDR were added to a total of 20 μl of sterilized water and ligated to each other using Ready-To-Go T4 DNA Ligase (Pharmacia Biotech). Each recombinant plasmid DNA solution thus obtained was used to transform Escherichia *coli* HB101. The plasmids pT796HLCDR, pT796HLCDRHV2 and pT796HLCDRHV4 shown in Fig. 38 were obtained.

Then, 3 μg of the plasmid pBSH10 obtained in Paragraph 1 (2) of Example 3 was added to 10 μl of 10 mM Tris-hydrochloride buffer (pH 7.5) containing 10 mM magnesium chloride and 1 mM DTT, 10 units of the restriction enzyme *Apa*I (Takara Shuzo) was further added, and the restriction was allowed to proceed at 37°C for 1 hour. The reaction mixture was subjected to ethanol precipitation, the precipitate was added to 10 μl of 50 mM Tris-hydrochloride buffer (pH 7.5) containing 100 mM sodium chloride, 10 mM magnesium chloride, 1 mM DTT, 100 μg/ml BSA and 0.01% Triton® X-100, 10 units of the restriction enzyme *Not*I (Takara Shuzo) was further added, and the reaction was allowed to proceed at 37°C for 1 hour. The reaction mixture was fractionated by agarose gel electrophoresis, and about 0.3 μg of an *Apa*I-*Not*I fragment about 0.44 kb in size was recovered.

Then, 0.1 μg of the *Apa*I-*Not*I fragment of pBSM10 and 0.1 μg of the *Apa*I-*Not*I fragment of pT796LCDR were added to a total of 20 μl of sterilized water and ligated to each other using Ready-To-Go T4 DNA Ligase (Pharmacia Biotech). The thus-obtained recombinant plasmid DNA solution was used to transform *Escherichia coli* HB101, and the plasmid pT796HLCDRH10 shown in Fig. 39 was obtained.

Then, 3 μg each of the plasmids pT796HLCDR, pT796HLCDRHV2, pT796HLCDRHV4 and pT796HLCDRH10 were respectively added to 10 μl of 50 mM Tris-hydrochloride buffer (pH 7.5) containing 100 mM sodium chloride, 10 mM magnesium chloride, 1 mM DTT and 100 μg/ml BSA, 10 units each of the restriction enzymes *Eco*RI (Takara Shuzo) and *Spl*I (Takara Shuzo) were further added, and the reaction was allowed to proceed at 37°C for 1 hour. Each reaction mixture was fractionated by agarose gel electrophoresis, and about 1 μg of an *Eco*RI-*Spl*I fragment about 9.15 kb in size was recovered.

Then, 5 μg of the plasmid pBSL3 obtained in Paragraph 1 (2) of Example 2 was added to 10 μl of 50 mM Tris-hydrochloride buffer (pH 7.5) containing 100 mM sodium chloride, 10 mM magnesium chloride, 1 mM DTT and 100 μg/ml BSA, 10 units each of the restriction enzymes *Eco*RI (Takara Shuzo) and *Spl*I (Takara Shuzo) were further added, and the reaction was allowed to proceed at 37°C for 1 hour. The reaction mixture was fractionated by agarose gel electrophoresis, and about 0.4 μg of an *Eco*RI-*Spl*I fragment about 0.39 kb in size was recovered.

Then, 0.1 μg of the *Eco*RI-*Spl*I fragment of each of pT796HLCDR, pT796HLCDRHV2, pT796HLCDRHV4 and pT796HLCDRH10 and 0.1 μg of the *Eco*RI-*Spl*I fragment of pBSL3 were added to a total of 20 μl of sterilized water and ligated to each other using Ready-To-Go DNA Ligase (Pharmacia Biotech). Each recombinant plasmid DNA solution thus obtained was used to transform *Escherichia coli* HB101. In this way, the plasmids pT796HCDR, pT796HCDRHV2, pT796HCDRHV4 and pT796HCDRH10 shown in Fig. 40 were obtained.

Then, 2 μg each of the plasmids pT796HCDR, pT796HCDRHV2, pT796HCDRHV4 and pT796HCDRH10 thus obtained were used for transient human CDR-grafted anti-$GM_2$ antibody expression and for culture supernatant human CDR-grafted anti-$GM_2$ antibody activity evaluation by the procedures described in Paragraphs 1 (5), 2 (2) and (3) of Example 2. After introduction of each plasmid, the culture supernatant was recovered at 72 hours, and the $GM_2$-binding activity and antibody concentration in the culture supernatant were determined by ELISA and the relative activity was calculated with the activity of the positive control chimera antibody taken as 100%. The results are shown in Fig. 41.

The results revealed that the amino acid residue replacements alone in replacement versions 2 and 4 have little influence on the restoration of the antigen-binding activity of the human CDR-grafted anti-$GM_2$ antibody but that the use of the pBSH10-derived human CDR-grafted antibody H chain V region constructed based on the known HMHCS of the human antiobdy H chain V region, contributes to the restoration of the antigen-binding activity.

In view of the above results, the human CDR-grafted anti-$GM_2$ antibody H chain V region constructed based on the known HMHCS of the human antibody H chain V region as shown in SEQ ID NO:7 was selected as a novel human CDR-grafted anti-$GM_2$ antibody H chain V region.

Then, for evaluating the human CDR-grafted anti-$GM_2$ antibody L chain V regions having various replacements, expression vectors, pT796HLCDRLV1, pT796HLCDRLV2, pT796HLCDRLV3, pT796HLCDRLV4, pT796HLCDRLV8, pT796HLCDRLm-2, pT796HLCDRLm-8, pT796HLCDRLm-28 and pT796HLCDRHSGL, were constructed in the following manner by replacing the mouse L chain V region of the vector pT796HCDRH10 for transient human CDR-grafted anti-$GM_2$ antibody expression obtained as mentioned above with the human CDR-grafted anti-$GM_2$ antibody L chain V regions having various replacements.

Thus, 3 μg of the plasmid pT796HCDRH10 was added to 10 μl of 50 mM Tris-hydrochloride buffer (pH 7.5) containing 100 mM sodium chloride, 10 mM magnesium chloride, 1 mM DTT and 100 μg/ml BSA, 10 units each of the restriction enzymes *Eco*RI (Takara Shuzo) and *Spl*I (Takara Shuzo) were further added, and the reaction was allowed to proceed at 37°C for 1 hour. The reaction mixture was fractionated by agarose gel electrophoresis, and about 1 μg of an *Eco*RI-*Spl*I fragment about 9.15 kb in size was recovered.

Then, 3 μg of the plasmid pBSLV1, pBSLV2, pBSLV3, pBSLV4, pBSLV8, pBSLm-2, pBSLm-8, pBSLm-28 or pBSHSGL obtained in Paragraph 1 (3) or (4) of Example 3 was added to 10 μl of 50 mM Tris-hydrochloride buffer (pH 7.5) containing 100 mM sodium chloride, 10 mM magnesium chloride, 1 mM DTT and 100 μg/ml BSA, 10 units each of the restriction enzymes *Eco*RI (Takara Shuzo) and *Spl*I (Takara Shuzo) were further added, and the reaction was allowed to proceed at 37°C for 1 hour. Each reaction mixture was fractionated by agarose gel electrophoresis, and about 0.3 μg of an *Eco*RI-*Spl*I fragment about 0.39 kb in size was recovered.

Then, 0.1 μg of the *Eco*RI-*Spl*I fragment of the pT796HCDRH10, and 0.1 μg of the *Eco*RI-*Spl*I fragment of each replacement version human CDR-grafted anti-$GM_2$ antibody L chain V region were added to a total of 20 μl of sterilized water and ligated to each other using Ready-To-Go T4 DNA Ligase (Pharmacia Biotech). Each recombinant plasmid DNA solution thus obtained was used to transform *Escherichia coli* HB101. In this way, the plasmids pT796HLCDRLV1, pT796HLCDRLV2, pT796HLCDRLV3, pT796HLCDRLV4, pT796HLCDRLV8, pT796HLCDRLm-2, pT796HLCDRLm-8, pT796HLCDRLm-28 and pT796HLCDRHSGL were obtained as shown in Fig. 42.

Then, 2 μg each of the thus-obtained plasmids pT796HLCDRLV1, pT796HLCDRLV2, pT796HLCDRLV3, pT796HLCDRLV4, pT796HLCDRLV8, pT796HLCDRLm-2, pT796HLCDRLm-8, pT796HLCDRLm-28 and pT796HLCDRHSGL and of the plasmid pT796HLCDR described in Example 2 of JP-A-6-205694 and capable of expressing human CDR-grafted anti-$GM_2$ antibody were used for transient human CDR-grafted anti-$GM_2$ antibody expression and for culture supernatant human CDR-grafted anti-$GM_2$ antibody activity evaluation by the procedures described in Paragraphs 1 (5) and 2 (2) and (3) of Example 2. After introduction of each plasmid, the culture supernatant was recovered at 72 hours, and the $GM_2$-binding activity and antibody concentration in the culture supernatant were determined by ELISA and the relative activity was calculated with the activity of the positive control chimera antibody taken as 100%. The results are shown in Fig. 43.

The results revealed that the amino acid residue replacements alone in replacement versions 1, 2, 3, 4 and 8 have little influence on the restoration of the antigen-binding activity of the human CDR-grafted anti-$GM_2$ antibody but that the amino acid residue replacements in replacement versions Lm-2 and Lm-8 contributes to the restoration of the antigen-binding activity. Furthermore, version Lm-28 having both the amino acid residue replacements of Lm-2 and Lm-8 showed a high level of antigen-biding activity almost comparable to that of the chimera antibody, revealing that those amino acid residues replaced in producing Lm-28 were very important from the antigen-binding activity viewpoint.

In view of the above results, the version Lm-28 human CDR-grafted anti-$GM_2$ antibody L chain V region shown in SEQ ID NO:8 was selected as a first novel human CDR-grafted anti-$GM_2$ antibody L chain V region.

It was further revealed that the antigen-binding activity can be restored when the pBSHSGL-derived human CDR-grafted anti-$GM_2$ antibody L chain V region, namely the human CDR-grafted anti-$GM_2$ antibody L chain V region constructed based on the known HMHCS of the human antibody L chain V region, is used.

In view of the above result, the human CDR-grafted anti-$GM_2$ antibody L chain V region constructed based on the known HMHCS of the human antibody L chain V region as set forth in SEQ ID NO:9 was selected as a second novel human CDR-grafted anti-$GM_2$ antibody L chain V region.

It is to be noted that in those human CDR-grafted anti-$GM_2$ antibody L chain V regions that showed high binding activity against $GM_2$, certain amino acid residues which cannot be specified by deduction from known human CDR-grafted antibody production examples have been replaced by amino acid residues found in the mouse L chain V region. Thus, obviously, it was very important, in human CDR-grafted anti-$GM_2$ antibody production, to identify these amino acid residues.

Furthermore, the fact that the human CDR-grafted anti-$GM_2$ antibodies having those human CDR-grafted anti-$GM_2$ antibody H chain and L chain V regions based on the known HMHCS of the human antibody V region showed high antigen binding activity is proof of the usefulness of the present process in human CDR-grafted antibody production.

3. Acquisition of cell lines for stable production of human CDR-grafted anti-$GM_2$ antibodies

Based on the results of Paragraph 2 (5) of Example 3, two cell lines, KM8966 and KM8967, capable of stably expressing KM8966, which has the amino acid sequence set forth in SEQ ID NO:7 as the H chain V region and the amino acid sequence set forth in SEQ ID NO:8 as the L chain V region, and KM8967, which has the amino acid sequence set forth in SEQ ID NO:7 as the H chain V region and the amino acid sequence set forth in SEQ ID NO:9 as the L chain V region, respectively as human CDR-grafted anti-$GM_2$ antibodies having higher antigen-binding activity than the human CDR-grafted anti-$GM_2$ antibody described in Example 2 of JP-A-6-205694 were obtained in the following manner.

Three μg each of the plasmids pT796HLCDRLm-28 and pT796HLCDRHSGL obtained in Paragraph 2 (5) of Example 3 were respectively added to 10 μl of 20 mM Tris-hydrochloride buffer (pH 8.5) containing 100 mM potassium chloride, 10 mM magnesium chloride and 1 mM DTT, 10 units of the restriction enzyme *Bam*HI (Takara Shuzo) was further added, and the reaction was allowed to proceed at 37°C for 1 hour. Each reaction mixture was subjected to ethanol precipitation, the precipitate was added to 10 μl of 50 mM Tris-hydrochloride buffer (pH 7.5) containing 100 mM sodium chloride, 10 mM magnesium chloride and 1 mM DTT, 10 units of the restriction enzyme *Xho*I (Takara Shuzo) was further added, and the reaction was allowed to proceed at 37°C for 1 hour. Each reaction mixture was fractionated by agarose gel electrophoresis, and about 1 μg of a *Bam*HI-*Xho*I fragment about 4.93 kb in size was recovered.

Then, 3 μg of the plasmid pKANTEX93 obtained in Example 1 was added to 10 μl of 20 mM Tris-hydrochloride buffer (pH 8.5) containing 100 mM potassium chloride, 10 mM magnesium chloride and 1 mM DTT, 10 units of the restriction enzyme *Bam*HI (Takara Shuzo) was further added, and the reaction was allowed to proceed at 37°C for 1 hour. The reaction mixture was subjected to ethanol precipitation, the precipitate was added to 10 μl of 50 mM Tris-hydrochloride buffer (pH 7.5) containing 100 mM sodium chloride, 10 mM magnesium chloride and 1 mM DTT, 10 units of the restriction enzyme *Xho*I (Takara Shuzo) was further added, and the reaction was allowed to proceed at 37°C for 1 hour. The reaction mixture was fractionated by agarose gel electrophoresis, and about 1 μg of a *Bam*HI-*Xho*I fragment about 8.68 kb in size was recovered.

Then, 0.1 μg of the *Bam*HI-*Xho*I fragment of pT796HLCDRLm-28 or pT796HLCDRHSGL and 0.1 μg of the *Bam*HI-*Xho*I fragment of pKANTEX93, each obtained as mentioned above, were added to a total of 20 μl of sterilized water and ligated to each other using Ready-To-Go T4 DNA Ligase (Pharmacia Biotech). Each recombinant plasmid DNA solution thus obtained was used to transform *Escherichia coli* HB101. In this way, the plasmids pKANTEX796HLCDRLm-28 and pKANTEX796HLCDRHSGL shown in Fig. 44 were obtained.

Then, 4 μg each of the above plasmids pKANTEX796HLCDRLm-28 and pKANTEX796HLCDRHSGL were respectively used to transform YB2/0 (ATCC CRL 1581) cells according to the procedure described in Paragraph 1 (4) of Example 2 and, after final selection using G418 (0.5 mg/ml) and MTX (200 nM), a transformant cell line, KM8966, capable of producing about 40 μg/ml of KM8966, i.e. the pKANTEX796HLCDRLm-28-derived human CDR-grafted anti-$GM_2$ antibody, and a transformant cell line, KM8967, capable of producing about 30 μg/ml of KM8967, i.e. the pKANTEX796HLCDRHSGL-derived human CDR-grafted anti-$GM_2$ antibody, were obtained.

The transformants KM8966 and KM8967 have been deposited with National Institute of Bioscience and Human-Technology, Agency of Industrial Science and Technology (Higashi 1-1-3, Tsukuba, Ibaraki, Japan; hereinafter the address is the same as this) on May 23, 1995 under the deposit numbers FERM BP-5105, and FERM BP-5106, respectively.

4. Purification of human CDR-grafted anti-$GM_2$ antibodies KM8966 and KM8967

The transformant cell lines KM8966 and 8967 obtained in Paragraph 3 of Example 3 were respectively suspended in GIT medium (Nippon Pharmaceutical) containing 0.5 mg/ml G418 and 200 nM MTX and, according to the procedure of Paragraph 11 of Example 1 of JP-A-6-205694, 18 mg of purified-human CDR-grafted anti-$GM_2$ antibody KM8966 and 12 mg of purified KM8967 were obtained each from about 0.5 liter of culture fluid. Three μg each of the purified human CDR-grafted anti-$GM_2$ antibodies obtained and the mouse-human chimeric anti-$GM_2$ antibody KM966 were subjected to electrophoresis by the known method [Laemli, U.K., *Nature*, 227, 680 (1979)] for molecular weight determination. The results are shown in Fig. 45. As shown in Fig. 45, under reducing conditions, both antibody H chains showed a molecular weight of about 50 kilodaltons and both antibody L chains showed a molecular weight of about 25 kilodaltons. Expression of H and L chains of correct molecular weights was thus confirmed. Under nonreducing conditions, both human CDR-grafted anti-$GM_2$ antibodies showed a molecular weight of about 150 kilodaltons and it was thus confirmed that antibodies each composed of two H chains and two L chains and having a correct size had been expressed. Furthermore, the H and L chains of each human CDR-grafted anti-$GM_2$ antibody were analyzed for N-terminal amino acid sequence by automatic Edman degradation using a protein sequencer (Applied Biosystems model 470A), whereby an amino acid sequence deducible from the base sequence of the V region DNA constructed was revealed.

5. *In vitro* reactivity of human CDR-grafted anti-$GM_2$ antibodies KM8966 and KM8967 against $GM_2$

The mouse-human chimeric anti-$GM_2$ antibody KM966 and the purified human CDR-grafted anti-$GM_2$ antibodies KM8966 and KM8967 were tested for reactivity against $GM_2$ by ELISA as described in Paragraph 1 (5) of Example 2. The results are shown in Fig. 46. $GM_2$ (N-acetyl-$GM_2$) used was purified from cultured cell line HPB-ALL [Oboshi *et al*., *Tanpakushitsu*, *Kakusan & Koso* (*Protein*, *Nucleic Acid & Enzyme*), 23, 697 (1978)] in accordance with the known method [*J. Biol. Chem*., 263, 10915 (1988)]. As shown, it was found that the purified human CDR-grafted anti-$GM_2$ antibody KM8966 exerted the binding activity comparable to that of the mouse-human chimeric anti-$GM_2$ antibody KM966. On the other hand, the binding activity of purified human CDR-grafted anti-$GM_2$ antibody KM8967 was about 1/4 to 1/5 of that of the mouse-human chimeric anti-$GM_2$ antibody KM966.

6. Reaction specificity of human CDR-grafted anti-$GM_2$ antibodies KM8966 and KM8967

The mouse-human chimeric anti-$GM_2$ antibody KM966 and the human CDR-grafted anti-$GM_2$ antibodies KM8966 and KM8967 were tested for reactivity against the gangliosides $GM_1$, N-acetyl-$GM_2$, N-glycolyl-$GM_2$, N-acetyl-$GM_3$, N-glycolyl-$GM_3$, $GD_{1a}$, $GD_{1b}$ (Iatron), $GD_2$, $GD_3$ (Iatron) and $GQ_{1b}$ (Iatron) by ELISA as described in Paragraph 1 (5) of Example 2. The results are shown in Fig. 47. $GM_1$ and $GD_{1a}$ were purified from bovine brain, N-acetyl-$GM_2$ from cultured cell line HPB-ALL [Oboshi *et al., Tanpakushitsu*, *Kakusan & Koso* (*Protein, Nucleic acid & Enzyme*), 23, 697 (1978)], N-glycolyl-$GM_2$ and N-glycolyl-$GM_3$ from mouse liver, N-acetyl-$GM_3$ canine erythrocytes, and $GD_2$ from cultured cell line IMR32 (ATCC CCL127), respectively by the per se known method [*J. Biol. Chem.*, 263, 10915 (1988)]. Each antibody was used in a concentration of 10 μg/ml.

As shown in Fig. 47, it was confirmed that the human CDR-grafted anti-$GM_2$ antibodies KM8966 and KM8967 react specifically with $GM_2$ (N-acetyl-$GM_2$ and N-glycolyl-$GM_2$) like the mouse-human chimeric anti-$GM_2$ antibody KM966.

7. Reactivity of human CDR-grafted anti-$GM_2$ antibodies KM8966 and KM8967 against cancer cells

The human lung small cell carcinoma culture cell line SBC-3 (JCRB 0818) ($1 \times 10^6$ cells) was suspended in PBS, the suspension was placed in a microtube (TREF) and centrifuged (1200 rpm, 2 minutes). To the thus-washed cells was added 50 μl (50 μg/ml) of the mouse-human chimeric anti-$GM_2$ antibody KM966 or the purified human CDR-grafted anti-$GM_2$ antibody KM8966 or KM8967, followed by stirring and 1 hour of standing at 4°C. After the above reaction step, the cells were washed three times with PBS, each time followed by centrifugation. Then, 20 μl of fluorescein isocyanate-labeled protein A (30-fold dilution, Boehringer Mannheim) was added and, after stirring, the reaction was allowed to proceed at 4°C for 1 hour. Thereafter, the cells were washed three times with PBS, each time followed by centrifugation, then further suspended in PBS and subjected to analysis using a flow cytometer, EPICS Elite (Coulter). In a control run, the above procedure was followed without addition of the human CDR-grafted anti-$GM_2$ antibody and analyzed. The results are shown in Fig. 48. It was found that the purified human CDR-grafted anti-$GM_2$ antibodies KM8966 and KM8967 strongly reacted with the human lung small cell carcinoma culture cell line SBC-3 like the mouse-human chimeric anti-$GM_2$ antibody KM966.

8. *In vitro* antitumor activity of human CDR-grafted anti-$GM_2$ antibodies KM8966 and KM8967: CDC activity

(1) Preparation of target cells

The target cells SBC-3, cultured in RPMI1640-FCS (10) medium supplemented with 10% FCS, were adjusted to a cell concentration of $5 \times 10^6$ cells/500 μl, 3.7 MBq of $Na_2{}^{51}CrO_4$ (Daiichi Pure Chemicals Co., Ltd.) was added thereto. Then, the reaction was allowed to proceed at 37°C for 1 hour, and the cells were washed three times with the medium. The cells were then allowed to stand in the medium at 4°C for 30 minutes and, after centrifugation, the medium was added to adjust the cell concentration to $1 \times 10^6$ cells/ml.

(2) Preparation of the complement

Sera from healthy subjects were combined and used as a complement source.

(3) CDC activity measurement

The mouse-human chimeric anti-$GM_2$ antibody KM966 or purified human CDR-grafted anti-$GM_2$ antibody KM8966 or KM8967 was added to wells of 96-well U-bottom plates within the final concentration range of 0.05 to 50 μg/ml and then 50 μl ($5 \times 10^4$ cells/well) of the target cells prepared in (1) were added to each well. The reaction was allowed to proceed at room temperature for 1 hour. After centrifugation, the supernatants were discarded, the human complement obtained in (2) was added to each well to give a final concentration of 15% v/v, and the reaction was allowed to proceed at 37°C for 1 hour. After centrifugation, the amount of $^{51}Cr$ in each supernatant was determined using a gamma counter. The amount of spontaneously dissociated $^{51}Cr$ was determined by adding to the target cells the medium alone in stead of the antibody and complement solutions and measuring the amount of $^{51}Cr$ in the supernatant in the same manner as mentioned above. The total amount of dissociated $^{51}Cr$ was determined by adding to the target cells 1 N hydrochloric acid in stead of the antibody and complement solutions and measuring the amount of $^{51}Cr$ in the supernatant in the same manner as mentioned above. The CDC activity was calculated as follows:

$$\text{CDC activity (\%)} = \frac{\text{Amount of } ^{51}\text{Cr in sample supernatant - Amount of } ^{51}\text{Cr spontaneously dissociated}}{\text{Total amount of } ^{51}\text{Cr dissociated - Amount of } ^{51}\text{Cr spontaneously dissociated}} \times 100$$

The results thus obtained are shown in Fig. 49. It was shown that CDC activity of the human CDR-grafted anti-$GM_2$ antibodies KM8966 and KM8967- was lower than that of the mouse-human chimeric anti-$GM_2$ antibody KM966. 9. *In vitro* antitumor activity of human CDR-grafted anti-$GM_2$ antibodies KM8966 and KM8967: ADCC activity

(1) Preparation of target cells

The target cells SBC-3 cultured in RPMI1640-FCS (10) medium supplemented with 10% FCS were adjusted to a cell concentration of $1 \times 10^6$ cells/500 μl, 3.7 MBq of $Na_2{}^{51}CrO_4$ (Daiichi Pure Chemicals Co., Ltd.) was added thereto. Then, the reaction was allowed to proceed at 37°C for 1 hour and the cells were washed three times with the medium. The cells were then allowed to stand in the medium at 4°C for 30 minutes and then, after centrifugation, the medium was added to adjust the cell concentration to $2 \times 10^5$ cells/ml.

(2) Preparation of effector cells

Human venous blood (50 ml) was collected, 0.5 ml of heparin sodium (Takeda Chemical Industries; 1,000 units/ml) was added, and the mixture was gently stirred. This mixture was overlaid on Polymorphprep® (Nycomed) and centrifuged to separate the lymphocyte layer (PBMC). The resulting lymphocytes were washed three times by centrifugation with RPMI1640 medium supplemented with 10% FCS, and the cells were suspended in the medium ($5 \times 10^6$ cells/ml) for use as effector cells.

(3) ADCC activity measurement

The mouse-human chimeric anti-$GM_2$ antibody KM966 or purified human CDR-grafted anti-$GM_2$ antibodies KM8966 or KM8967 was added to wells of 96-well U-bottom plates within the final concentration range of 0.05 to 50 μg/ml and then 50 μl ($1 \times 10^4$ cells/well) of the target cell suspension prepared in (1) and 100 μl ($5 \times 10^5$ cells/well) of the effector cell suspension prepared in (2) were added to each well. The reaction was allowed to proceed at 37°C for 4 hours and, after centrifugation, the amount of $^{51}Cr$ in each supernatant was measured using a gamma counter. The amount of spontaneously dissociated $^{51}Cr$ was determined by adding to the target cells the medium alone in lieu of the antibody and effector cells and measuring the amount of $^{51}Cr$ in the supernatant in the same manner as mentioned above. The total amount of dissociated $^{51}Cr$ was determined by adding to the target cells 1 N hydrochloric acid in lieu of the antibody and effector cells and measuring the amount of $^{51}Cr$ in the supernatant in the same manner as mentioned above. The ADCC activity was calculated as follows:

$$\text{ADCC activity (\%)} = \frac{\text{Amount of } ^{51}\text{Cr in sample supernatant - Amount of } ^{51}\text{Cr spontaneously dissociated}}{\text{Total amount of } ^{51}\text{Cr dissociated - Amount of } ^{51}\text{Cr spontaneously dissociated}} \times 100$$

The results thus obtained are shown in Fig. 50. The human CDR-grafted anti-$GM_2$ antibody KM8966 showed ADCC activity comparable to that of the mouse-human chimeric anti-$GM_2$ antibody KM966, whereas the human CDR-grafted anti-$GM_2$ antibody KM8967 showed ADCC activity slightly lower than that of the mouse-human chimeric an-

ti-$GM_2$ antibody KM966.

EXAMPLE 4

Production of human CDR-grafted anti-$GM_2$ antibodies II

The human CDR-grafted anti-$GM_2$ antibodies KM8966 and KM8967 showed antigen binding activity (ELISA), binding specificity and ADCC activity comparable to those of the mouse-human chimeric anti-$GM_2$ antibody KM966, while its CDC activity was lower than that of the chimeric antibody. In order to improve the CDC activity, human CDR-grafted anti-$GM_2$ antibodies were produced in the following manner.

1. Modification of human CDR-grafted anti-$GM_2$ antibody KM8966 H chain V region

Among the human CDR-grafted anti-$GM_2$ antibodies prepared in Example 3, the antibody KM8966 showing higher CDC activity was subjected to amino acid residue replacements at the H chain V region (SEQ ID NO:7) in order to improve CDC activity. The amino acid residues to be replaced were selected at random with reference to the results of various replacement obtained in Example 3 and a computer model for the V region of mouse antibody KM796. Replacements were introduced by PCR method using as a template 1 ng of the plasmid pBSH10 containing the human CDR-grafted anti-$GM_2$ antibody H chain V region obtained in Paragraph 1 (2) of Example 3 and using as a primer antisense and sense synthetic DNA containing mutations described in Paragraph 1 (3) of Example 3.

The reaction was carried out in the same manner as described in Paragraph 1 (3) of Example 3 using the synthetic DNA of SEQ ID NO:62 as the mutant antisense primer and the synthetic DNA of SEQ ID NO:63 as the mutant sense primer to obtain the plasmid pBSHM1 containing version HM1, shown in SEQ ID NO:64, of the human CDR-grafted anti-$GM_2$ antibody H chain V region. In the amino acid sequence of the version HM1, arginine in position 38, alanine in position 40, glutamine in position 43 and glycine in position 44 in the FR shown in SEQ ID NO:7 were replaced by lysine, serine, lysine and serine, respectively, that are found in the mouse antibody KM796 H chain V region.

The plasmid pBSHM2 containing version HM2, shown in SEQ ID NO:10, of the human CDR-grafted anti-$GM_2$ antibody H chain V region was obtained following the reaction described in Paragraph 1 (3) of Example 3 using the synthetic DNA of SEQ ID NO:65 as the mutant antisense primer and the synthetic DNA of SEQ ID NO: 66 as the mutant sense primer. In the amino acid sequence of the version HM2, arginine in position 38 and alanine in position 40 in the FR shown in SEQ ID NO:7 were replaced by lysine and serine, respectively, that are found in the mouse antibody KM796 H chain V region.

The plasmid pBSHM3 containing version HM3, shown in SEQ ID NO:69, of the human CDR-grafted anti-$GM_2$ antibody H chain V region was obtained following the reaction described in Paragraph 1 (3) of Example 3 using the synthetic DNA of SEQ ID NO:67 as the mutant antisense primer and the synthetic DNA of SEQ ID NO:68 as the mutant sense primer. In the amino acid sequence of the version HM3, valine in position 68 and isoleucine in position 70 in the FR shown in SEQ ID NO:7 were replaced by alanine and leucine, respectively, that are found in the mouse antibody KM796 H chain V region.

The plasmid pBSHM31 containing version HM31, shown in SEQ ID NO:70, of the human CDR-grafted anti-$GM_2$ antibody H chain V region was obtained following the reaction described in Paragraph 1 (3) of Example 3 using 1 ng of the plasmid pBSHM3 as the template, the synthetic DNA of SEQ ID NO:62 as the mutant antisense primer and the synthetic DNA of SEQ ID NO:63 as the mutant sense primer. In the amino acid sequence of the version HM31, arginine in position 38, alanine in position 40, glutamine in position 43 and glycine in position 44 in the FR of the version HM3 were replaced by lysine, serine, lysine and serine, respectively, that are found in the mouse antibody KM796 H chain V region.

Further, the plasmid pBSHM32 containing version HM32, shown in SEQ ID NO:71, of the human CDR-grafted anti-$GM_2$ antibody H chain V region was obtained following the reaction described in Paragraph 1 (3) of Example 3 using 1 ng of the plasmid pBSHM3 as the template, the synthetic DNA of SEQ ID NO:65 as the mutant antisense primer and the synthetic DNA of SEQ ID NO:66 as the mutant sense primer. In the amino acid sequence of the version HM32, arginine in position 38 and alanine in position 40 in the FR of the version HM3 were replaced by lysine and serine, respectively, that are found in the mouse antibody KM796 H chain V region.

2. Evaluation of CDC activity of human CDR-grafted anti-$GM_2$ antibodies having various replacements in the human CDR-grafted anti-$GM_2$ antibody H chain V region

(1) Construction of expression vectors

Expression vectors for various human CDR-grafted anti-$GM_2$ antibodies containing the H chain V region of human CDR-grafted anti-$GM_2$ antibodies having various replacements obtained in Paragraph 1 of Example 4 and the L chain V region of KM8966 (SEQ ID NO:8) were prepared in the following manner.

Three µg each of the plasmids pBSHM1, pBSHM2, pBSHM3, pBSHM31 and pBSHM32 obtained in Paragraph 1 of Example 4 were dissolved in 10 µl of 10 mM Tris-hydrochloride buffer (pH 7.5) containing 10 mM magnesium chloride and 1 mM DTT, 10 units of *Apa*I (Takara Shuzo) were added thereto and the mixture was allowed to react at 37°C for 1 hour. The resulting mixture was subjected to ethanol precipitation and the thus-obtained precipitate was dissolved in 10 µl of 50 mM Tris-hydrochloride buffer (pH 7.5) containing 100 mM sodium chloride, 10 mM magnesium chloride, 1 mM DTT, 100 µg/ml BSA and 0.01% of Triton® X-100. Ten units of *Not*I (Takara Shuzo) were further added thereto to allow the mixture to react at 37°C for 1 hour. The reaction mixture was fractionated by agarose gel electrophoresis to recover about 0.2 µg of the ApaI-NotI fragment of about 0.44 kb.

Then, 3 µg of the plasmid pKANTEX796HLCDRLm-28 obtained in Paragraph 3 (3) of Example 3 was dissolved in 10 µl of 10 mM Tris-hydrochloride buffer (pH 7.5) containing 10 mM magnesium chloride and 1 mM DTT, 10 units of *Apa*I (Takara Shuzo) were added thereto and the mixture was allowed to react at 37°C for 1 hour. The resulting mixture was subjected to ethanol precipitation and the thus-obtained precipitate was dissolved in 10 µl of 50 mM Tris-hydrochloride buffer (pH 7.5) containing 100 mM sodium chloride, 10 mM magnesium chloride, 1 mM DTT, 100 µg/ml BSA and 0.01% of Triton® X-100. 10 units of *Not*I (Takara Shuzo) were added thereto to allow the mixture to react at 37°C for 1 hour. The reaction mixture was fractionated by agarose gel electrophoresis to recover about 1 µg of the *Apa*I-*Not*I fragment of about 13.14 kb.

About 0.1 µg each of the thus-obtained *Apa*I-*Not*I fragment of pBSHM1, pBSHM2, pBSHM3, pBSHM31 and pBSHM32 and 0.1 µg of the *Apa*I-*Not*I fragment of pKANTEX796HLCDRLm-28 were added in a total of 20 µl of sterilized water and ligated to each other using Ready-To-Go T4 DNA Ligase (Pharmacia Biotech). Each of the resulting recombinant plasmid DNA solutions was used to transform *Escherichia coli* HB101 and plasmids, pKANTEX796HM1Lm-28, pKANTEX796HM2Lm-28, pKANTEX796HM3Lm-28, pKANTEX796HM31Lm-28 and pKANTEX796HM32Lm-28 shown in Fig. 51 were obtained.

(2) Expression of replacement versions of human CDR-grafted anti-$GM_2$ antibodies

Four µg each of the plasmids pKANTEX796BM1Lm-28, pKANTEX796HM2Lm-28, pKANTEX796HM3Lm-28, pKANTEX796HM31Lm-28 and pKANTEX796HM32Lm-28 obtained in Paragraph 2 (1) of Example 4 were used to transform YB2/0 cells (ATCC CRL 1581) in accordance with the method as described in Paragraph 1 (4) of Example 2. The cells were ultimately selected using G418 (0.5 mg/ml) and MTX (200 nM) to obtain about 2 to 5 µg/ml of transformants capable of producing human CDR-grafted anti-$GM_2$ antibodies derived from the corresponding expression vectors.

(3) Purification of replacement versions of human CDR-grafted anti-$GM_2$ antibodies

Cells of each transformant obtained in Paragraph 2 (2) of Example 4 were suspended in GIT medium (Nihon Pharmaceutical) containing 0.5 mg/ml G418 and 200 nM MTX and about 1 to 3 mg of purified human CDR-grafted anti-$GM_2$ antibodies were obtained from about 0.6 liter of the culture broth in accordance with the method described in Paragraph 11 of Example 1 of JP-A-6-205694. The human CDR-grafted anti-$GM_2$ antibodies derived from the plasmids pKANTEX796HM1Lm-28, pKANTEX796HM2Lm-28, pKANTEX796HM3Lm-28, pKANTEX796HM31Lm-28 and pKANTEX796HM32Lm-28 are hereinafter referred to as "M1-28", "M2-28", "M3-28", "M31-28" and "M32-28", respectively. 4 µg each of the purified human CDR-grafted anti-$GM_2$ antibodies, the human CDR-grafted anti-$GM_2$ antibody KM8966 and the mouse-human chimeric anti-$GM_2$ antibody KM966 were electrophoresed by the conventional method [Laemmli: *Nature*, 227, 680 (1970)] for molecular weight checking. The results are shown in Fig. 52. As shown in Fig. 52, under reducing conditions, the molecular weight of the antibody H chain was about 50 KDa and the molecular weight of the antibody L chain was about 25 KDa, thus confirming the expression of the H chain and L chain having the correct molecular weight. Under nonreducing conditions, the molecular weight of the human CDR-grafted anti-$GM_2$ antibodies was about 150 KDa, confirming that the antibody expressed was composed of two H chains and two L chains and was correct in size. The N-terminal amino acid sequence of the H and L chains of each purified human CDR-grafted anti-$GM_2$ antibodies was examined by automatic Edman degradation using a protein sequencer (Applied Biosystems model 470A). As a result, it was confirmed that the amino acid sequence was consistent with that deduced from the synthesized V region DNA sequence.

(4) CDC activity of replacement versions of human CDR-grafted anti-$GM_2$ antibodies

CDC activity of the replacement versions of the human CDR-grafted anti-$GM_2$ antibodies obtained in Paragraph 2 (3) of Example 4, the human CDR-grafted anti-$GM_2$ antibody KM8966 and the mouse-human chimeric anti-$GM_2$

antibody KM966 was measured in accordance with the method described in Paragraph 8 of Example 3. The results are shown in Fig. 53. As shown in Fig. 53, it was found that, among the replacement versions of the human CDR-grafted anti-$GM_2$ antibodies, the human CDR-grafted anti-$GM_2$ antibody M2-28 derived from the plasmid pKANTEX796HM2Lm-28 showed the highest CDC activity which was higher than that of the human CDR-grafted anti-$GM_2$ antibody KM8966 prepared in Example 3. This result indicates that the replaced amino acid residues of the version HM2 among the various replacement versions prepared in Paragraph 1 of Example 4 play an important role for improving CDC activity. It was assumed from the computer model for the V region of mouse antibody KM796 that the replacement of the amino acid residues of the version HM2 would influence on the entire structure of the V region since these amino acid residues are located at the site which interacts with the L chain V region. Recent study of the production of human CDR-grafted antibody reveals that the amino acid residues which affect the structure of the antibody varies in each antibody. No method for precisely predicting such amino acid residues has been established and the above results provide a significant finding for the production of the human CDR-grafted antibody.

The human CDR-grafted anti-$GM_2$ antibody M2-28 derived from the plasmid pKANTEX796HM2Lm-28 was designated as KM8970 and the antibody KM8970-producing transformant KM8970 has been deposited with National Institute of Bioscience and Human-Technology, Agency of Industrial Science and Technology as of May 9, 1996 under the deposit number FERM BP-5528.

3. Modification of human CDR-grafted anti-$GM_2$ antibody KM8966 L chain V region

The human CDR-grafted anti-$GM_2$ antibody KM8966 prepared in Example 3 was subjected to amino acid residue replacements in the L chain V region (SEQ ID NO:8) to improve CDC activity. As an amino acid residue to be replaced, serine residue in position 59 was selected based on the results of various replacements obtained in Paragraph 1 (3) of Example 3 which suggested that it was important to support the structure of CDR2 for the human CDR-grafted anti-$GM_2$ antibody activity. Replacements were introduced by PCR method using as a template 1 ng of the plasmid pBSLm-28 containing the human CDR-grafted anti-$GM_2$ antibody L chain V region obtained in Paragraph 1 (3) of Example 3 and using as a primer antisense and sense synthetic DNA containing mutations described in Paragraph 1 (3) of Example 3.

The reaction was carried out in the same manner as described in Paragraph 1 (3) of Example 3 using the synthetic DNA of SEQ ID NO:72 as the mutant antisense primer and the synthetic DNA of SEQ ID NO:73 as the mutant sense primer to obtain the plasmid pBSLm-28 No.1, containing version Lm-28 No.1, shown in SEQ ID NO:11, of the human CDR-grafted anti-$GM_2$ antibody L chain V region. In the amino acid sequence of the version Lm-28 No.1, serine in position 59 in the FR shown in SEQ ID NO:83 was replaced by alanine that is found in the mouse antibody KM796 L chain V region.

4. Evaluation of CDC activity of human CDR-grafted anti-$GM_2$ antibody having new replacement in human CDR-grafted anti-$GM_2$ antibody L chain V region

(1) Construction of expression vectors

Expression vectors for the human CDR-grafted anti-$GM_2$ antibody containing the human CDR-grafted anti-$GM_2$ antibody L chain V region having the replacement obtained in Paragraph 3 of Example 4 and the human CDR-grafted anti-$GM_2$ antibody H chain V region were obtained in the following manner.

Six μg of the plasmid pBSLm-28 No.1 obtained in Paragraph 3 of Example 4 was dissolved in 10 μl of 50 mM Tris-hydrochloride buffer (pH 7.5) containing 100 mM sodium chloride, 10 mM magnesium chloride, 1 mM DTT and 100 μg/ml BSA. 10 units each of *Eco*RI (Takara Shuzo) and *Spl*I (Takara Shuzo) were added thereto to allow the mixture to react at 37°C for 1 hour. The reaction mixture was fractionated by agarose gel electrophoresis to recover about 0.4 μg of the *Eco*RI-*Spl*I fragment of about 0.39 kb.

Then, 3 μg each of the plasmid pKANTEX796HLCDRIm-28 obtained in Paragraph 3 of Example 3 and the plasmids pKANTEX796HM1Lm-28, pKANTEX796HM2Lm-28 and pKANTEX796HM3Lm-28 obtained in Paragraph 2 (1) of Example 4 were dissolved in 10 μl of 50 mM Tris-hydrochloride buffer (pH 7.5) containing 100 mM sodium chloride, 10 mM magnesium chloride and 1 mM DTT and 100 μg/ml BSA, 10 units each of *Eco*RI (Takara Shuzo) and *Spl*I were added thereto and the mixture was allowed to react at 37°C for 1 hour. The reaction mixture was fractionated by agarose gel electrophoresis to recover about 1 μg of the *Eco*RI-*Spl*I fragment of about 13.19 kb.

A 0.1 μg portion each of the thus-obtained *Eco*RI-*Spl*I fragment of pBSLm-28 No.1 and 0.1 μg of the *Eco*RI-*Spl*I of pKANTEX796HLCDRLm-28, pKANTEX796HM1Lm-28, pKANTEX796HM2Lm-28 and pKANTEX796HM3Lm-28 were added in a total of 20 μl of sterilized water and ligated to each other using Ready-To-Go T4 DNA Ligase (Pharmacia Biotech). Each of the resulting recombinant plasmid DNA solutions was used to transform *Escherichia coli* HB101 and the plasmids pKANTEX796HLm-28 No.1, pKANTEX796HM1Lm-28 No.1, pKANTEX796HM2Lm-28 No.1 and pKANTEX796HM3Lm-28 No.1 shown in Fig. 54 were obtained.

(2) Expression of human CDR-grafted anti-$GM_2$ antibodies having replacements in the L chain V region

Four μg each of the plasmids pKANTEX796HLm-28 No.1, pKANTEX796HM1Lm-28 No.1, pKANTEX796HM2Lm-28 No.1 and pKANTEX796HM3Lm-28 No.1 obtained in Paragraph 4 (1) of Example 4 was used to transform YB2/0 cells (ATCC CRL 1581) in accordance with the method as described in Paragraph 11 of Example 1. The cells were ultimately selected using G418 (0.5 mg/ml) and MTX (200 nM) to obtain about 2 to 5 μg/ml of transformants capable of producing human CDR-grafted anti-$GM_2$ antibodies derived from the corresponding expression vectors.

(3) Purification of human CDR-grafted anti-$GM_2$ antibodies having replacements in the L chain V region

Cells of each transformant obtained in Paragraph 4 (2) of Example 4 were suspended in GIT medium (Nihon Pharmaceutical) containing 0.5 mg/ml G418 and 200 nM MTX and about 1 to 3 mg of purified human CDR-grafted anti-$GM_2$ antibodies were obtained from about 0.6 liter of the culture broth in accordance with the method described in Paragraph 11 of Example 1 of JP-A-6-205694. The human CDR-grafted anti-$GM_2$ antibodies derived from the plasmids pKANTEX796HLm-28 No.1, pKANTEX796HM1Lm-28 No.1, pKANTEX796HM2Lm-28 No.1 and pKANTEX796HM3Lm-28 No.1 are hereinafter referred to as "h796H-No.1", "M1-No.1", "M2-No.1" and "M3-No.1", respectively. Four μg each of the purified human CDR-grafted anti-$GM_2$ antibodies and the mouse-human chimeric anti-$GM_2$ antibody KM966 was electrophoresed by the conventional method [Laemmli: *Nature*, 227, 680 (1970)] for molecular weight checking. The results are shown in Fig. 55. As shown in Fig. 55, under reducing conditions, the molecular weight of the antibody H chain was about 50 KDa and the molecular weight of the antibody L chain was about 25 KDa, thus confirming the expression of the H chain and L chain having the correct molecular weight. Under nonreducing conditions, the molecular weight of the human CDR-grafted anti-$GM_2$ antibodies was about 150 KDa, confirming that the antibody expressed was composed of two H chains and two L chains and was correct in size. The N-terminal amino acid sequence of the H and L chains of each purified human CDR-grafted anti-$GM_2$ antibodies was examined by automatic Edman degradation using a protein sequencer (Applied Biosystems model 470A). As a result, it was confirmed that the amino acid sequence was consistent with that deduced from the synthesized V region DNA sequence.

(4) CDC activity of human CDR-grafted anti-$GM_2$ antibodies having replacements in the L chain V region

CDC activity of the human CDR-grafted anti-$GM_2$ antibodies having replacements in the L chain V region obtained in Paragraph 4 (3) of Example 4, the human CDR-grafted anti-$GM_2$ antibody KM8970, the human CDR-grafted anti-$GM_2$ antibody KM8966 and the mouse-human chimeric anti-$GM_2$ antibody KM966 was measured in accordance with the method described in Paragraph 8 of Example 3. The results are shown in Fig. 56. Comparing CDC activity of KM8966 with that of h796H-No.1, it was found that the replacement introduced into only the L chain V region showed improved CDC activity. Among the replaced antibodies having replacements in both of the L chain V region and the H chain V region, M2-No.1 having replacement in the human CDR-grafted anti-$GM_2$ antibody KM8970 H and L chain V region obtained in Paragraph 2 of Example 4 showed the highest CDC activity, which was comparable to or higher than that of KM8970. These results indicates that the replaced amino acid residue in position 59 in the FR of the L chain V region prepared in Paragraph 3 of Example 4 played an important role for improving its CDC activity and it interacted with the replaced amino acid residue in the H chain V region of KM8970 for improving its CDC activity cooperatively. It was not assumed from the computer model for the V region of mouse antibody KM796 that the replacement of the amino acid residue in position 59 in the FR of the version Lm-28 No.1 would be involved in direct action with antigen $GM_2$ and interaction with each CDR residue. However, the above results suggested that they were quite important for maintaining the entire structure of the whole V region. This knowledge cannot be predicted from the known production method of a humanized antibody, and the above findings will provide an important indication for the production of human CDR-grafted antibody.

The human CDR-grafted anti-$GM_2$ antibody M2-No.1 derived from the plasmid pKANTEX796HM2Lm-28 No. 1 was designated as KM8969 and the antibody KM8969-producing transformant KM8969 has been deposited with National Institute of Bioscience and Human-Technology, Agency of Industrial Science and Technology as of May 9, 1996 under the deposit number FERM BP-5527.

5. *In vitro* reactivity of human CDR-grafted anti-$GM_2$ antibodies KM8969 and KM8970 with $GM_2$

Reactivities of the mouse-human chimeric anti-$GM_2$ antibody KM966 and the human CDR-grafted anti-$GM_2$

antibodies KM8969 and KM8970 with $GM_2$ were measured in accordance with the method described in Paragraph 1 (5) of Example 2. The results are shown in Fig. 57. As shown in Fig. 57, the human CDR-grafted anti-$GM_2$ antibodies KM8969 and KM8970 showed binding activity comparable to that of the mouse-human chimeric anti-$GM_2$ antibody KM966.

6. Reaction specificity of human CDR-grafted anti-$GM_2$ antibodies KM8969 and KM8970

The mouse-human chimeric anti-$GM_2$ antibody KM966 and the human CDR-grafted anti-$GM_2$ antibodies KM8969 and KM8970 were examined for reactivity with various gangliosides in accordance with the method described in Paragraph 6 of Example 3. The results are shown in Fig. 58. As shown in Fig. 58, it was found that the human CDR-grafted anti-$GM_2$ antibodies KM8969 and KM8970 specifically reacted with $GM_2$ (N-acetyl $GM_2$ and N-glycolyl $GM_2$) like the mouse-human chimeric anti-$GM_2$ antibody KM966.

7. Reactivity of human CDR-grafted anti-$GM_2$ antibodies-KM8969 and KM8970 with cancer cells

The mouse-human chimeric anti-$GM_2$ antibody KM966 and the human CDR-grafted anti-$GM_2$ antibodies KM8969 and KM8970 were examined for reactivity with the human lung small cell carcinoma cell line SBC-3 (JCRB 0818) using fluorescein isocyanate-labeled rabbit anti-human IgG antibody (Dako) as a second antibody in accordance with the method described in Paragraph 7 of Example 3. The results are shown in Fig. 59. As shown in Fig. 59, the human CDR-grafted anti-$GM_2$ antibodies KM8969 and KM8970 strongly reacted with the human lung small cell carcinoma cell line SBC-3 like the mouse-human chimeric anti-$GM_2$ antibody KM966.

8. *In vitro* antitumor effect of human CDR-grafted anti-$GM_2$ antibodies KM8969 and KM8970: antibody dependent cell mediated cytotoxicity (ADCC)

The mouse-human chimeric anti-$GM_2$ antibody KM966 and the human CDR-grafted anti-$GM_2$ antibodies KM8966, KM8969 and KM8970 were examined for ADCC activity against the human lung small cell carcinoma cell line SBC-3 (JCRB 0818) in accordance with the method described in Paragraph 9 of Example 3. The results are shown in Fig. 123. As shown in Fig. 123, the human CDR-grafted anti-$GM_2$ antibodies KM8969 and KM8970 showed ADCC activity comparable to that of the mouse-human chimeric anti-$GM_2$ antibody KM966.

9. Comparison of *in vitro* anti-tumor activities of humanized anti-$GM_2$ antibodies: comparison of CDC activity

CDC activities of various humanized anti-$GM_2$ antibodies (KM966, KM8966, KM8969 and KM8970) established in the aforementioned Inventive Examples 3 and 4 were compared by prolonging the reaction time. Illustratively, the reaction time of the method described in the item 8 of Inventive Example 3 after addition of the human complement was set to 4 hours. The results are shown in Fig. 61. As shown in Fig. 61, it was revealed that the CDC activity of each of these humanized antibodies increases by the 4 hours of reaction and, at an antibody concentration of 5 μg/ml or more, the mouse-human chimeric anti-$GM_2$ antibody KM966 and the human CDR-grafted anti-$GM_2$ antibodies KM8966, KM8969 and KM8970 show almost the same level of CDC activity. Particularly, KM8969 showed the highest CDC activity which was about 1/2 of that of the mouse-human chimeric anti-$GM_2$ antibody KM966, so that it was revealed that a human CDR-grafted anti-$GM_2$ antibody having further high CDC activity was able to be produced by the examination of Inventive Example 4.

Thus, production method of human CDR-grafted anti-$GM_2$ antibodies and evaluation of their various activities have been described, and these results show that the established human CDR-grafted anti-$GM_2$ antibodies are useful for the treatment of human cancers.

By the present invention, human CDR-grafted antibodies to ganglioside $GM_2$, whose binding activity and binding specificity for $GM_2$ and anti-tumor effect upon ganglioside $GM_2$-positive cells are comparable to the levels of chimeric human antibodies, and the production method thereof are provided.

## Claims

**1.** A human CDR-grafted antibody which specifically reacts with ganglioside $GM_2$ wherein said antibody comprises CDR1, CDR2 und CDR3 of a heavy chain (H chain) variable region (V region) comprising amino acid sequences of SEQ ID NO:1, SEQ ID NO:2 und SEQ ID NO:3 and CDR1, CDR2 und CDR3 of a light chain (L chain) V region comprising amino acid sequences of SEQ ID NO:4, SEQ ID NO:5 und SEQ ID NO:6 and frameworks (FR) of Kabat's Human Sub Group (HSG), which comprises an amino acid sequence in which at least one amino acid of positions 38, 40, 67, 72, 84 and 98 in the FR of the H chain V region and positions 4, 11, 15, 35, 42, 46, 59, 69, 70, 71, 72, 76, 77 and 103 in the FR of the L chain V region is replaced by another amino acid.

**2.** The human CDR-grafted antibody according to claim 1, wherein said H chain C region of the antibody is derived from an antibody belonging to the human antibody IgG class.

**3.** The human CDR-grafted antibody of claim 2, wherein the H chain C region of the antibody is a $C\gamma1$ region and the L chain C region is a $C\kappa$ region.

**4.** The human CDR-grafted antibody according to claim 3, which is the antibody comprising the H chain V region of the antibody having an amino acid sequence of SEQ ID NO:7 and the L chain V region of the antibody having an amino acid sequence of SEQ ID NO:8.

**5.** The human CDR-grafted antibody according to claim 3, which is the antibody comprising the H chain V region of the antibody having an amino acid sequence of SEQ ID NO:7 and the L chain V region of the antibody having an amino acid sequence of SEQ ID NO:9.

**6.** The human CDR-grafted antibody according to claim 3, which is the antibody comprising the H chain V region of the antibody having an amino acid sequence of SEQ ID NO:10 and the L chain V region of the antibody having an amino acid sequence of SEQ ID NO:8.

7. The human CDR-grafted antibody according to claim 3, which is the antibody comprising the H chain V region of the antibody having an amino acid sequence of SEQ ID NO:10 and the L chain V region of the antibody having an amino acid sequence of SEQ ID NO:11.

8. The human CDR-grafted antibody according to claim 1 or 2, which is KM8966 obtainable from the deposited transformant cell line having the accession number FERM BP-5105 comprising the H chain V region of the antibody having an amino acid sequence of SEQ ID NO:7 and the L chain V region of the antibody having an amino acid sequence of SEQ ID NO:8.

9. The human CDR-grafted antibody according to claim 1 or 2, which is KM8967 obtainable from the deposited transformant cell line having the accession number FERM BP-5106 comprising the H chain V region of the antibody having an amino acid sequence of SEQ ID NO:7 and the L chain V region of the antibody having an amino acid sequence of SEQ ID NO:9.

10. The human CDR-grafted antibody according to claim 1 or 2, which is KM8970 obtainable from the deposited transformant cell line having the accession number FERM BP-5528 comprising the H chain V region of the antibody having an amino acid sequence of SEQ ID NO:10 and the L chain V region of the antibody having an amino acid sequence of SEQ ID NO:8.

11. The human CDR-grafted antibody according to claim 1 or 2, which is KM8969 obtainable from the deposited transformant cell line having the accession number FERM BP-5527 comprising the H chain V region of the antibody having an amino acid sequence of SEQ ID NO:10 and the L chain V region of the antibody having an amino acid sequence of SEQ ID NO:11.

12. A DNA fragment encoding an amino acid sequence of the H chain V region an L chain V region of the antibody according to any of claims 1 to 11.

13. A recombinant vector comprising the DNA fragment according to claim 12.

14. The recombinant vector according to claim 13 which is derived from a tandem cassette vector for expressing a human chimeric antibody and a human CDR-grafted antibody.

15. A transformant comprising the recombinant vector according to claim 13 or 14.

16. A transformant cell line KM8966 having the accession number FERM BP-5105, which produces the antibody KM8966 according to claim 8.

17. A transformant cell line KM8967 having the accession number FERM BP-5106, which produces the antibody KM8967 according to claim 9.

18. A transformant cell line KM8970 having the accession number FERM BP-5528, which produces the antibody KM8970 according to claim 10.

19. A transformant cell line KM8969 having the accession number FERM BP-5527, which produces the antibody KM8969 according to claim 11.

20. A method for producing the antibodies according to any one of claims 1 to 11 using said transformant according to any one of claims 16 to 20.

21. An anti-tumor agent comprising the antibody of any one of claims 1 to 11 as an active ingredient.

22. A diagnostic agent for cancer comprising the antibody of any one of claims 1 to 11 as an active ingredient.

23. The use of an antibody of any of claims 1 to 11 for preparing a pharmaceutical composition for treating human cancer.

**Patentansprüche**

1. Humaner Graft-CDR-Antikörper, der mit Gangliosid $GM_2$ spezifisch reagiert, wobei der Antikörper umfasst:

   CDR1, CDR2 und CDR3 aus der variablen Region (V-Region) einer schweren Kette (H-Kette), welche die Aminosäuresequenzen SEQ ID NO:1, SEQ ID NO:2 und SEQ ID NO:3 umfassen; und
   CDR1, CDR2 und CDR3 aus der V-Region einer leichten Kette (L-Kette), welche die Aminosäuresequenzen SEQ ID NO:4, SEQ ID NO:5 und SEQ ID NO:6 umfassen; und
   Gerüstregionen (FR) aus Kabats humaner Untergruppe (HSG), welche eine Aminosäuresequenz umfassen, in der wenigstens eine Aminosäure an den Positionen 38, 40, 67, 72, 84 und 98 in der FR aus der H-Ketten-V-Region und an den Positionen 4, 11, 15, 35, 42,46, 59, 69, 70, 71, 72, 76, 77 und 103 der FR aus der L-Ketten-V-Region durch eine andere Aminosäure ersetzt ist.

2. Humaner Graft-CDR-Antikörper nach Anspruch 1, wobei die H-Ketten-C-Region des Antikörpers von einem zur humanen Antikörper-IgG-Klasse gehörenden Antikörper stammt.

3. Humaner Graft-CDR-Antikörper nach Anspruch 2, wobei die H-Ketten-C-Region des Antikörpers eine Cγ1-Region und die L-Ketten-C-Region eine Cκ-Region ist.

4. Humaner Graft-CDR-Antikörper nach Anspruch 3, wobei der Antikörper die H-Ketten-V-Region des Antikörpers mit einer Aminosäuresequenz SEQ ID NO:7 und die L-Ketten-V-Region des Antikörpers mit einer Aminosäuresequenz SEQ ID NO:8 umfasst.

5. Humaner Graft-CDR-Antikörper nach Anspruch 3, wobei der Antikörper die H-Ketten-V-Region des Antikörpers mit einer Aminosäuresequenz SEQ ID NO:7 und die L-Ketten-V-Region des Antikörpers mit einer Aminosäuresequenz SEQ ID NO:9 umfasst.

6. Humaner Graft-CDR-Antikörper nach Anspruch 3, wobei der Antikörper die H-Ketten-V-Region des Antikörpers mit einer Aminosäuresequenz SEQ ID NO:10 und die L-Ketten-V-Region des Antikörpers mit einer Aminosäuresequenz SEQ ID NO:8 umfasst.

7. Humaner Graft-CDR-Antikörper nach Anspruch 3, wobei der Antikörper die H-Ketten-V-Region des Antikörpers mit einer Aminosäuresequenz SEQ ID NO:10 und die L-Ketten-V-Region des Antikörpers mit einer Aminosäuresequenz SEQ ID NO:11 umfasst.

8. Humaner Graft-CDR-Antikörper nach Anspruch 1 oder 2, nämlich KM8966, der durch die hinterlegte Transformantenzelllinie mit der Hinterlegungsnummer FERM BP-5105 erhältlich ist und die H-Ketten-V-Region des Antikörpers mit einer Aminosäuresequenz SEQ ID NO:7 und die L-Ketten-V-Region des Antikörpers mit einer Aminosäuresequenz SEQ ID NO:8 umfasst.

9. Humaner Graft-CDR-Antikörper nach Anspruche 1 öder 2, nämlich KM8967, der durch die hinterlegte Transformantenzelllinie mit der Hinterlegungsnummer FERM BP-5106 erhältlich ist und die H-Ketten-V-Region des Antikörpers mit einer Aminosäuresequenz SEQ ID NO:7 und die L-Ketten-V-Region des Antikörpers mit einer Aminosäuresequenz SEQ ID NO:9 umfasst

10. Humaner Graft-CDR-Antikörper nach Anspruch 1 oder 2, nämlich KM8970, der durch die hinterlegte Transformantenzelllinie mit der Hinterlegungsnummer FERM BP-5528 erhältlich ist und die H-Ketten-V-Region des Antikörpers mit einer Aminosäuresequenz SEQ ID NO: 10 und die L-Ketten-V-Region des Antikörpers mit einer Aminosäuresequenz SEQ ID NO:8 umfasst.

11. Humaner Graft-CDR-Antikörper nach Anspruch 1 oder 2, nämlich KM8969, der durch die hinterlegte Transformantenzelllinie mit der Hinterlegungsnummer FERM BP-5527 erhältlich ist und die H-Ketten-V-Region des Antikörpers mit einer Aminosäuresequenz SEQ ID NO:10 und die L-Ketten-V-Region des Antikörpers mit einer Aminosäuresequenz SEQ ID NO:11 umfasst.

12. DNA-Fragment, das eine Aminosäuresequenz der H-Ketten-V-Region und L-Ketten-V-Region des Antikörpers nach einem der Ansprüche 1 bis 11 kodiert.

13. Rekombinanter Vektor, der das DNA-Fragment nach Anspruch 12 umfasst.

14. Rekombinanter Vektor nach Anspruch 13, der von einem Tandemkassettenvektor für die Expression eines humanen chimären Antikörpers und eines humanen Graft-CDR-Antikörpers stammt.

15. Transformante, die den rekombinanten Vektor nach Anspruch 13 oder 14 umfasst.

16. Transformantenzelllinie KM8966 mit der Hinterlegungsnummer FERM BP-5105, die den Antikörper KM8966 nach Anspruch 8 produziert.

17. Transformantenzelllinie KM8967 mit der Hinterlegungsnummer FERM BP-5106, die den Antikörper KM8967 nach Anspruch 9 produziert.

18. Transformantenzelllinie KM8970 mit der Hinterlegungsnummer FERM BP-5528, die den Antikörper KM8970 nach Anspruch 10 produziert.

19. Transformantenzelllinie KM8969 mit der Hinterlegungsnummer FERM BP-5527, die den Antikörper KM8969 nach Anspruch 11 produziert.

20. Verfahren zur Herstellung der Antikörper nach einem der Ansprüche 1 bis 11, wobei die Transformante nach einem der Ansprüche 16 bis 20 verwendet wird.

21. Antitumormittel, das den Antikörper nach einem der Ansprüche 1 bis 11 als aktiven Inhaltsstoff umfasst.

22. Mittel für die Krebsdiagnose, das den Antikörper nach einem der Ansprüche 1 bis 11 als aktiven Inhaltsstoff umfasst.

23. Verwendung eines Antikörpers nach einem der Ansprüche 1 bis 11 zur Herstellung einer pharmazeutischen Zusammensetzung zur Behandlung von Humankrebs.

**Revendications**

1. Anticorps à greffage de CDR humain qui réagit spécifiquement avec le ganglioside $GM_2$, où ledit anticorps comprend la CDR 1, la CDR 2 et la CDR 3 d'une région variable (région V) de chaîne lourde (chaîne H) comprenant les séquences d'acides aminés de SEQ ID NO:1, SEQ ID NO:2 et SEQ ID NO:3 et la CDR 1, la CDR 2 et la CDR 3 d'une région V de chaîne légère (chaîne L) comprenant les séquences d'acides aminés de SEQ ID NO:4, SEQ ID NO:5 et SEQ ID NO:6 et les ossatures (FR) du sous-groupe humain (HSG) de Kabat, qui comprennent une séquence d'acides aminés dans laquelle au moins un acide aminé des positions 38, 40, 67, 72, 84 et 98 dans la FR de la région V de chaîne H et des positions 4, 11, 15, 35, 42, 46, 59, 69, 70, 71, 72, 76, 77 et 103 dans la FR de la région V de chaîne L est remplacé par un autre acide aminé.

2. Anticorps à greffage de CDR humain selon la revendication 1, où ladite région C de chaîne H de l'anticorps est dérivée d'un anticorps appartenant à la classe IgG des anticorps humains.

3. Anticorps à greffage de CDR humain selon la revendication 2 où la région C de chaîne H de l'anticorps est une région Cγ1 et la région C de chaîne L est une région Cκ.

4. Anticorps à greffage de CDR humain selon la revendication 3 où ledit anticorps comprend la région V de chaîne H de l'anticorps ayant une séquence d'acides aminés de SEQ ID NO:7 et la région V de chaîne L de l'anticorps ayant une séquence d'acides aminés de SEQ ID NO:8.

5. Anticorps à greffage de CDR humain selon la revendication 3 où ledit anticorps comprend la région V de chaîne H de l'anticorps ayant une séquence d'acides aminés de SEQ ID NO:7 et la région V de chaîne L de l'anticorps ayant une séquence d'acides aminés de SEQ ID NO:9.

6. Anticorps à greffage de CDR humain selon la revendication 3 où ledit anticorps comprend la région V de chaîne H de l'anticorps ayant une séquence d'acides aminés de SEQ ID NO:10 et la région V de chaîne L de l'anticorps ayant une séquence d'acides aminés de SEQ ID NO:8.

**7.** Anticorps à greffage de CDR humain selon la revendication 3 où ledit anticorps comprend la région V de chaîne H de l'anticorps ayant une séquence d'acides aminés de SEQ ID NO:10 et la région V de chaîne L de l'anticorps ayant une séquence d'acides aminés de SEQ ID NO:11.

**8.** Anticorps à greffage de CDR humain selon la revendication 1 ou 2 qui est KM8966 qui peut être obtenu à partir de la lignée cellulaire de transformant déposée ayant le numéro de dépôt FERM BP-5105 comprenant la région V de chaîne H de l'anticorps ayant une séquence d'acides aminés de SEQ ID NO:7 et la région V de chaîne L de l'anticorps ayant une séquence d'acides aminés de SEQ ID NO:8.

**9.** Anticorps à greffage de CDR humain selon la revendication 1 ou 2 qui est KM8967 qui peut être obtenu à partir de la lignée cellulaire de transformant déposée ayant le numéro de dépôt FERM BP-5106 comprenant la région V de chaîne H de l'anticorps ayant une séquence d'acides aminés de SEQ ID NO:7 et la région V de chaîne L de l'anticorps ayant une séquence d'acides aminés de SEQ ID NO:9.

**10.** Anticorps à greffage de CDR humain selon la revendication 1 ou 2 qui est KM8970 qui peut être obtenu à partir de la lignée cellulaire de transformant déposée ayant le numéro de dépôt FERM BP-5528 comprenant la région V de chaîne H de l'anticorps ayant une séquence d'acides aminés de SEQ ID NO:10 et la région V de chaîne L de l'anticorps ayant une séquence d'acides aminés de SEQ ID NO:8.

**11.** Anticorps à greffage de CDR humain selon la revendication 1 ou 2 qui est KM8969 qui peut être obtenu à partir de la lignée cellulaire de transformant déposée ayant le numéro de dépôt FERM PB-5527 comprenant la région V de chaîne H de l'anticorps ayant une séquence d'acides aminés de SEQ ID NO:10 et la région V de chaîne L de l'anticorps ayant une séquence d'acides aminés de SEQ ID NO:11.

**12.** Fragment d'ADN codant une séquence d'acides aminés de la région V de chaîne H et de la région V de chaîne L de l'anticorps selon l'une quelconque des revendications 1 à 11.

**13.** Vecteur recombiné comprenant le fragment d'ADN selon la revendication 12.

**14.** Vecteur recombiné selon la revendication 13 qui est dérivé d'un vecteur à cassette en tandem pour exprimer un anticorps chimère humain et un anticorps à greffage de CDR humain.

**15.** Transformant comprenant le vecteur recombiné selon la revendication 13 ou 14.

**16.** Lignée cellulaire de transformant KM8966 ayant le numéro de dépôt FERM BP-5105 qui produit l'anticorps KM8966 selon la revendication 8.

**17.** Lignée cellulaire de transformant KM8967 ayant le numéro de dépôt FERM BP-5106 qui produit l'anticorps KM8967 selon la revendication 9.

**18.** Lignée cellulaire de transformant KM8970 ayant le numéro de dépôt FERM BP-5528 qui produit l'anticorps KM8970 selon la revendication 10.

**19.** Lignée cellulaire de transformant KM8969 ayant le numéro de dépôt FERM BP-5527 qui produit l'anticorps KM8969 selon la revendication 11.

**20.** Procédé pour produire les anticorps selon l'une quelconque des revendications 1 à 11 au moyen dudit transformant selon l'une quelconque des revendications 16 à 20.

**21.** Agent anti-tumoral comprenant l'anticorps selon l'une quelconque des revendications 1 à 11 comme ingrédient actif.

**22.** Agent de diagnostic pour le cancer comprenant l'anticorps selon l'une quelconque des revendications 1 à 11 comme ingrédient actif.

**23.** Utilisation d'un anticorps selon l'une quelconque des revendications 1 à 11 pour préparer une composition pharmaceutique pour traiter un cancer humain.

FIG. 1

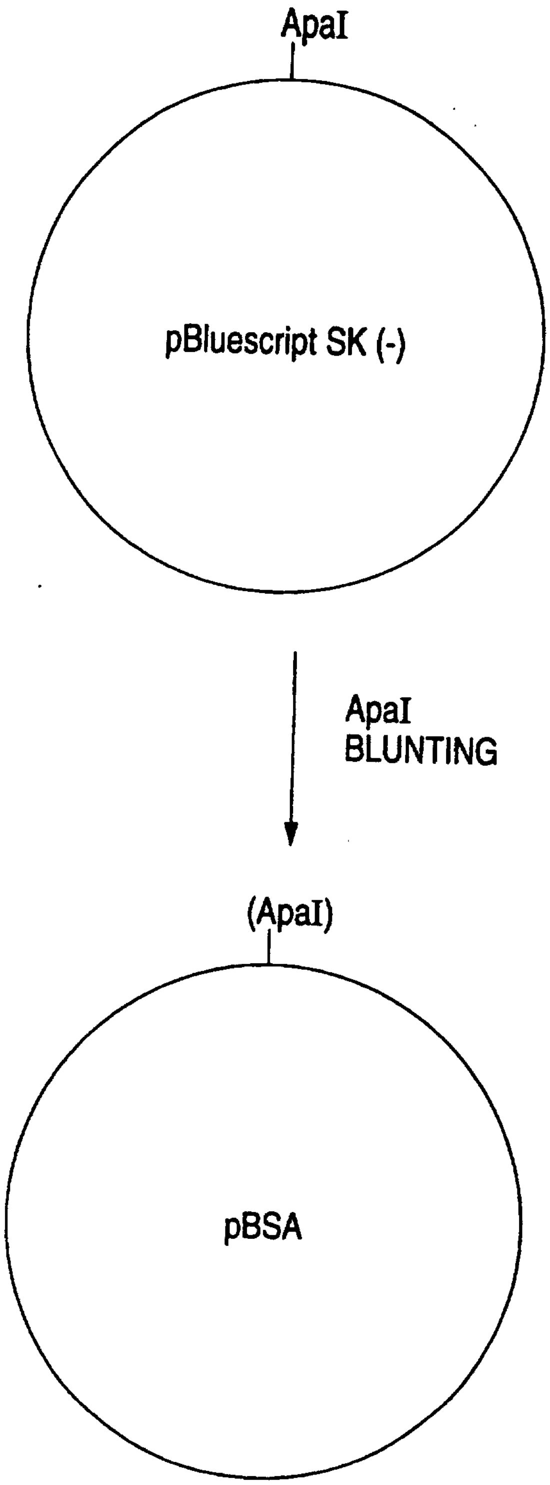

*FIG. 2*

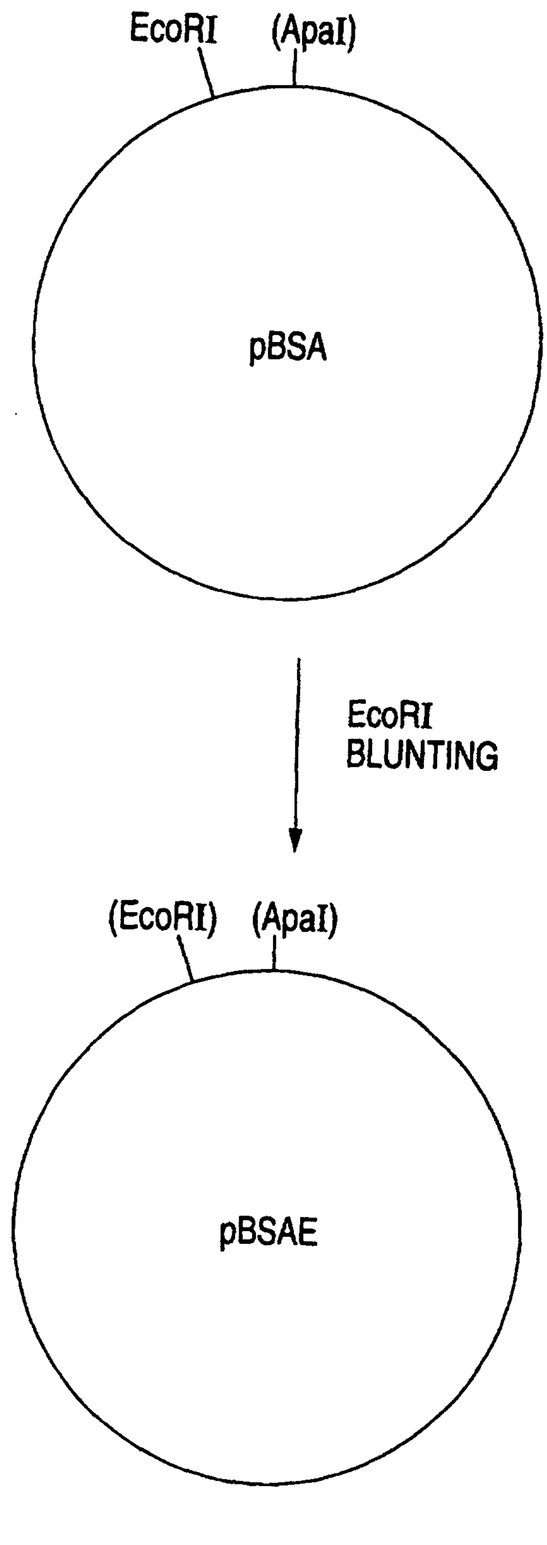

FIG. 3

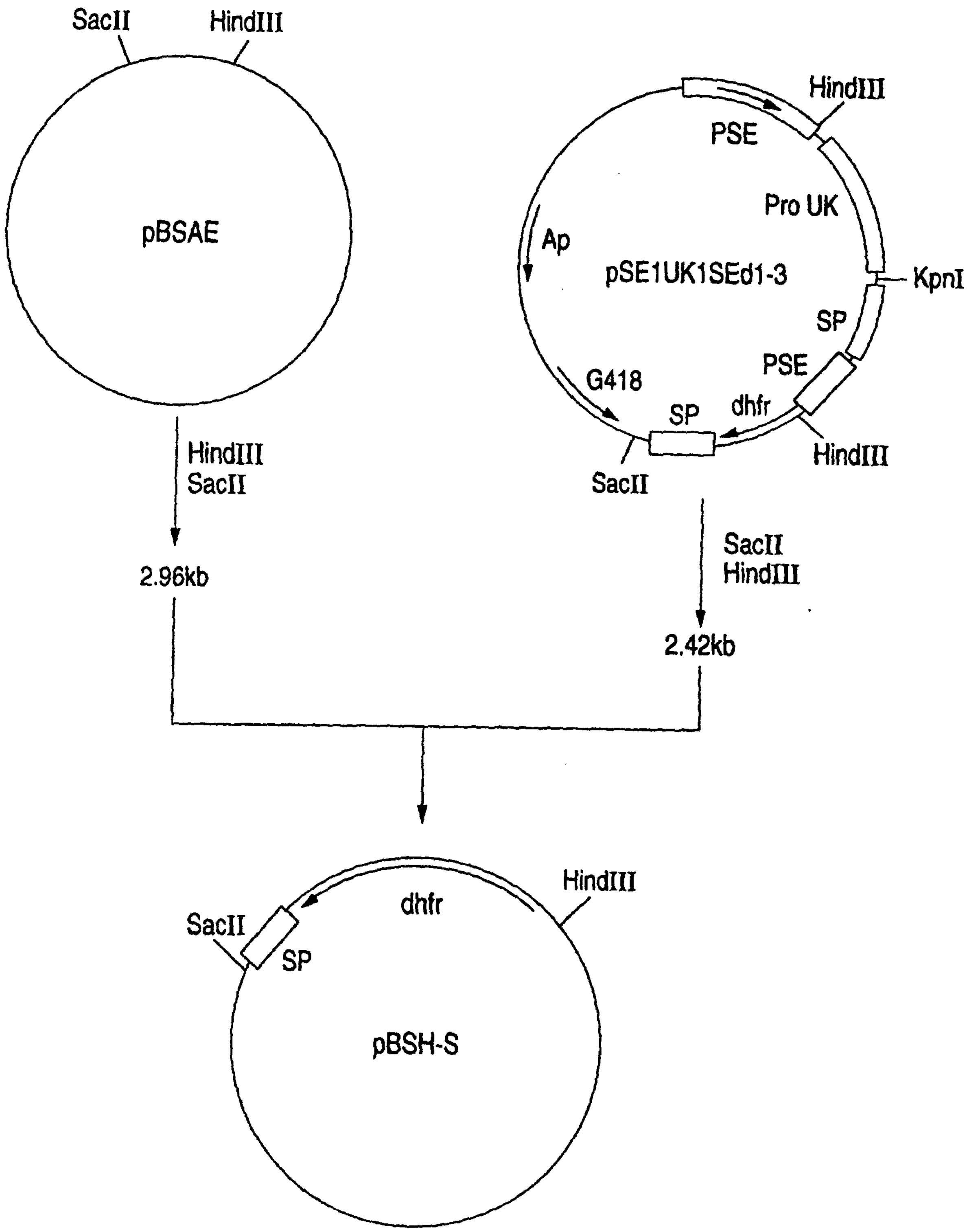
SacII
HindIII
pBSAE
HindIII
SacII
2.96kb
HindIII
PSE
Pro UK
Ap
pSE1UK1SEd1-3
KpnI
SP
PSE
G418
SP
dhfr
HindIII
SacII
SacII
HindIII
2.42kb
HindIII
dhfr
SacII
SP
pBSH-S

*FIG. 4*

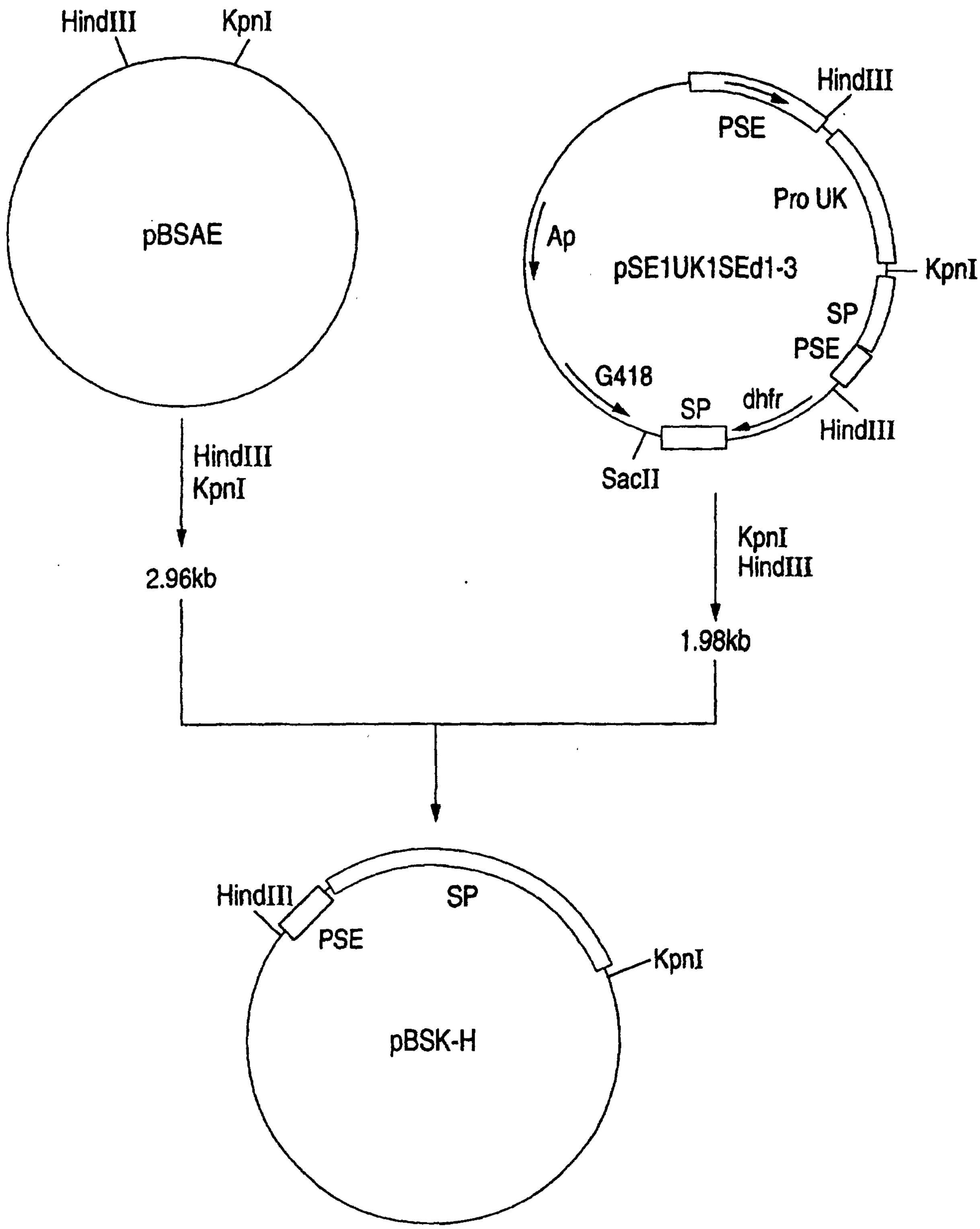
HindIII
KpnI
pBSAE
HindIII
KpnI
2.96kb
HindIII
PSE
Pro UK
Ap
pSE1UK1SEd1-3
KpnI
SP
PSE
G418
SP
dhfr
HindIII
SacII
KpnI
HindIII
1.98kb
HindIII
SP
PSE
KpnI
pBSK-H

FIG. 5

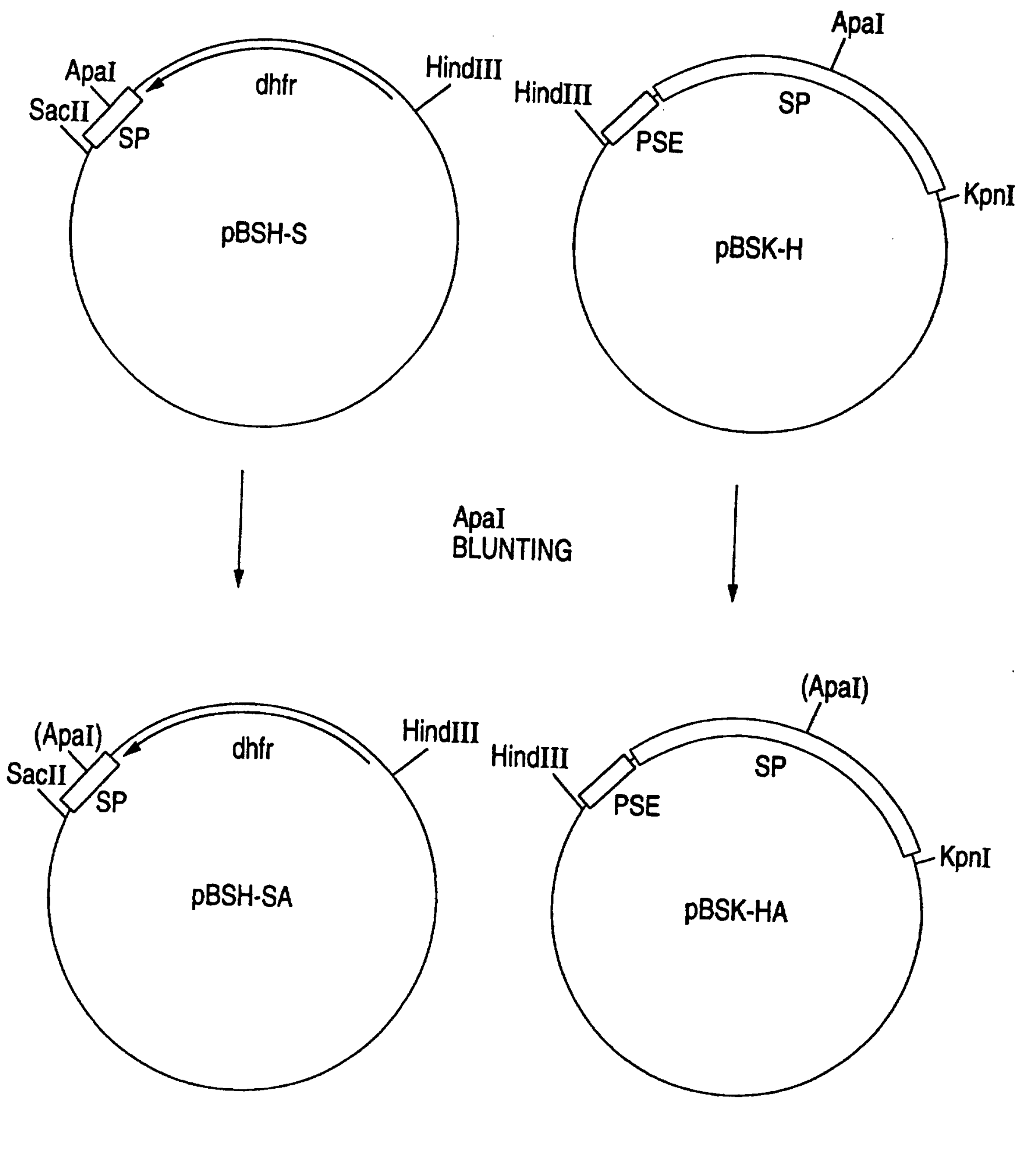
ApaI
SacII
dhfr
HindIII
SP
pBSH-S
ApaI
HindIII
SP
PSE
KpnI
pBSK-H
ApaI
BLUNTING
(ApaI)
SacII
dhfr
HindIII
SP
pBSH-SA
(ApaI)
HindIII
SP
PSE
KpnI
pBSK-HA

FIG. 6

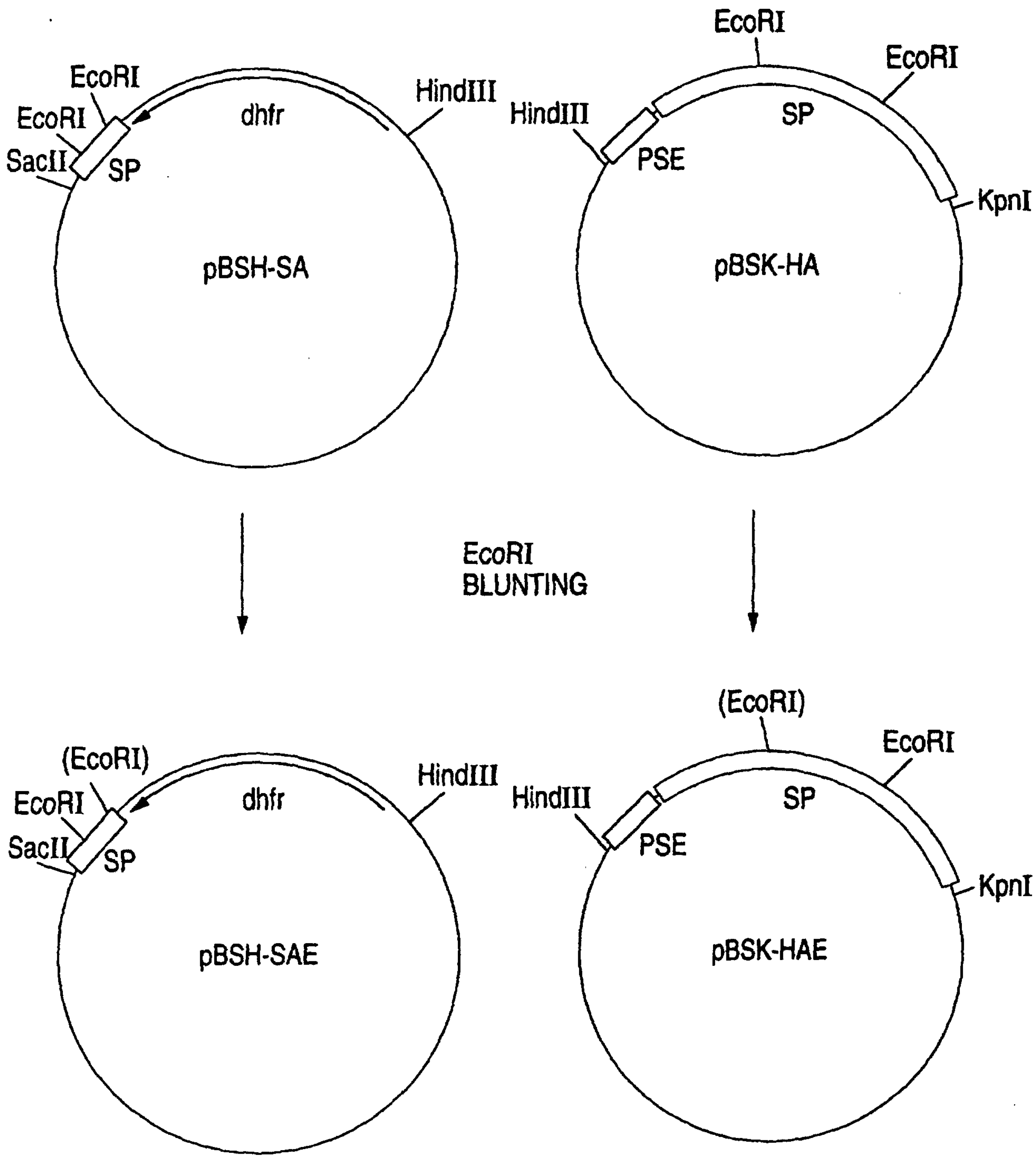
EcoRI
EcoRI
SacII
SP
dhfr
HindIII
pBSH-SA
EcoRI
EcoRI
HindIII
SP
PSE
KpnI
pBSK-HA
EcoRI
BLUNTING
(EcoRI)
EcoRI
SacII
SP
dhfr
HindIII
pBSH-SAE
(EcoRI)
EcoRI
HindIII
SP
PSE
KpnI
pBSK-HAE

FIG. 7

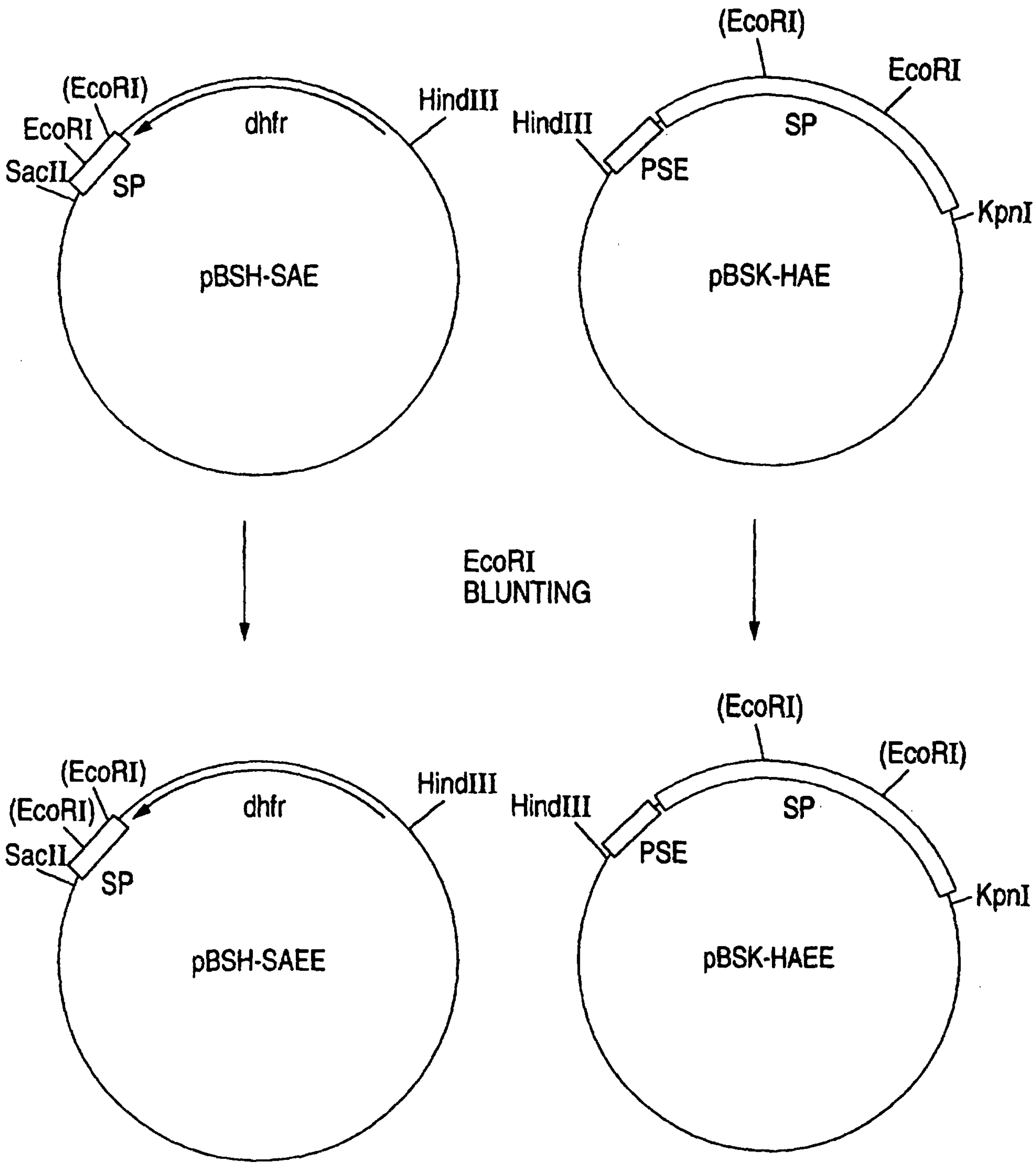
(EcoRI)
EcoRI
SacII
SP
dhfr
HindIII
pBSH-SAE
(EcoRI)
EcoRI
HindIII
SP
PSE
KpnI
pBSK-HAE
EcoRI
BLUNTING
(EcoRI)
(EcoRI)
SacII
SP
dhfr
HindIII
pBSH-SAEE
(EcoRI)
(EcoRI)
HindIII
SP
PSE
KpnI
pBSK-HAEE

FIG. 8

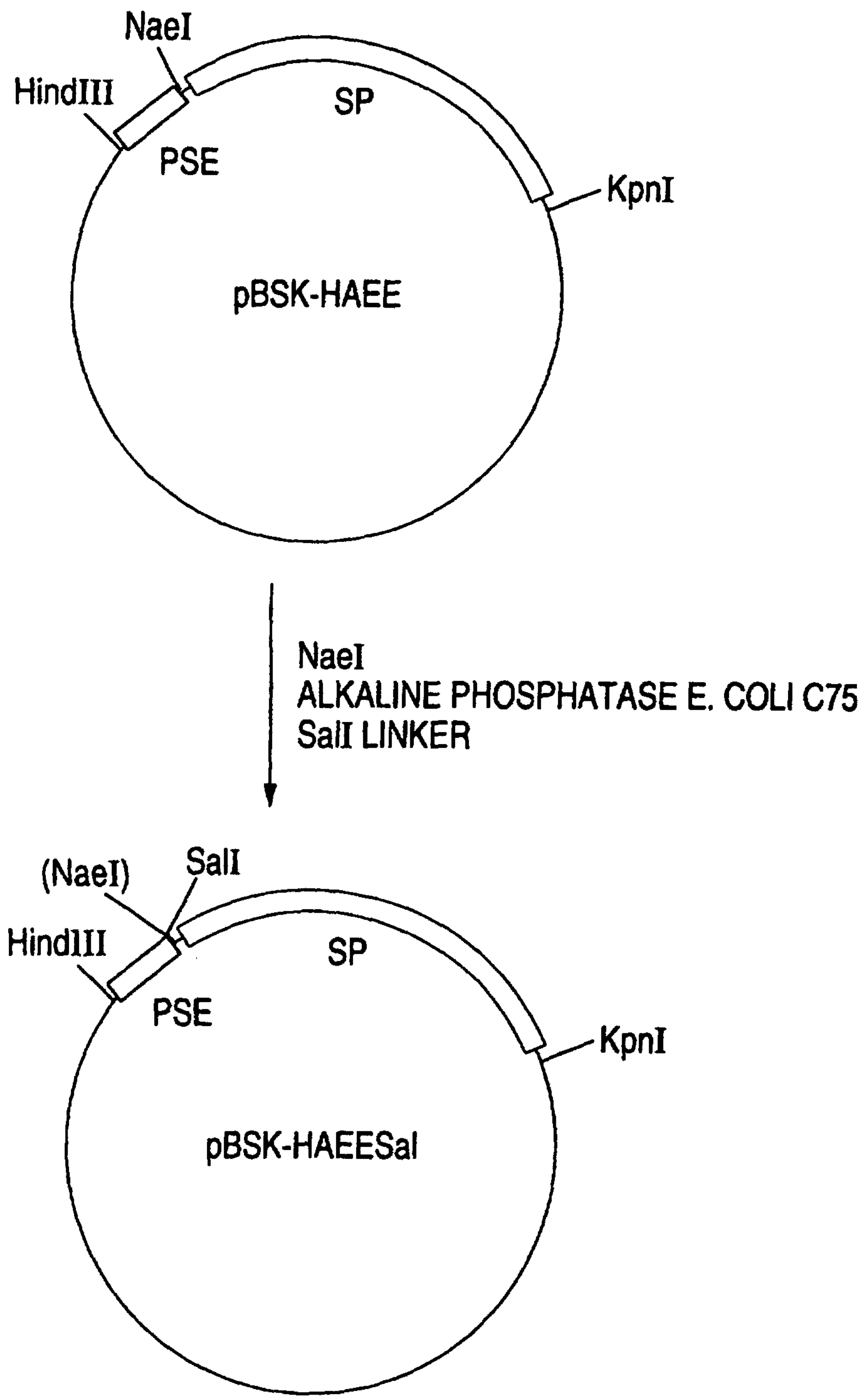
NaeI
HindIII
SP
PSE
KpnI
pBSK-HAEE
NaeI
ALKALINE PHOSPHATASE E. COLI C75
SalI LINKER
(NaeI)
SalI
HindIII
SP
PSE
KpnI
pBSK-HAEESal

FIG. 9

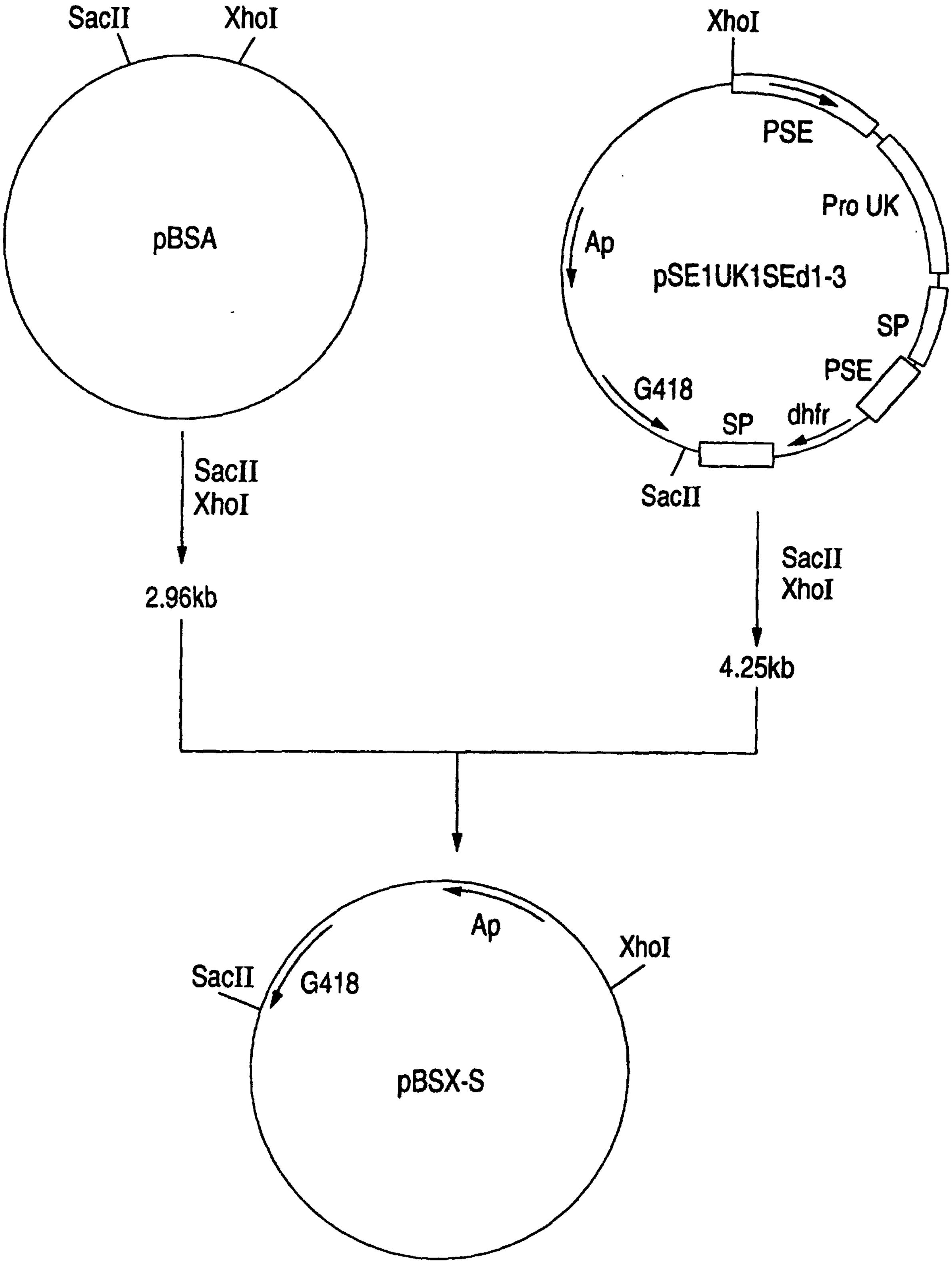
SacII
XhoI
pBSA
XhoI
PSE
Pro UK
Ap
pSE1UK1SEd1-3
SP
PSE
G418
SP
dhfr
SacII
SacII
XhoI
2.96kb
SacII
XhoI
4.25kb
Ap
XhoI
SacII
G418
pBSX-S

FIG. 10

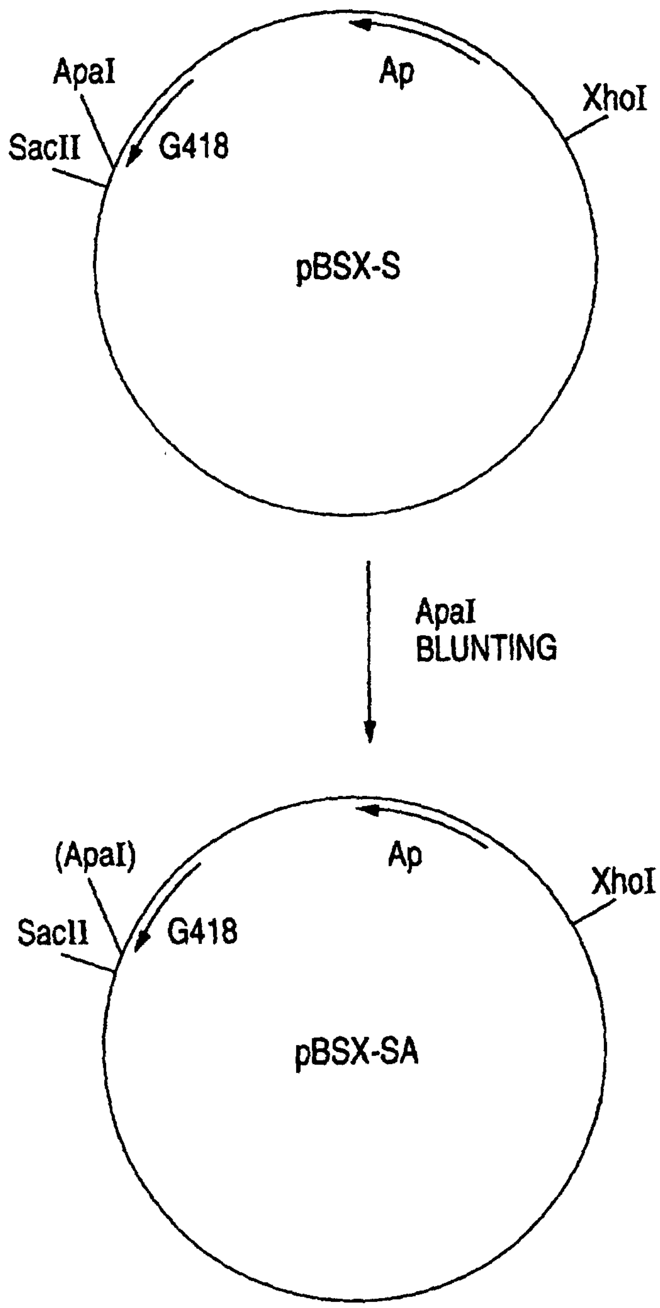
ApaI
Ap
XhoI
SacII
G418
pBSX-S
ApaI
BLUNTING
(ApaI)
Ap
XhoI
SacII
G418
pBSX-SA

*FIG. 11*

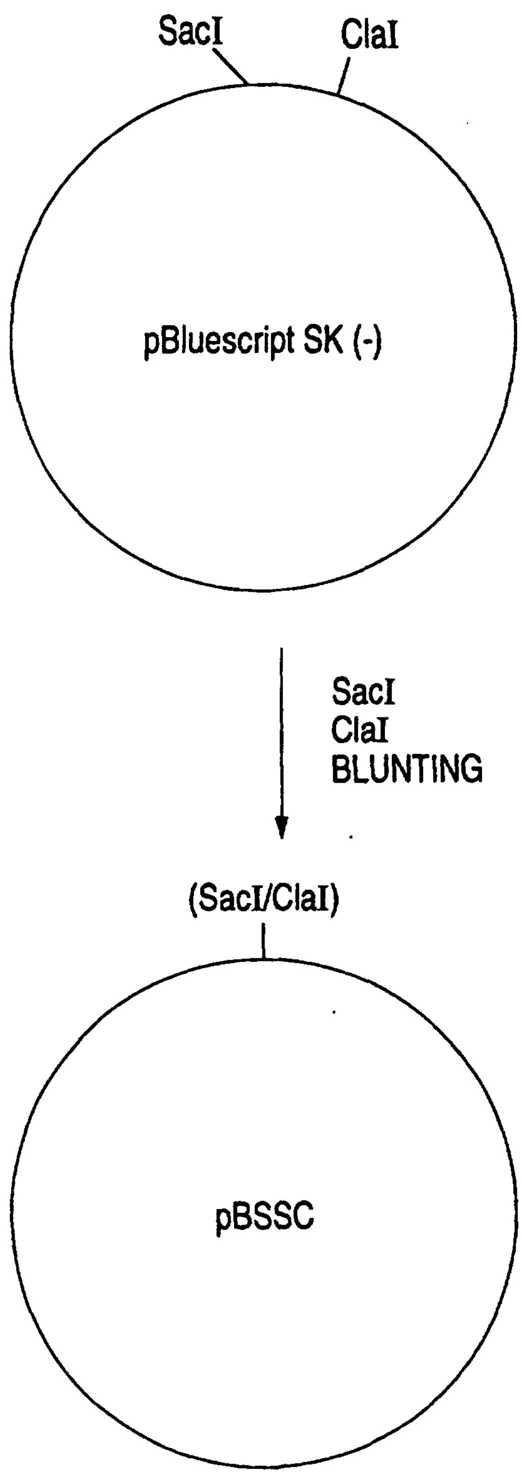
SacI
ClaI
pBluescript SK (-)
SacI
ClaI
BLUNTING
(SacI/ClaI)
pBSSC

FIG. 12

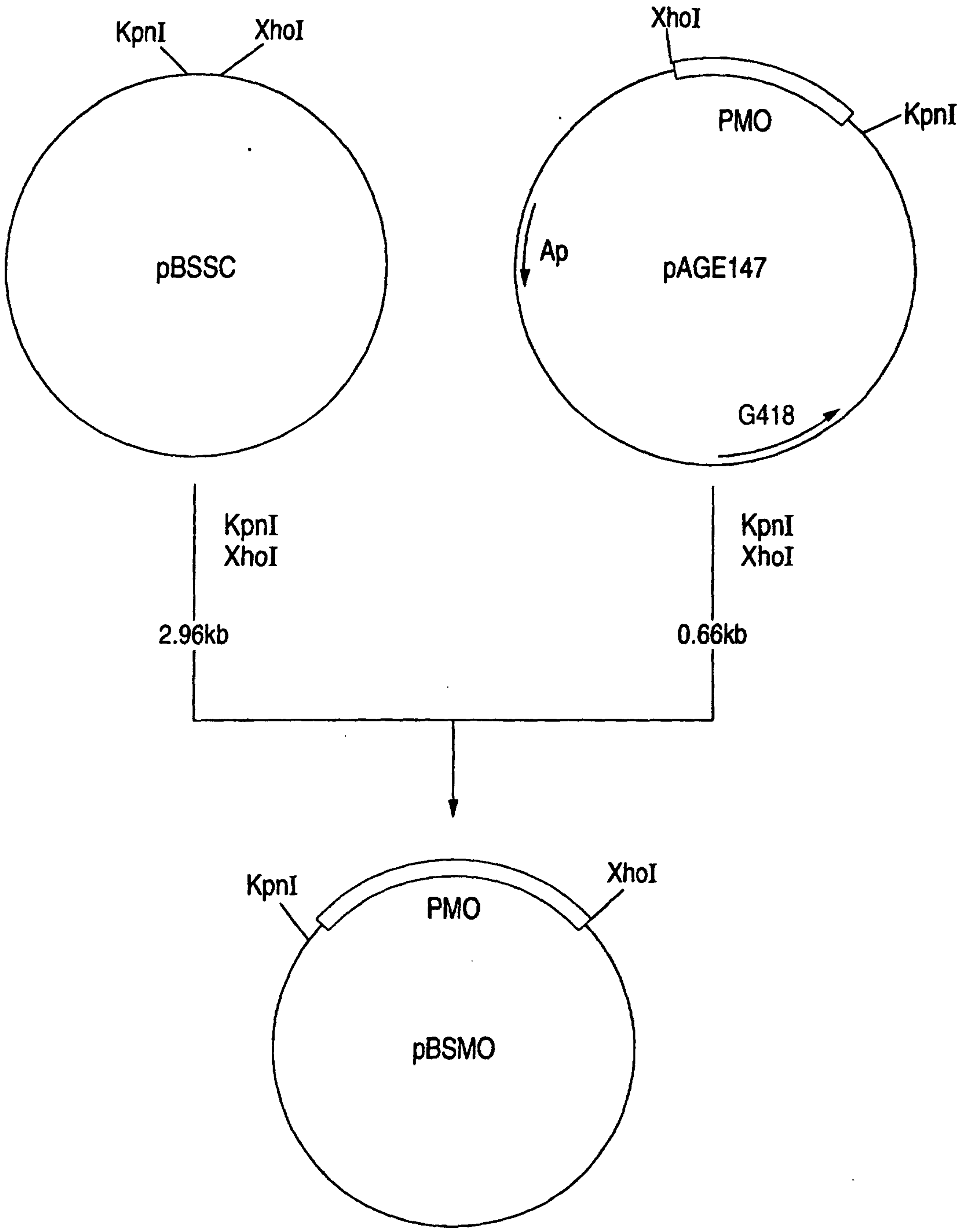
KpnI
XhoI
pBSSC
XhoI
PMO
KpnI
Ap
pAGE147
G418
KpnI
XhoI
2.96kb
KpnI
XhoI
0.66kb
KpnI
PMO
XhoI
pBSMO

*FIG. 13*

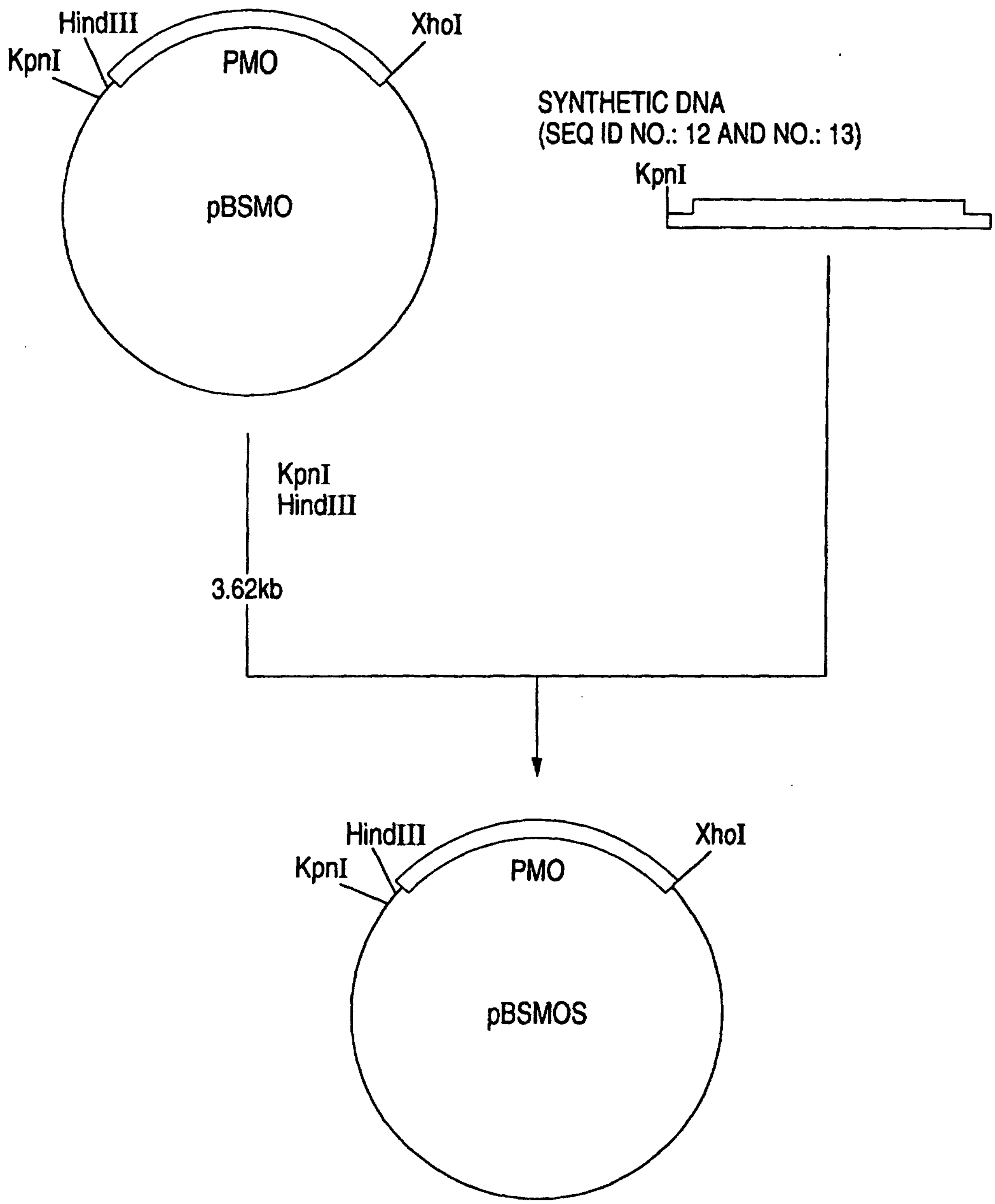
HindIII
KpnI
PMO
XhoI
pBSMO
SYNTHETIC DNA
(SEQ ID NO.: 12 AND NO.: 13)
KpnI
KpnI
HindIII
3.62kb
HindIII
KpnI
PMO
XhoI
pBSMOS

FIG. 14

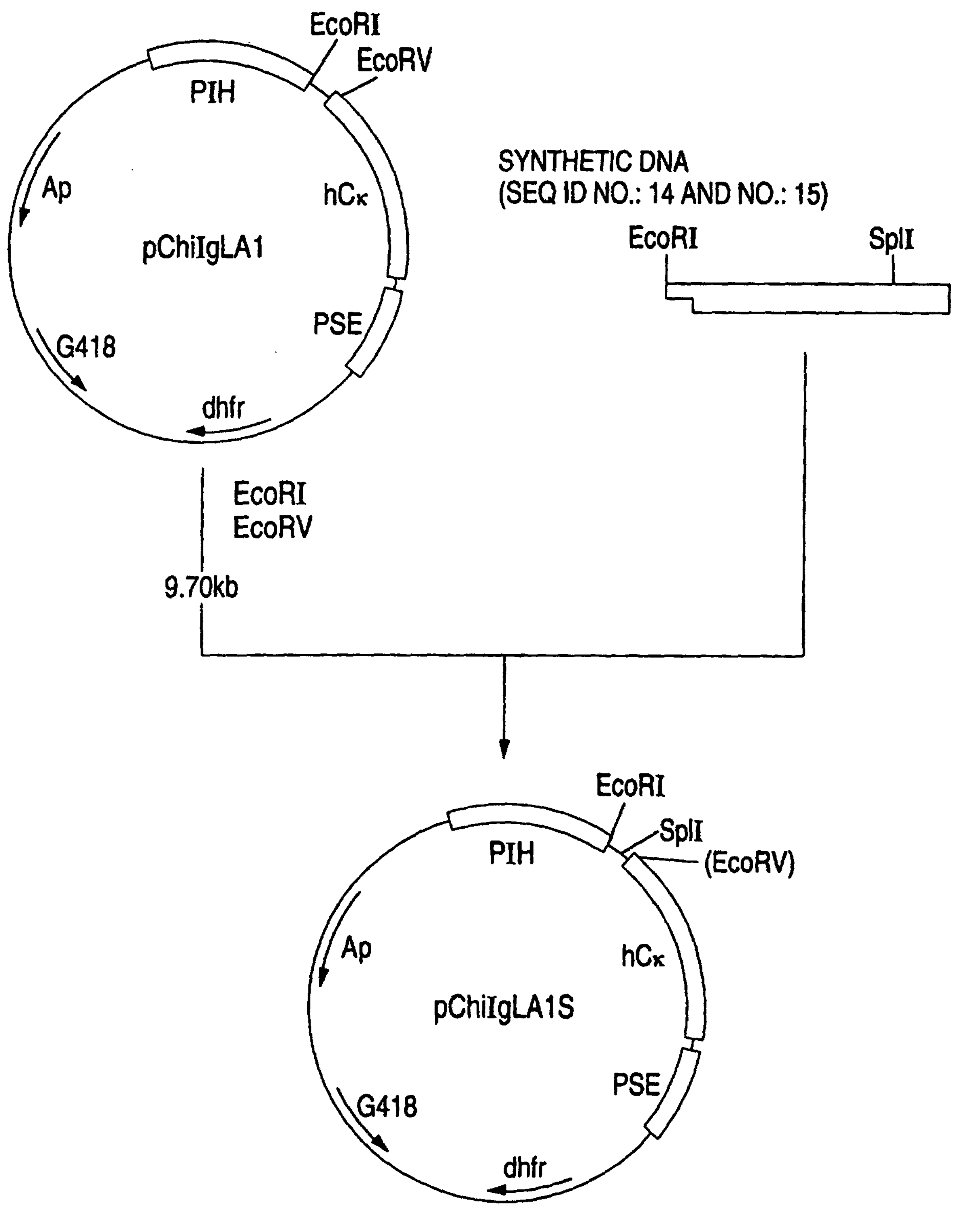
EcoRI
EcoRV
PIH
Ap
hCκ
pChiIgLA1
G418
PSE
dhfr
SYNTHETIC DNA
(SEQ ID NO.: 14 AND NO.: 15)
EcoRI
SplI
EcoRI
EcoRV
9.70kb
EcoRI
SplI
(EcoRV)
PIH
Ap
hCκ
pChiIgLA1S
G418
PSE
dhfr

FIG. 15

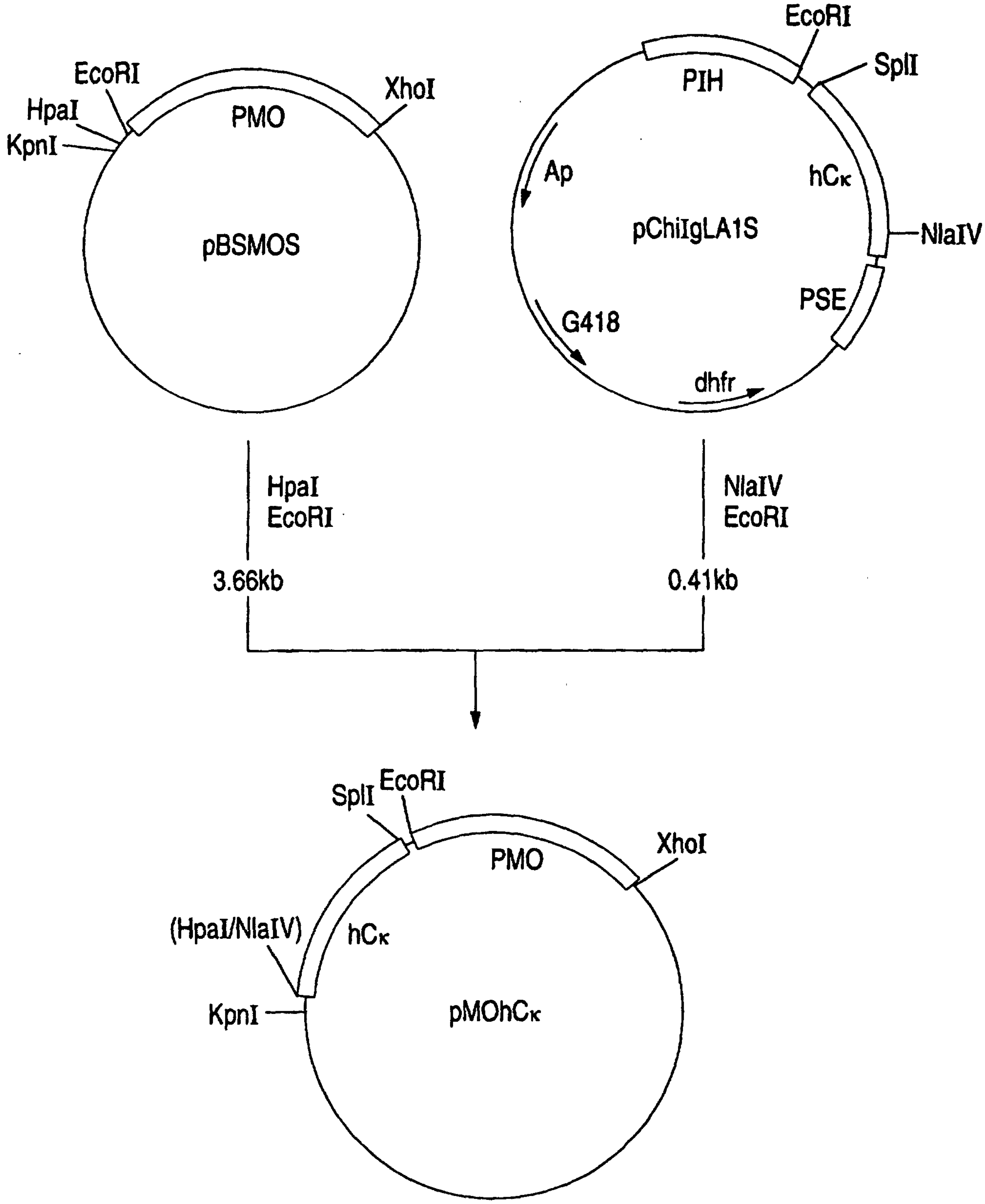
EcoRI
HpaI
KpnI
PMO
XhoI
pBSMOS
EcoRI
SplI
PIH
Ap
hCκ
pChiIgLA1S
NlaIV
PSE
G418
dhfr
HpaI
EcoRI
NlaIV
EcoRI
3.66kb
0.41kb
SplI
EcoRI
PMO
XhoI
(HpaI/NlaIV)
hCκ
KpnI
pMOhCκ

FIG. 16

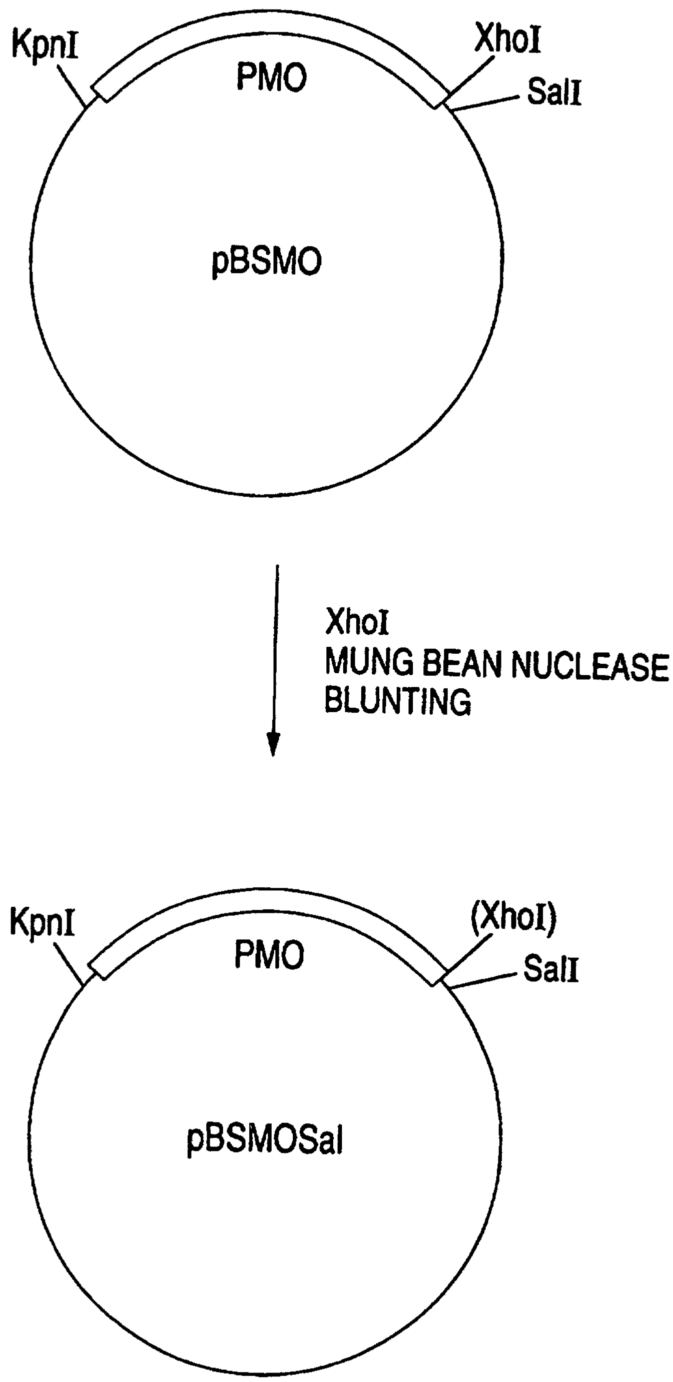
KpnI
PMO
XhoI
SalI
pBSMO
XhoI
MUNG BEAN NUCLEASE
BLUNTING
KpnI
PMO
(XhoI)
SalI
pBSMOSal

*FIG. 17*

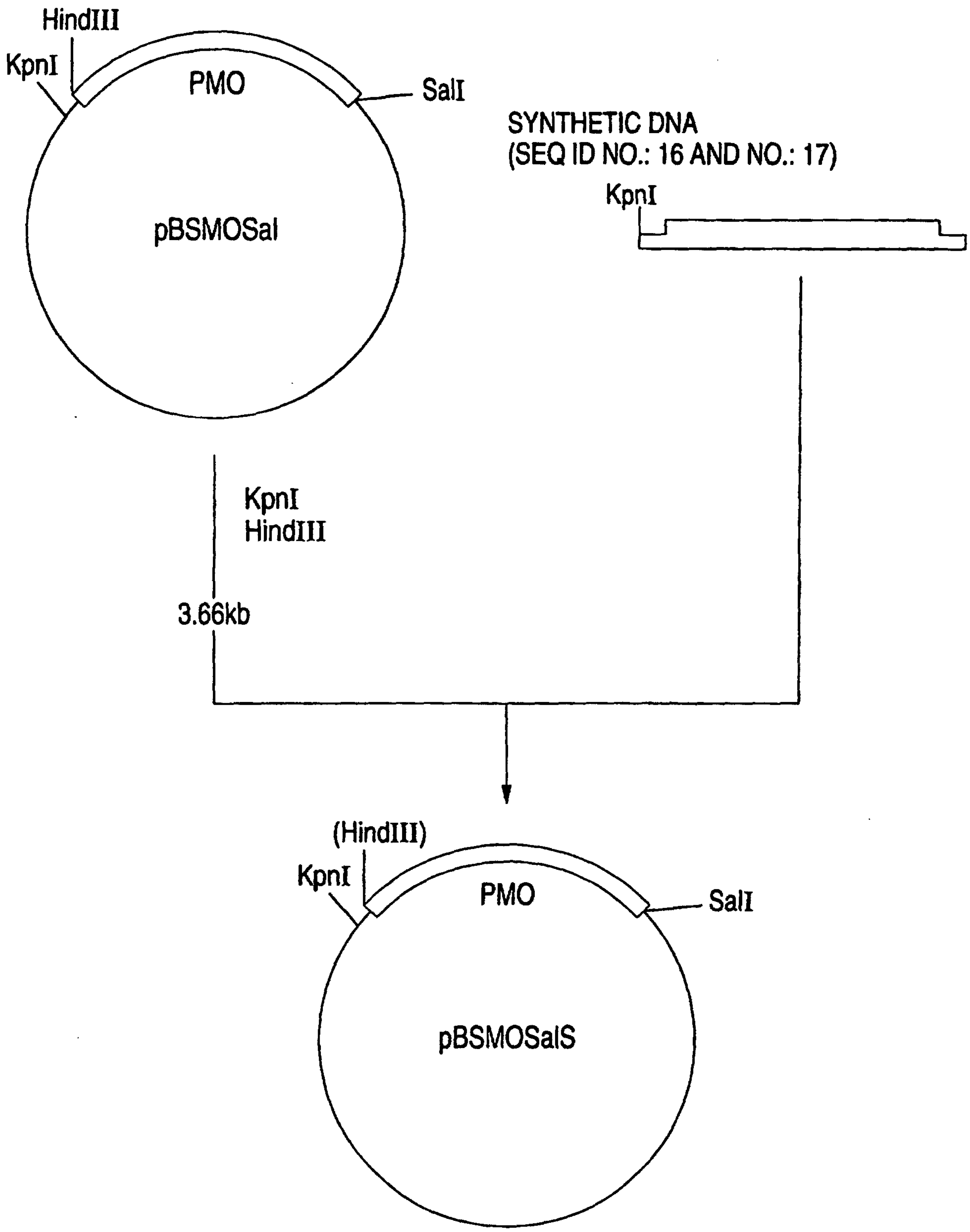
HindIII
KpnI
PMO
SalI
pBSMOSal
SYNTHETIC DNA
(SEQ ID NO.: 16 AND NO.: 17)
KpnI
KpnI
HindIII
3.66kb
(HindIII)
KpnI
PMO
SalI
pBSMOSalS

FIG. 18

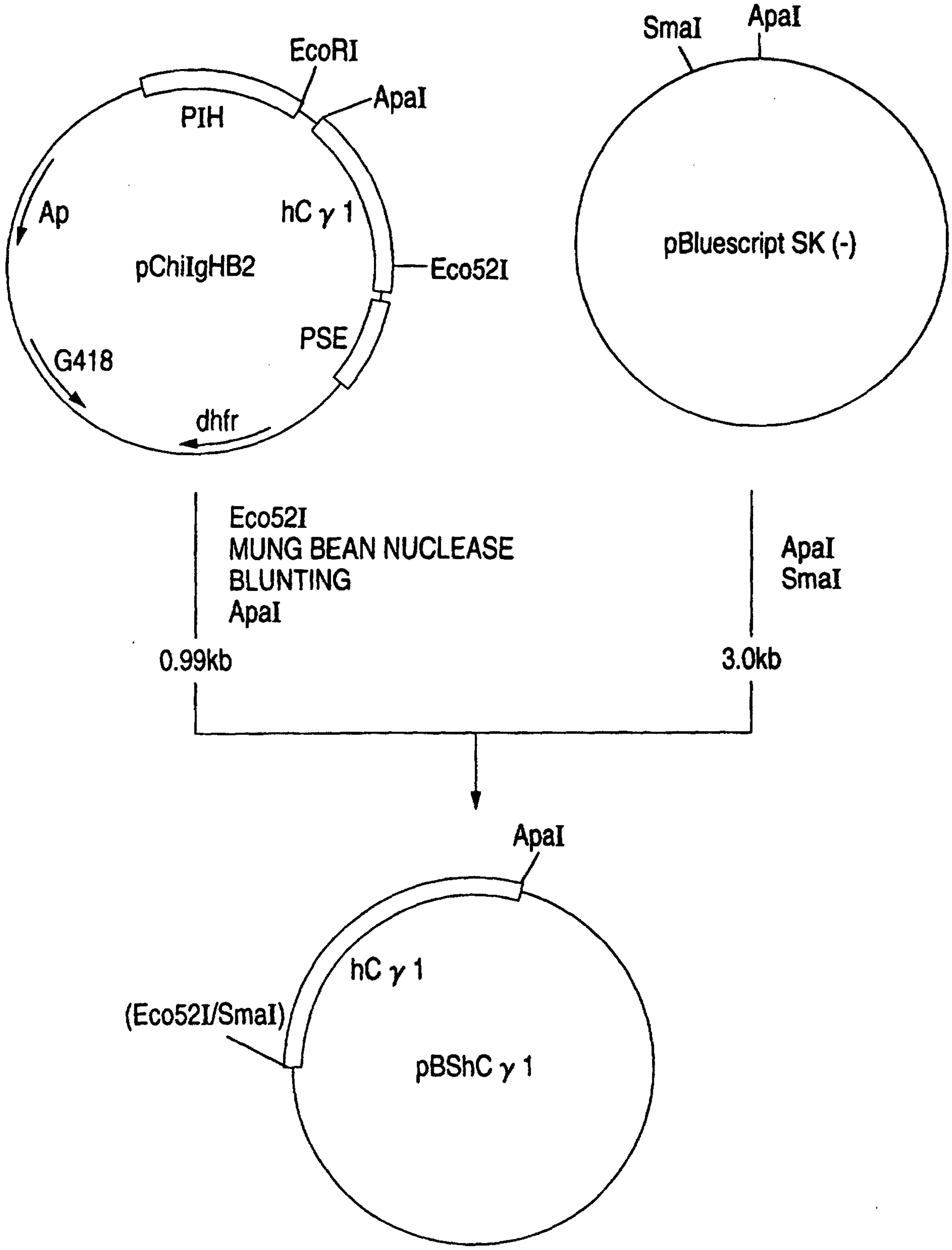
EcoRI
ApaI
PIH
Ap
hC γ 1
pChiIgHB2
Eco52I
PSE
G418
dhfr
SmaI
ApaI
pBluescript SK (-)
Eco52I
MUNG BEAN NUCLEASE
BLUNTING
ApaI
0.99kb
ApaI
SmaI
3.0kb
ApaI
hC γ 1
(Eco52I/SmaI)
pBShC γ 1

FIG. 19

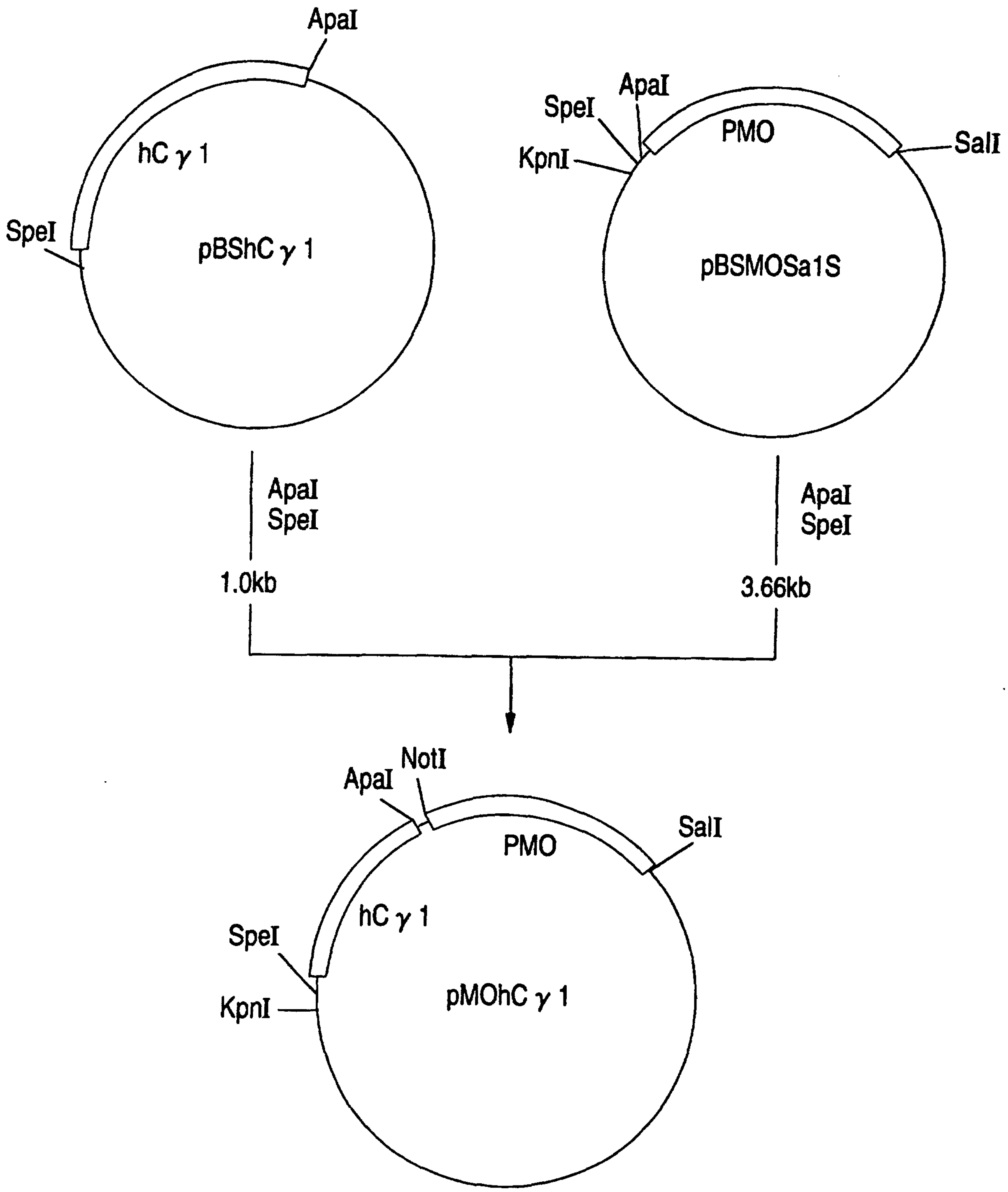
ApaI
hCγ1
SpeI
pBShCγ1
ApaI
SpeI
KpnI
PMO
SalI
pBSMOSa1S
ApaI
SpeI
1.0kb
ApaI
SpeI
3.66kb
NotI
ApaI
PMO
SalI
hCγ1
SpeI
KpnI
pMOhCγ1

*FIG. 20*

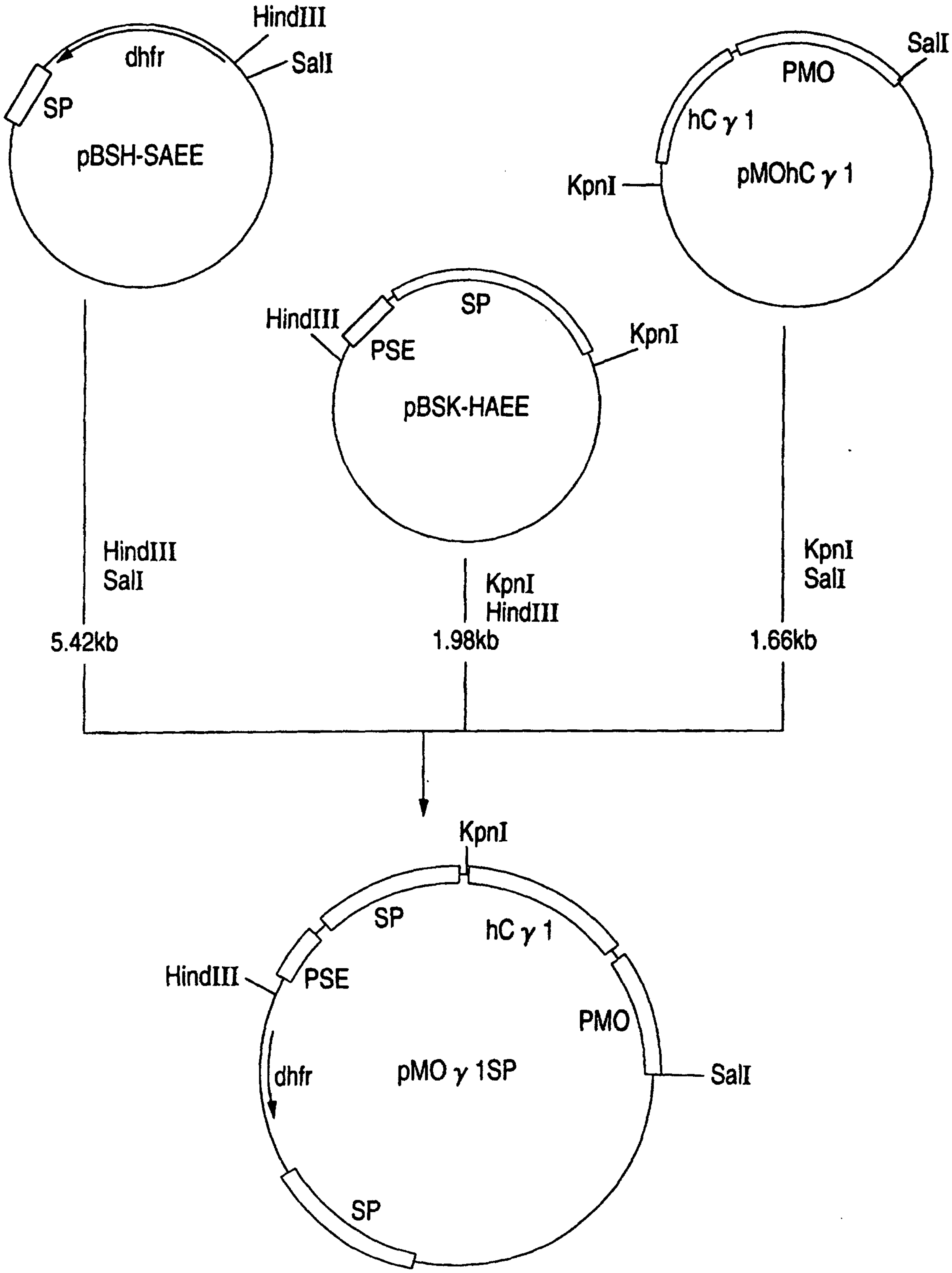
HindIII
SalI
dhfr
SP
pBSH-SAEE
SalI
PMO
hC γ 1
KpnI
pMOhC γ 1
HindIII
SP
PSE
KpnI
pBSK-HAEE
HindIII
SalI
5.42kb
KpnI
HindIII
1.98kb
KpnI
SalI
1.66kb
KpnI
SP
hC γ 1
HindIII
PSE
PMO
dhfr
pMO γ 1SP
SalI
SP

FIG. 21

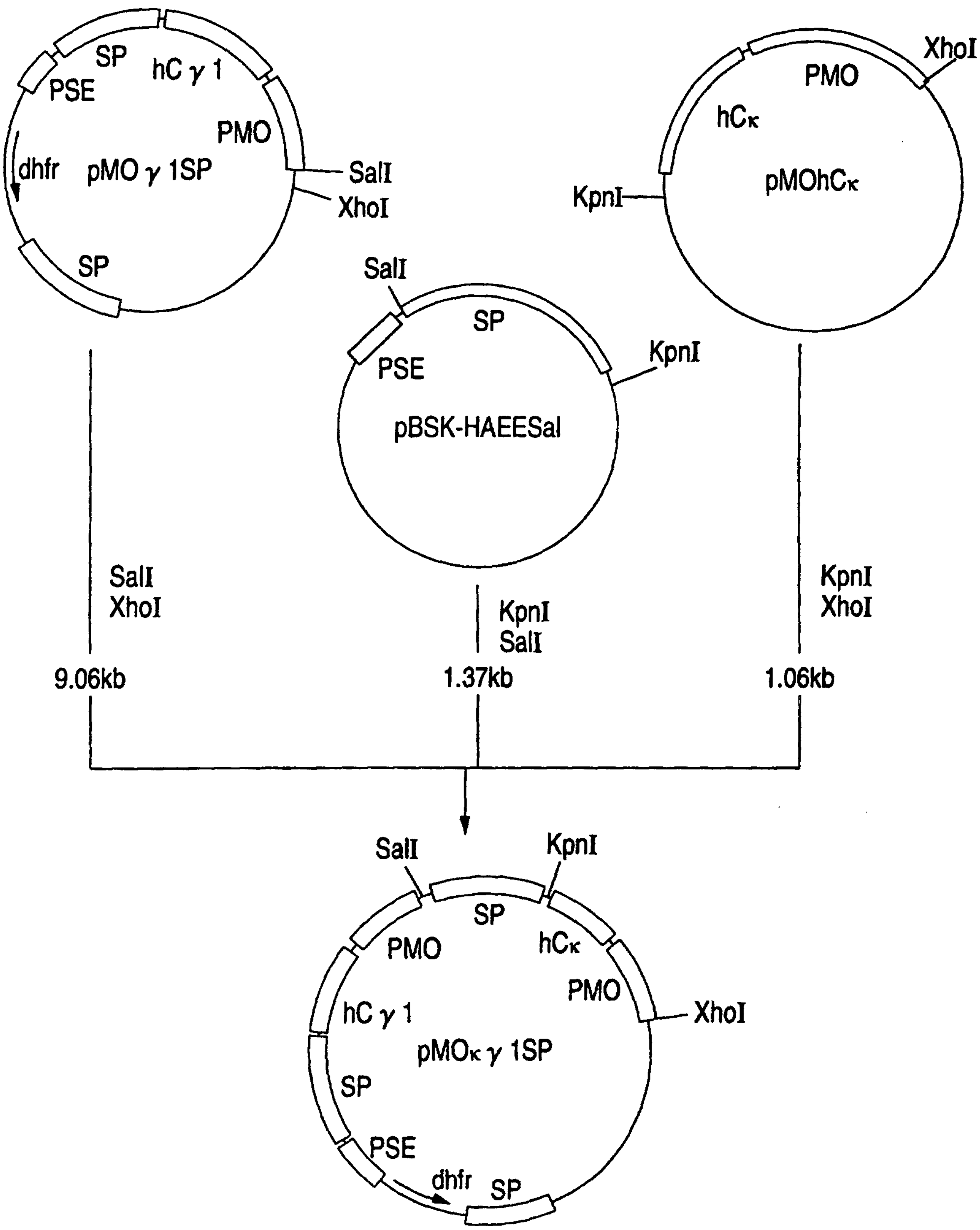
SP
hC γ 1
PSE
PMO
dhfr
pMO γ 1SP
SalI
XhoI
SP
XhoI
PMO
hCκ
pMOhCκ
KpnI
SalI
SP
KpnI
PSE
pBSK-HAEESal
SalI
XhoI
KpnI
SalI
KpnI
XhoI
9.06kb
1.37kb
1.06kb
SalI
KpnI
SP
PMO
hCκ
PMO
XhoI
hC γ 1
pMOκ γ 1SP
SP
PSE
dhfr
SP

FIG. 22

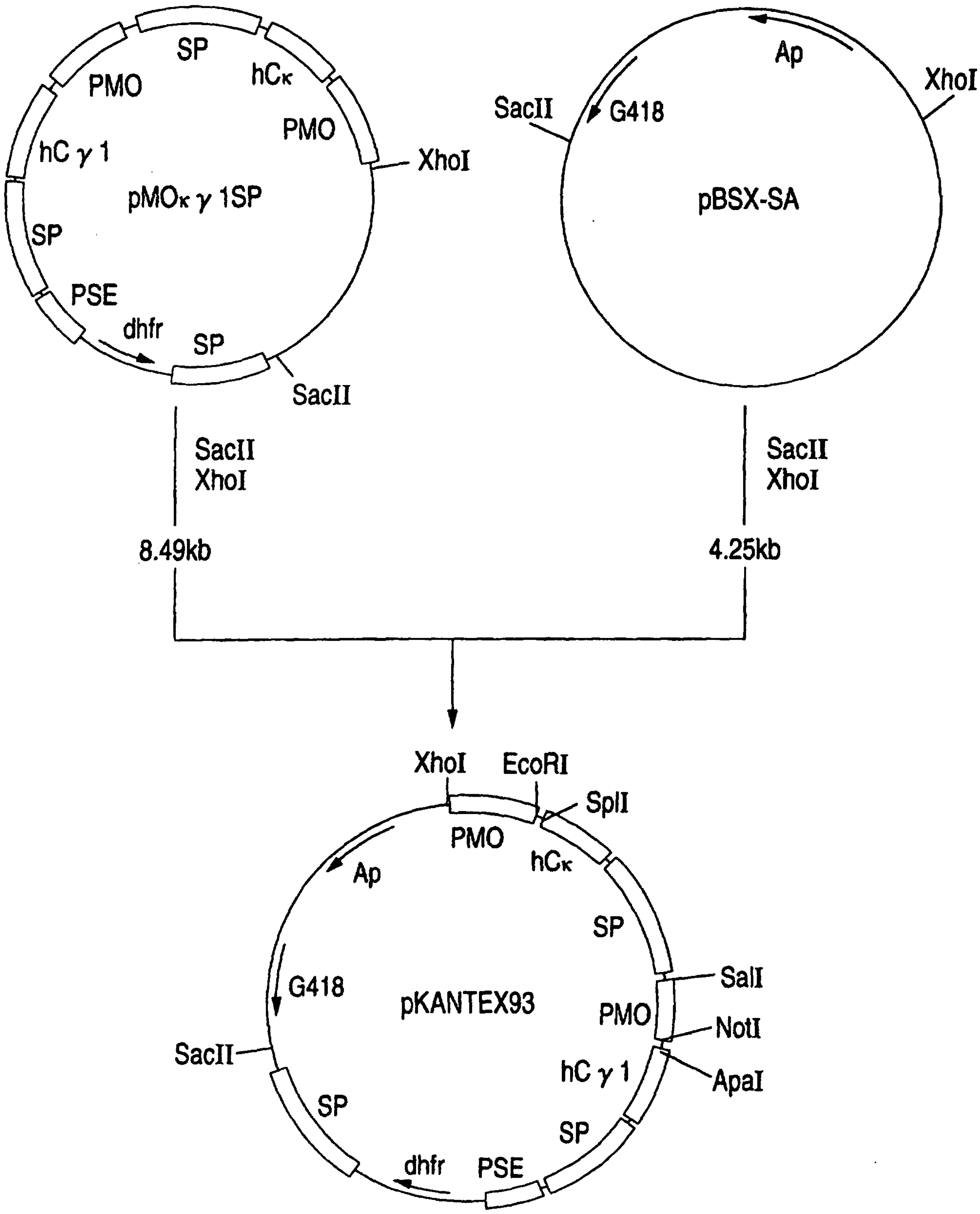
SP
PMO
hCκ
PMO
XhoI
hCγ1
pMOκγ1SP
SP
PSE
dhfr
SP
SacII
Ap
XhoI
SacII
G418
pBSX-SA
SacII
XhoI
8.49kb
SacII
XhoI
4.25kb
XhoI
EcoRI
SplI
PMO
Ap
hCκ
SP
SalI
G418
pKANTEX93
PMO
NotI
SacII
hCγ1
ApaI
SP
SP
dhfr
PSE

FIG. 23

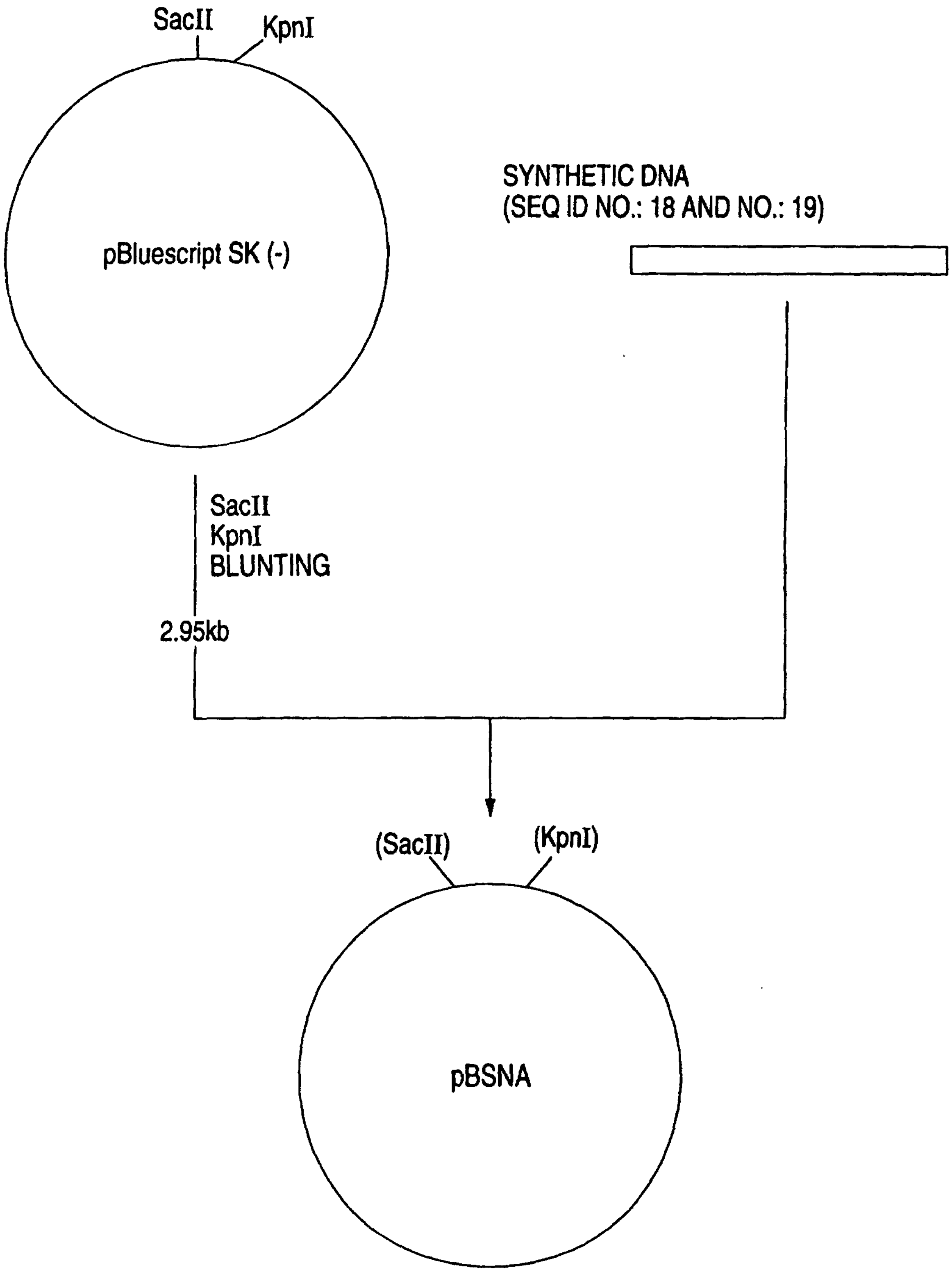
SacII
KpnI
pBluescript SK (-)
SYNTHETIC DNA
(SEQ ID NO.: 18 AND NO.: 19)
SacII
KpnI
BLUNTING
2.95kb
(SacII)
(KpnI)
pBSNA

FIG. 24

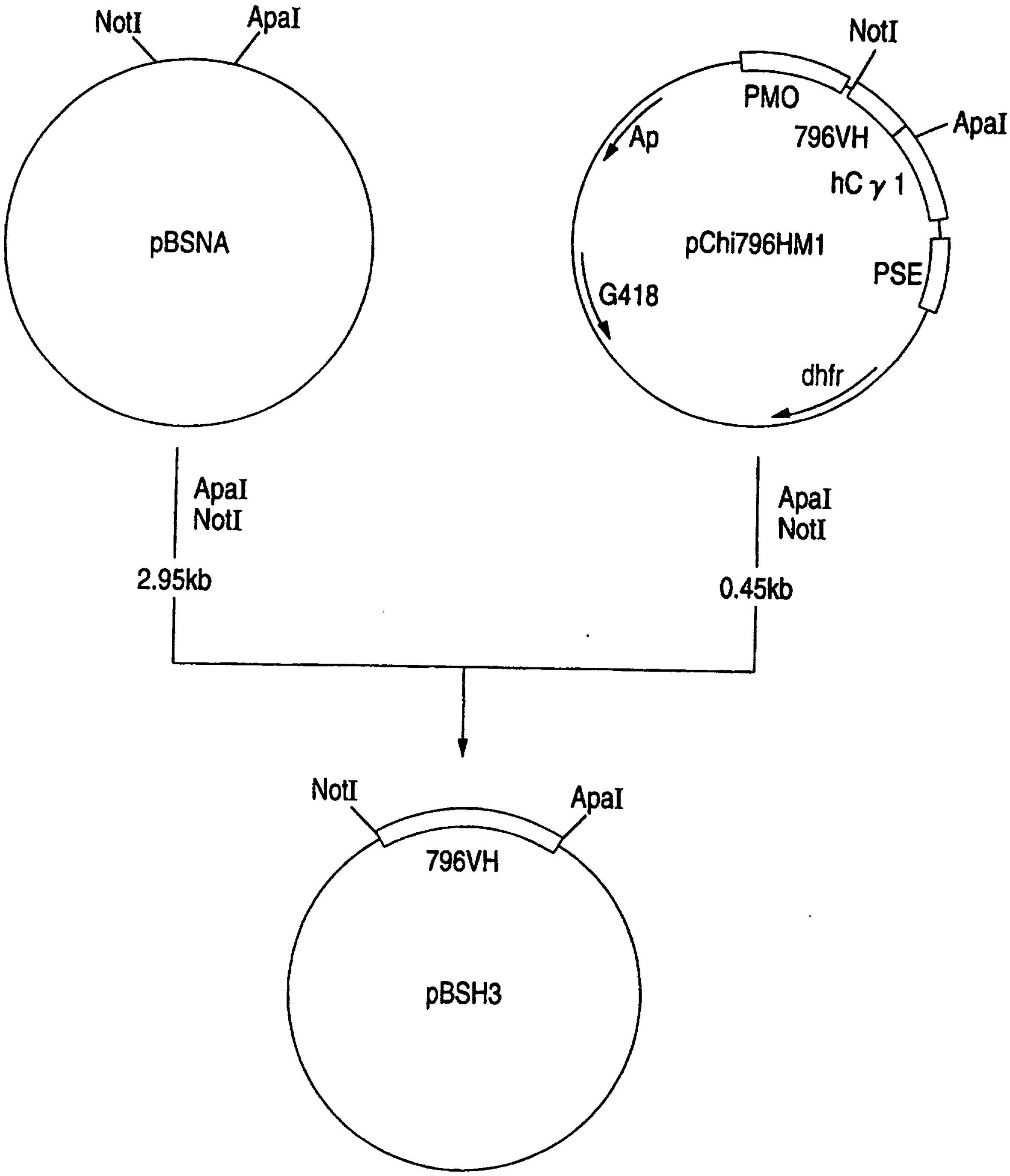
NotI
ApaI
pBSNA
NotI
PMO
ApaI
Ap
796VH
hC γ 1
pChi796HM1
PSE
G418
dhfr
ApaI
NotI
2.95kb
ApaI
NotI
0.45kb
NotI
ApaI
796VH
pBSH3

FIG. 25

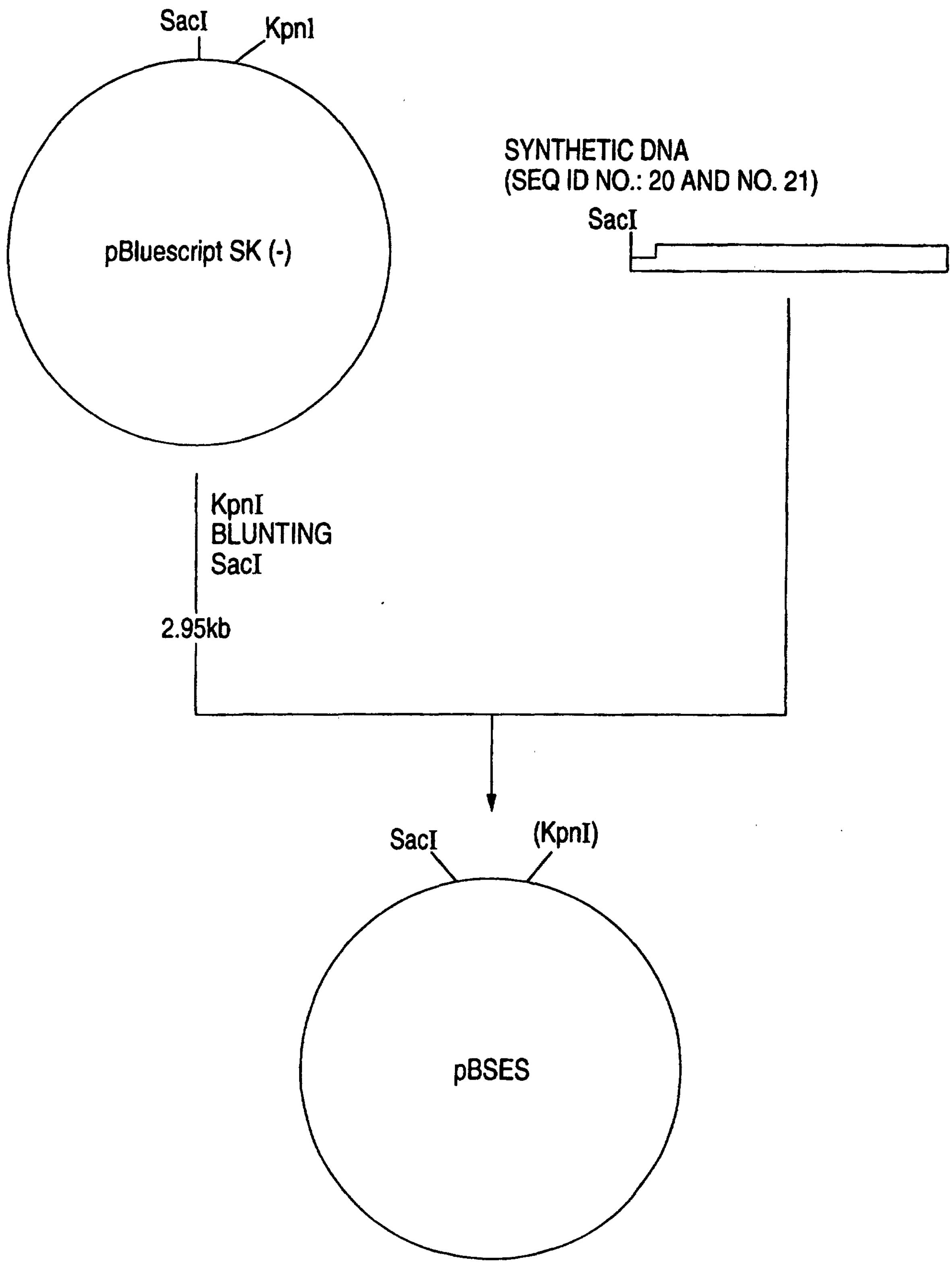
SacI
KpnI
pBluescript SK (-)
SYNTHETIC DNA
(SEQ ID NO.: 20 AND NO. 21)
SacI
KpnI
BLUNTING
SacI
2.95kb
SacI
(KpnI)
pBSES

FIG. 26

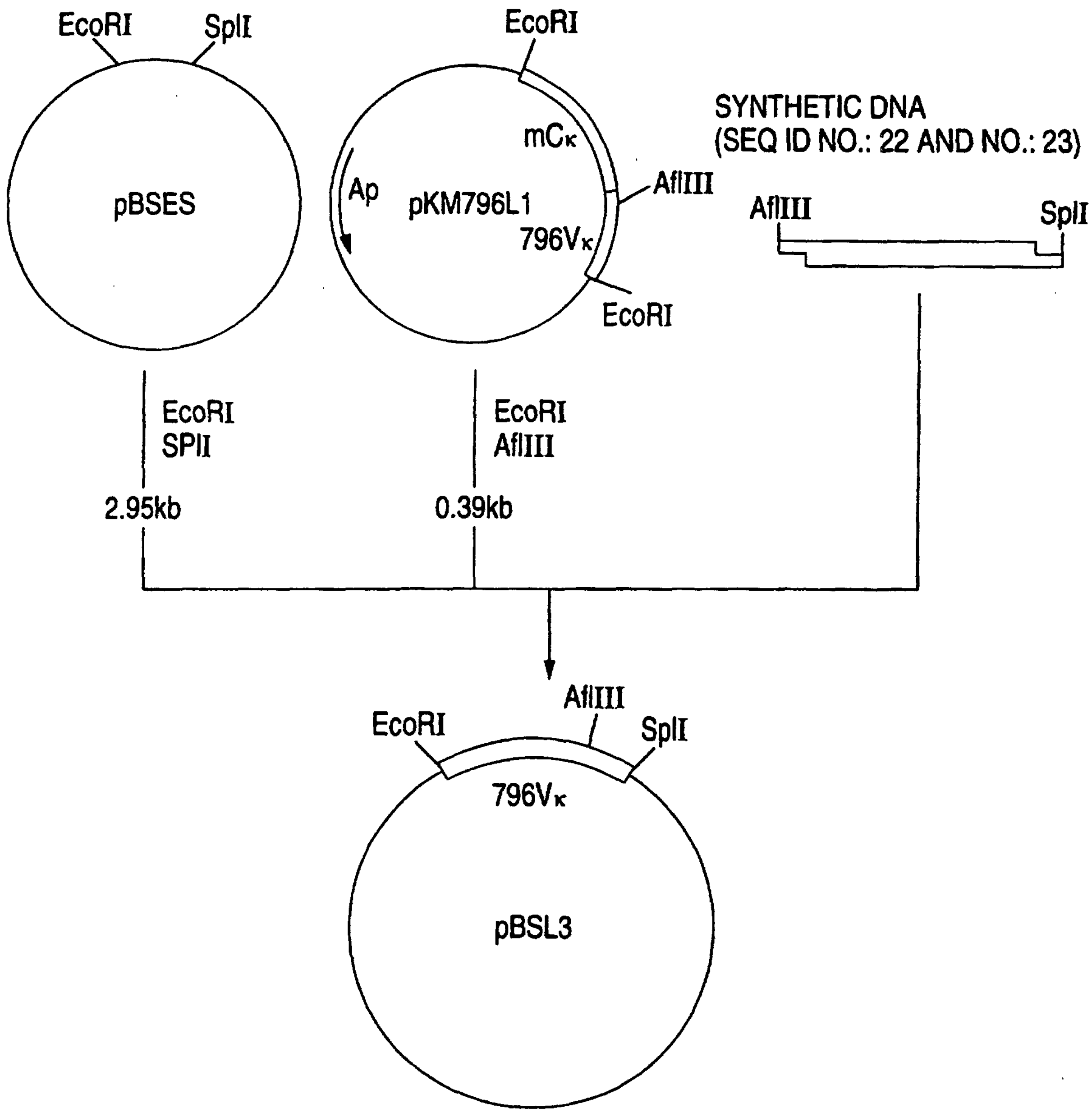
EcoRI
SplI
pBSES
EcoRI
mCκ
Ap
pKM796L1
AflIII
796Vκ
EcoRI
SYNTHETIC DNA
(SEQ ID NO.: 22 AND NO.: 23)
AflIII
SplI
EcoRI
SPlI
2.95kb
EcoRI
AflIII
0.39kb
AflIII
EcoRI
SplI
796Vκ
pBSL3

FIG. 27

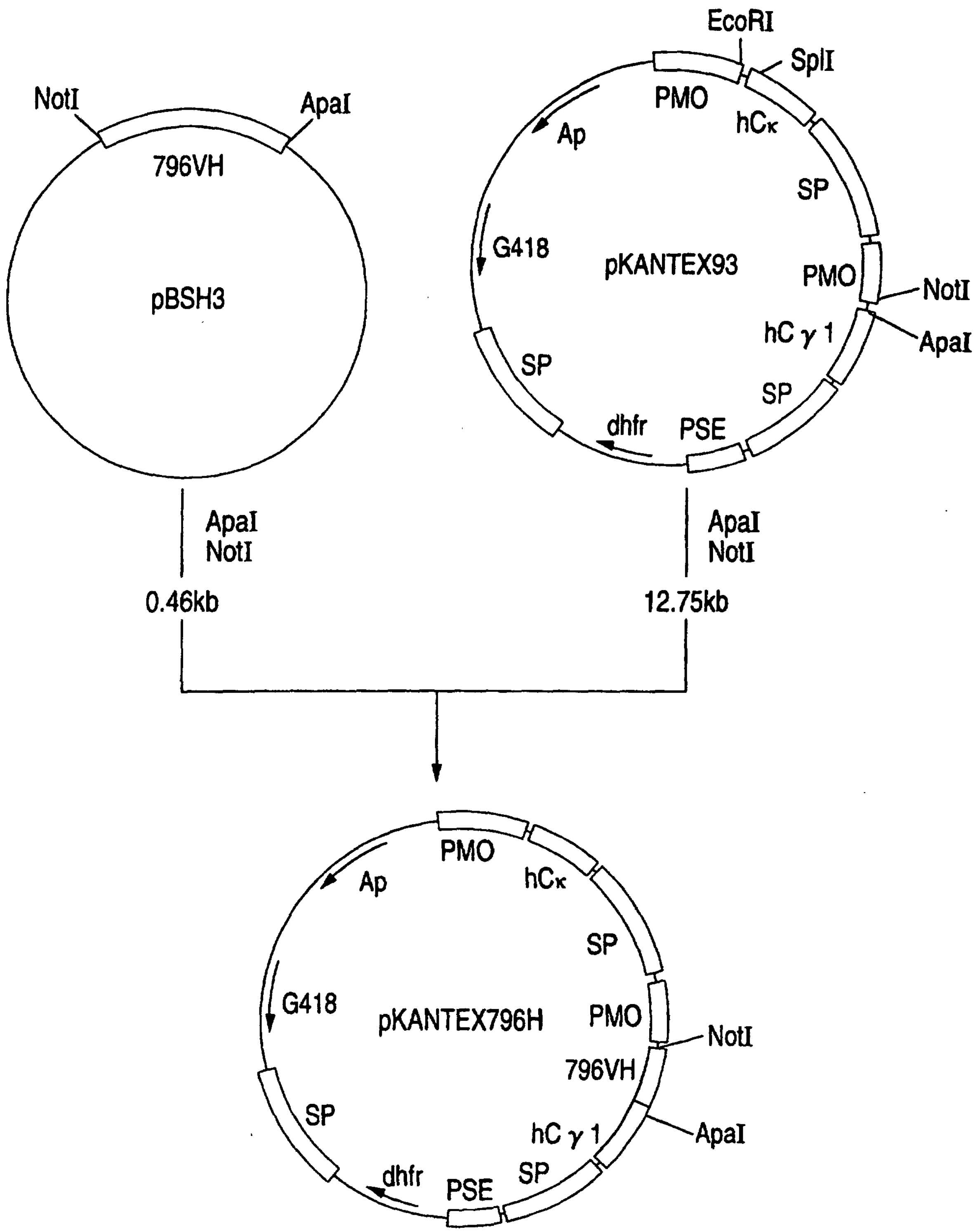
NotI
ApaI
796VH
pBSH3
EcoRI
SplI
PMO
hCκ
Ap
SP
G418
pKANTEX93
PMO
NotI
ApaI
hCγ1
SP
SP
dhfr
PSE
ApaI
NotI
0.46kb
ApaI
NotI
12.75kb
PMO
hCκ
Ap
SP
G418
pKANTEX796H
PMO
NotI
796VH
SP
hCγ1
ApaI
SP
dhfr
PSE

FIG. 28

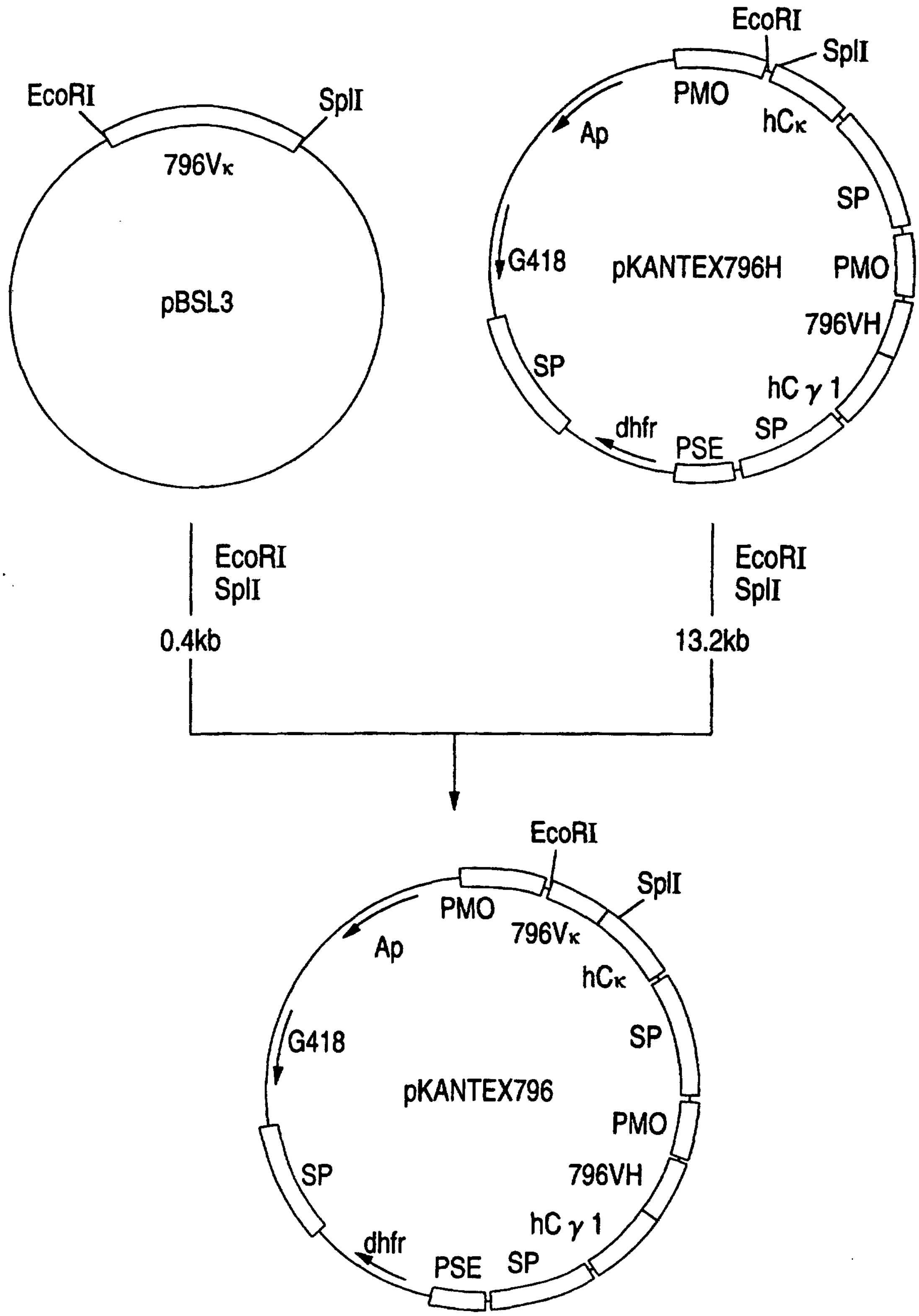
EcoRI
SplI
796Vκ
pBSL3
EcoRI
SplI
PMO
hCκ
Ap
SP
G418
pKANTEX796H
PMO
796VH
SP
hC γ 1
dhfr
PSE
SP
EcoRI
SplI
0.4kb
EcoRI
SplI
13.2kb
EcoRI
SplI
PMO
796Vκ
Ap
hCκ
G418
SP
pKANTEX796
PMO
796VH
SP
hC γ 1
dhfr
PSE
SP

FIG. 29

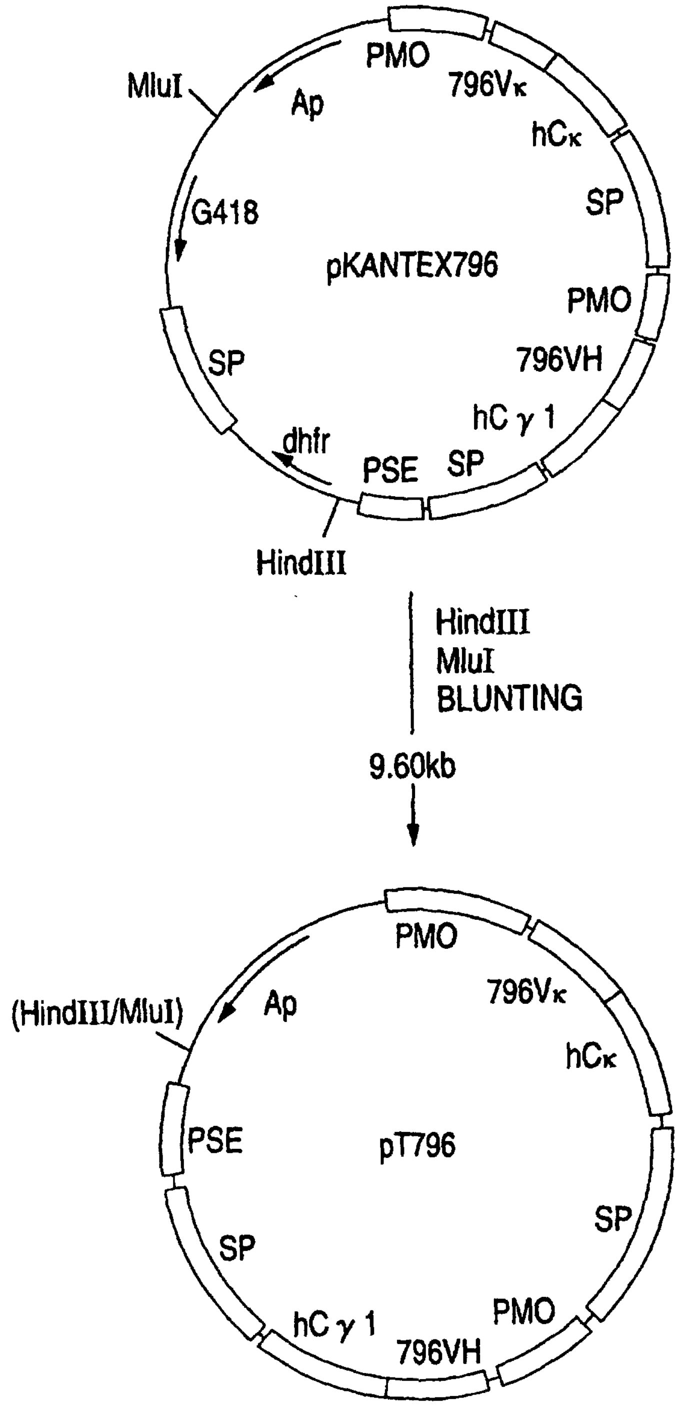
MluI
PMO
Ap
796Vκ
hCκ
G418
SP
pKANTEX796
PMO
SP
796VH
hCγ1
dhfr
PSE
SP
HindIII
HindIII
MluI
BLUNTING
9.60kb
PMO
796Vκ
(HindIII/MluI)
Ap
hCκ
PSE
pT796
SP
SP
PMO
hCγ1
796VH

FIG. 30

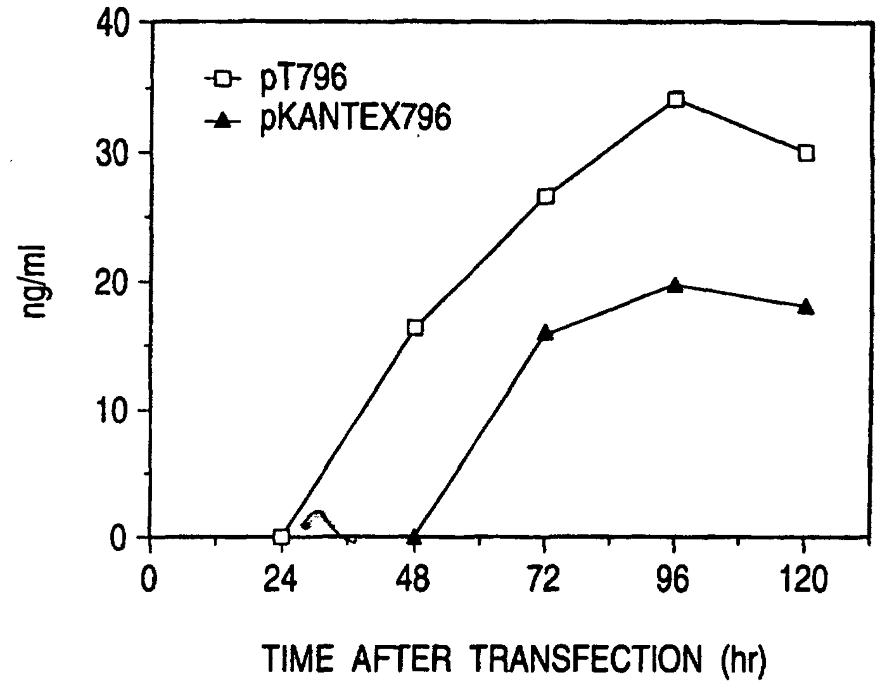
40
30
20
10
0
ng/ml
pT796
pKANTEX796
0
24
48
72
96
120
TIME AFTER TRANSFECTION (hr)

*FIG. 31*

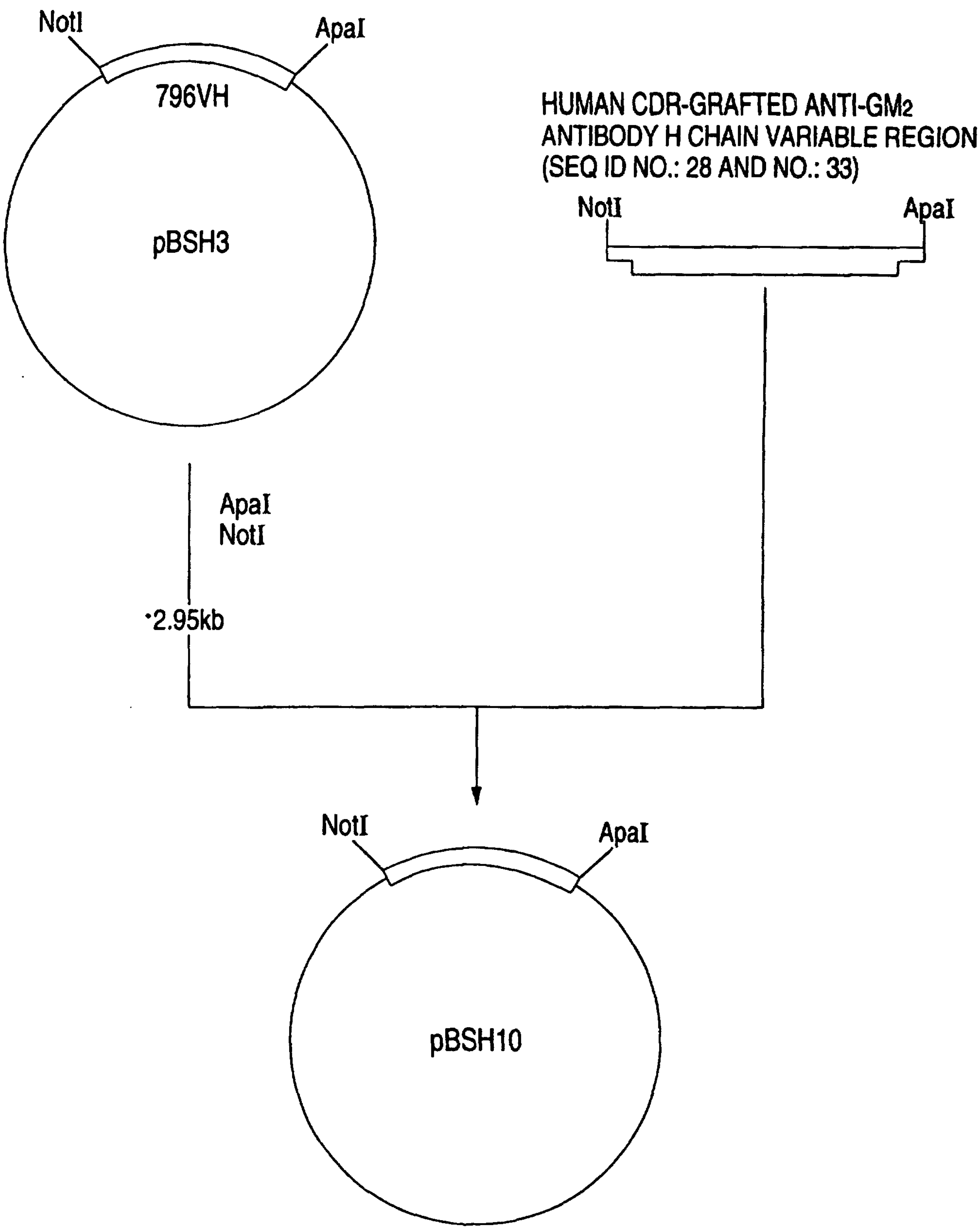
NotI
ApaI
796VH
pBSH3
HUMAN CDR-GRAFTED ANTI-GM2
ANTIBODY H CHAIN VARIABLE REGION
(SEQ ID NO.: 28 AND NO.: 33)
NotI
ApaI
ApaI
NotI
2.95kb
NotI
ApaI
pBSH10

FIG. 32

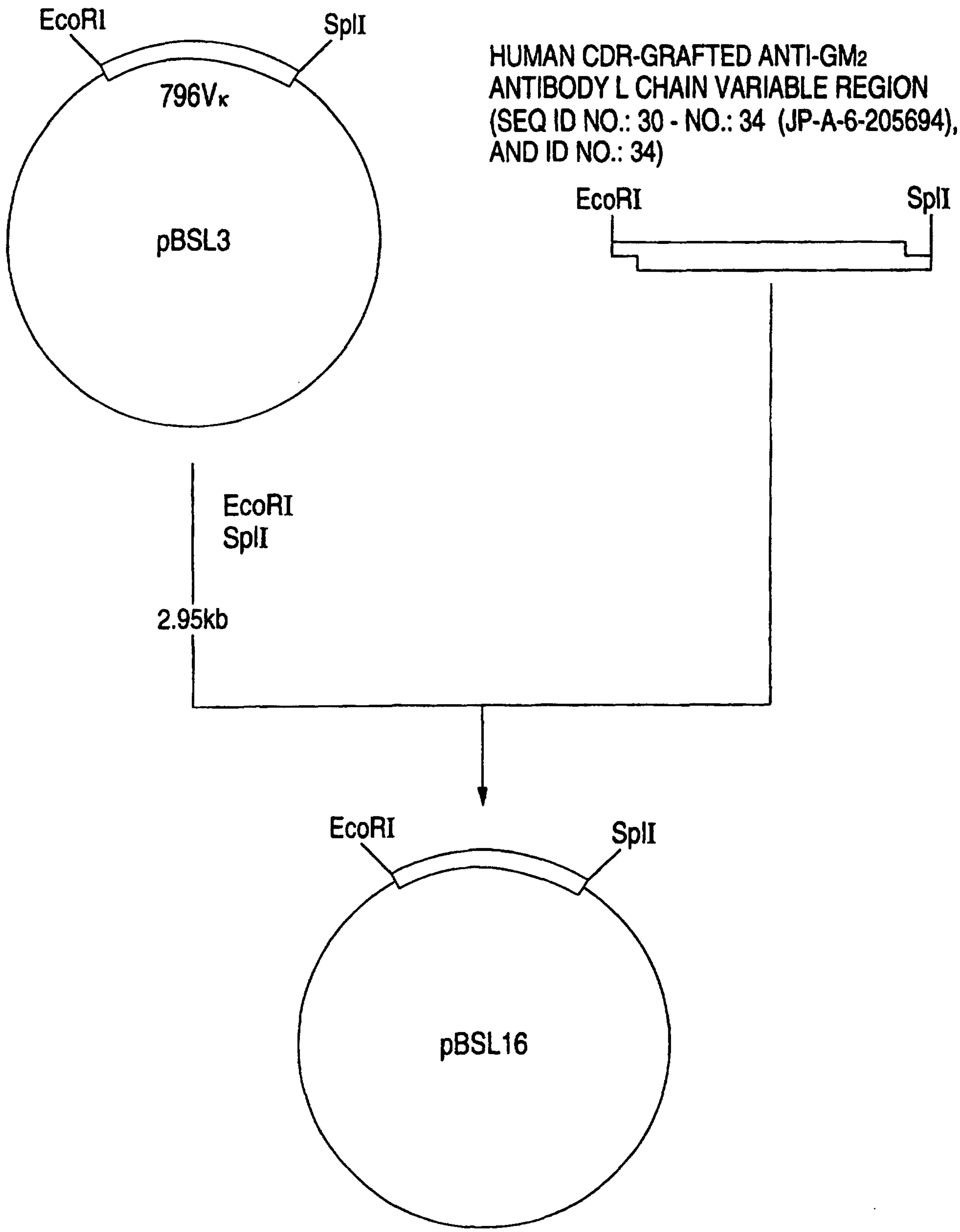
EcoRI
SplI
796Vκ
pBSL3
HUMAN CDR-GRAFTED ANTI-GM2
ANTIBODY L CHAIN VARIABLE REGION
(SEQ ID NO.: 30 - NO.: 34 (JP-A-6-205694),
AND ID NO.: 34)
EcoRI
SplI
EcoRI
SplI
2.95kb
EcoRI
SplI
pBSL16

FIG. 33

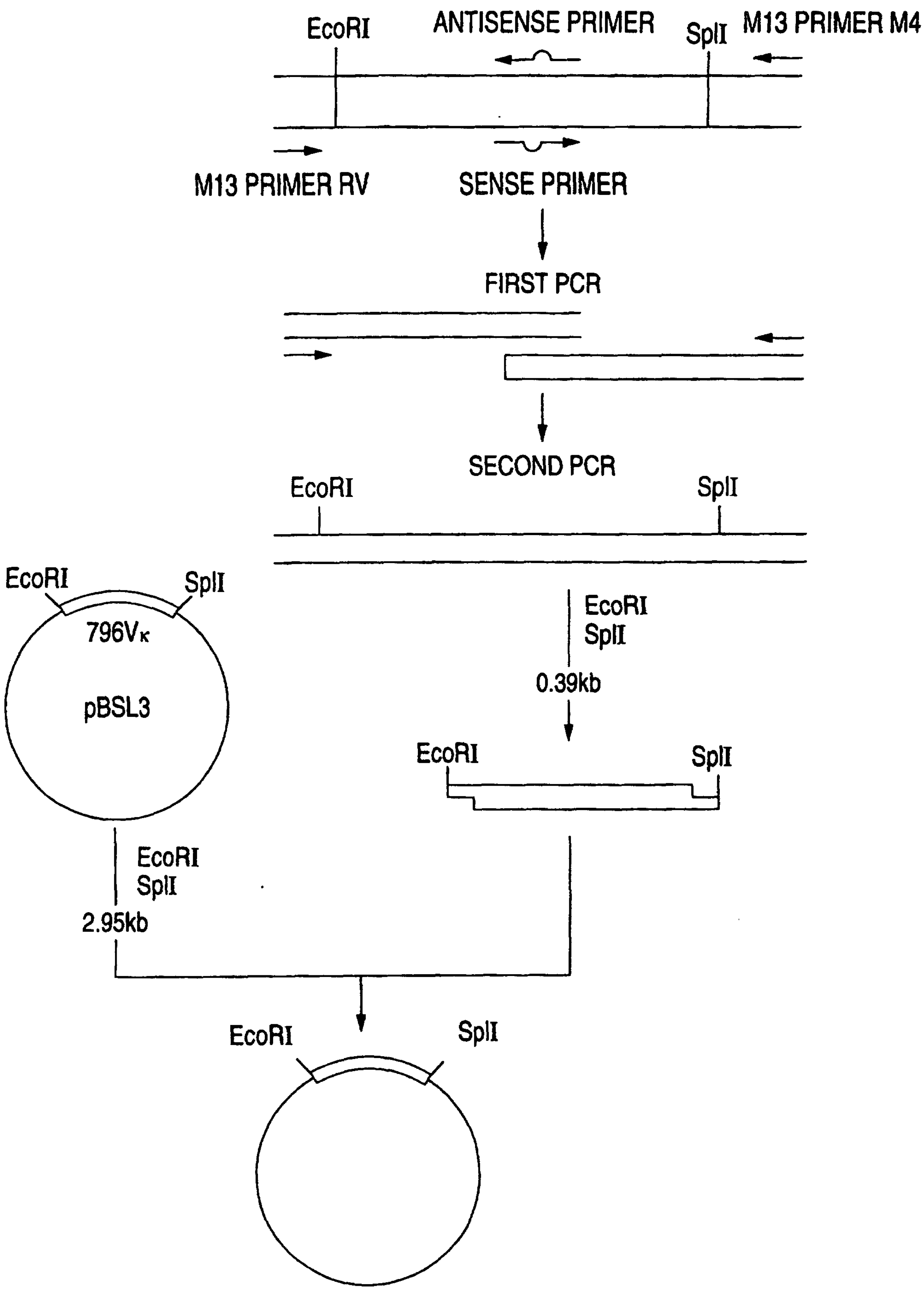
EcoRI
ANTISENSE PRIMER
SplI
M13 PRIMER M4
M13 PRIMER RV
SENSE PRIMER
FIRST PCR
SECOND PCR
EcoRI
SplI
EcoRI
SplI
796Vκ
pBSL3
EcoRI
SplI
0.39kb
EcoRI
SplI
EcoRI
SplI
2.95kb
EcoRI
SplI

FIG. 34

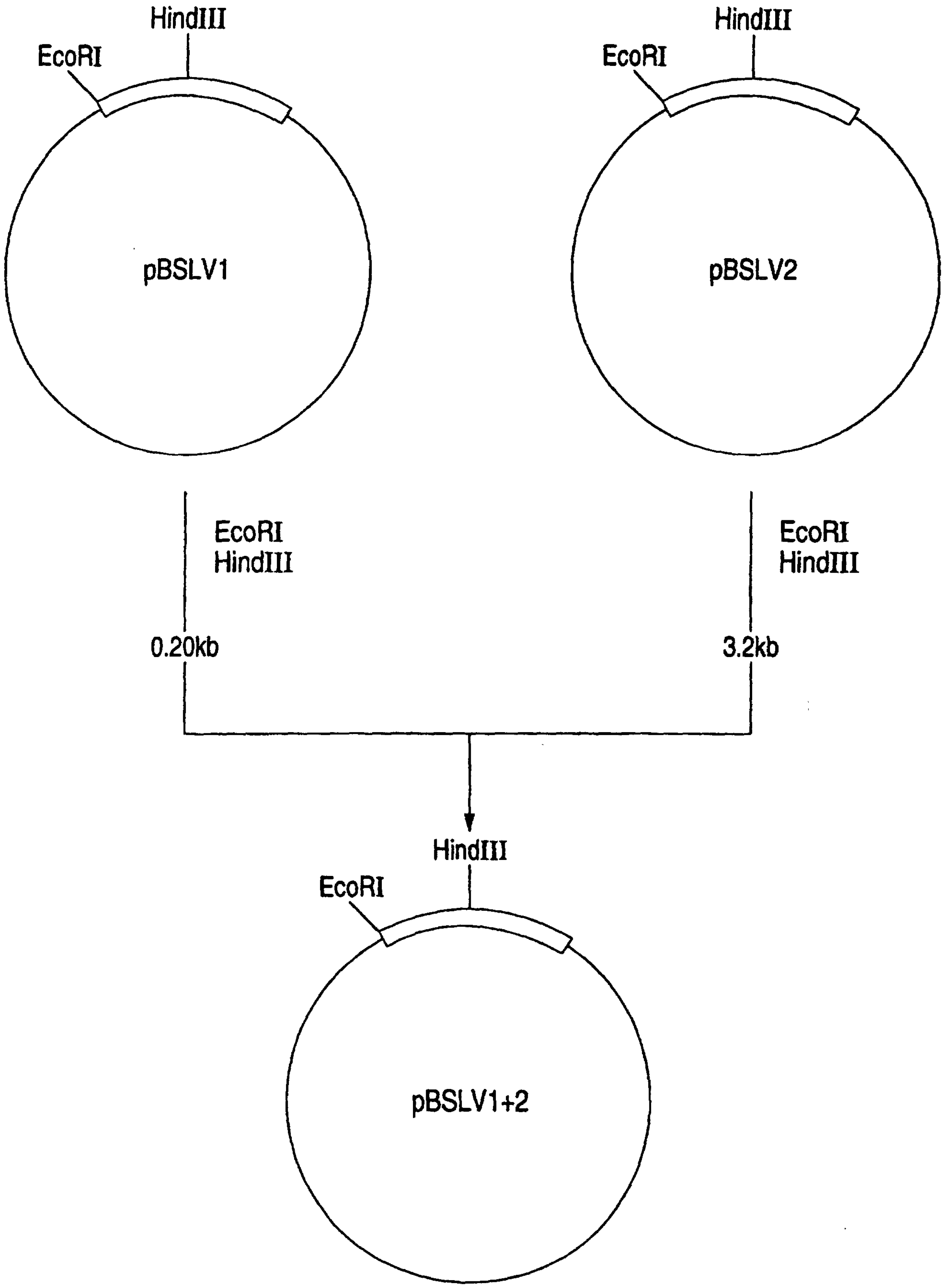
HindIII
EcoRI
pBSLV1
HindIII
EcoRI
pBSLV2
EcoRI
HindIII
0.20kb
EcoRI
HindIII
3.2kb
HindIII
EcoRI
pBSLV1+2

FIG. 35

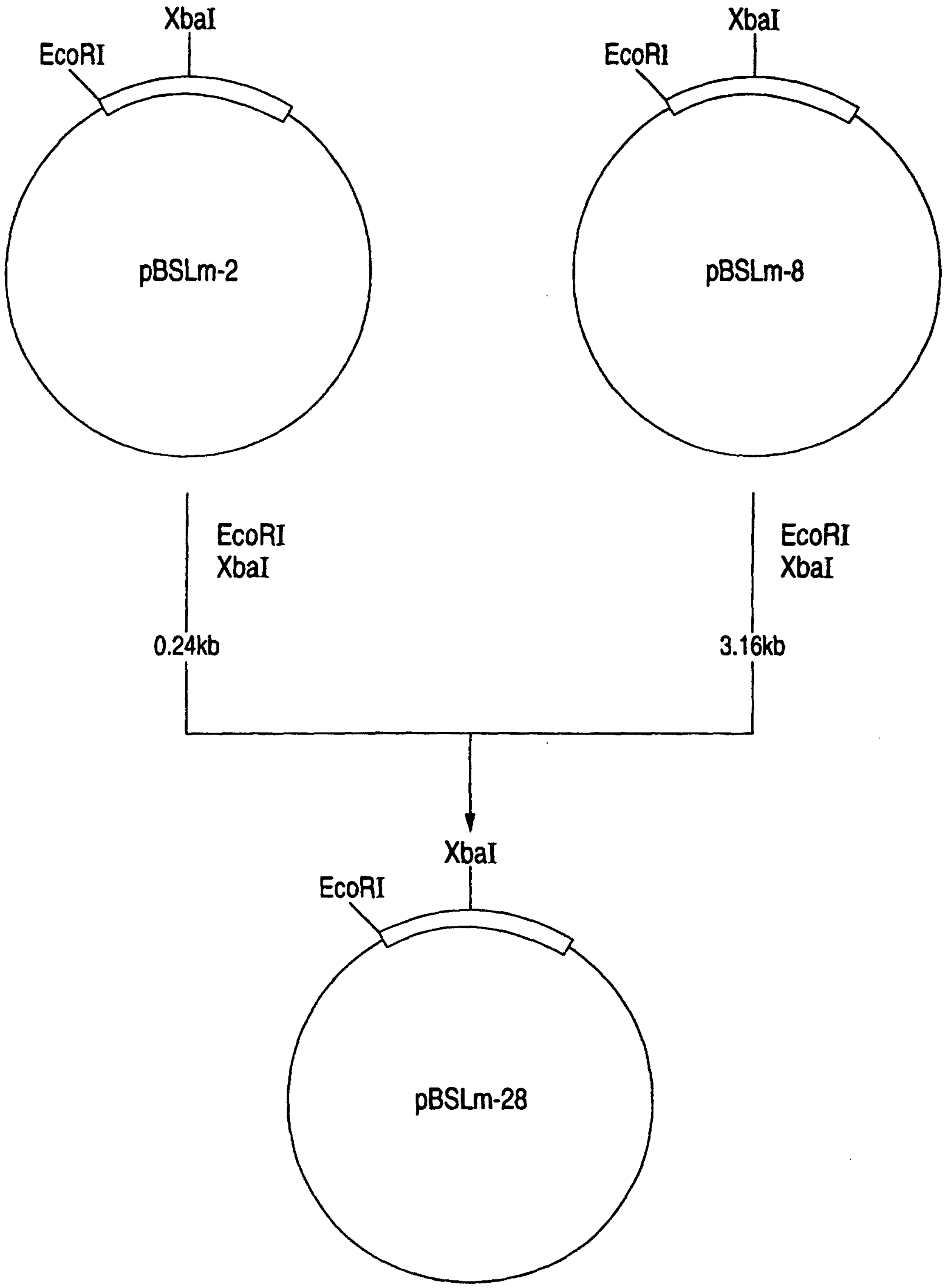
XbaI
EcoRI
pBSLm-2
XbaI
EcoRI
pBSLm-8
EcoRI
XbaI
0.24kb
EcoRI
XbaI
3.16kb
XbaI
EcoRI
pBSLm-28

FIG. 36

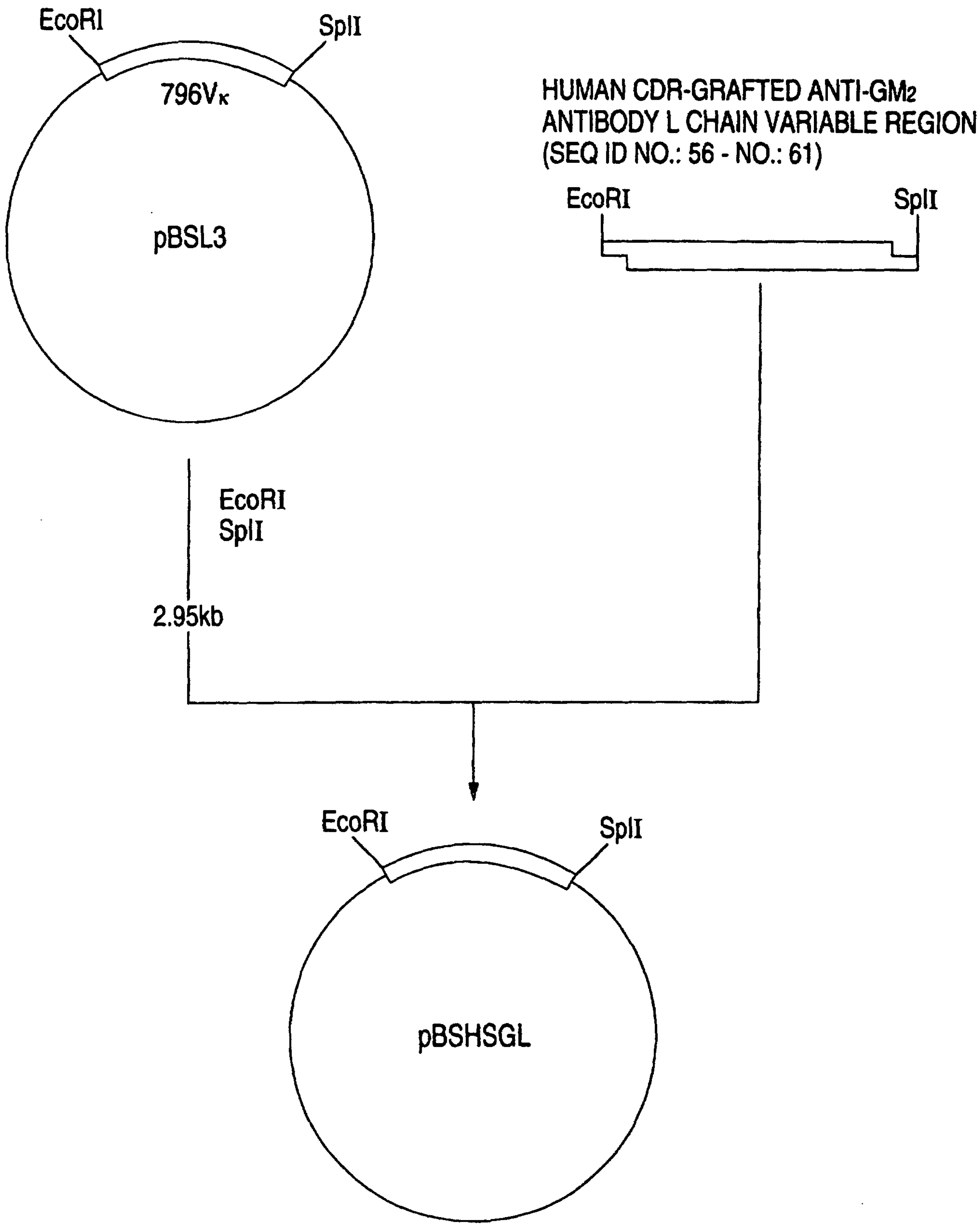
EcoRI
SplI
796Vκ
pBSL3
HUMAN CDR-GRAFTED ANTI-GM2
ANTIBODY L CHAIN VARIABLE REGION
(SEQ ID NO.: 56 - NO.: 61)
EcoRI
SplI
EcoRI
SplI
2.95kb
EcoRI
SplI
pBSHSGL

FIG. 37

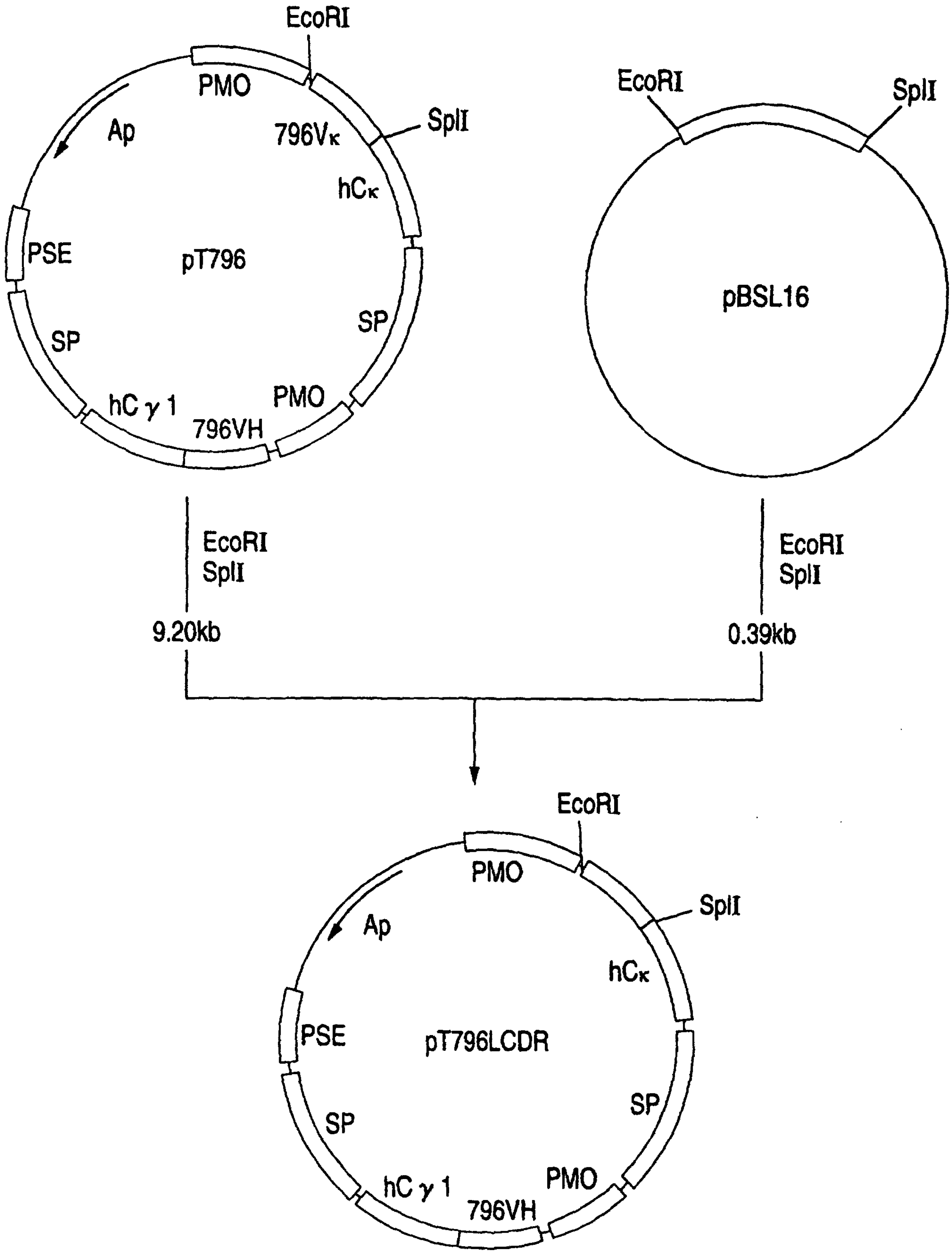
EcoRI
PMO
SplI
796Vκ
hCκ
Ap
PSE
pT796
SP
SP
PMO
hC γ 1
796VH
EcoRI
SplI
pBSL16
EcoRI
SplI
9.20kb
EcoRI
SplI
0.39kb
EcoRI
PMO
SplI
Ap
hCκ
PSE
pT796LCDR
SP
SP
PMO
hC γ 1
796VH

FIG. 38

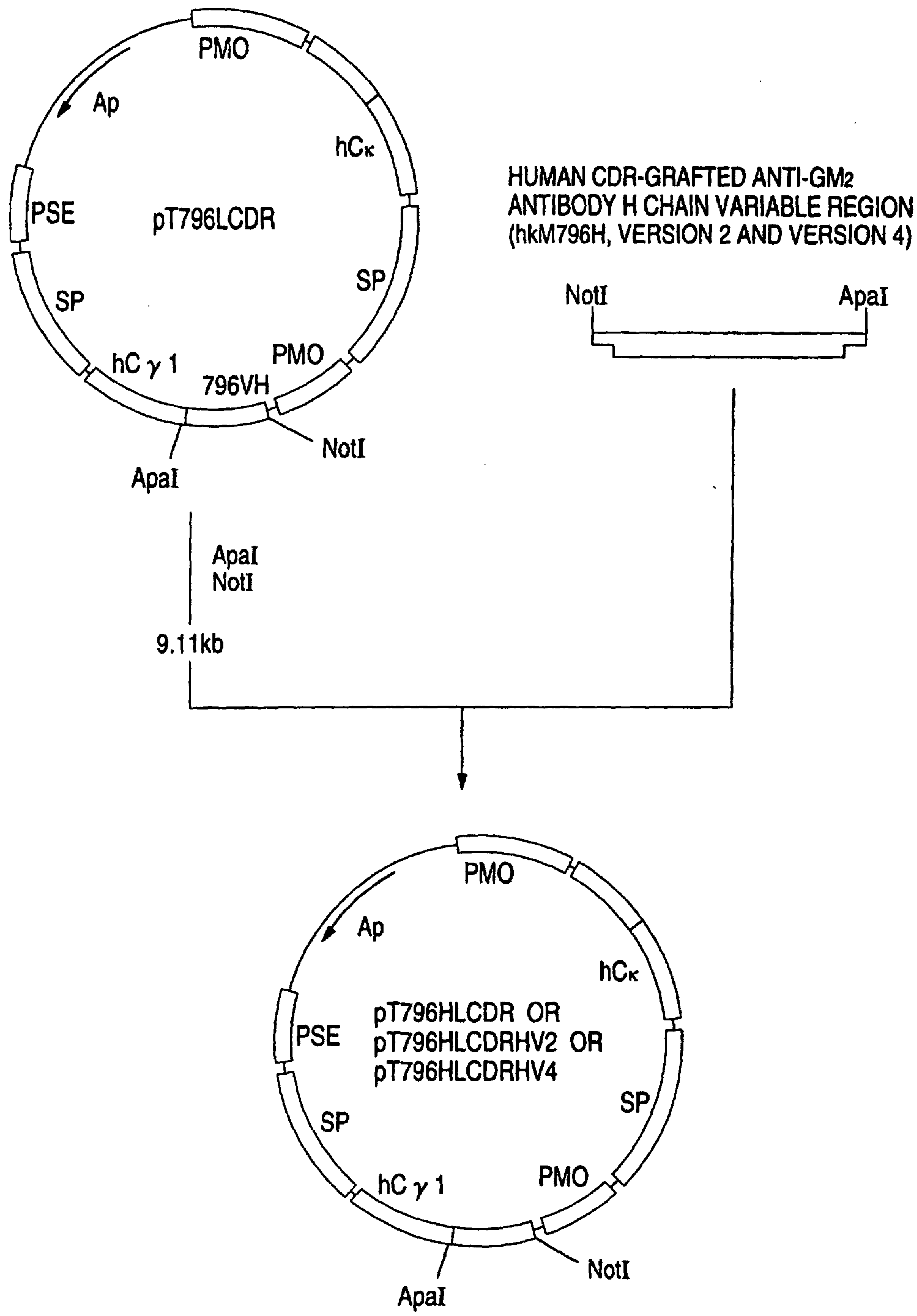
PMO
Ap
hCκ
PSE
pT796LCDR
SP
SP
hCγ1
PMO
796VH
NotI
ApaI
HUMAN CDR-GRAFTED ANTI-GM2
ANTIBODY H CHAIN VARIABLE REGION
(hkM796H, VERSION 2 AND VERSION 4)
NotI
ApaI
ApaI
NotI
9.11kb
PMO
Ap
hCκ
pT796HLCDR OR
pT796HLCDRHV2 OR
pT796HLCDRHV4
PSE
SP
SP
hCγ1
PMO
NotI
ApaI

*FIG. 39*

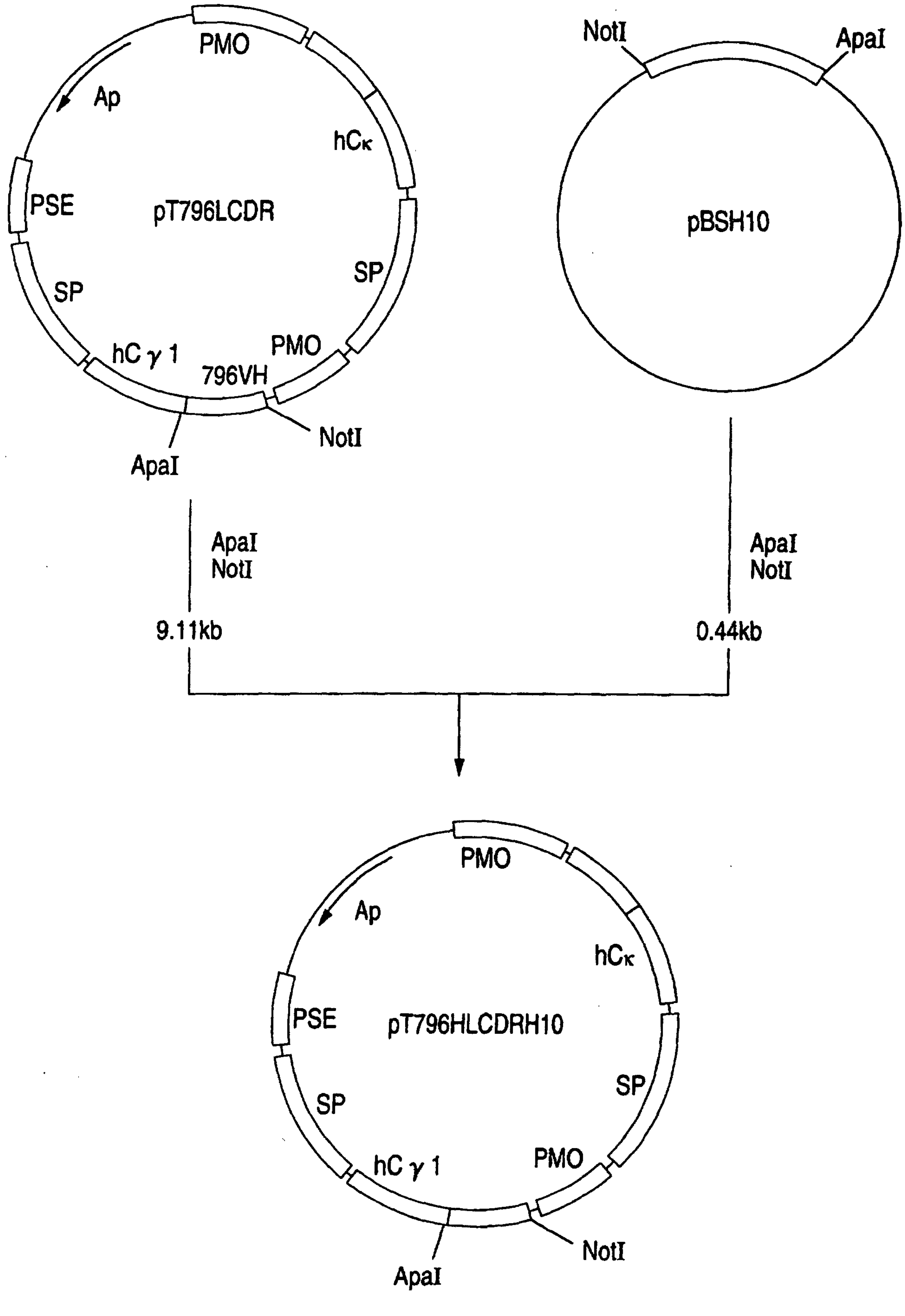
PMO
Ap
hCκ
PSE
pT796LCDR
SP
SP
hC γ 1
PMO
796VH
NotI
ApaI
NotI
ApaI
pBSH10
ApaI
NotI
9.11kb
ApaI
NotI
0.44kb
PMO
Ap
hCκ
PSE
pT796HLCDRH10
SP
SP
hC γ 1
PMO
NotI
ApaI

FIG. 40

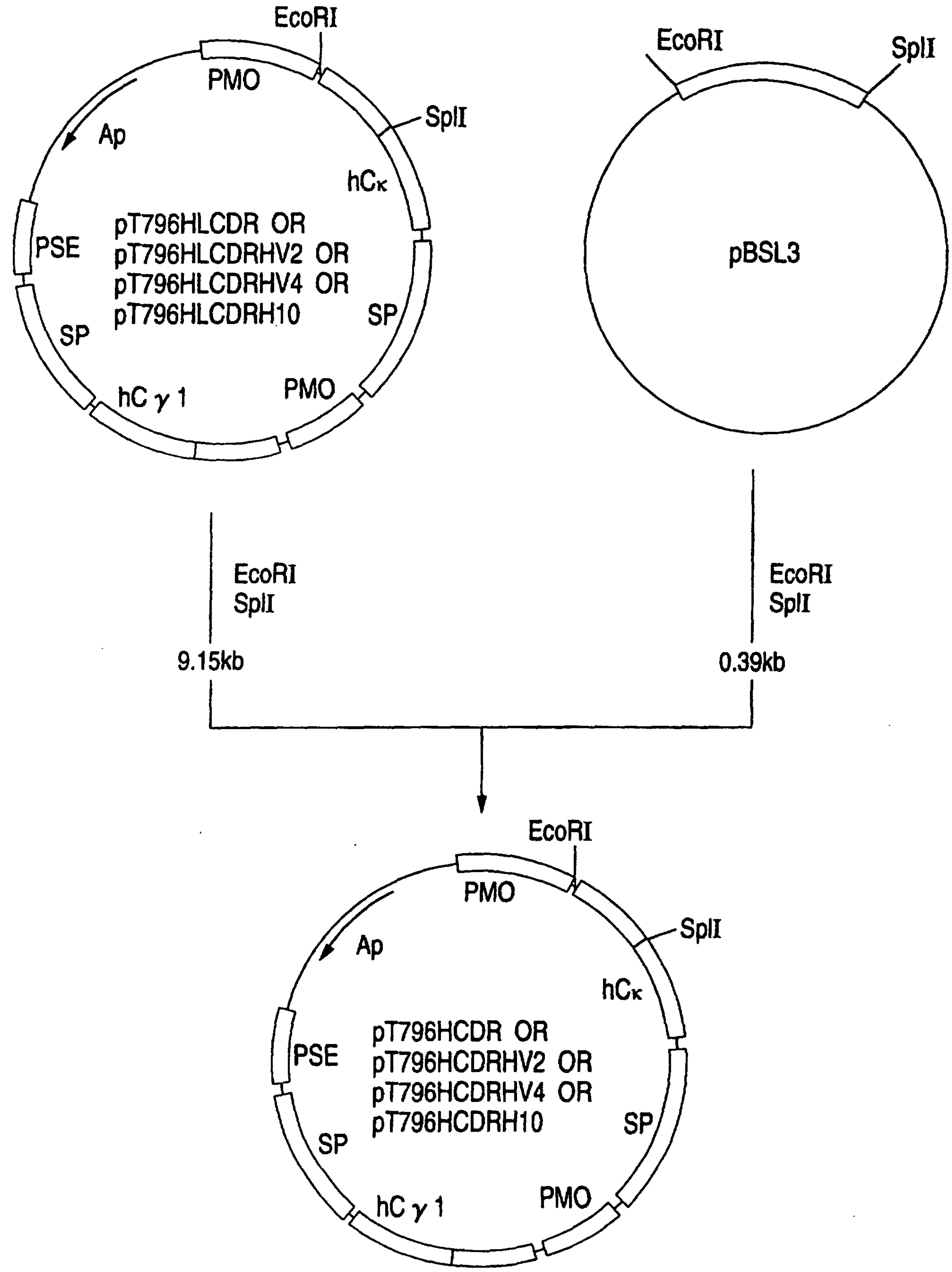
EcoRI
PMO
SplI
Ap
hCκ
PSE
pT796HLCDR OR
pT796HLCDRHV2 OR
pT796HLCDRHV4 OR
pT796HLCDRH10
SP
SP
hCγ1
PMO
EcoRI
SplI
pBSL3
EcoRI
SplI
9.15kb
EcoRI
SplI
0.39kb
EcoRI
PMO
SplI
Ap
hCκ
PSE
pT796HCDR OR
pT796HCDRHV2 OR
pT796HCDRHV4 OR
pT796HCDRH10
SP
SP
hCγ1
PMO

FIG. 41

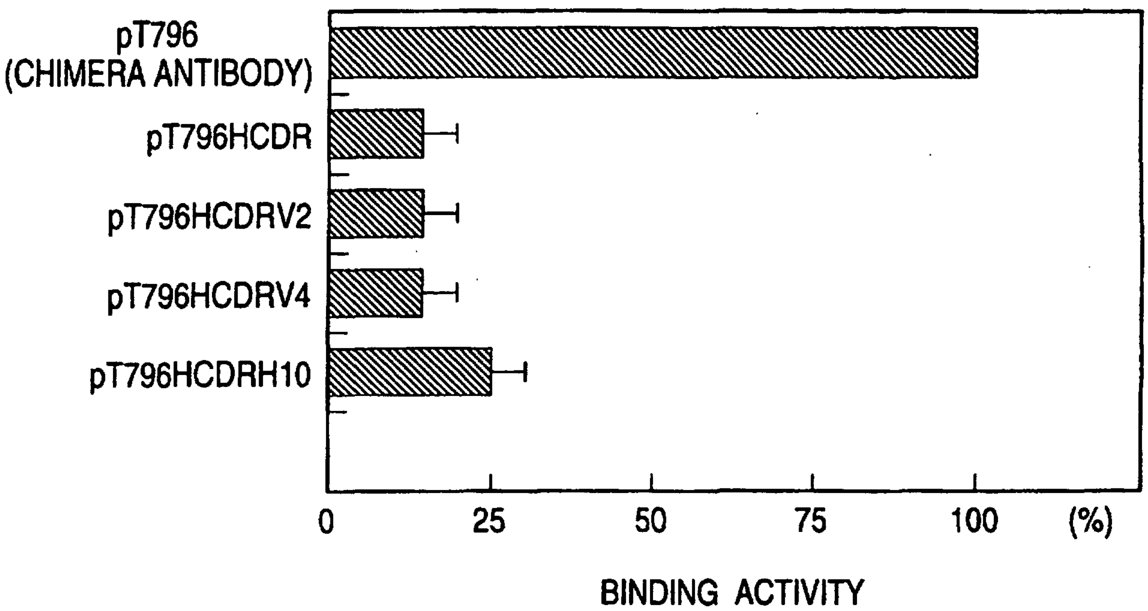
pT796
(CHIMERA ANTIBODY)
pT796HCDR
pT796HCDRV2
pT796HCDRV4
pT796HCDRH10
0
25
50
75
100
(%)
BINDING ACTIVITY

FIG. 42

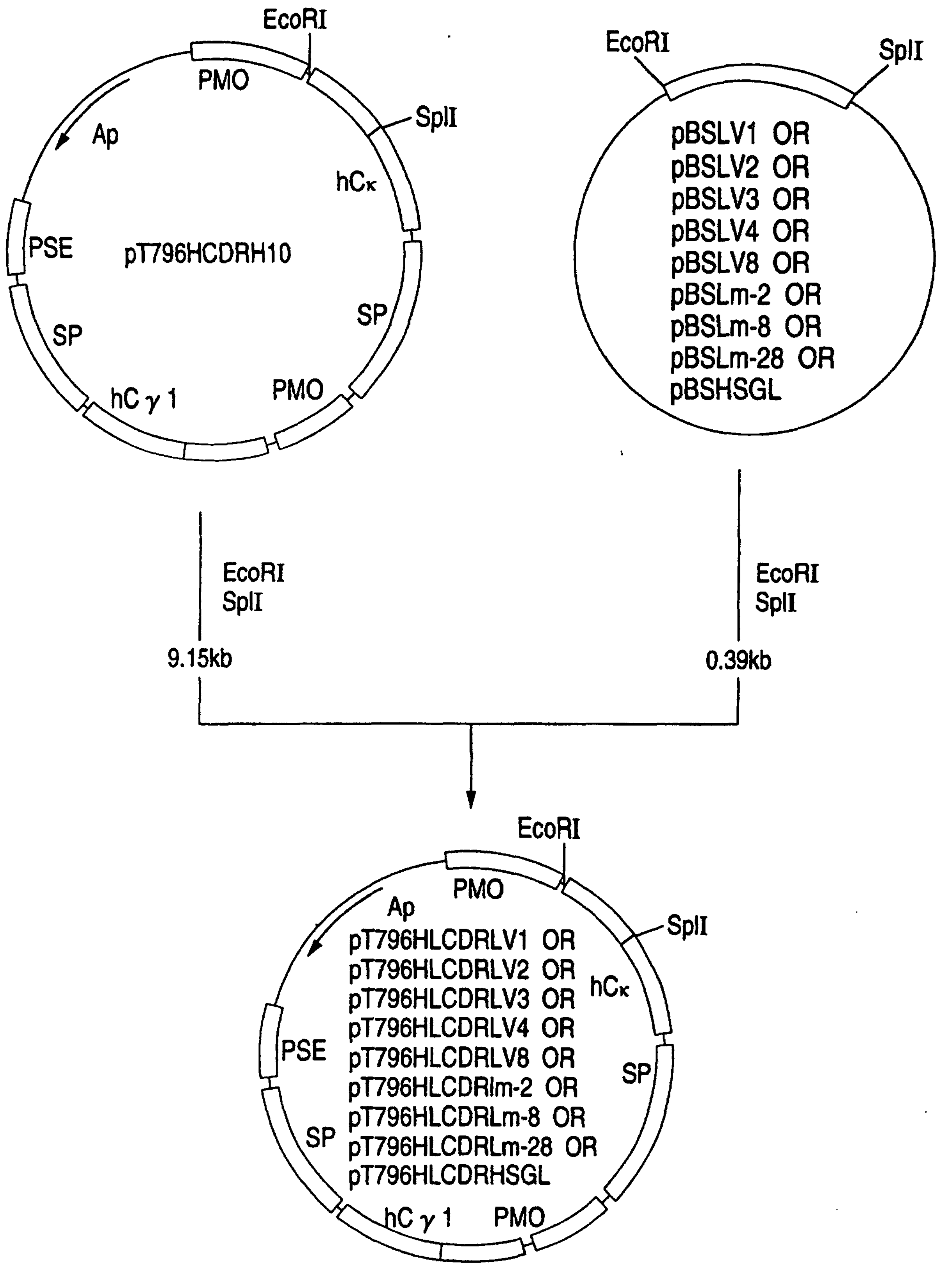
EcoRI
PMO
SplI
Ap
hCκ
PSE
pT796HCDRH10
SP
SP
hC γ 1
PMO
EcoRI
SplI
pBSLV1 OR
pBSLV2 OR
pBSLV3 OR
pBSLV4 OR
pBSLV8 OR
pBSLm-2 OR
pBSLm-8 OR
pBSLm-28 OR
pBSHSGL
EcoRI
SplI
9.15kb
EcoRI
SplI
0.39kb
EcoRI
PMO
Ap
SplI
pT796HLCDRLV1 OR
pT796HLCDRLV2 OR
pT796HLCDRLV3 OR
hCκ
pT796HLCDRLV4 OR
PSE
pT796HLCDRLV8 OR
SP
pT796HLCDRlm-2 OR
pT796HLCDRLm-8 OR
SP
pT796HLCDRLm-28 OR
pT796HLCDRHSGL
hC γ 1
PMO

FIG. 43

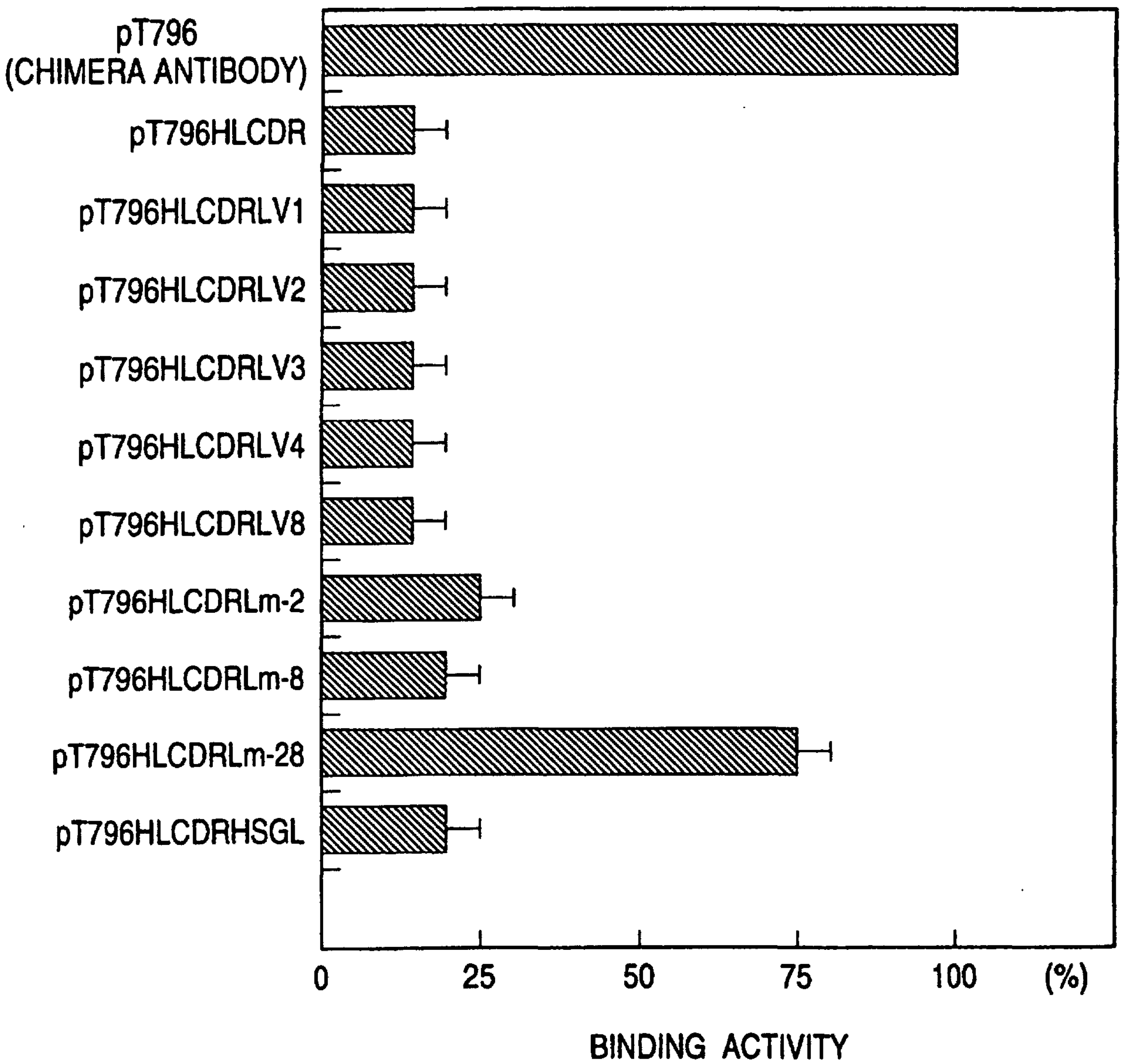
pT796
(CHIMERA ANTIBODY)
pT796HLCDR
pT796HLCDRLV1
pT796HLCDRLV2
pT796HLCDRLV3
pT796HLCDRLV4
pT796HLCDRLV8
pT796HLCDRLm-2
pT796HLCDRLm-8
pT796HLCDRLm-28
pT796HLCDRHSGL
0
25
50
75
100
(%)
BINDING ACTIVITY

*FIG. 44*

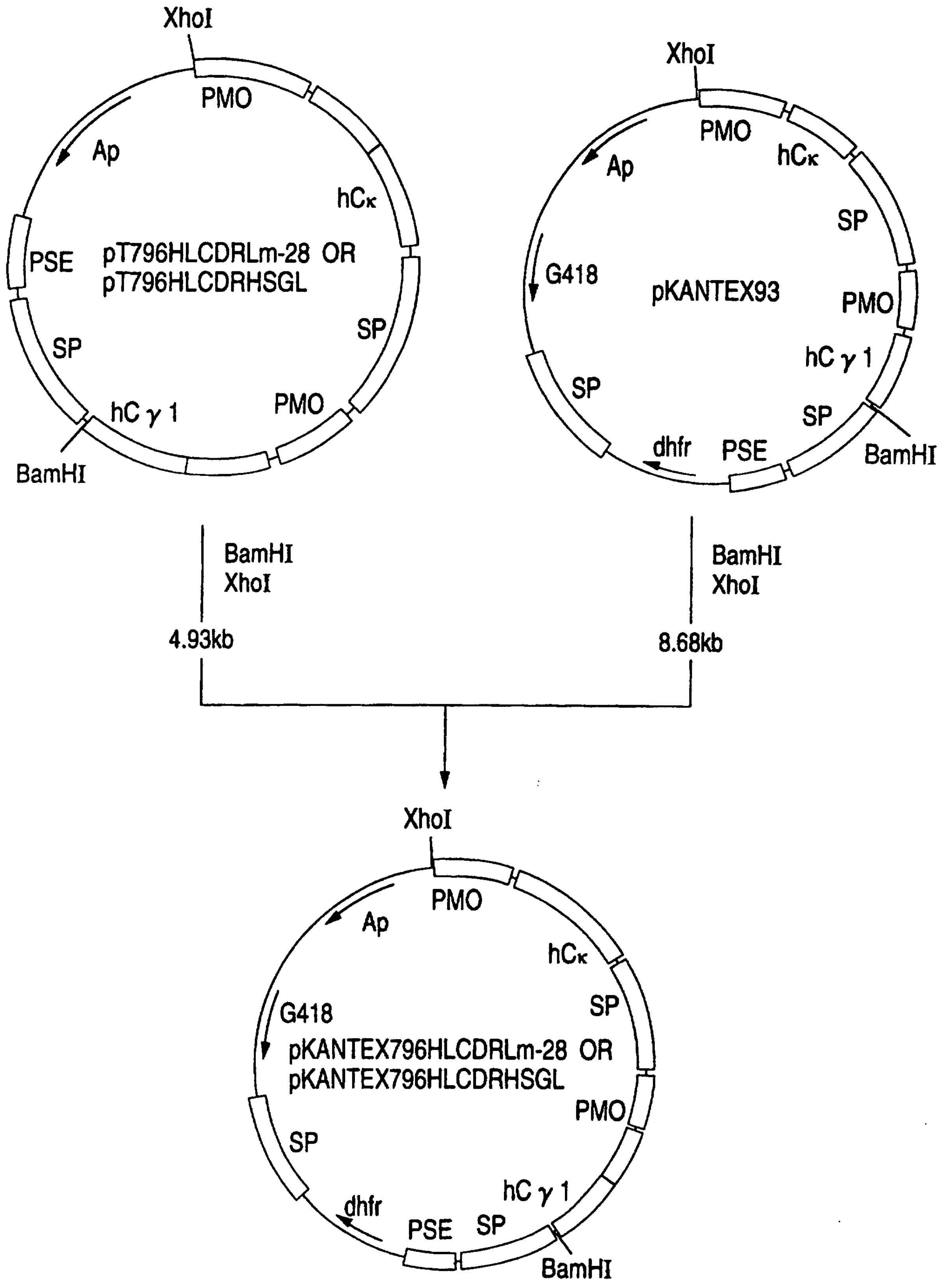
XhoI
PMO
Ap
hCκ
PSE
pT796HLCDRLm-28 OR
pT796HLCDRHSGL
SP
SP
hC γ 1
PMO
BamHI
XhoI
PMO
hCκ
Ap
SP
G418
pKANTEX93
PMO
hC γ 1
SP
SP
dhfr
PSE
BamHI
BamHI
XhoI
4.93kb
BamHI
XhoI
8.68kb
XhoI
PMO
Ap
hCκ
G418
SP
pKANTEX796HLCDRLm-28 OR
pKANTEX796HLCDRHSGL
PMO
SP
hC γ 1
dhfr
PSE
SP
BamHI

FIG. 45

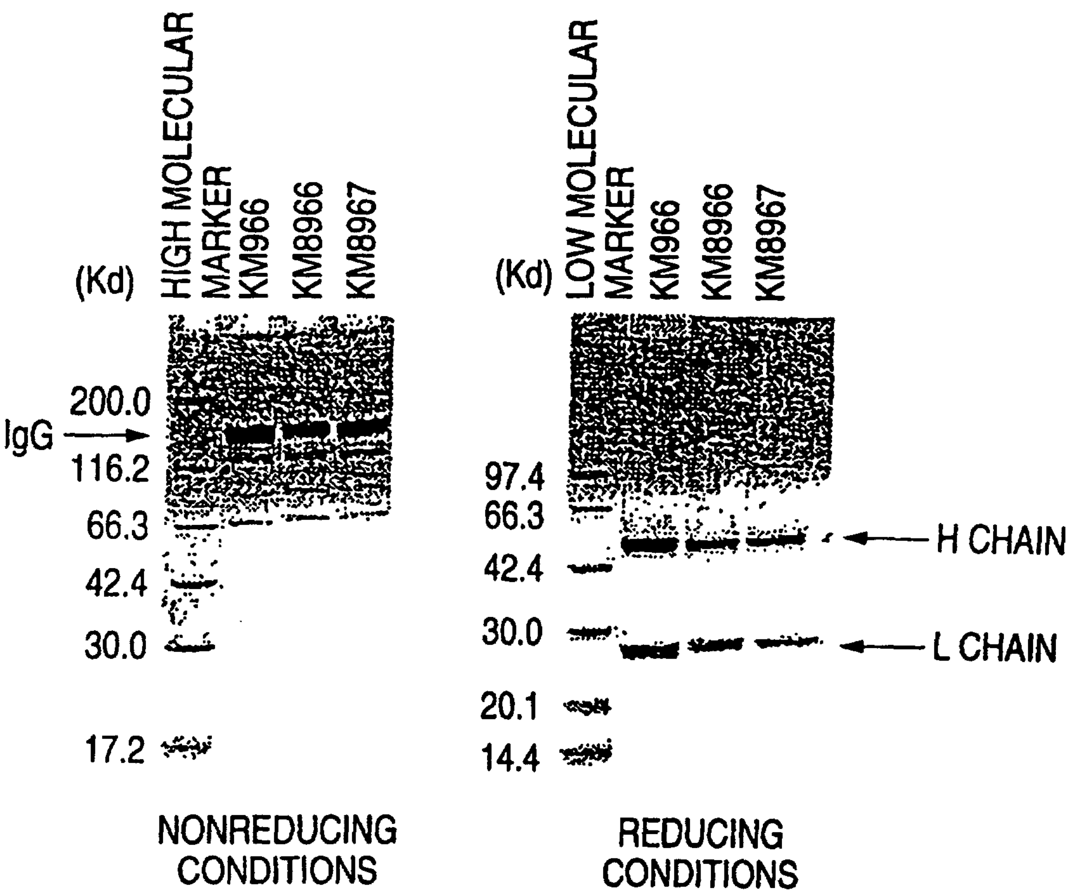
(Kd)
HIGH MOLECULAR MARKER
KM966
KM8966
KM8967
200.0
IgG
116.2
66.3
42.4
30.0
17.2
NONREDUCING CONDITIONS
(Kd)
LOW MOLECULAR MARKER
KM966
KM8966
KM8967
97.4
66.3
42.4
30.0
20.1
14.4
H CHAIN
L CHAIN
REDUCING CONDITIONS

FIG. 46

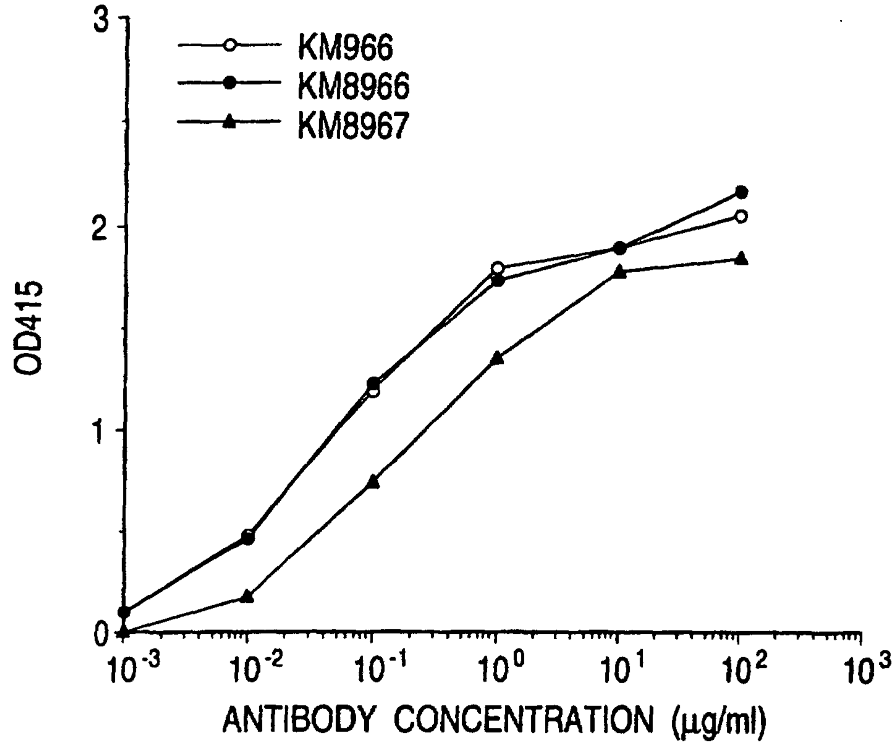
KM966
KM8966
KM8967
3
2
1
0
OD415
$10^{-3}$
$10^{-2}$
$10^{-1}$
$10^{0}$
$10^{1}$
$10^{2}$
$10^{3}$
ANTIBODY CONCENTRATION (μg/ml)

FIG. 47

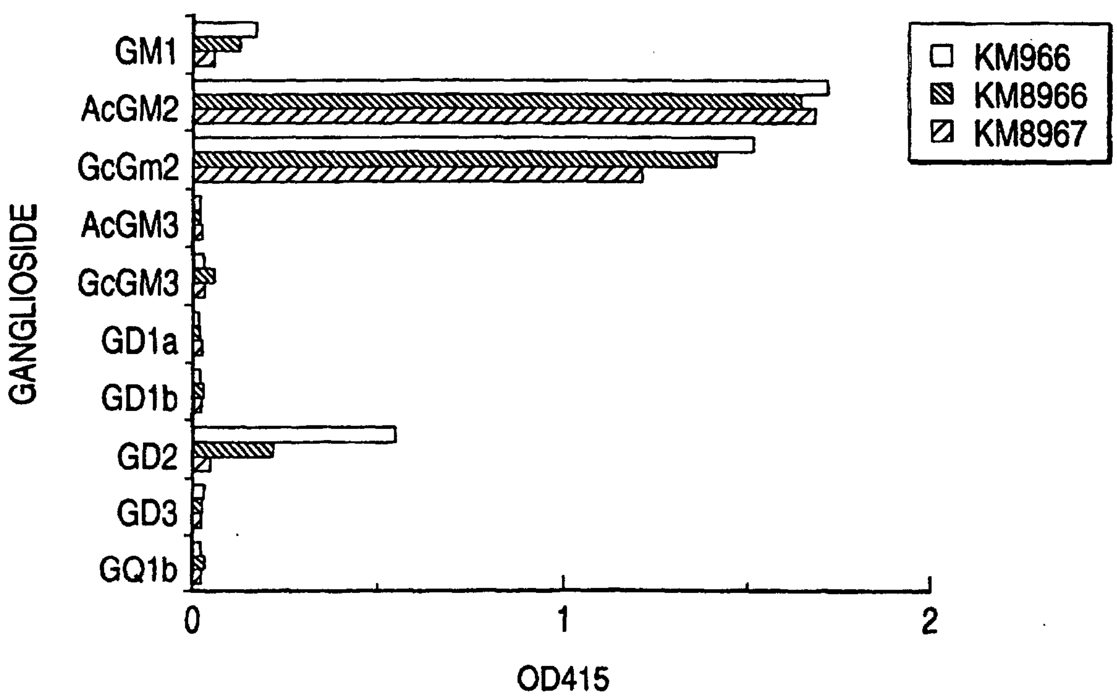
GM1
AcGM2
GcGm2
AcGM3
GcGM3
GD1a
GD1b
GD2
GD3
GQ1b
GANGLIOSIDE
KM966
KM8966
KM8967
0
1
2
OD415

FIG. 48

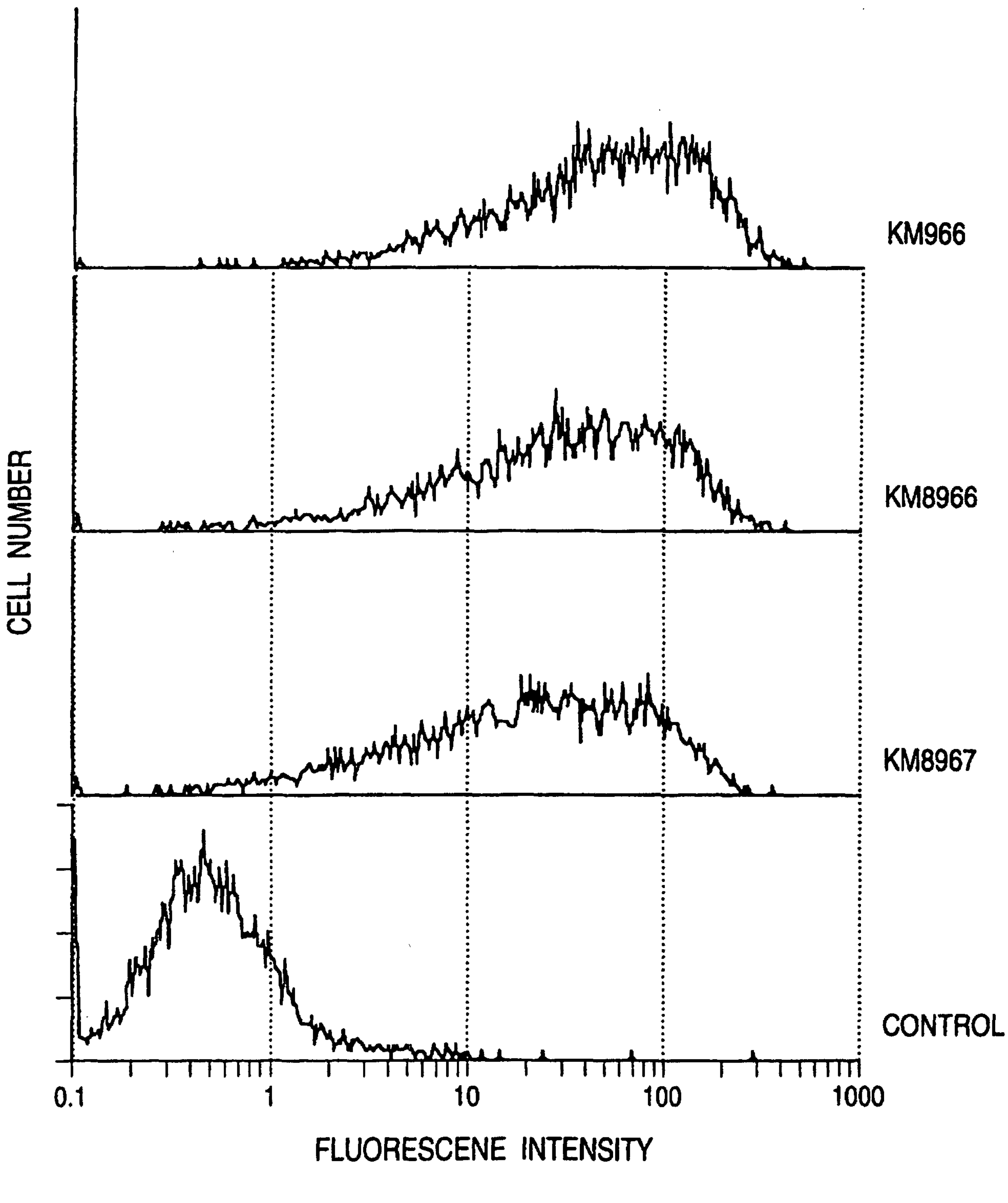
CELL NUMBER
KM966
KM8966
KM8967
CONTROL
0.1
1
10
100
1000
FLUORESCENE INTENSITY

FIG. 49

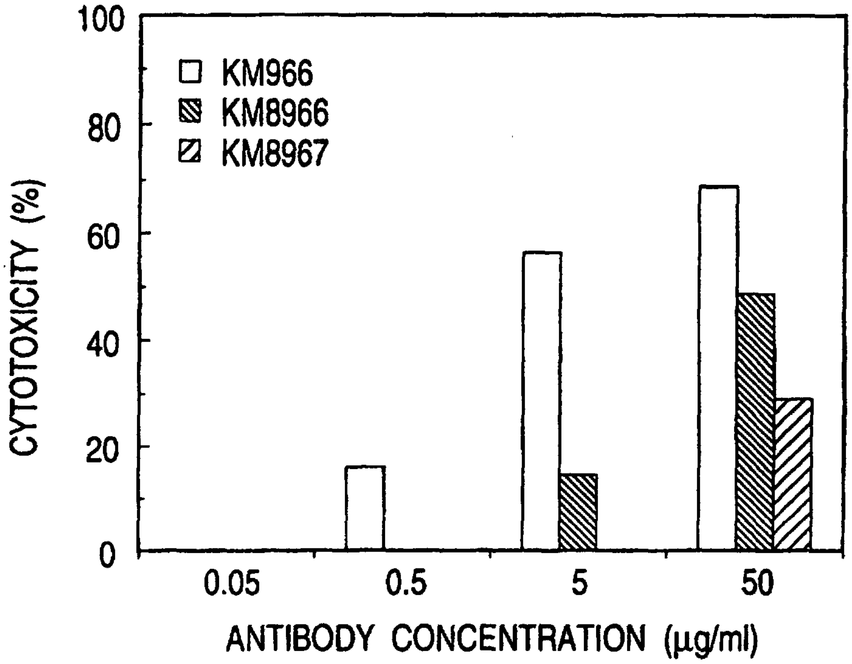
100
80
60
40
20
0
CYTOTOXICITY (%)
KM966
KM8966
KM8967
0.05
0.5
5
50
ANTIBODY CONCENTRATION (µg/ml)

FIG. 50

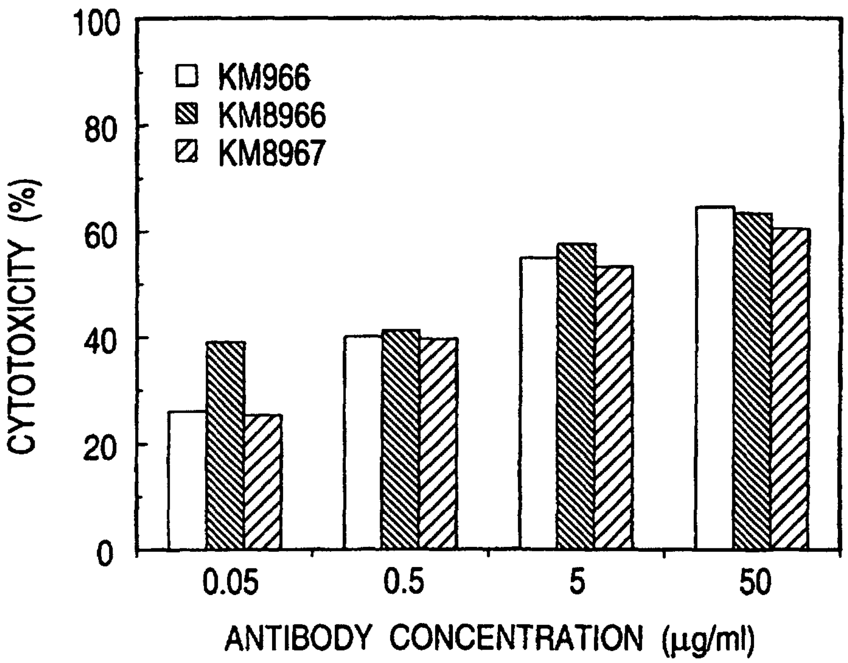
100
80
60
40
20
0
CYTOTOXICITY (%)
KM966
KM8966
KM8967
0.05
0.5
5
50
ANTIBODY CONCENTRATION (µg/ml)

*FIG. 51*

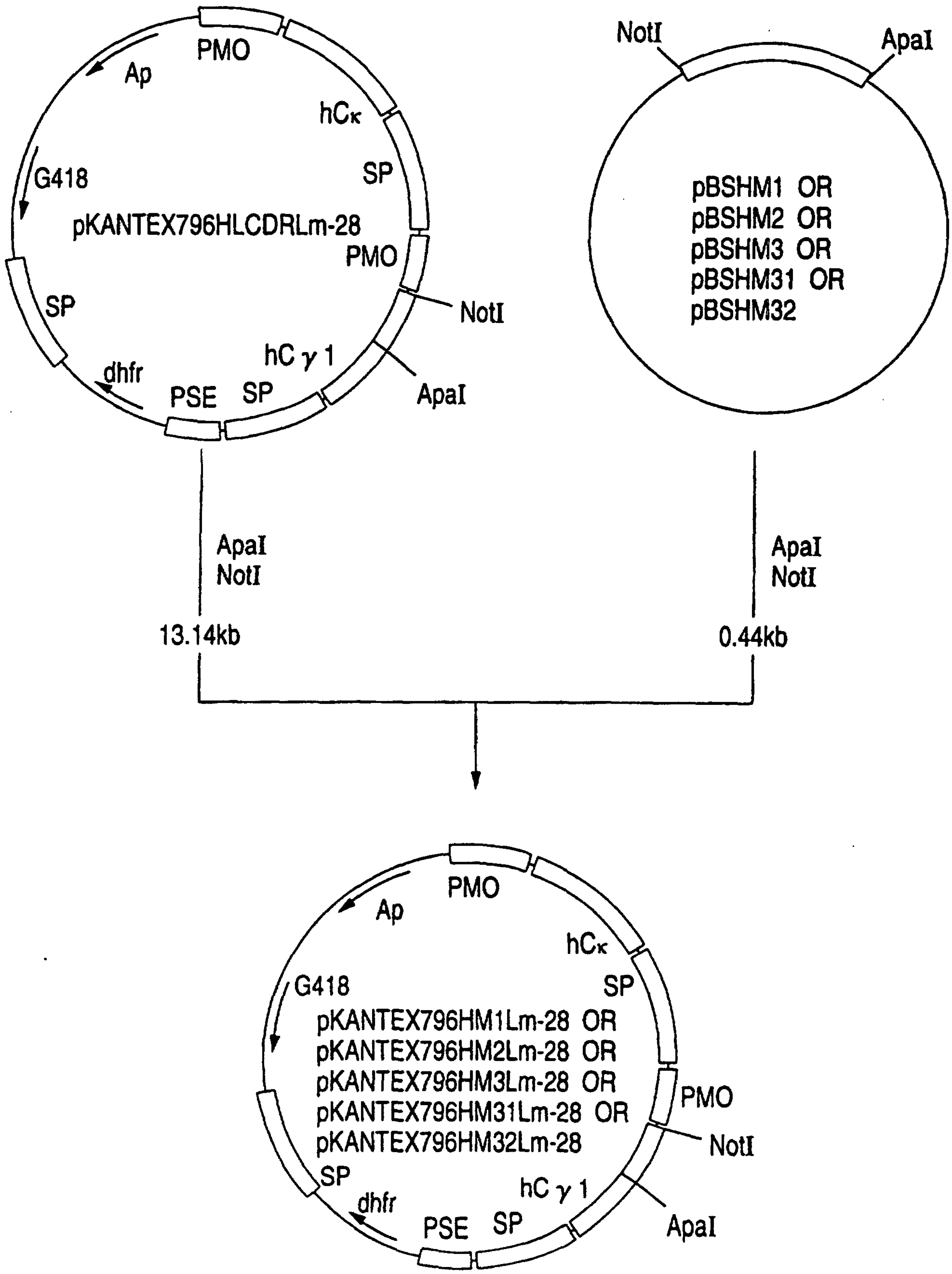
PMO
Ap
hCκ
SP
G418
pKANTEX796HLCDRLm-28
PMO
NotI
SP
hC γ 1
dhfr
PSE
SP
ApaI
NotI
ApaI
pBSHM1 OR
pBSHM2 OR
pBSHM3 OR
pBSHM31 OR
pBSHM32
ApaI
NotI
13.14kb
ApaI
NotI
0.44kb
PMO
Ap
hCκ
SP
G418
pKANTEX796HM1Lm-28 OR
pKANTEX796HM2Lm-28 OR
pKANTEX796HM3Lm-28 OR
pKANTEX796HM31Lm-28 OR
pKANTEX796HM32Lm-28
PMO
NotI
SP
hC γ 1
dhfr
PSE
SP
ApaI

FIG. 52

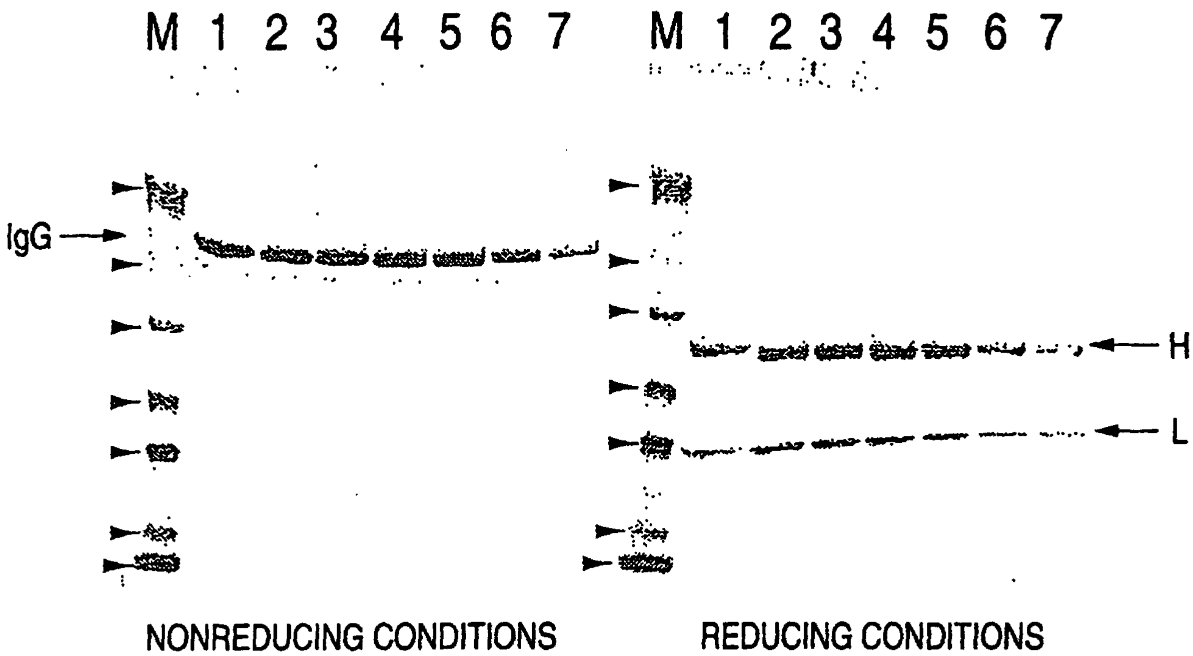
M 1 2 3 4 5 6 7 M 1 2 3 4 5 6 7
IgG
H
L
NONREDUCING CONDITIONS
REDUCING CONDITIONS

FIG. 53

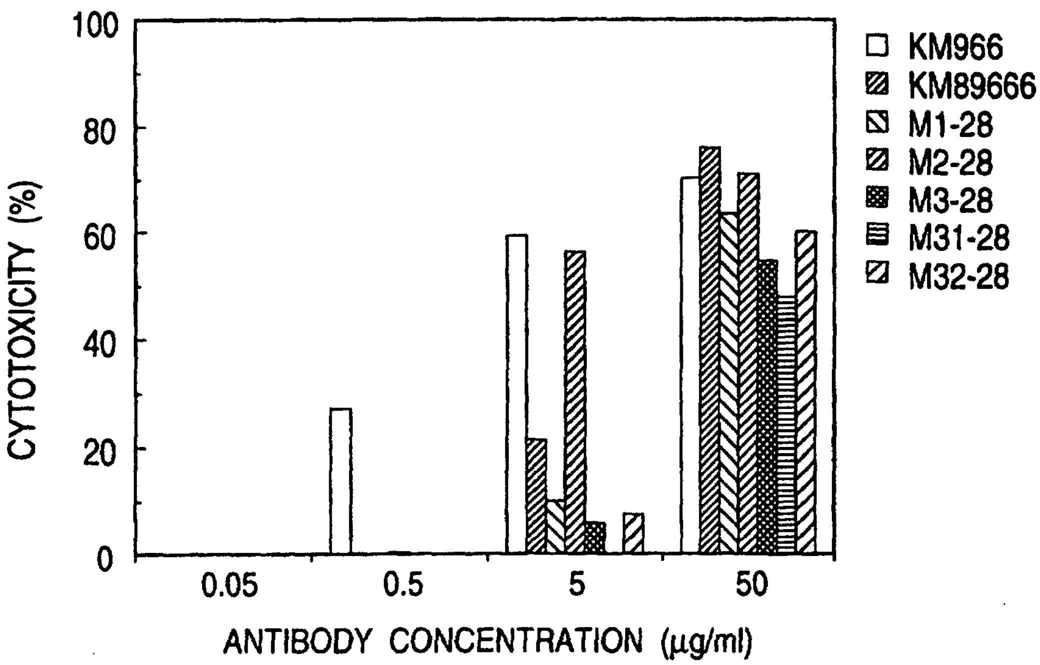
100
80
60
40
20
0
CYTOTOXICITY (%)
0.05
0.5
5
50
ANTIBODY CONCENTRATION (µg/ml)
KM966
KM89666
M1-28
M2-28
M3-28
M31-28
M32-28

*FIG. 54*

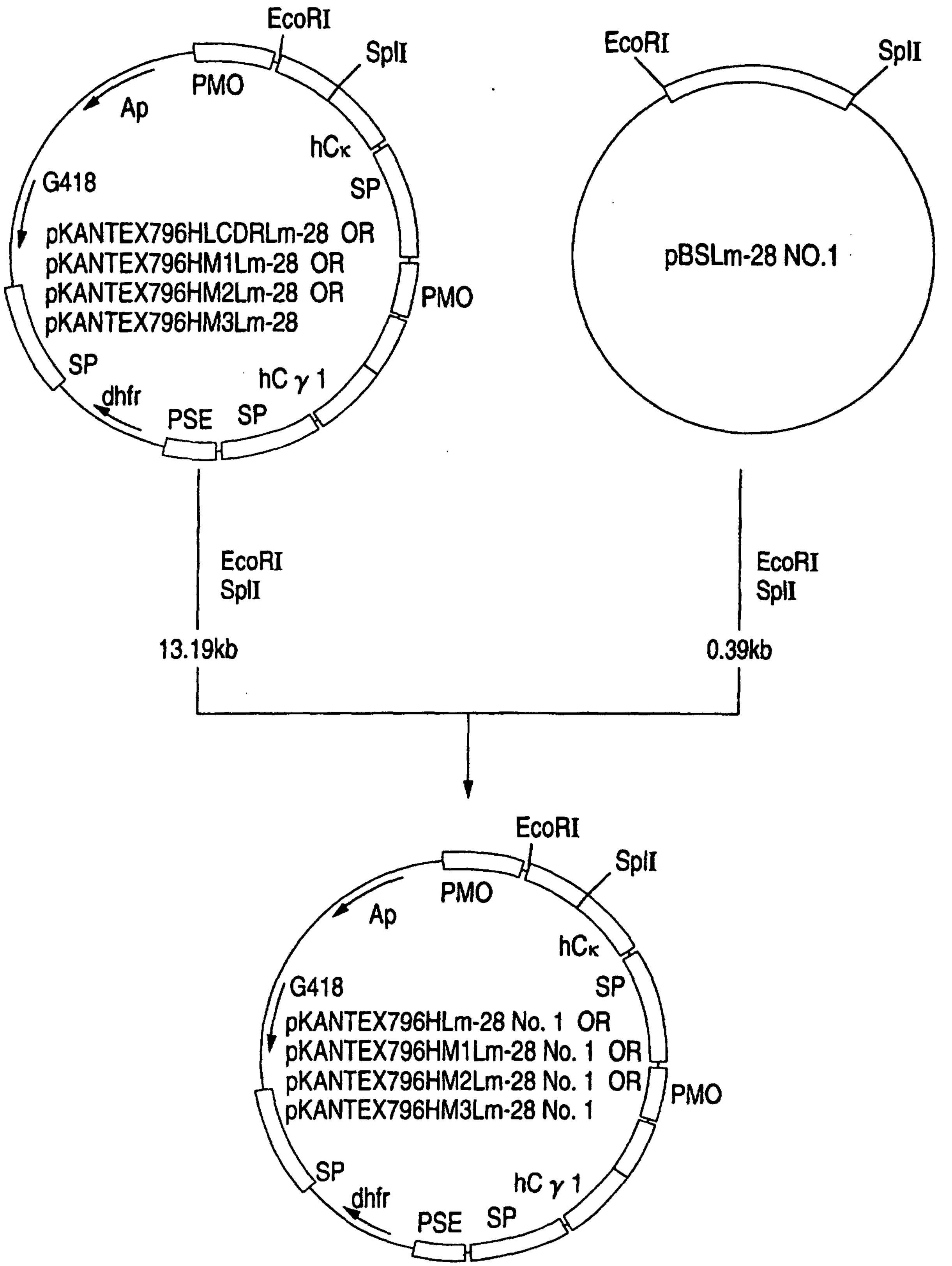
EcoRI
SplI
PMO
Ap
hCκ
G418
SP
pKANTEX796HLCDRLm-28 OR
pKANTEX796HM1Lm-28 OR
pKANTEX796HM2Lm-28 OR
pKANTEX796HM3Lm-28
PMO
SP
dhfr
hC γ 1
PSE
SP
EcoRI
SplI
pBSLm-28 NO.1
EcoRI
SplI
13.19kb
EcoRI
SplI
0.39kb
EcoRI
SplI
PMO
Ap
hCκ
G418
SP
pKANTEX796HLm-28 No. 1 OR
pKANTEX796HM1Lm-28 No. 1 OR
pKANTEX796HM2Lm-28 No. 1 OR
pKANTEX796HM3Lm-28 No. 1
PMO
SP
dhfr
hC γ 1
PSE
SP

FIG. 55

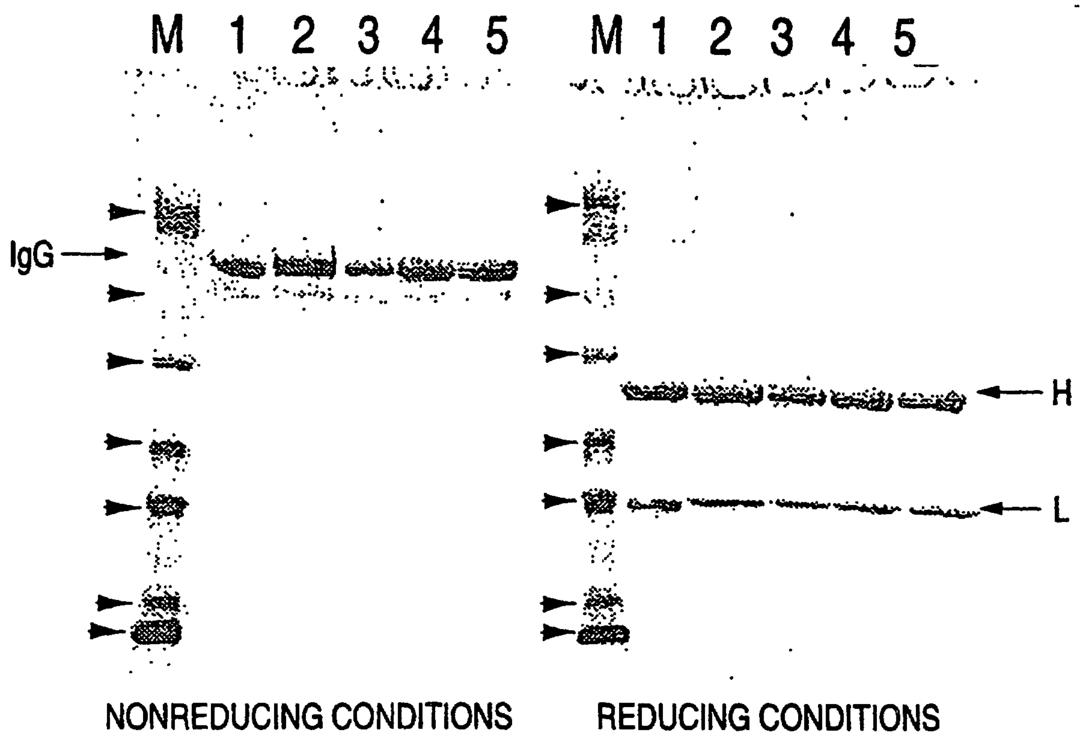
M 1 2 3 4 5 M 1 2 3 4 5
IgG
H
L
NONREDUCING CONDITIONS
REDUCING CONDITIONS

FIG. 56

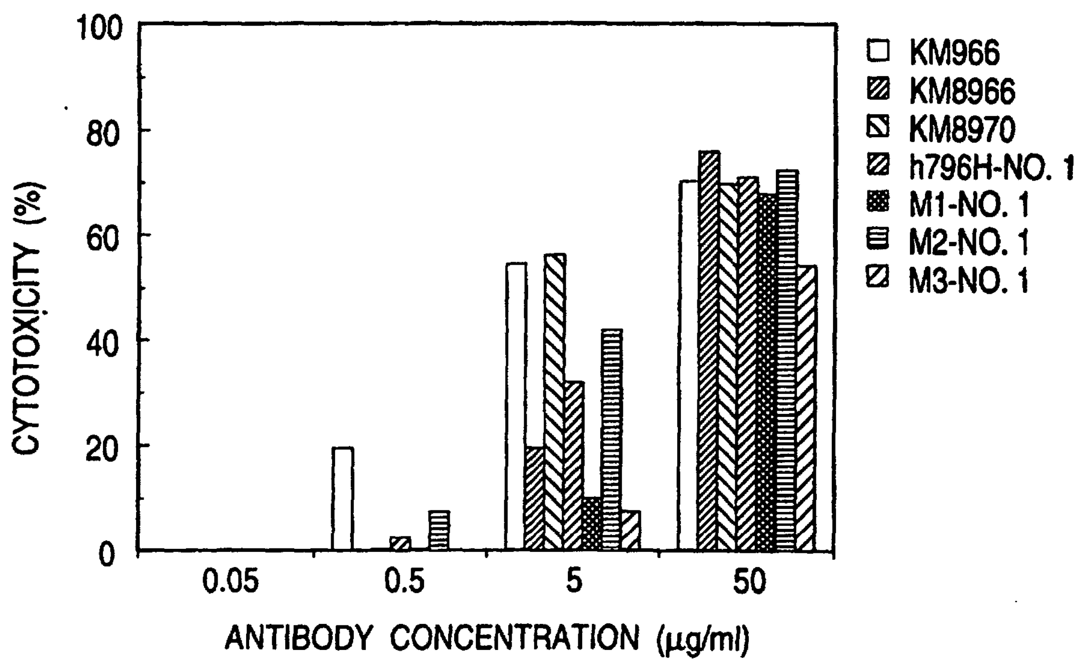
100
80
60
40
20
0
CYTOTOXICITY (%)
0.05
0.5
5
50
ANTIBODY CONCENTRATION (μg/ml)
KM966
KM8966
KM8970
h796H-NO. 1
M1-NO. 1
M2-NO. 1
M3-NO. 1

*FIG. 57*

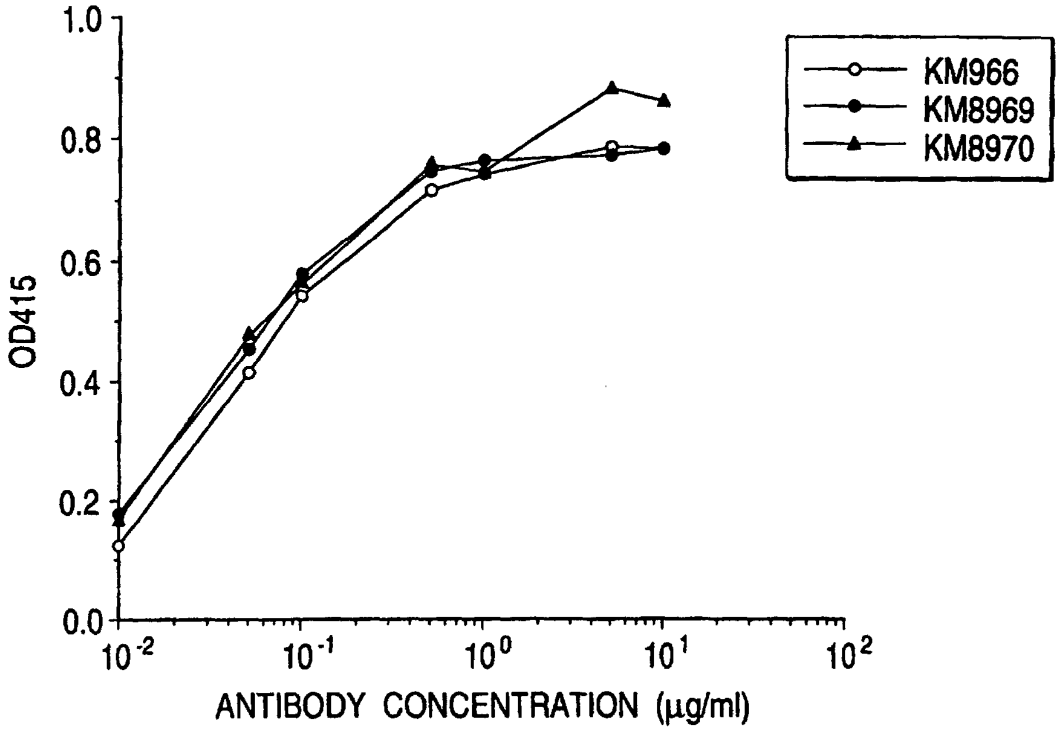
1.0
0.8
0.6
0.4
0.2
0.0
OD415
KM966
KM8969
KM8970
$10^{-2}$
$10^{-1}$
$10^{0}$
$10^{1}$
$10^{2}$
ANTIBODY CONCENTRATION (μg/ml)

*FIG. 58*

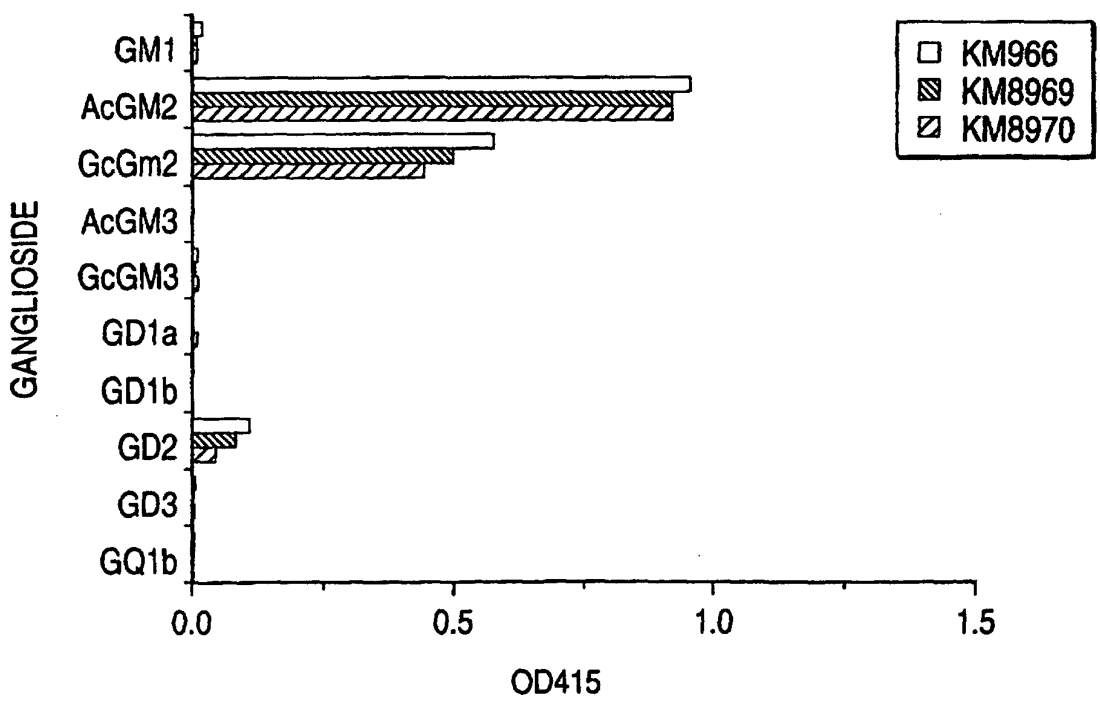
GM1
AcGM2
GcGm2
AcGM3
GcGM3
GD1a
GD1b
GD2
GD3
GQ1b
GANGLIOSIDE
KM966
KM8969
KM8970
0.0
0.5
1.0
1.5
OD415

FIG. 59

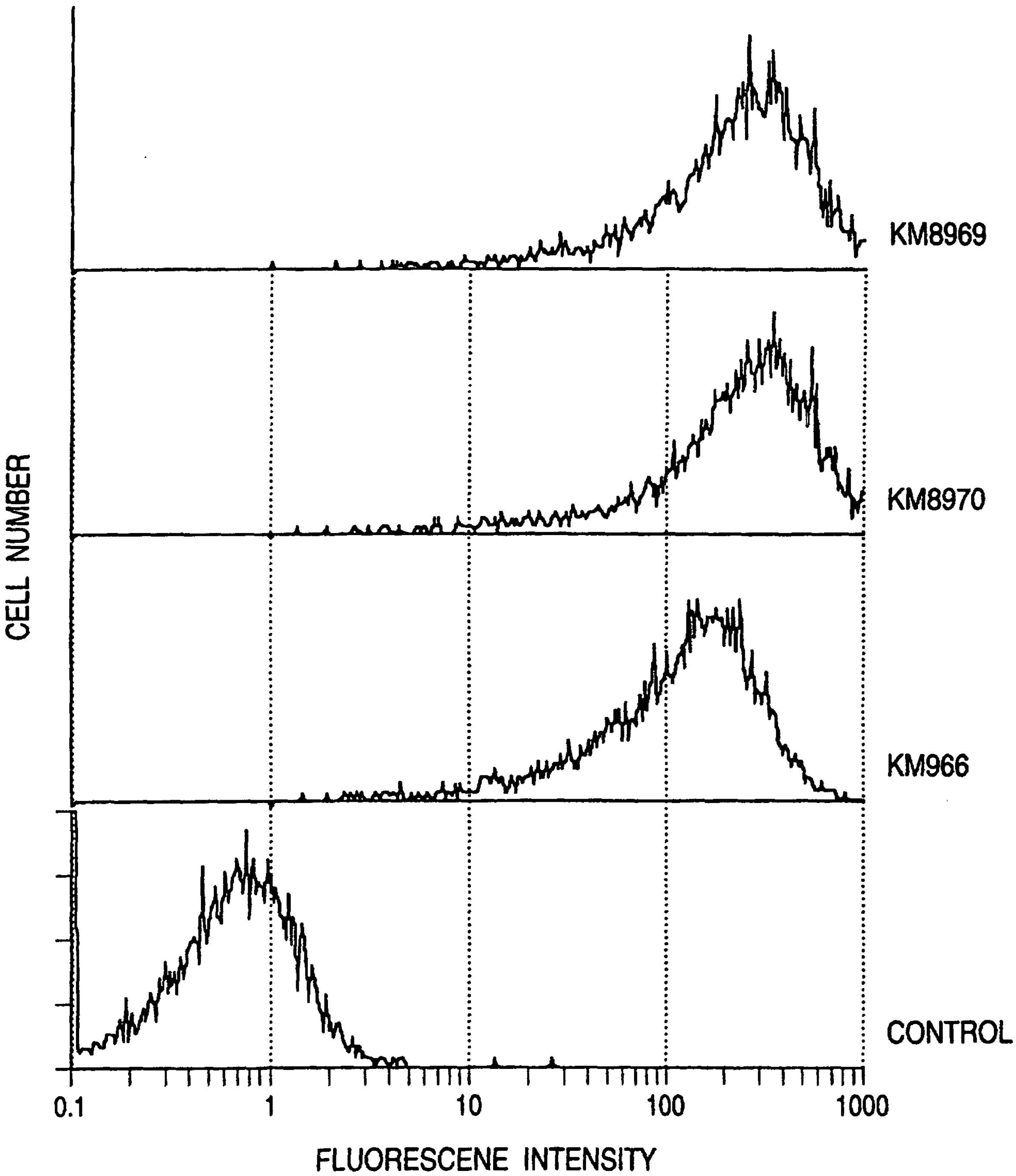
CELL NUMBER
KM8969
KM8970
KM966
CONTROL
0.1
1
10
100
1000
FLUORESCENE INTENSITY

FIG. 60

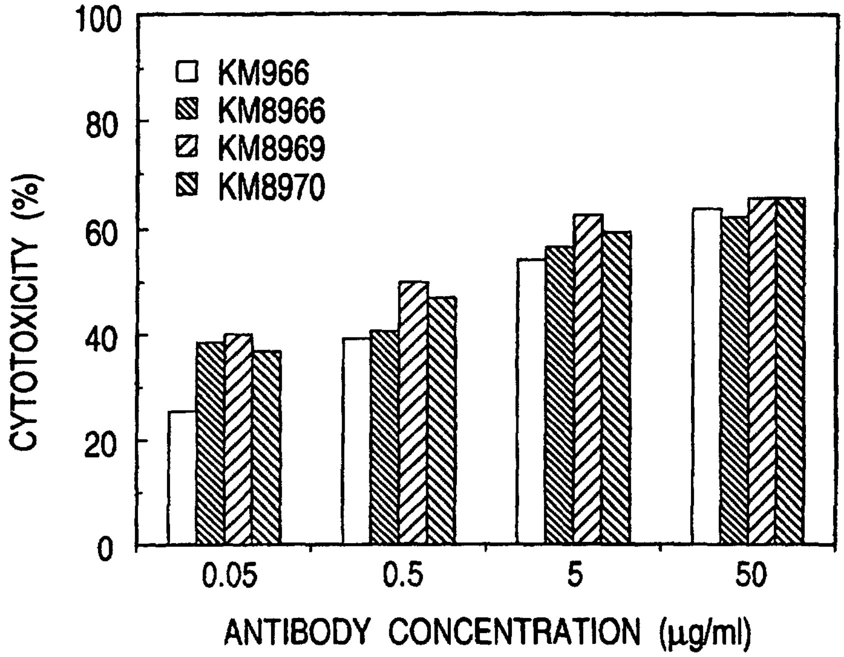
KM966
KM8966
KM8969
KM8970
CYTOTOXICITY (%)
100
80
60
40
20
0
0.05
0.5
5
50
ANTIBODY CONCENTRATION (μg/ml)

FIG. 61

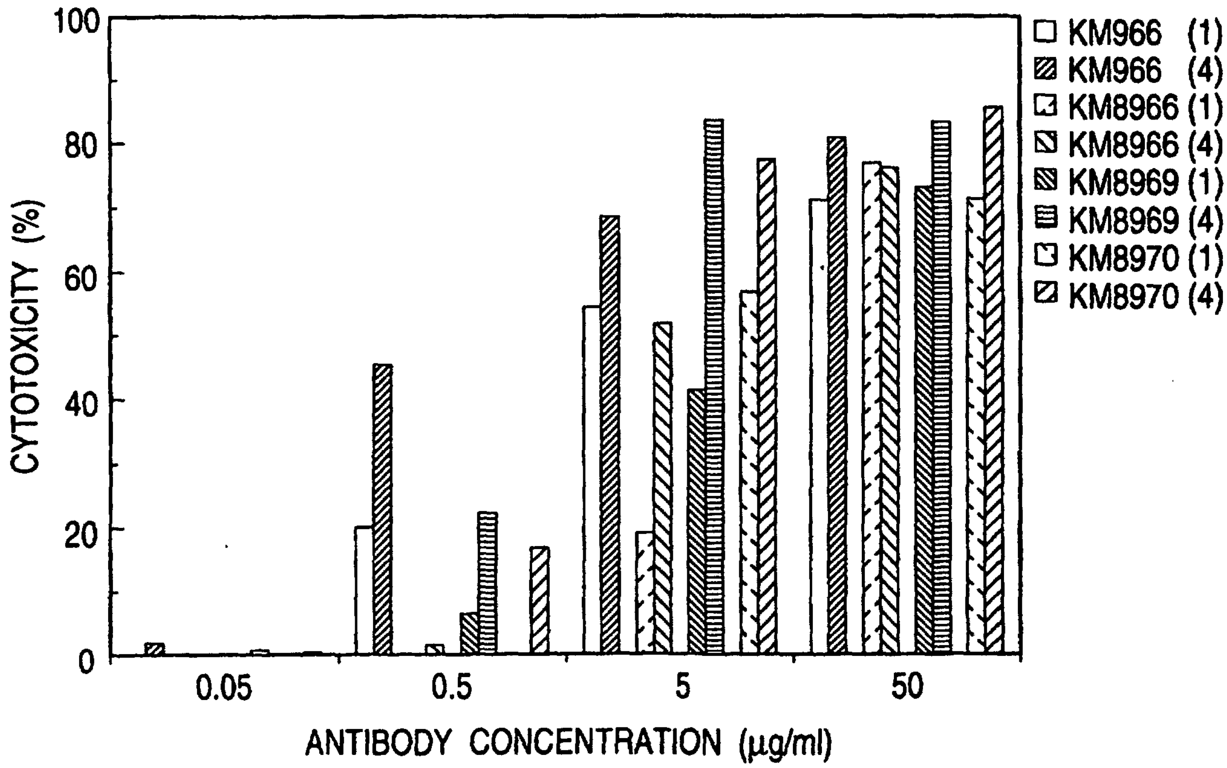
KM966 (1)
KM966 (4)
KM8966 (1)
KM8966 (4)
KM8969 (1)
KM8969 (4)
KM8970 (1)
KM8970 (4)
CYTOTOXICITY (%)
100
80
60
40
20
0
0.05
0.5
5
50
ANTIBODY CONCENTRATION (μg/ml)